# EUROPEAN PATENT SPECIFICATION

(11) **EP 3 848 363 B1**
(45) Date of publication and mention of the grant of the patent: **19.07.2023**
(21) Application number: 19856867.7
(22) Date of filing: 09.09.2019
(51) Int. Cl.: C07D 405/04, C07D 405/14, C07D 491/048, C07D 493/04, A61P 35/00, A61K 31/4525, A61K 31/454, A61K 31/4545, A61K 31/4741, A61K 31/496, A61K 31/5377, A61K 31/541

(54) **TRICYCLIC SUBSTITUTED PIPERIDINE DIONE COMPOUND**
TRICYCLISCHE SUBSTITUIERTE PIPERIDINDIONVERBINDUNG
COMPOSÉ DE PIPÉRIDINE DIONE SUBSTITUÉ TRICYCLIQUE

(30) Priority: 07.09.2018 CN 201811044122; 14.11.2018 CN 201811353938; 22.03.2019 CN 201910223413
(43) Date of publication of application: 14.07.2021
(73) Proprietor: Medshine Discovery Inc., Nanjing, Jiangsu 210032 (CN)
(72) Inventor: LUO, Yunfu, Shanghai 200131 (CN); LEI, Maoyi, Shanghai 200131 (CN); WANG, Yong, Shanghai 200131 (CN); LI, Jian, Shanghai 200131 (CN); CHEN, Shuhui, Shanghai 200131 (CN)
(74) Representative: Dehns
(86) International application number: PCT/CN2019/104989
(87) International publication number: WO 2020/048546

(56) References cited:
- WO-A1-2017/197046
- CN-A- 101 080 400
- CN-A- 102 245 023
- CN-A- 102 379 874
- CN-A- 108 409 718

## Description

### Reference to Related Application

The present disclosure claims the following right of priority:
CN 201811044122.5, filing date of 2018-09-07;
CN 201811353938.6, filing date of 2018-11-14;
CN 201910223413.9, filing date of 2019-03-22.

### Technical Field

The present disclosure relates to a series of tricyclic substituted piperidine dione compounds and applications thereof in the preparation of medicines for treating diseases related to CRBN protein, and specifically to a derivative compound represented by formula (I) or a pharmaceutically acceptable salt thereof.

### Background

Thalidomide, with the trade name Thalomid, was first synthesized by the German company Grünenthal. From the second half of the 1950s to the early 1960s, it was sold as a sedative in more than 40 countries and widely used as an antiemetic drug for pregnant women. It eventually led to the tragedy of tens of thousands of infants with phocomelia (disorders of morphogenesis) and withdraw from the market.

Since the "thalomid" incident, the teratogenic mechanism of thalidomide has aroused great interest among scientific researchers. It has been confirmed that the protein Cereblon (CRBN) is a target protein for the teratogenic effects of thalidomide. Thalidomide combines with CRBN, DDB1 (Damaged DNA Binding Protein 1), CUL4A (Cullin-4A) and Cullins 1 regulator (ROC1) to form an E3 ubiquitin ligase complex, which ubiquitinates a variety of substrate proteins to form a ubiquitinated chain, so that the substrate proteins are recognized and hydrolyzed by the proteasome. Domide drugs are called Immunomodulatory Drugs (IMiDs), which activate the ubiquitination of the transcription factors IKZF1 and IKZF3 by the E3 ubiquitin ligase complex formed by domide drugs with CRBN, and then the ubiquitinated transcription factors are recognized and degraded by the proteasome. Thereby, domide drugs have a toxic effect on multiple myeloma. The loss of these two transcription factors will stop the growth of myeloma. Now the domide drugs such as lenalidomide and pomalidomide are the first-line drugs for the treatment of multiple myeloma.

CRBN is a protein consisting of 442 amino acids conserved from plants to human and located on the p26.3 short arm of human chromosome 3 and has a molecular weight of 51 kDa. In human, the CRBN gene has been identified as a candidate gene for autosomal recessive inheritance non-syndromic mild mental retardation (ARNSMR). CRBN is widely expressed in testis, spleen, prostate, liver, pancreas, placenta, kidney, lung, skeletal muscle, ovary, small intestine, peripheral blood leukocytes, colon, brain and retina, and the expression in brain tissue (including retina) and testis is significantly higher than other tissues.

CRBN, as an important target of anti-tumor and immunomodulator drugs, has been demonstrated to have clear efficacy in multiple myeloma, chronic lymphocytic leukemia and other hematological malignancies, leprosy erythema nodosum and other skin diseases, and systemic lupus erythematosus and other autoimmune diseases. Domide drugs have relatively more side effects, especially peripheral neuropathy. There is an urgent need to develop CRBN modulator drugs with no teratogenic effects, less peripheral neuropathy, stronger immunomodulatory effects, and higher anti-tumor activities to improve clinical efficacy, reduce clinical side effects, and facilitate long-term use by patients.

WO2017/197046A1 provides Degronimers that have carbon-linked E3 Ubiquitin Ligase targeting moieties. CN102379874A provides a compound in the preparation of a medicament for the treatment of multiple myeloma. CN101080400A relates to a method for preparing 3- (4-amino-1-oxoisoindolin-2-yl) -piperidine-2,6-dione which is used for reducing the level or activity of tumor necrosis factor alpha in a mammal. CN108409718Arelates to a class of aromatic nitrogen mustard-amine amine conjugates and pharmaceutically acceptable salts thereof, a process for the preparation thereof, and pharmaceutical uses thereof. CN102245023A relates to novel substituted dioxopiperidylphthalimide derivatives and their pharmaceutically acceptable salts.

### Content of the present invention

The present disclosure provides a compound represented by formula (I), or a pharmaceutically acceptable salt thereof, 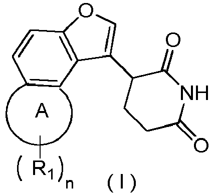 wherein,
n is selected from 0, 1, 2 and 3;
R₁ is selected from H, halogen, OH, NH₂, CN, C₃₋₆ cycloalkyl, C₁₋₆ alkyl, and C₁₋₆ alkoxy and the NH₂, C₃₋₆ cycloalkyl, C₁₋₆ alkyl and C₁₋₆ alkoxy are optionally substituted with 1, 2 or 3 Rₐ;
ring A is selected from 5- to 6-membered heteroaryl, phenyl, C₄₋₆ cycloalkyl, and 4-to 7-membered heterocycloalkyl;
Rₐ is independently selected from F, Cl, Br, I, OH, NH₂, C₁₋₁₀ alkylamino, C₁₋₁₀ alkoxy, -C(=O)O-C₁₋₁₀ alkyl, 4- to 10-membered heterocycloalkyl, 4- to 10-membered heterocycloalkylamino, C₄₋₇ cycloalkylamino, C₄₋₇ cycloalkylmethylamino, wherein the NH₂, C₁₋₁₀ alkylamino, C₁₋₁₀ alkoxy, -C(=O)O-C₁₋₁₀ alkyl, 4- to 10-membered heterocycloalkyl, 4-to 10-membered heterocycloalkylamino, C₄₋₇ cycloalkylamino and C₄₋₇ cycloalkylmethylamino are optionally substituted with 1, 2 or 3 R;
R is independently selected from F, Cl, Br, I, OH, NH₂, Me, 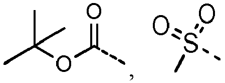 and 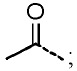
the 5- to 6-membered heteroaryl, 4- to 7-membered heterocycloalkyl, 4- to 10-membered heterocycloalkyl and 4- to 10-membered heterocycloalkylamino comprise 1, 2, 3 or 4 heteroatoms or heteroatom groups independently selected from -NH-, -O-, -S-, 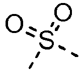 and N, respectively.

The present disclosure provides a compound represented by formula (I) or a pharmaceutically acceptable salt thereof, 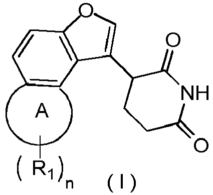 wherein,
n is selected from 0, 1, 2 and 3;
R₁ is selected from H, halogen, OH, NH₂ and C₁₋₆ alkyl, wherein the C₁₋₆ alkyl is optionally substituted with 1, 2 or 3 Rₐ;
ring A is selected from 5- to 6-membered heteroaryl, phenyl, C₄₋₆ cycloalkyl, and 4- to 7-membered heterocycloalkyl;
Rₐ is selected from F, Cl, Br, I, OH and NH₂;
the 5- to 6-membered heteroaryl and 4- to 7-membered heterocycloalkyl comprise 1, 2, 3 or 4 heteroatoms or heteroatom groups independently selected from -NH-, -O-, -S- and N, respectively.

In some embodiments of the present disclosure, the above-mentioned Rₐ is selected from F, Cl, Br, I, OH, NH₂, C₁₋₆ alkylamino, C₁₋₆ alkoxy, -C(=O)O-C₁₋₆ alkyl, 4- to 7-membered heterocycloalkyl, 4- to 7-membered heterocycloalkylamino, C₄₋₇ cycloalkylamino and C₄₋₇ cycloalkylmethylamino, wherein the NH₂, C₁₋₆ alkylamino, C₁₋₆ alkoxy, -C(=O)O-C₁₋₆ alkyl, 4-to 7-membered heterocycloalkyl, C₄₋₇ cycloalkylamino, 4- to 7-membered heterocycloalkylamino and C₄₋₇ cycloalkylmethylamino are optionally substituted with 1, 2 or 3 R.

In some embodiments of the present disclosure, the above-mentioned Rₐ is selected from F, Cl, Br, I, OH, NH₂, C₁₋₃ alkylamino, C₁₋₃ alkoxy, -C(=O)O-C₁₋₄ alkyl, pyrrolidinyl, morpholinyl, piperazinyl, piperidinyl, 3-azabicyclo[3,1,0] hexyl, thiomorpholine-1,1-dioxide group, cyclohexylamino, piperidinylamino, tetrahydropyranyl, tetrahydropyranylamino and cyclohexylmethylamino, wherein the NH₂, C₁₋₃ alkylamino, C₁₋₃ alkoxy, -C(=O)O-C₁₋₄ alkyl, tetrahydropyrrolyl, morpholinyl, piperazinyl, piperidinyl, 3-azabicyclo[3,1,0]hexyl, thiomorpholine-1,1-dioxide group, cyclohexylamino, piperidinylamino, tetrahydropyranyl, tetrahydropyranylamino and cyclohexylmethylamino are optionally substituted with 1, 2 or 3 R.

In some embodiments of the present disclosure, the above-mentioned Rₐ is selected from 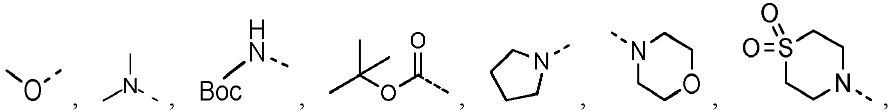 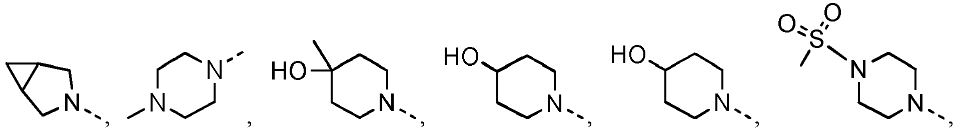 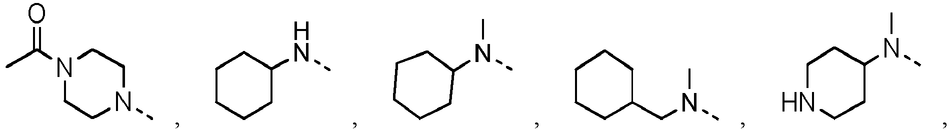 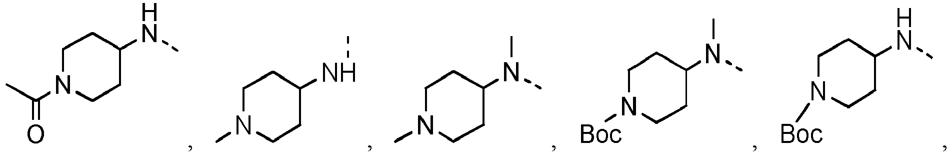 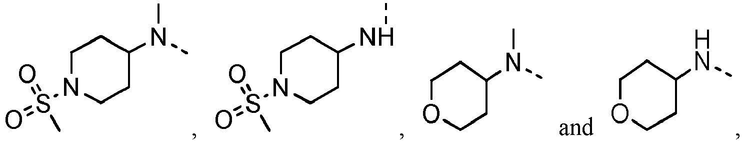 and other variables are as defined in the present disclosure.

In some embodiments of the present disclosure, the above-mentioned R₁ is selected from H, NH₂, CN, C₃₋₆ cycloalkyl, C₁₋₃ alkyl, and C₁₋₃ alkoxy, wherein the NH₂, C₃₋₆ cycloalkyl, C₁₋₃ alkyl, and C₁₋₃ alkoxy are optionally substituted with 1, 2 or 3 Rₐ, and other variables are as defined in the present disclosure.

In some embodiments of the present disclosure, the above-mentioned R₁ is selected from H, F, Cl, Br, I, OH, NH₂, C₁₋₃alkyl, wherein the C₁₋₃ alkyl is optionally substituted with 1, 2 or 3 Rₐ, and other variables are as defined in the present disclosure.

In some embodiments of the present disclosure, the above-mentioned R₁ is selected from F, Cl, Br, I, OH, NH₂, C₁₋₃alkyl, wherein the C₁₋₃ alkyl is optionally substituted with 1, 2 or 3 Rₐ, and other variables are as defined in the present disclosure, and other variables are as defined in the present disclosure.

In some embodiments of the present disclosure, the above-mentioned R₁ is selected from H, F, Cl, Br, I, OH, NH₂ and Me, wherein the Me is optionally substituted with 1, 2 or 3 Rₐ, and other variables are as defined in the present disclosure.

In some embodiments of the present disclosure, the above-mentioned R₁ is selected from F, Cl, Br, I, OH, NH₂ and Me, wherein the Me is optionally substituted with 1, 2 or 3 Rₐ, and other variables are as defined in the present disclosure.

6. The compound as defined in claim 5 or a pharmaceutically acceptable salt thereof, wherein R₁ is selected from H, Me, CN, 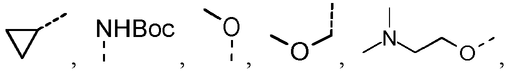 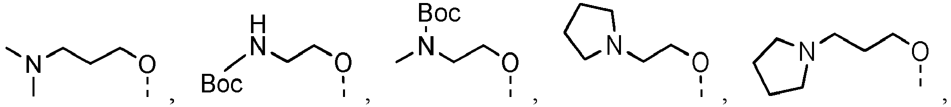 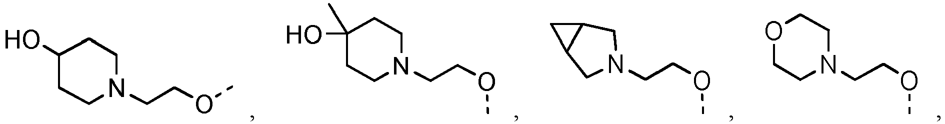 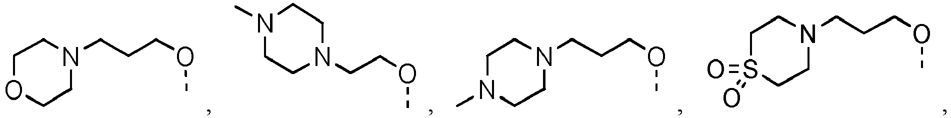 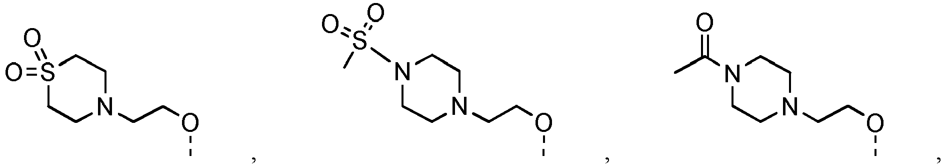 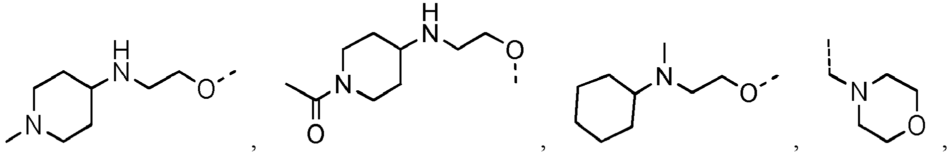 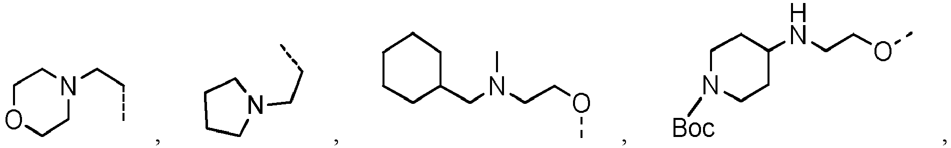 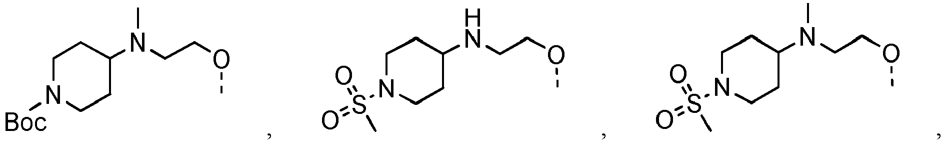 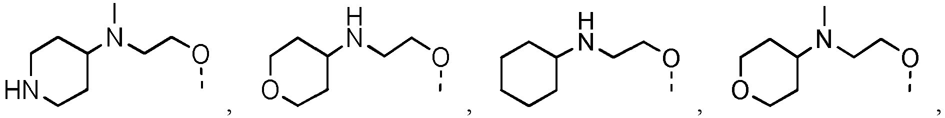 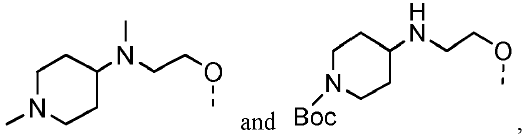 and other variables are as defined in the present disclosure.

In some embodiments of the present disclosure, the above-mentioned R₁ is selected from H and Me, and other variables are as defined in the present disclosure.

In some embodiments of the present disclosure, the above-mentioned R₁ is selected from Me, and other variables are as defined in the present disclosure.

In some embodiments of the present disclosure, the above-mentioned ring A is selected from phenyl, pyridyl, pyrrolyl, pyrazolyl, 1,3-dioxolanyl, morpholinyl, oxazolyl cyclobutyl, oxepanyl, furanyl, tetrahydrofuranyl and 1,4-oxazepinyl, and other variables are as defined in the present disclosure.

8. The compound as defined in claim 7 or a pharmaceutically acceptable salt thereof, wherein the ring Ais selected from phenyl, pyridyl, pyrrolyl, pyrazolyl, 1,3-dioxolanyl, furanyl and tetrahydrofuranyl, and other variables are as defined in the present disclosure.

In some embodiments of the present disclosure, the above-mentioned ring A is selected from phenyl, 1,3- dioxolane, morpholinyl, oxazolyl cyclobutyl, oxepanyl and 1,4-oxazepinyl, and other variables are as defined in the present disclosure.

In some embodiments of the present disclosure, the above-mentioned ring A is selected from phenyl, pyridyl, 1,3- dioxolane, morpholinyl, oxazolyl cyclobutyl, oxepanyl, tetrahydrofuranyl adn 1,4-oxazepinyl, and other variables are as defined in the present disclosure.

In some embodiments of the present disclosure, the above-mentioned ring A is selected from phenyl, pyridyl, 1,3- dioxolane and tetrahydrofuranyl, and other variables are as defined in the present disclosure.

In some embodiments of the present disclosure, the above-mentioned structural unit 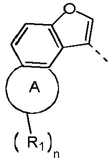 is selected from 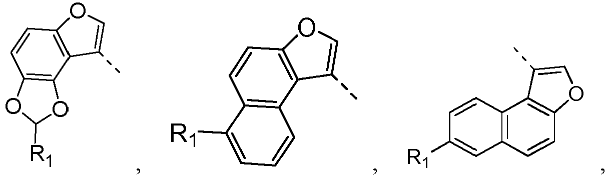 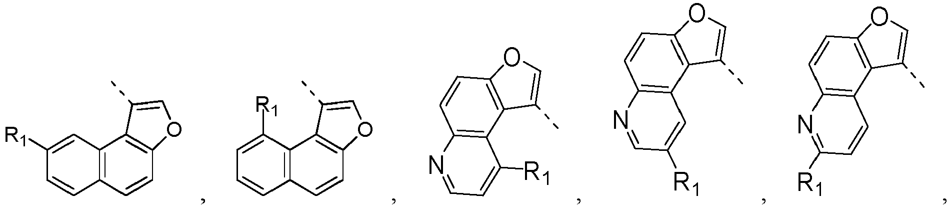 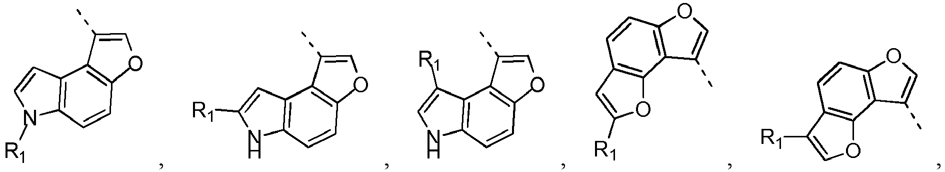 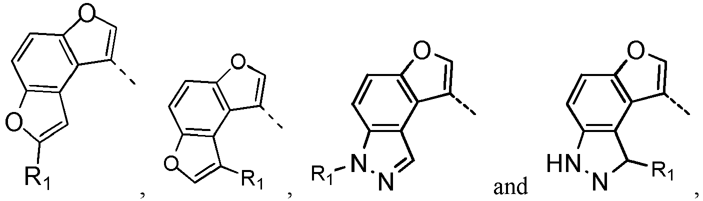 and other variables are as defined in the present disclosure.

In some embodiments of the present disclosure, the above-mentioned structural unit 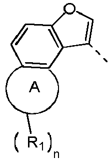 is selected from 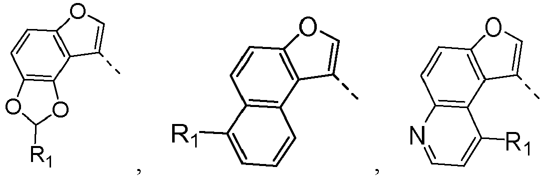 and 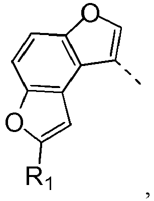 and other variables are as defined in the present disclosure.

In some embodiments of the present disclosure, the above-mentioned structural unit 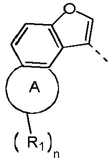 is selected from 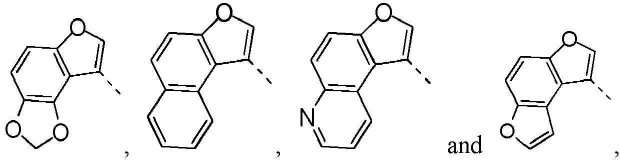 and other variables are as defined in the present disclosure.

In some embodiments of the present disclosure, the above-mentioned structural unit 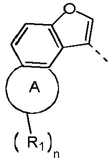 is selected from 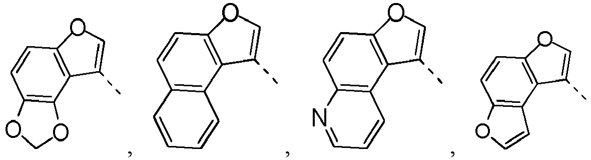 and 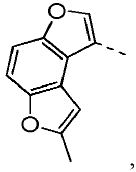 and other variables are as defined in the present disclosure.

There are still some embodiments of the present disclosure derived from any combination of the above-mentioned variables.

In some embodiments of the present disclosure, the above-mentioned compound or pharmaceutically acceptable salt thereof is selected from 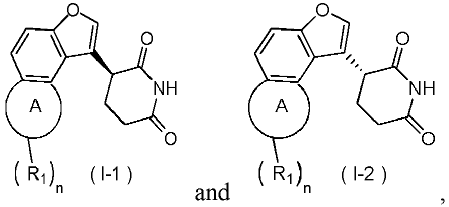 wherein, n, R₁ and ring A are as defined in the present disclosure.

In some embodiments of the present disclosure, the above-mentioned compound or pharmaceutically acceptable salt thereof is selected from 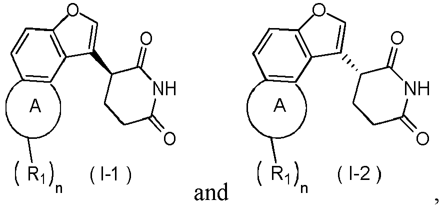 wherein, n, R₁, ring A, and structural unit 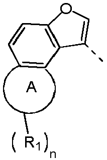 are as defined in the present disclosure.

The present disclosure also provides the following compounds or pharmaceutically acceptable salts thereof

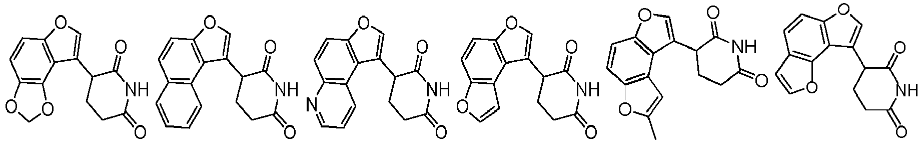

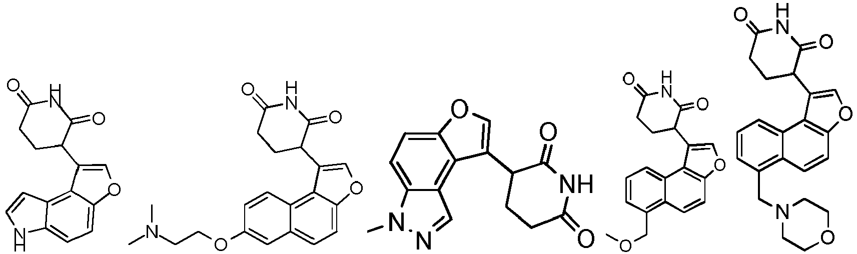

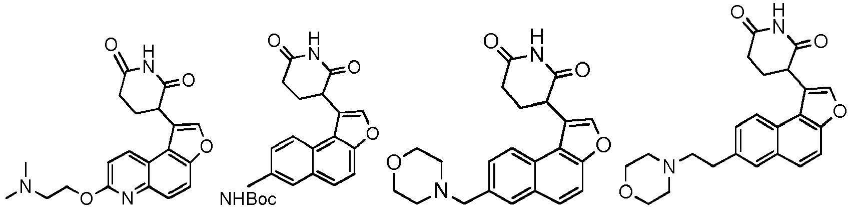

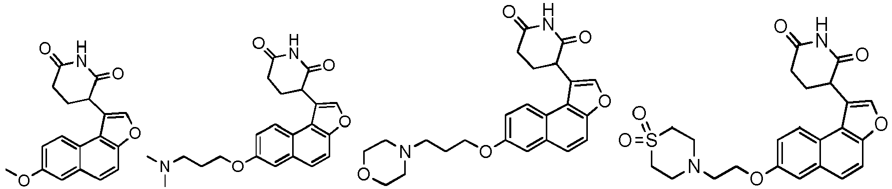

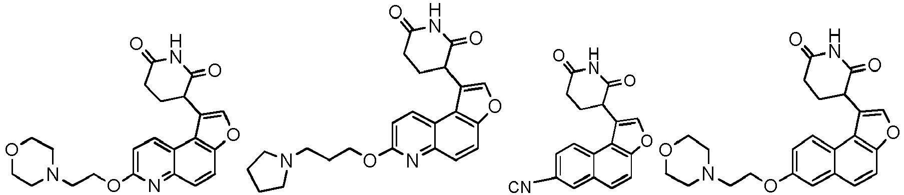

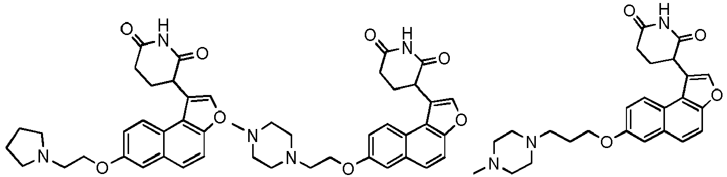

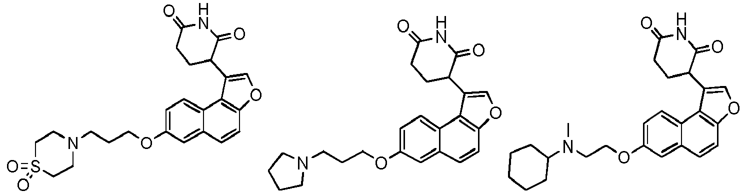

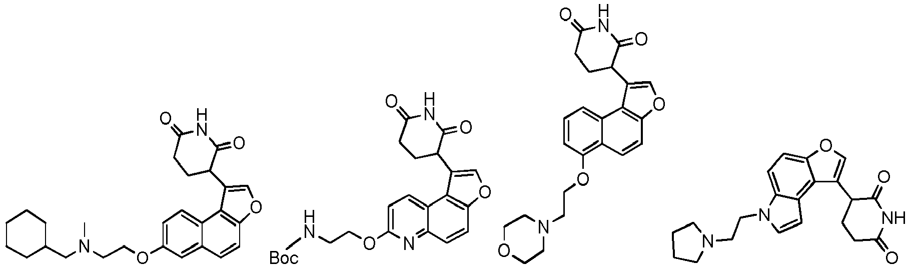

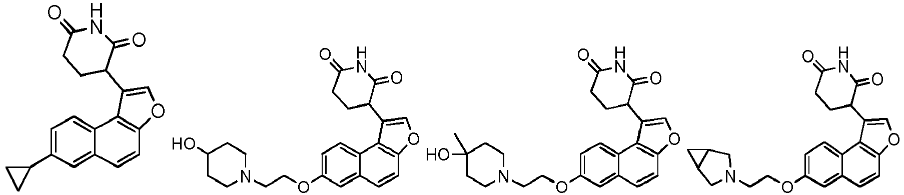

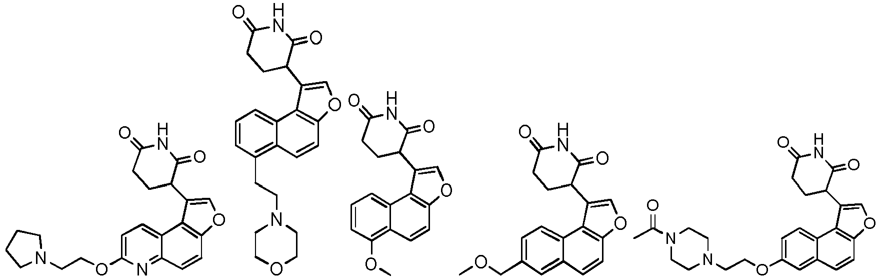

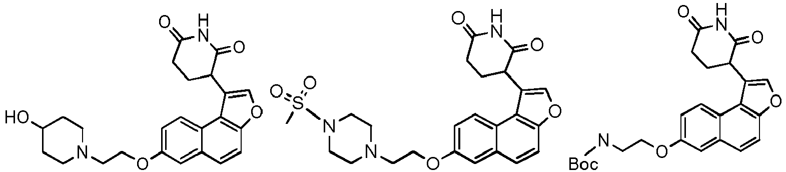

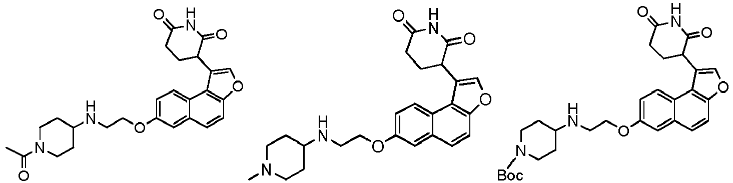

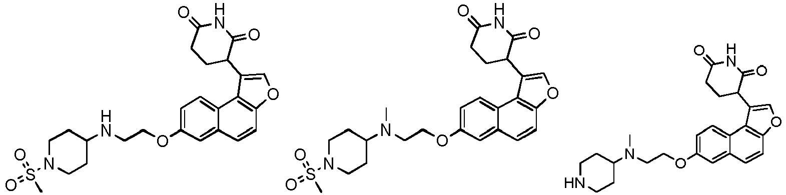

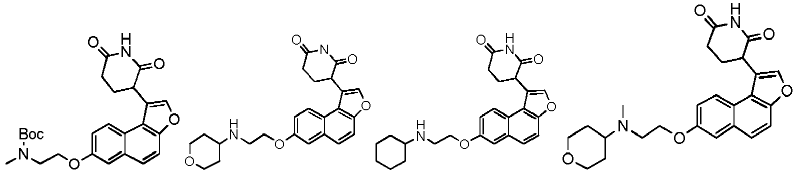

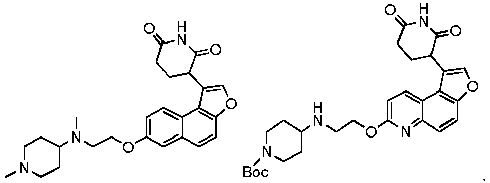

In some embodiments of the present disclosure, the above-mentioned compound or pharmaceutically acceptable salt thereof is selected from

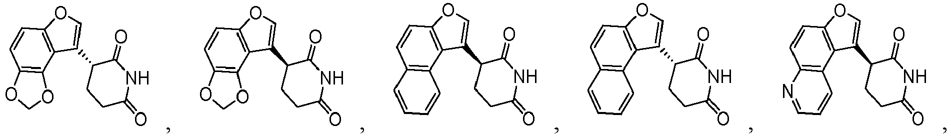

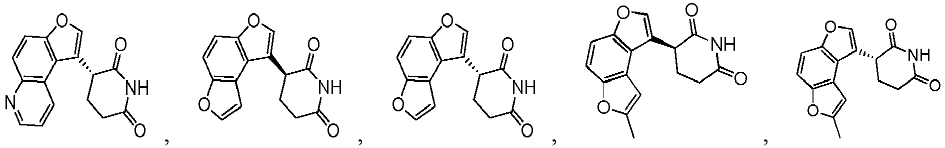

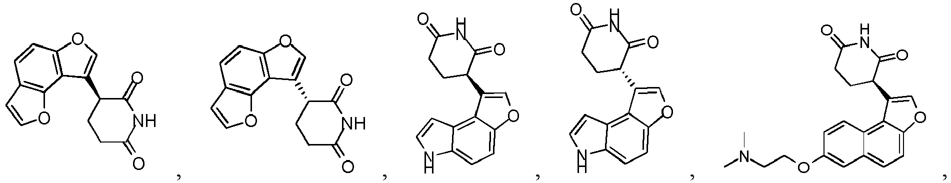

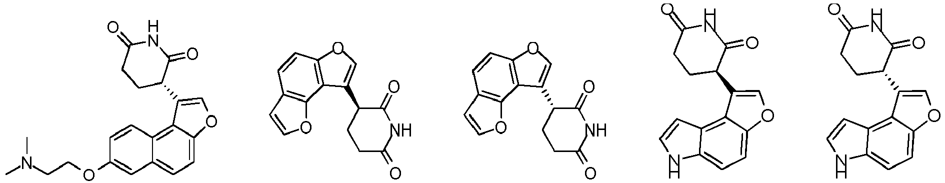

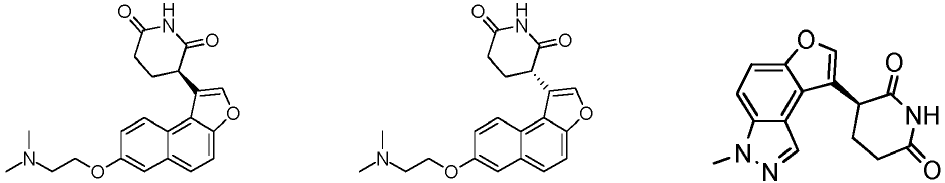

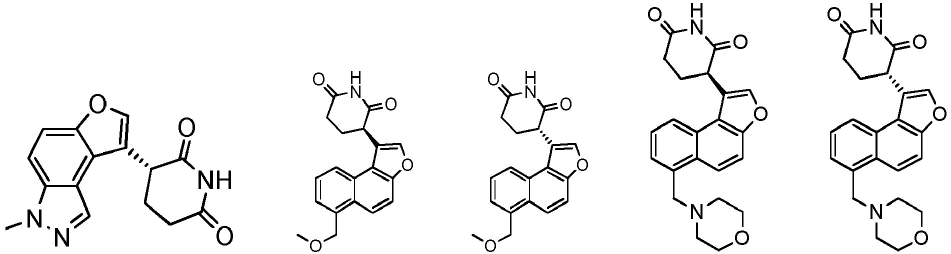

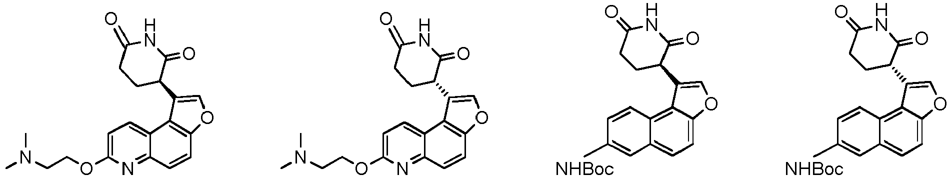

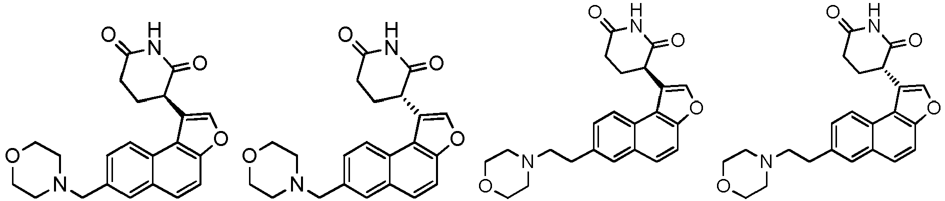

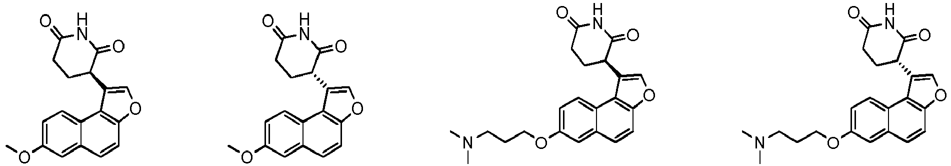

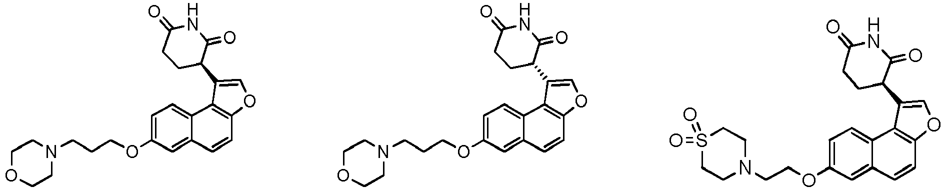

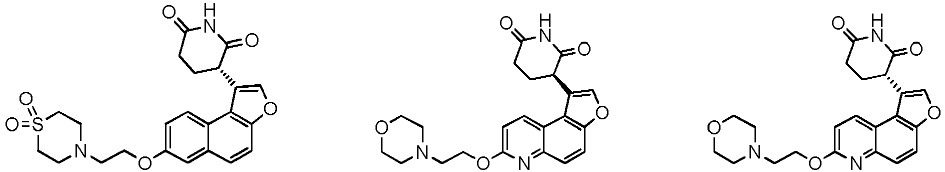

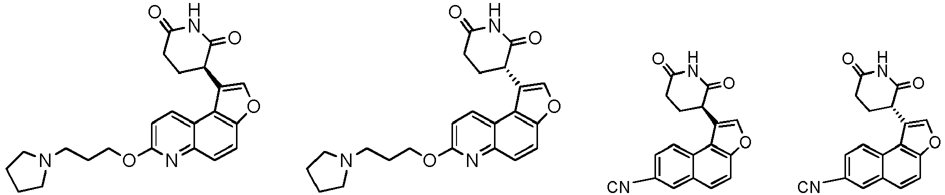

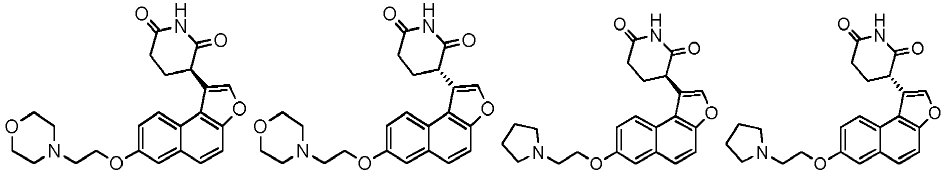

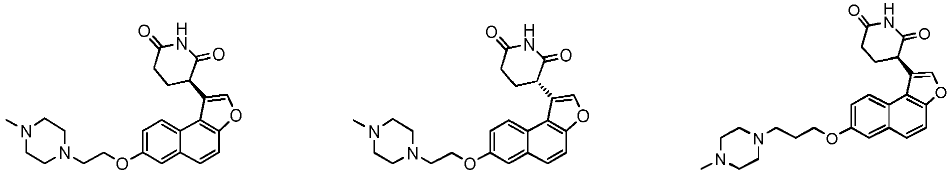

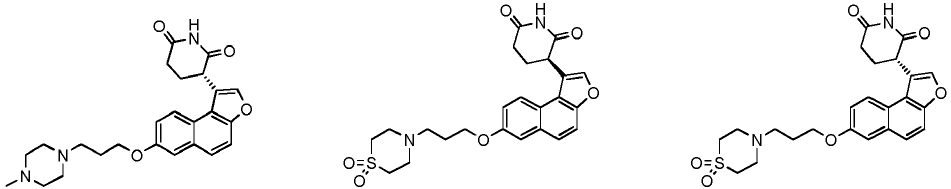

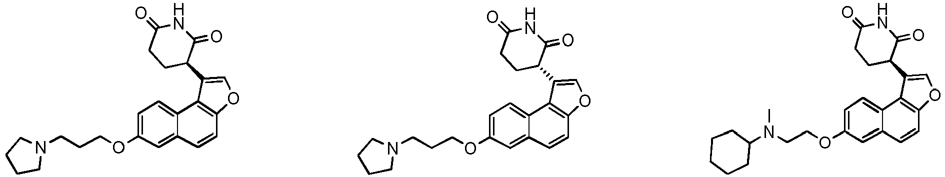

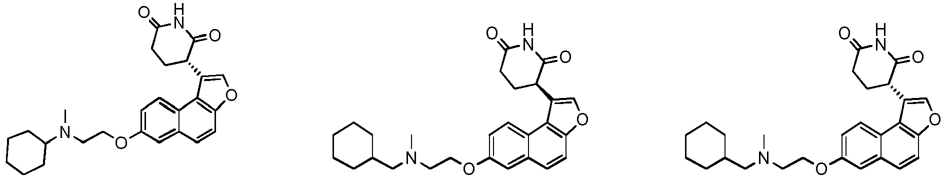

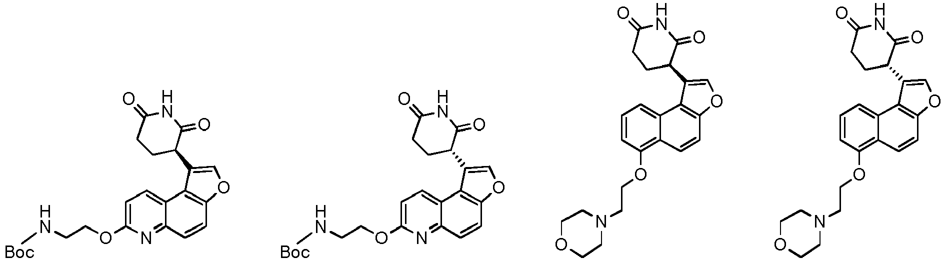

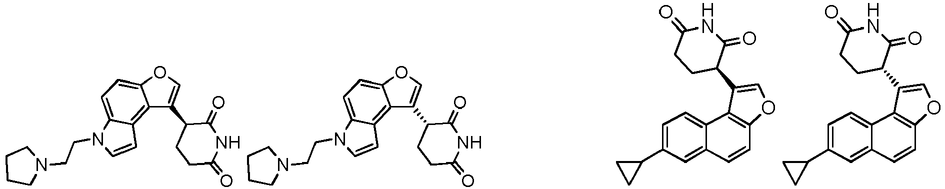

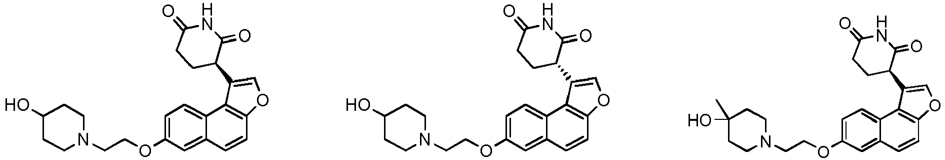

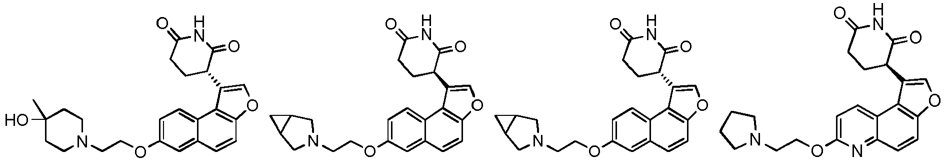

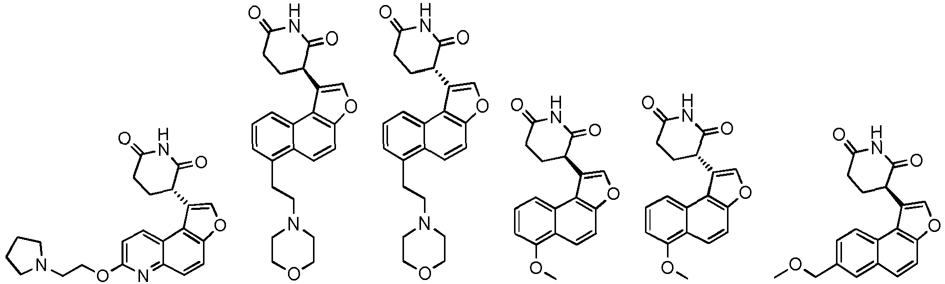

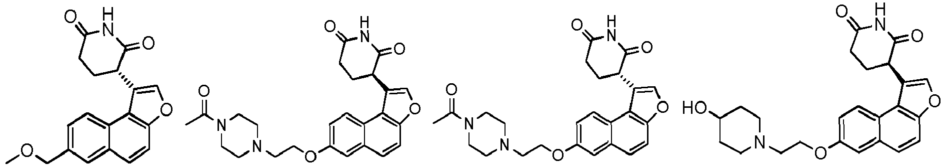

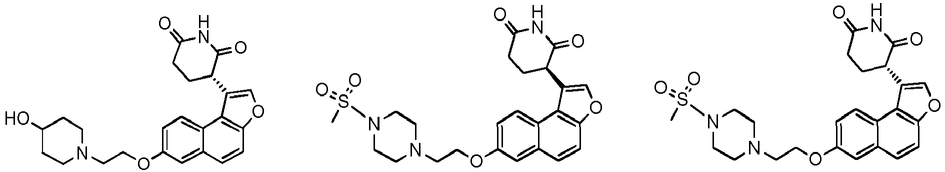

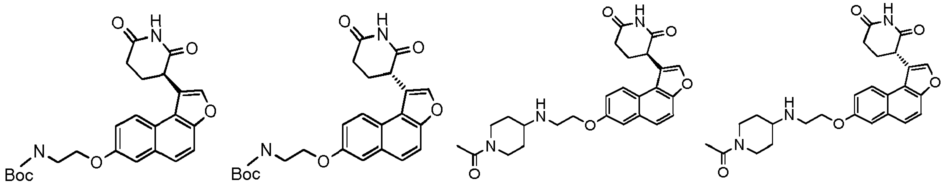

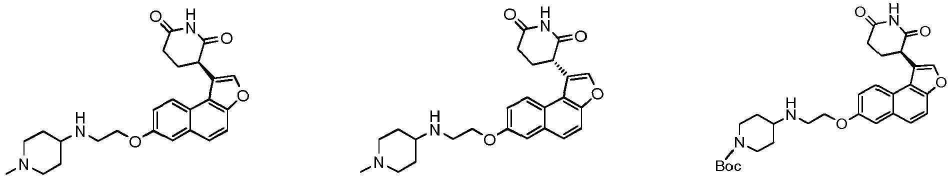

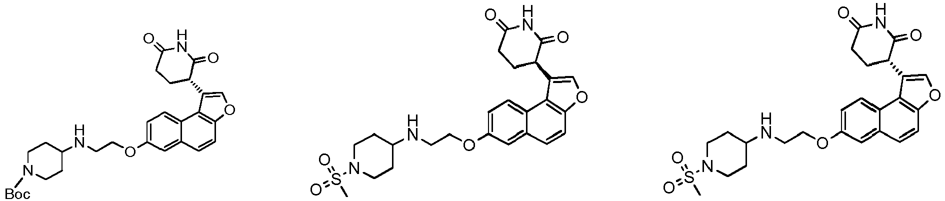

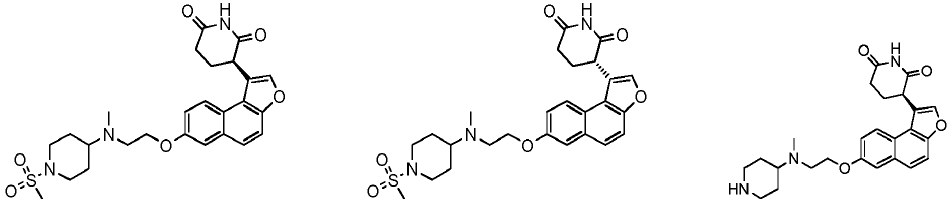

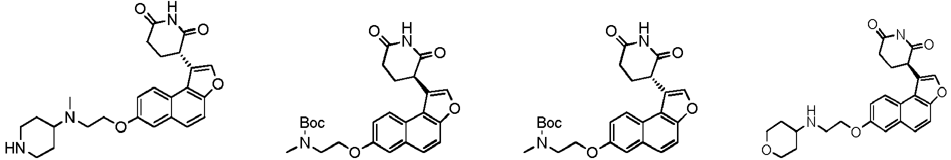

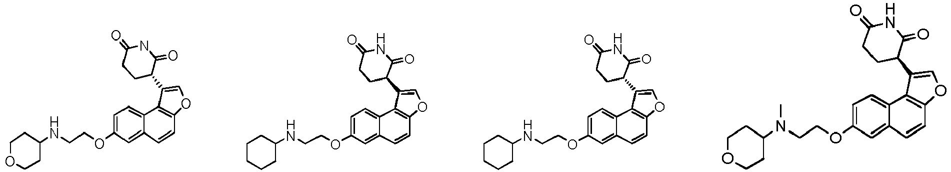

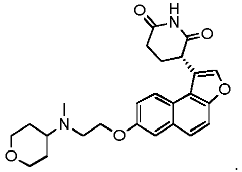

The present disclosure also provides a pharmaceutical composition comprising a therapeutically effective amount of the above-mentioned compound or pharmaceutically acceptable salt thereof as an active ingredient and a pharmaceutically acceptable carrier.

The present disclosure also provides the applications of the above-mentioned compound or pharmaceutically acceptable salt thereof in the preparation of medicines for treating diseases related to CRBN protein.

The present disclosure also provides the applications of the above-mentioned composition in the preparation of medicines for treating diseases related to CRBN protein.

### Definition and Description

Unless otherwise stated, the following terms and phrases used herein are intended to have the following meanings. A specific term or phrase should not be considered uncertain or unclear unless specifically defined, but should be understood in its ordinary meaning. When a trade name appears herein, it is intended to refer to the corresponding commodity or an active ingredient thereof.

The term "pharmaceutically acceptable" as used herein refers to those compounds, materials, compositions and/or dosage forms, which are, within the scope of sound medical judgment, suitable for use in contact with human and animal tissues, without excessive toxicity, irritation, allergic reactions or other problems or complications, which is commensurate with a reasonable benefit/risk ratio.

The term "pharmaceutically acceptable salt" refers to a salt of the compound of the present disclosure, which is prepared from the compound having specific substituents found in the present disclosure with relatively non-toxic acids or bases. When compounds of the present disclosure contain relatively acidic functional groups, base addition salts can be obtained by contacting the neutral form of such compounds with a sufficient amount of base, either in pure solution or a suitable inert solvent. Pharmaceutically acceptable base addition salts include sodium, potassium, calcium, ammonium, organic amine or magnesium salts or similar salts. When compounds of the present disclosure contain relatively basic functional groups, acid addition salts can be obtained by contacting the neutral form of such compounds with a sufficient amount of acid, either in pure solution or a suitable inert solvent. Examples of pharmaceutically acceptable acid addition salts include salts of inorganic acids, which include, for example, hydrochloric acid, hydrobromic acid, nitric acid, carbonic acid, bicarbonate, phosphoric acid, monohydrogen phosphate, dihydrogen phosphate, sulfuric acid, hydrogen sulfate, hydroiodic acid and phosphorous acid; and salts of organic acids, which include, for example, acetic acid, propionic acid, isobutyric acid, maleic acid, malonic acid, benzoic acid, succinic acid, suberic acid, fumaric acid, lactic acid, mandelic acid, phthalic acid, benzenesulfonic acid, p-toluenesulfonic acid, citric acid, tartaric acid, and methanesulfonic acid; and also include salts of amino acids (such as arginine), and salts of organic acids such as glucuronic acid. Certain specific compounds of the present disclosure contain basic and acidic functional groups and thus can be converted to any base or acid addition salt.

The pharmaceutically acceptable salts of the present disclosure can be synthesized from a parent compound containing acid radicals or base radicals by conventional chemical methods. In general, the method for preparing such salts comprises: in water or an organic solvent or a mixture of both, reacting these compounds in free acid or base forms with a stoichiometric amount of a suitable base or acid to prepare the salts.

The compounds of the present disclosure may exist in specific geometric or stereoisomeric forms. The present disclosure contemplates all such compounds, including cis and trans isomers, (-)- and (+)-enantiomers, (R)- and (*S*)-enantiomers, diastereomers, (D)-isomers, (*L*)-isomers, and racemic mixtures and other mixtures thereof, such as enantiomerically or diastereomerically enriched mixtures, all of which fall within the scope of the present disclosure. Additional asymmetric carbon atoms may be present in a substituent such as an alkyl group. All these stereoisomers and mixtures thereof are included in the scope of the present disclosure.

Unless otherwise stated, the term "enantiomer" or "optical isomers" refers to stereoisomers that are mirror images of each other.

Unless otherwise stated, the term "diastereomers" refers to stereoisomers in which molecules have two or more chiral centers and are not mirror images of each other.

Unless otherwise stated, "(+)" represents right-handed, "(-)" represents left-handed, and "(±)" means racemic.

Unless otherwise stated, the wedge-shaped solid bond ( 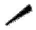 ) and the wedge-shaped dotted bond ( 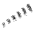 ) represent the absolute configuration of a stereoscopic center; the straight solid bond ( 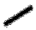 ) and straight dotted bond ( 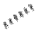 ) represent the relative configuration of a stereoscopic center; the wavy line ( 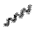 ) represents the wedge-shaped solid bond ( 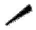 ) or the wedge-shaped dotted bond ( 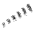 ); or the wavy line ( 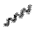 ) represents the straight solid bond ( 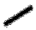 ) and the straight dotted bond ( 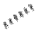 ).

Unless otherwise specified, when a group has one or more connectable sites, any one or more sites of the group can be connected to other groups through chemical bonds. The chemical bonds between the sites and other groups can be represented by a straight solid bond ( 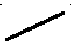 ), a straight dashed bond ( 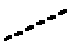 ), or a wavy line ( 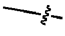 ). For example, the straight solid bond in -OCH₃ means that the group is connected to other groups through the oxygen atom in the group; the straight dashed bond in 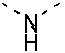 means that the group is connected to other groups through the two ends of the nitrogen atom in the group; the wavy line in 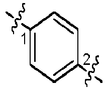 means that the group is connected to other groups through the 1 and 2 carbon atoms in the phenyl group.

The compounds of the present disclosure may exist in specific tautomeric forms. Unless otherwise stated, the term "tautomer" or "tautomeric form" means that at room temperature, isomers with different functional groups are in dynamic equilibrium and can be quickly converted to each other. Where tautomerization is possible (such as in solution), a chemical equilibrium of tautomers can be achieved. For example, proton tautomers (also known as prototropic tautomers) include interconversion via migration of a proton, such as keto-enol isomerization and imine-enamine isomerization. Valence tautomers include some interconversions by recombination of some of bond-forming electrons. A specific example of keto-enol tautomerization is the interconversion between two tautomers, pentane-2,4-dione and 4-hydroxypent-3-en-2-one.

Unless otherwise stated, the terms "rich in one isomer", "isomer enriched", "rich in one enantiomer" or "enantiomerically enriched" refer to the content of one of the isomers or enantiomers is less than 100%, and the content of the isomer or enantiomer is greater than or equal to 60%, or greater than or equal to 70%, or greater than or equal to 80%, or greater than or equal to 90%, or greater than or equal to 95%, or greater than or equal to 96%, or greater than or equal to 97%, or greater than or equal to 98%, or greater than or equal to 99%, or greater than or equal to 99.5%, or greater than or equal to 99.6%, or greater than or equal to 99.7%, or greater than or equal to 99.8%, or greater than or equal to 99.9%.

Unless otherwise stated, the term "isomer excess" or "enantiomeric excess" refers to the difference between the relative percentages of two isomers or two enantiomers. For example, if the content of one isomer or enantiomer is 90%, and the content of the other isomer or enantiomer is 10%, the isomer or enantiomer excess (ee value) is 80%.

Optically active (*R*)- and (*S*)-isomers and *D* and *L* isomers can be prepared using chiral synthesis or chiral reagents or other conventional techniques. If a particular enantiomer of a compound of the present disclosure is desired, it can be prepared by asymmetric synthesis or derivatization with a chiral auxiliary, wherein the resulting diastereomeric mixture is separated and the auxiliary groups are cleaved to provide pure desired enantiomers. Alternatively, where the molecule contains a basic functional group (such as an amino group) or an acidic functional group (such as a carboxyl group), diastereomeric salts can be formed with an appropriate optically active acid or base, followed by resolution of the diastereomers using conventional methods well known in the art, and subsequent recovery of the pure enantiomers. In addition, separation of enantiomers and diastereomers is frequently accomplished using chromatography, which uses chiral stationary phases, optionally in combination with chemical derivatization methods (e.g., formation of carbamates from amines).

The compounds of the present disclosure may contain unnatural proportions of atomic isotopes at one or more of the atoms constituting the compound. For example, the compounds may be radiolabeled with radioactive isotopes, such as tritium (³H), iodine-125 (¹²⁵I) or C-14 (¹⁴C). For another example, the hydrogen can be substituted by heavy hydrogen to form deuterated drugs. The bond formed by deuterium and carbon is stronger than the bond formed by ordinary hydrogen and carbon. Compared with undeuterated drugs, deuterated drugs have reduced toxic side effects, increased drug stability, enhanced efficacy, prolonged biological half-life of drugs and other advantages. All isotopic variations of the compounds of the present disclosure, whether radioactive or not, are intended to be encompassed within the scope of the present disclosure. "Optional" or "optionally" means that the subsequently described event or circumstance may, but not necessarily occur, and that the description includes instances where said event or circumstance occurs and instances where said event or circumstance does not occur.

The term "substituted" means that any one or more hydrogen atoms on the designated atom is substituted by a substituent, which may include heavy hydrogen and hydrogen variants, provided that the valence state of the designated atom is normal, and the substituted compound is stable. Where the substituent is oxygen (i.e., =O), it means that two hydrogen atoms are substituted. Oxygen substitution does not occur on aromatic groups. The term "optionally substituted" means that it may or may not be substituted. Unless otherwise specified, the type and number of substituents may be arbitrary on the basis that they can be achieved in chemistry.

Where any variable (such as R) appears more than once in the composition or structure of a compound, its definition in each case is independent. Thus, for example, if a group is substituted with 0-2 R, the group can optionally be substituted with up to two R, and R in each case has independent options. In addition, combinations of substituents and/or variants thereof are permissible only if such combinations result in stable compounds.

When the number of a linking group is 0, such as -(CRR)₀-, it means that the linking group is a single bond.

When a substituent is vacant, it means that the substituent does not exist. For example, when X is vacant in A-X, it means that the structure is actually A. When the substituents listed do not indicate through which atom they are connected to the substituted group, such substituents can be bonded through any of the atoms thereof, for example, pyridyl as a substituent can be attached to the substituted group via any carbon atom on the pyridine ring.

Unless otherwise specified, the number of atoms in a ring is usually defined as the member number of the ring. For example, "5- to 7-membered ring" means a "ring" with 5-7 atoms arranging in a circle.

Unless otherwise specified, the term "C₁₋₆ alkyl" is used to represent a linear or branched saturated hydrocarbon group consisting of 1 to 6 carbon atoms. The C₁₋₆ alkyl includes C₁₋₅, C₁₋₄, C₁₋₃, C₁₋₂, C₂₋₆, C₂₋₄, C₆ and C₅ alkyl; It can be monovalent (such as methyl), divalent (such as methyl) or multivalent (such as methine). Examples of C₁₋₆ alkyl include, but are not limited to, methyl (Me), ethyl (Et), propyl (including n-propyl and isopropyl), butyl (including n-butyl, isobutyl, s-butyl and t-butyl), pentyl (including n-pentyl, isopentyl and neopentyl) and hexyl.

Unless otherwise specified, the term "C₁₋₄ alkyl" is used to represent a linear or branched saturated hydrocarbon group consisting of 1 to 4 carbon atoms. The C₁₋₄ alkyl includes C₁₋₂, C₁₋₃, C₂₋₃ alkyl group, etc.; It can be monovalent (such as methyl), divalent (such as methyl) or multivalent (such as methine). Examples of C₁₋₄ alkyl include but are not limited to methyl (Me), ethyl (Et), propyl (including n-propyl and isopropyl), butyl (including *n*-butyl, isobutyl, *s*-butyl and *t*-butyl), etc.

Unless otherwise specified, the term "C₁₋₃ alkyl" is used to represent a linear or branched saturated hydrocarbon group consisting of 1 to 3 carbon atoms. The C₁₋₃ alkyl includes C₁₋₂ and C₂₋₃ alkyl; It can be monovalent (such as methyl), divalent (such as methyl) or multivalent (such as methine). Examples of C₁₋₃ alkyl include, but are not limited to, methyl (Me), ethyl (Et) and propyl (including *n*-propyl and isopropyl).

Unless otherwise specified, the term "C₁₋₁₀ alkylamino" means those alkyl groups containing 1 to 10 carbon atoms that are connected to the rest of the molecule through an amino group. The C₁₋₁₀ alkylamino includes C₁₋₁₀, C₁₋₉, C₁₋₈, C₁₋₇, C₁₋₆, C₁₋₅, C₁₋₄, C₁₋₃, C₁₋₂, C₂₋₆, C₂₋₄, C₆, C₅, C₄, C₂ alkylamino, etc. Examples of C₁₋₁₀ alkylamino include, but are not limited to -NHCH₃, -N(CH₃)₂, -NHCH₂CH₃, -N(CH₃)CH₂CH₃, -N(CH₂CH₃)(CH₂CH₃), -NHCH₂CH₂CH₃, -NHCH₂(CH₃)₂, -NHCH₂CH₂CH₂CH₃, etc.

Unless otherwise specified, the term "C₁₋₆ alkylamino" means those alkyl groups containing 1 to 6 carbon atoms that are connected to the rest of the molecule through an amino group. The C₁₋₆ alkylamino includes C₁₋₄, C₁₋₃, C₁₋₂, C₂₋₆, C₂₋₄, C₆, C₅, C₄, C₃, C₂ alkylamino, etc. Examples of C₁₋₆ alkylamino include, but are not limited to -NHCH₃, -N(CH₃)₂, -NHCH₂CH₃, -N(CH₃)CH₂CH₃, -N(CH₂CH₃)(CH₂CH₃), -NHCH₂CH₂CH₃, -NHCH₂(CH₃)₂, -NHCH₂CH₂CH₂CH₃, etc.

Unless otherwise specified, the term "C₁₋₃ alkylamino" means those alkyl groups containing 1 to 3 carbon atoms that are connected to the rest of the molecule through an amino group. The C₁₋₃ alkylamino includes C₁₋₂, C₃, C₂ alkylamino, etc. Examples of C₁₋₃ alkylamino include, but are not limited to -NHCH₃, -N(CH₃)₂, -NHCH₂CH₃, -N(CH₃)CH₂CH₃, -NHCH₂CH₂CH₃, -NHCH₂(CH₃)₂, etc.

Unless otherwise specified, the term "C₁₋₁₀ alkoxy" means those alkyl groups containing 1 to 10 carbon atoms that are connected to the rest of the molecule through one oxygen atom. The C₁₋₁₀ alkoxy group includes C₁₋₉, C₁₋₈, C₁₋₇, C₁₋₆, C₁₋₅, C₁₋₄, C₁₋₃, C₁₋₂, C₂₋₆, C₂₋₄, C₆, C₅, C₄, C₃ alkoxy, etc. Examples of C₁₋₁₀ alkoxy include but are not limited to methoxy, ethoxy, propoxy (including n-propoxy and isopropoxy), butoxy (including *n*-butoxy, isobutoxy, *s*-butoxy and *t*-butoxy), pentyloxy (including *n*-pentyloxy, isopentyloxy and neopentyloxy), hexyloxy, etc.

Unless otherwise specified, the term "C₁₋₆ alkoxy" means those alkyl groups containing 1 to 6 carbon atoms that are connected to the rest of the molecule through one oxygen atom. The C₁₋₆ alkoxy includes C₁₋₄, C₁₋₃, C₁₋₂, C₂₋₆, C₂₋₄, C₆, C₅, C₄, C₃ alkoxy, etc. Examples of C₁₋₆ alkoxy include but are not limited to methoxy, ethoxy, propoxy (including n-propoxy and isopropoxy), butoxy (including n-butoxy, isobutoxy, s-butoxy and t-butoxy), pentyloxy (including n-pentyloxy, isopentyloxy and neopentyloxy), hexyloxy, etc.

Unless otherwise specified, the term "C₂₋₄ alkoxy" means those alkyl groups containing 2 to 4 carbon atoms that are connected to the rest of the molecule through one oxygen atom. The C₂₋₄ alkoxy includes C₂₋₃, C₂₋₄, C₂, C₄, C₃ alkoxy, etc. Examples of C₂₋₄ alkoxy include but are not limited to methoxy, ethoxy, propoxy (including n-propoxy and isopropoxy), butoxy (including *n*-butoxy, isobutoxy, *s*-butoxy and *t*-butoxy), pentyloxy (including *n*-pentyloxy, isopentyloxy and neopentyloxy), hexyloxy, etc.

Unless otherwise specified, the term "C₁₋₃ alkoxy" means those alkyl groups containing 1 to 3 carbon atoms that are connected to the rest of the molecule through one oxygen atom. The C₁₋₃ alkoxy group includes C₁₋₂, C₂₋₃, C₃, C₂ alkoxy, etc. Examples of C₁₋₃ alkoxy include but are not limited to methoxy, ethoxy, propoxy (including n-propoxy and isopropoxy), etc.

Unless otherwise specified, the term "halo" or "halogen" by itself or as part of another substituent means a fluorine, chlorine, bromine or iodine atom.

Unless otherwise specified, "C₃₋₆ cycloalkyl" means a saturated cyclic hydrocarbon group consisting of 3 to 6 carbon atoms, which comprises a monocyclic and bicyclic ring system, and the C₃₋₆ cycloalkyl includes C₃₋₅ and C₅₋₆ cycloalkyl, etc.; It can be monovalent, bivalent or multivalent. Examples of C₃₋₆ cycloalkyl include, but are not limited to, cyclobutyl, cyclopentyl and cyclohexyl, etc.

Unless otherwise specified, "C₄₋₆ cycloalkyl" means a saturated cyclic hydrocarbon group consisting of 4 to 6 carbon atoms, which comprises a monocyclic and bicyclic ring system, wherein the bicyclic ring system includes a spiro ring, a fused ring and a bridged ring and the C₄₋₆ cycloalkyl includes C₄₋₅, C₅₋₆ cycloalkyl, etc.; It can be monovalent, bivalent or multivalent. Examples of C₃₋₆ cycloalkyl include, but are not limited to, cyclopropyl, cyclobutyl, cyclopentyl and cyclohexyl.

Unless otherwise specified, the term "4- to 10-membered heterocycloalkyl" by itself or in combination with other terms respectively represents a saturated cyclic group consisting of 4 to 10 ring atoms, of which 1, 2, 3 or 4 ring atoms are heteroatoms independently selected from O, S and N, and the rest of which are carbon atoms, wherein the nitrogen atom is optionally quaternized, and the nitrogen and sulfur heteroatoms can be optionally oxidized (i.e., NO and S(O)ₚ, wherein p is 1 or 2). It comprises a monocyclic, bicyclic and tricyclic ring system, wherein the bicyclic and tricyclic system include a spiro ring, a fused ring, and a bridged ring. In addition, in terms of the "4- to 10-membered heterocycloalkyl", the heteroatom may occupy the connection position of the heterocycloalkyl to the remainder of the molecule. The 4- to 10-membered heterocycloalkyl includes 4- to 8-membered, 4- to 6-membered, 4- to 7-membered, 4- to 9-membered, 4-membered, 5-membered, 6-membered heterocycloalkyl group, etc. Examples of 4- to 10-membered heterocycloalkyl include, but are not limited to, pyrrolidinyl, pyrazolidinyl, imidazolidinyl, tetrahydrothienyl (including tetrahydrothien-2-yl and tetrahydrothien-3-yl), tetrahydrofuranyl (including tetrahydrofuran-2-yl), tetrahydropyranyl, piperidinyl (including 1-piperidinyl, 2-piperidinyl, and 3-piperidinyl), piperazinyl (including 1-piperazinyl and 2-piperazinyl), morpholinyl (including 3-morpholinyl and 4-morpholinyl), dioxanyl, dithianyl, isoxazolidinyl, isothiazolidinyl, 1,2-oxazinyl, 1,2-thiazinyl, hexahydropyridazinyl, homopiperazinyl, homopiperidinyl or dioxepanyl, etc.

Unless otherwise specified, the term "4- to 7-membered heterocycloalkyl" by itself or in combination with other terms respectively represents a saturated cyclic group consisting of 4 to 7 ring atoms, of which 1, 2, 3 or 4 ring atoms are heteroatoms independently selected from O, S and N, and the rest of which are carbon atoms, wherein the nitrogen atom is optionally quaternized, and the nitrogen and sulfur heteroatoms can be optionally oxidized (i.e., NO and S(O)ₚ, wherein p is 1 or 2). It comprises a monocyclic and bicyclic ring system, wherein the bicyclic system includes a spiro ring, a fused ring, and a bridged ring. In addition, in terms of the "4- to 7-membered heterocycloalkyl", the heteroatom may occupy the connection position of the heterocycloalkyl to the remainder of the molecule. The 4- to 7-membered heterocycloalkyl includes 4- to 5-membered, 4- to 6-membered, 5- to 6-membered, 5- to 7-membered, 4-membered, 5-membered and 6-membered heterocycloalkyl group, etc. Examples of 4- to 7-membered heterocycloalkyl include, but are not limited to, azetidinyl, oxetanyl, thiatanyl, pyrrolidinyl, pyrazolidinyl, imidazolidinyl, tetrahydrothienyl (including tetrahydrothiophene-2-yl and tetrahydrothiophen-3-yl), tetrahydrofuranyl (including tetrahydrofuran-2-yl), tetrahydropyranyl, piperidinyl (including 1-piperidinyl, 2-piperidinyl and 3-piperidinyl), piperazinyl (including 1-piperazinyl and 2-piperazinyl), morpholinyl (including 3-morpholinyl and 4-morpholinyl), dioxanyl, dithianyl, isoxazolidinyl, isothiazolidinyl, 1,2-oxazinyl, 1,2-thiazinyl, hexahydropyridazinyl, homopiperazinyl, homopiperidinyl or oxepanyl.

Unless otherwise specified, the terms "5- to 6--membered heteroaryl ring" and "5- to 6-membered heteroaryl" of the present disclosure can be used interchangeably, and the term "5- to 6-membered heteroaryl" represents a monocyclic group having a conjugated π-electron system and consisting of 5 to 6 ring atoms, of which 1, 2, 3, or 4 ring atoms are heteroatoms independently selected from O, S, and N, and the rest of which are carbon atoms, wherein nitrogen atoms are optionally quaternized, and nitrogen and sulfur heteroatoms can be optionally oxidized (i.e., NO and S(O)ₚ, wherein p is 1 or 2). The 5- to 6-membered heteroaryl can be connected to the remainder of the molecule via a heteroatom or a carbon atom. The 5-to 6-membered heteroaryl includes 5-membered and 6-membered heteroaryl. Examples of the 5- to 6-membered heteroaryl include, but are not limited to, pyrrolyl (including N-pyrrolyl, 2-pyrrolyl and 3-pyrrolyl), pyrazolyl (including 2-pyrazolyl and 3-pyrazolyl), imidazolyl (including *N*-imidazolyl, 2-imidazolyl, 4-imidazolyl and 5-imidazolyl), oxazolyl (including 2-oxazolyl, 4-oxazolyl and 5-oxazolyl), triazolyl (1*H*-1,2,3-triazolyl, 2*H*-1,2,3-triazolyl, 1*H-*1,2,4-triazolyl and 4*H*-1,2,4-triazolyl), tetrazolyl, isoxazolyl (3-isoxazolyl, 4-isoxazolyl and 5-isoxazolyl), thiazolyl (including 2-thiazolyl, 4-thiazolyl and 5-thiazolyl), furyl (including 2-furanyl and 3-furanyl), thienyl (including 2-thienyl and 3-thienyl), pyridyl (including 2 -pyridyl, 3-pyridyl and 4-pyridyl), pyrazinyl or pyrimidinyl (including 2-pyrimidyl and 4-pyrimidyl).

Unless otherwise specified, Cₙ₋ₙ₊ₘ or Cₙ-Cₙ₊ₘ includes any specific case of n to n + m carbons, for example, C₁₋₁₂ includes C₁, C₂, C₃, C₄, C₅, C₆, C₇, C₈, C₉, C₁₀, C₁₁ and C₁₂, and also includes any range from n to n + m, for example, C₁₋₁₂ includes C₁₋₃, C₁₋₆, C₁₋₉, C₃₋₆, C₃₋₉, C₃₋₁₂, C₆₋₉, C₆₋₁₂, and C₉₋₁₂; Similarly, n-membered to n + m-membered means that the number of atoms in the ring is n to n + m, for example, a 3- to 12-membered ring includes a 3-membered ring, a 4-membered ring, a 5-membered ring, a 6-membered ring, a 7-membered ring, a 8-membered ring, a 9-membered ring, a 10-membered ring, a 11-membered ring, and a 12-membered ring, and also includes any range from n to n + m, for example, a 3- to 12-membered ring includes a 3- to 6-membered ring, a 3- to 9-membered ring, a 5- to 6-membered ring, a 5-to 7-membered ring, a 6- to 7-membered ring, a 6- to 8-membered ring, and a 6- to 10-membered ring.

The term "leaving group" refers to a functional group or atom that can be substituted by another functional group or atom through a substitution reaction (e.g., an affinity substitution reaction). For example, representative leaving groups include trifluoromethanesulfonate; chlorine, bromine and iodine; sulfonates, such as methanesulfonate, tosylate, p-bromobenzenesulfonate, and p-toluenesulfonate; and acyloxy, such as acetoxy and trifluoroacetoxy.

The term "protecting group" includes, but is not limited to, "amino protecting group", "hydroxy protecting group" or "mercapto protecting group". The term "amino protecting group" refers to a protecting group suitable for preventing side reactions occur at the nitrogen atom of an amino group. Representative amino protecting groups include, but are not limited to: formyl; acyl, such as alkanoyl (e.g., acetyl, trichloroacetyl or trifluoroacetyl); alkoxycarbonyl, such as tert-butoxycarbonyl (Boc); aryl methoxycarbonyl, such as benzyloxycarbonyl (Cbz) and 9-fluorenylmethoxycarbonyl (Fmoc); aryl methyl, such as benzyl (Bn), triphenyl methyl (Tr), 1,1-bis-(4'-methoxyphenyl)methyl; silyl, such as trimethylsilyl (TMS) and tert-butyldimethylsilyl (TBS). The term "hydroxyl protecting group" refers to a protecting group suitable for preventing side reactions of a hydroxyl group. Representative hydroxyl protecting groups include, but are not limited to: alkyl, such as methyl, ethyl and tert-butyl; acyl, such as alkanoyl (e.g., acetyl); arylmethyl, such as benzyl (Bn), p-methoxybenzyl (PMB), 9-fluorenylmethyl (Fm) and diphenylmethyl (DPM); silyl, such as trimethylsilyl (TMS) and tert-butyldimethylsilyl (TBS).

The compounds of the present disclosure can be prepared by various synthetic methods well known to a person skilled in the art, including the specific embodiments listed below, the embodiments formed by the combination with other chemical synthesis methods, and equivalent alternative embodiments well known to a person skilled in the art, wherein the preferred embodiments include but are not limited to the examples of the present disclosure.

The solvents used in the present disclosure are commercially available. The present disclosure uses the following abbreviations: aq represents water; HATU represents O-(7-azabenzotriazol-1-yl)-N,N,N',N'-tetramethylurea hexafluorophosphate; EDC represents N-(3-dimethylaminopropyl)-N'-ethylcarbodiimide hydrochloride; m-CPBA represents 3-chloroperoxybenzoic acid; eq represents equivalent; CDI represents carbonyldiimidazole; DCM represents dichloromethane; PE represents petroleum ether; DIAD represents diisopropyl azodicarboxylate; DMF represents N,N-dimethylformamide; DMSO represents dimethyl sulfoxide; EtOAc represents ethyl acetate; EtOH represents ethanol; MeOH represents methanol; CBz represents benzyloxycarbonyl, which is an amine protecting group; BOC represents tert-butoxycarbonyl, which is an amine protecting group; HOAc represents acetic acid; NaCNBH₃ represents sodium cyanoborohydride; r.t. represents room temperature; O/N represents overnight; THF represents tetrahydrofuran; Boc₂O represents di-tert-butyl dicarbonate; TFA represents trifluoroacetic acid; DIPEA represents diisopropylethylamine; SOCl₂ represents thionyl chloride; CS₂ represents carbon disulfide; TsOH represents p-toluenesulfonic acid; NFSI represents N-fluoro-N-(benzenesulfonyl)benzenesulfonamide; NCS represents 1-chloropyrrolidine-2,5-dione; *n*-Bu₄NF represents tetrabutylammonium fluoride; iPrOH represents 2-propanol; mp represents melting point; LDA represents lithium diisopropylamide; M represents mol/L.

Compounds are named according to conventional naming principles in the field or using ChemDraw^{®} software, and commercially available compounds are named using supplier catalog names.

### Technical Effects

The compound of the present disclosure exhibits a significant down-regulation effect on IKZF3 protein level in multiple myeloma cells MM.1S; the compound of the present disclosure exhibits an excellent inhibitory effect on cell proliferation in the lymphoma cell lines such as OCI-LY10, DOHH2 and Mino. In rodent mice, the pharmacokinetic properties of the compound of the present disclosure are very good; the compound **WX002** of the present disclosure has shown a significant tumor-shrinking effect on the human lymphoma OCI-LY10 in vivo pharmacodynamic model.

### Brief description of the drawings

Figure **1** shows the changes of IKZF3 protein levels in the cells detected by WB after treating multiple myeloma cells MM.1S with the compounds WX001 to WX008 of the present disclosure at a concentration of 100 nM;
Figure **2** shows the changes of IKZF3 protein levels in the cells detected by WB after treating multiple myeloma cells MM.1S with the compounds WX009 to WX030 of the present disclosure at a concentration of 100 nM;
Figure **3** shows the changes of IKZF3 protein levels in the cells detected by WB after treating multiple myeloma cells MM.1S with the compounds WX031 to WX056 of the present disclosure at a concentration of 100 nM.

### Detailed description of the preferred embodiment

### Example 1: WX001

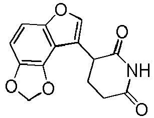

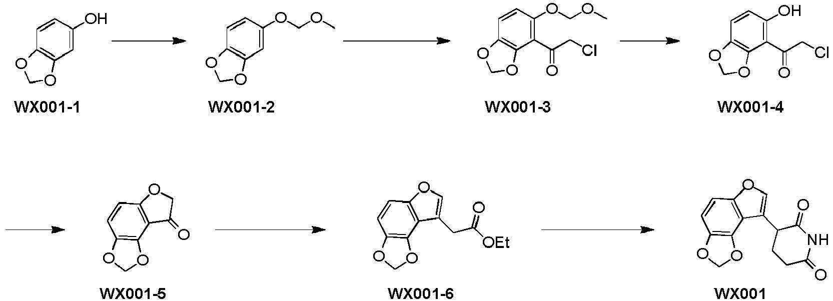

### Step 1: Synthesis of intermediate WX001-2

At room temperature, **WX001-1** (11 g, 79.64 mmol) was dissolved in dichloromethane (100 mL), and then diisopropylethylamine (36.03 g, 278.74 mmol, 48.55 mL) was added. The reaction mixture was cooled to 0 °C, and then chloromethyl ether (10.7 g, 132.90 mmol, 10.09 mL) was added. The reaction mixture was warmed to room temperature and stirred and reacted for 2 hours. After completion of the reaction, water (100 mL) was added for dilution, the organic phase was collected after separation, and the aqueous phase was extracted with dichloromethane (50 mL × 2). The organic phase was combined, dried over anhydrous sodium sulfate, filtered and concentrated under reduced pressure to obtain a residue, which was purified by column chromatography (eluent: petroleum ether/ethyl acetate = 100/0-9/1, volume ratio) to obtain the intermediate **WX001-2.** ¹H NMR (400 MHz, CDCl₃) δ: 6.72 (d, *J =* 8.4 Hz, 1H), 6.63 (d, *J =* 2.4 Hz, 1H), 6.50 (dd, *J =* 2.4, 8.5 Hz, 1H), 5.93 (s, 2H), 5.09 (s, 2H), 3.49 (s, 3H).

### Step 2: Synthesis of intermediate WX001-3

At room temperature, the intermediate **WX001-2** (2.5 g, 13.72 mmol) was dissolved in tetrahydrofuran (30 mL), the reaction mixture was cooled to -60 °C under nitrogen atmosphere, and then *n*-butyllithium (2.5 M, 5.49 mL) was added, and the reaction mixture was stirred and reacted at-60 °C for 10 minutes, then 2-chloro-N-methoxy-N-methylacetamide (2.27 g, 16.47 mmol) was added, and the reaction mixture was warmed to room temperature and was stirred and reacted for additional 2 hours. After completion of the reaction, water (100 mL) was add and extraction with ethyl acetate (50 mL × 2) was performed. The organic phase was combined, dried over anhydrous sodium sulfate and filtered, and concentrated under reduced pressure to obtain the intermediate **WX001-3.** ¹H NMR (400 MHz, CDCl₃) δ: 6.84 (d, *J =* 8.4 Hz, 1H), 6.64 (d, *J =* 7.6 Hz, 1H), 6.05 (s, 2H), 5.17 (s, 2H), 4.65 (s, 2H), 3.50 (s, 3H).

### Step 3: Synthesis of intermediate WX001-4

At room temperature, hydrochloric acid/ethyl acetate (4 M, 40 mL) was added to intermediate **WX001-3** (3 g, 11.60 mmol), and the reaction mixture was stirred at room temperature for 12 hours. After the completion of the reaction, the reaction mixture was directly concentrated under reduced pressure, and the resulting residue was purified by column chromatography (eluent: petroleum ether/ethyl acetate = 100/0-4/1, volume ratio) to obtain the intermediate **WX001-4.** ¹H NMR (400 MHz, CDCl₃) δ: 11.23 (s, 1H), 7.01 (d, *J =* 8.4 Hz, 1H), 6.47 (d, *J=* 8.8 Hz, 1H), 6.09 (s, 2H), 4.77 (s, 2H).

### Step 4: Synthesis of intermediate WX001-5

At room temperature, the intermediate **WX001-4** (800 mg, 3.73 mmol) was dissolved in acetonitrile (40 mL), then potassium carbonate (1.03 g, 7.46 mmol) was added, and the reaction mixture was stirred at room temperature for 3 hours. After completion of the reaction, the reaction mixture was filtered, the filtrate was concentrated under reduced pressure, and the resulting residue was purified by column chromatography (eluent: petroleum ether/ethyl acetate = 100/0-4/1, volume ratio) to obtain the intermediate **WX001-5.** ¹H NMR (400 MHz, CDCl₃) δ: 7.06 (d, *J=* 8.8 Hz, 1H), 6.53 (d, *J=* 8.8 Hz, 1H), 6.13 (s, 2H), 4.63 (s, 2H).

### Step 5: Synthesis of intermediate WX001-6

At room temperature, the intermediate **WX001-5** (260 mg, 1.46 mmol) was dissolved in toluene (10 mL), then ethyl (triphenylphosphine) acetate (762.69 mg, 2.19 mmol) was added, and the reaction mixture was heated under nitrogen atmosphere to 120 °C and stirred and reacted for 48 hours. After completion of the reaction, the reaction mixture was concentrated under reduced pressure, methyl *tert*-butyl ether (10 mL) was added to the resulting residue, stirred at room temperature for 30 minutes, then filtered, and the filter cake was discarded. The solvent was removed from the resulting filtrate under reduced pressure and the resulting residue was purified by column chromatography (eluent: petroleum ether/ethyl acetate = 100/0-9/1, volume ratio) to obtain the intermediate **WX001-6.** ¹H NMR (400 MHz, CDCl₃) δ: 7.54 (s, 1H), 6.94 (d, *J =* 8.8 Hz, 1H), 6.83 (d, *J =* 8.8 Hz, 1H), 6.03 (s, 2H), 4.22 (q, *J =* 7.2 Hz, 2H), 3.76 (s, 2H), 1.29 (t, *J=* 7.2 Hz, 3H).

### Step 6: Synthesis of compound WX001

At room temperature, the intermediate **WX001-6** (170 mg, 684.85 µmol) was dissolved in tetrahydrofuran (10 mL), then potassium *tert*-butoxide (76.85 mg, 684.85 µmol) was added, and then the solution of acrylamide (48.68 mg, 684.85 µmol) in tetrahydrofuran (0.5 mL) was added dropwise, and the reaction mixture was stirred at room temperature for 0.5 hours under nitrogen atmosphere. After completion of the reaction, water (10 mL) was add and extraction with ethyl acetate (10 mL × 3) was performed. The organic phase was combined, dried over anhydrous sodium sulfate and filtered, and concentrated under reduced pressure to obtain a residue. The resulting residue was purified by column chromatography (eluent: petroleum ether/ethyl acetate = 100/0-1/4, volume ratio) to obtain the compound **WX001.** MS-ESI *m*/*z:* 274.0 [M+H]⁺. ¹H NMR (400 MHz, DMSO_*d*₆) δ: 10.89 (s, 1H), 7.86 (s, 1H), 7.07 (d, *J=* 8.4 Hz, 1H), 6.96 (d, *J=* 8.8 Hz, 1H), 6.08-5.98 (m, 2H), 4.05 (dd, *J* = 4.8, 12.8 Hz, 1H), 2.85-2.71 (m, 1H), 2.69-2.53 (m, 1H), 2.27 (dq, *J =* 4.5, 13.0 Hz, 1H), 2.12-2.02 (m, 1H).

### Example 2: WX002

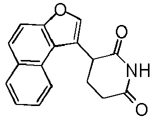

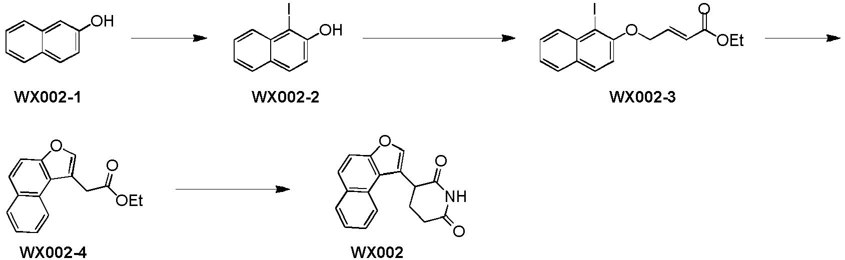

### Step 1: Synthesis of intermediate WX002-2

At room temperature, **WX002-1** (5 g, 34.68 mmol) was dissolved in dichloromethane (70 mL), and then N-iodosuccinimide (7.80 g, 34.68 mmol) and p-toluenesulfonic acid (1.98 g, 10.40 mmol) were added successively, and the reaction mixture was stirred at room temperature for 30 minutes. After completion of the reaction, water (50 mL) was added for dilution, the organic phase was collected after separation, and the aqueous phase was extracted with dichloromethane (50 mL × 2). The organic phase was combined, dried over anhydrous sodium sulfate and filtered, and the solvent was removed from the filtrate under reduced pressure to obtain the target intermediate **WX002-2.** ¹HNMR (400 MHz, CDCl₃) δ: 7.96 (d, *J =* 8.4 Hz, 1H), 7.76 (dd, *J =* 3.2, 8.4 Hz, 2H), 7.58 (t, *J =* 7.7 Hz, 1H), 7.46-7.37 (m, 1H), 7.32-7.24 (m, 1H), 4.95 (s, 1H).

### Step 2: Synthesis of intermediate WX002-3

At room temperature, the intermediate **WX002-2** (9 g, 33.33 mmol) was dissolved in acetonitrile (150 mL), then potassium carbonate (9.21 g, 66.65 mmol) and ethyl 4-bromocrotonate (6.43 g, 33.33 mmol, 4.60 mL) were added successively, and the reaction mixture was stirred at room temperature for 12 hours. After completion of the reaction, the reaction solution was filtered, the filtrate was collected, and the filter cake was washed with ethyl acetate (30 mL × 2). The filtrate and washings were combined, the solvent was removed under reduced pressure, and the resulting residue was purified by column chromatography (eluent: petroleum ether/ethyl acetate = 1/0-20/1, volume ratio) to obtain the target intermediate **WX002-3.** ¹H NMR (400 MHz, CDCl₃) δ: 8.17 (d, *J=* 8.4 Hz, 1H), 7.81 (d, *J* = 8.8 Hz, 1H), 7.75 (d, *J =* 8.0 Hz, 1H), 7.57 (ddd, *J =* 1.2, 7.0, 8.5 Hz, 1H), 7.42 (ddd, *J =* 1.1, 6.9, 8.1 Hz, 1H), 7.19-7.10 (m, 2H), 6.46 (td, *J =* 2.1, 15.7 Hz, 1H), 4.90 (dd, *J=* 2.0, 3.6 Hz, 2H), 4.25 (q, *J=* 7.2 Hz, 2H), 1.33 (t, *J =* 7.1 Hz, 3H).

### Step 3: Synthesis of intermediate WX002-4

At room temperature and under nitrogen atmosphere, the intermediate **WX002-3** (3 g, 7.85 mmol) was dissolved in N,N-dimethylformamide (50 mL), and then sodium carbonate (2.08 g, 19.62 mmol), palladium acetate (88.11 mg, 392.47 µmol), tetrabutylammonium chloride (2.40 g, 8.63 mmol) and sodium formate (533.82 mg, 7.85 mmol) were added successively, and the reaction mixture was heated to 80 °C and stirred and reacted for 2 hours. After completion of the reaction, the reaction mixture was cooled to room temperature, water (100 mL) and ethyl acetate (100 mL) were added for dilution, the organic phase was collected after separation, and the aqueous phase was extracted with ethyl acetate (50 mL × 2). The organic phase was combined, washed with half-saturated brine (50 mL × 2), dried over anhydrous sodium sulfate and filtered, and the solvent was removed from the filtrate under reduced pressure. The resulting residue was purified by column chromatography (eluent: petroleum ether/ethyl acetate = 1/0-20/1, volume ratio) to obtain the intermediate **WX002-4.** ¹H NMR (400 MHz, CDCl₃) δ: 8.25 (d, *J =* 8.4 Hz, 1H), 7.97 (d, *J =* 8.4 Hz, 1H), 7.80-7.72 (m, 2H), 7.69-7.64 (m, 1H), 7.60 (dt, *J =* 1.3, 7.7 Hz, 1H), 7.53-7.46 (m, 1H), 4.24 (q, *J =* 7.1 Hz, 2H), 4.09 (s, 2H), 1.28 (t, *J =* 7.2 Hz, 3H).

### Step 4: Synthesis of compound WX002

At room temperature and under nitrogen atmosphere, the intermediate **WX002-4** (1.4 g, 5.51 mmol) was dissolved in tetrahydrofuran (70 mL), and then acrylamide (391.34 mg, 5.51 mmol) and potassium tert-butoxide (617.81 mg, 5.51 mmol) were added successively, and the reaction mixture was stirred and reacted at room temperature for 3 hours. After completion of the reaction, water (100 mL) and ethyl acetate (30 mL) were added for dilution, the organic phase was collected after separation, and the aqueous phase was extracted with ethyl acetate (30 mL × 2). The organic phase was combined, dried over anhydrous sodium sulfate and filtered, and the solvent was removed from the filtrate under reduced pressure. The resulting residue was purified by column chromatography (eluent: petroleum ether/ethyl acetate = 1/0-2/3, volume ratio), and then purified again by preparative HPLC (mobile phase: acetonitrile/water; acid system: 0.05% HCl) to obtain the compound **WX002.** MS-ESI *m*/*z:* 280.0 [M+H]⁺. ¹H NMR (400 MHz, DMSO_*d*₆) δ: 10.95 (s, 1H), 8.18 (d, *J =* 8.6 Hz, 1H), 8.06 (d, *J=* 8.2 Hz, 1H), 8.01 (s, 1H), 7.90-7.84 (m, 1H), 7.82-7.77 (m, 1H), 7.62-7.56 (m, 1H), 7.55-7.49 (m, 1H), 4.68 (dd, *J=* 4.6, 12.2 Hz, 1H), 2.89-2.82 (m, 1H), 2.71-2.58 (m, 1H), 2.48-2.36 (m, 1H), 2.35-2.22 (m, 1H).

### Example 3: Hydrochloride of WX003

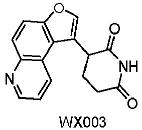

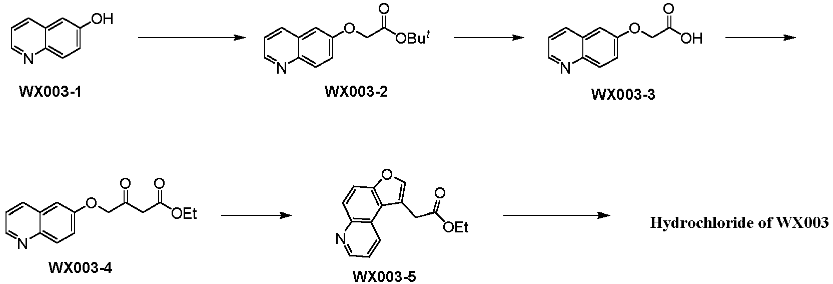

### Step 1: Synthesis of intermediate WX003-2

At room temperature and under nitrogen atmosphere, **WX003-1** (9.5 g, 65.45 mmol) and *tert*-butyl 2-bromoacetate (14.04 g, 71.99 mmol, 10.64 mL) were dissolved in N,N-dimethylformamide (100 mL) and then potassium carbonate (9.05 g, 65.45 mmol) was slowly added in batches. The reaction mixture was heated to 60 °C and stirred and reacted for 12 hours, then the reaction system was cooled to 30 °C, sodium hydride (2.62 g, 65.45 mmol, purity: 60%) was slowly added in batches, and the resulting reaction mixture was heated to 60 °C again and stirred and reacted for additional 2 hours. After completion of the reaction, the reaction mixture was cooled to room temperature, quenched by adding water (100 mL), and extracted with ethyl acetate (200 mL × 2). The organic phase was combined, washed with half-saturated brine (150 mL × 3), dried over anhydrous sodium sulfate and filtered, and the solvent was removed from the filtrate under reduced pressure. The resulting residue was purified by column chromatography (eluent: ethyl acetate) to obtain the intermediate **WX003-2.** ¹H NMR (400 MHz, CDCl₃) δ: 8.81 (dd, *J=* 1.6, 4.3 Hz, 1H), 8.10-8.03 (m, 2H), 7.48 (dd, *J =* 2.8, 9.2 Hz, 1H), 7.38 (dd, *J =* 4.3, 8.3 Hz, 1H), 7.03 (d, *J* = 2.8 Hz, 1H), 4.67 (s, 2H), 1.52 (s, 9H).

### Step 2: Synthesis of intermediate WX003-3

At room temperature, the solution of hydrogen chloride in ethyl acetate (4 M, 50 mL) was added to intermediate **WX003-2** (5 g, 16.91 mmol), and the reaction mixture was stirred at room temperature for 12 hours. After completion of the reaction, the solvent was directly removed from the reaction solution under reduced pressure to obtain the target intermediate **WX003-3.** ¹H NMR (400 MHz, DMSO_*d*₆) δ: 9.08 (dd, *J=* 1.4, 5.3 Hz, 1H), 8.93 (d, *J* = 8.4 Hz, 1H), 8.33 (d, *J =* 9.2 Hz, 1H), 7.96 (dd, *J =* 5.1, 8.4 Hz, 1H), 7.80 (dd, *J =* 2.7, 9.2 Hz, 1H), 7.70 (d, *J=* 2.4 Hz, 1H), 4.91 (s, 2H).

### Step 3: Synthesis of intermediate WX003-4

At room temperature and under nitrogen atmosphere, intermediate **WX003-3** (3.5 g, 14.60 mmol) was added to acetonitrile (35 mL) in a first reaction flask. After cooling to 0 °C, triethylamine (2.96 g, 29.21 mmol, 4.07 mL) and carbonyldiimidazole (4.74 g, 29.21 mmol) were added successively, and the reaction mixture was warmed to room temperature and stirred and reacted for 2 hours. At room temperature, monoethyl malonate potassium salt (4.97 g, 29.21 mmol) was added to acetonitrile (70 mL) in another reaction flask, and then triethylamine (5.47 g, 54.04 mmol, 7.52 mL) and magnesium chloride (3.75 g, 39.43 mmol) were added successively, and the reaction mixture was stirred and reacted at room temperature for 2 hours. Finally, at 0 °C, the reaction mixture in the second reaction flask was added dropwise to the first reaction flask, and the reaction mixture was slowly warmed to room temperature and the reaction was stirred and reacted for additional 10 hours. After completion of the reaction, the reaction was quenched by adding water (180 mL), and extracted with ethyl acetate (60 mL × 2). The organic phase was combined, washed with half-saturated brine (50 mL × 3), dried over anhydrous sodium sulfate and filtered, and the solvent was removed from the filtrate under reduced pressure. The resulting residue was purified by column chromatography (eluent: petroleum ether/ethyl acetate = 1/0-0/1, volume ratio) to obtain the intermediate **WX003-4.** ¹H NMR (400 MHz, CDCl₃) δ: 8.83-8.77 (m, 1H), 8.07-8.00 (m, 2H), 7.44-7.34 (m, 2H), 7.03 (d, *J=* 2.8 Hz, 1H), 4.79 (s, 2H), 4.28-4.15 (m, 2H), 3.67 (s, 2H), 1.36-1.21 (m, 3H).

### Step 4: Synthesis of intermediate WX003-5

At room temperature and under nitrogen atmosphere, the intermediate **WX003-4** (1.5 g, 5.49 mmol) was dissolved in toluene (3 mL), and polyphosphoric acid (2 mL) was added. The reaction mixture was heated to 110 °C and stirred and reacted for 0.5 hours. After completion of the reaction, the reaction solution was poured into ice water (80 mL) for quenching, adjusted with saturated sodium bicarbonate solution to achieve pH 7-8, and extracted with ethyl acetate (100 mL × 2). The organic phase was combined, dried over anhydrous sodium sulfate and filtered, and the solvent was removed from the filtrate under reduced pressure. The resulting residue was purified by column chromatography (eluent: petroleum ether/ethyl acetate = 1/0-1/1, volume ratio) to obtain the intermediate **WX003-5.** ¹H NMR (400 MHz, CDCl₃) δ: 8.76 (dd, *J =* 1.6, 4.3 Hz, 1H), 8.44 (d, *J =* 8.5 Hz, 1H), 7.87 (d, *J =* 9.2 Hz, 1H), 7.74-7.68 (m, 1H), 7.66 (s, 1H), 7.33 (dd, *J =* 4.0, 8.4 Hz, 1H), 4.05 (q, *J* = 7.2 Hz, 2H), 3.88 (s, 2H), 1.08 (t, *J=* 7.2 Hz, 3H).

### Step 5: Synthesis of compound WX003

At room temperature, the intermediate **WX003-5** (60.00 mg, 206.84 µmol) was dissolved in tetrahydrofuran (3.5 mL), acrylamide (14.70 mg, 206.84 µmol) was added. After cooling to 0 °C, potassium *tert*-butoxide (23.21 mg, 206.84 µmol) was added, and the reaction mixture was stirred and reacted for 1 hour at 0 °C. After completion of the reaction, the reaction was quenched by adding water (50 mL), and extracted with ethyl acetate (100 mL × 2). The organic phase was combined, washed with half-saturated brine (50 mL × 3), dried over anhydrous sodium sulfate and filtered, and the solvent was removed from the filtrate under reduced pressure. The resulting residue was purified by preparative HPLC (mobile phase: acetonitrile/water; acid system: 0.05% HCl) to obtain the hydrochloride of the target compound **WX003.** MS-ESI m/z: 281.0 [M+H]⁺. ¹H NMR (400 MHz, DMSO_*d*₆) δ: 10.96 (s, 1H), 9.14-9.03 (m, 2H), 8.30 (d, *J =* 9.2 Hz, 1H), 8.25 (s, 1H), 8.15 (d, *J* = 9.2 Hz, 1H), 7.90 (dd, *J* = 4.9, 8.4 Hz, 1H), 4.75 (dd, *J=* 4.4, 12.8 Hz, 1H), 2.94-2.78 (m, 2H), 2.71-2.60 (m, 1H), 2.35-2.23 (m, 1H).

### Example 4: WX004

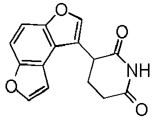

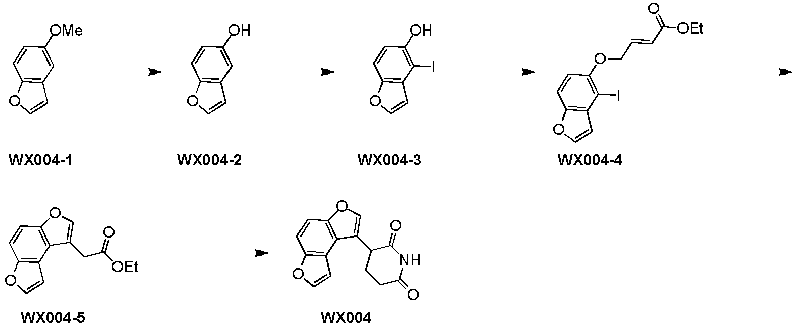

### Step 1: Synthesis of intermediate WX004-2

At room temperature, **WX004-1** (0.5 g, 3.37 mmol) was dissolved in dichloromethane (15 mL), cooled to -78 °C, then boron tribromide (1.69 g, 6.75 mmol, 650.34 µL) was added, and the reaction mixture was slowly warmed to 0 °C and stirred and reacted for 4 hours at 0 °C. After completion of the reaction, the reaction mixture was diluted by adding dichloromethane (50 mL), then slowly poured into 1 M aqueous sodium hydroxide solution (20 mL), adjusted with 1 M aqueous hydrochloric acid to achieve pH 6-7. The organic phase was collected after separation. The organic phase was dried over anhydrous sodium sulfate and filtered, and the solvent was removed from the filtrate under reduced pressure. The resulting residue was purified by column chromatography (eluent: petroleum ether/ethyl acetate = 100/0-9/1, volume ratio) to obtain the intermediate **WX004-2.** MS-ESI m/z: 133.1[M-H]⁻.

### Step 2: Synthesis of intermediate WX004-3

At room temperature, the intermediate **WX004-2** (0.2 g, 1.49 mmol) was dissolved in dichloromethane (3 mL), then N-iodosuccinimide (335.46 mg, 1.49 mmol) was added, and the reaction mixture was stirred and reacted at room temperature for 12 hours. After completion of the reaction, the reaction was quenched by adding saturated aqueous sodium sulfite solution (80 mL), and extracted with ethyl acetate (80 mL × 2). The organic phase was combined, dried over anhydrous sodium sulfate and filtered, and the solvent was removed from the filtrate under reduced pressure. The resulting residue was purified by preparative thin layer chromatography (eluent: petroleum ether/ethyl acetate = 3/1, volume ratio) to obtain the intermediate **WX004-3.** MS-ESI m/z: 259.0 [M-H]⁻. ¹H NMR (400 MHz, CDCl₃) δ: 7.65 (d, *J=* 2.0 Hz, 1H), 7.36 (d, *J=* 8.8 Hz, 1H), 6.98 (d, *J=* 8.8 Hz, 1H), 6.61 (dd, *J=* 0.8, 2.0 Hz, 1H), 5.20 (s, 1H).

### Step 3: Synthesis of intermediate WX004-4

At room temperature, the intermediate **WX004-3** (0.2 g, 769.15 µmol, purity: 88%) was dissolved in acetonitrile (4 mL), and then potassium carbonate (159.46 mg, 1.15 mmol) and ethyl 4-bromocrotonate (178.17 mg, 922.98 µmol) were added successively, and the reaction mixture was stirred and reacted at room temperature for 12 hours. After completion of the reaction, water (50 mL) was add and extraction with ethyl acetate (80 mL × 2) was performed. The organic phase was combined, dried over anhydrous sodium sulfate and filtered, and the solvent was removed from the filtrate under reduced pressure. The resulting residue was purified by preparative thin layer chromatography (eluent: petroleum ether/ethyl acetate = 3/1, volume ratio) to obtain the intermediate **WX004-4.** MS-ESI m/z: 373.0 [M+H]⁺. ¹H NMR (400 MHz, CDCl₃) δ: 7.67 (d, *J=* 2.4 Hz, 1H), 7.40 (d, *J=* 8.8 Hz, 1H), 7.10 (td, *J=* 4.0, 15.6 Hz, 1H), 6.82 (d, *J=* 9.2 Hz, 1H), 6.70 (dd, *J =* 0.8, 2.0 Hz, 1H), 6.39 (td, *J =* 2.0, 15.6 Hz, 1H), 4.78 (dd, *J* = 2.2, 3.8 Hz, 2H), 4.24 (q, *J* = 7.2 Hz, 2H), 1.32 (t, *J* = 7.0 Hz, 3H).

### Step 4: Synthesis of intermediate WX004-5

At room temperature and under nitrogen atmosphere, the intermediate **WX004-4** (0.16 g, 429.93 µmol, purity: 82%) was dissolved in N,N-dimethylformamide (10 mL), and then palladium acetate (9.65 mg, 42.99 µmol), sodium carbonate (113.92 mg, 1.07 mmol), tetrabutylammonium chloride (131.43 mg, 472.92 µmol) and sodium formate (29.24 mg, 429.93 µmol) were added successively, and the reaction mixture was heated to 80 °C and stirred and reacted at 80 °C for 12 hours. After completion of the reaction, the reaction was quenched by adding water (50 mL), and extracted with ethyl acetate (80 mL × 2). The organic phase was combined, washed with half-saturated brine (50 mL × 3), dried over anhydrous sodium sulfate and filtered, and the solvent was removed from the filtrate under reduced pressure. The resulting residue was purified by preparative thin layer chromatography (eluent: petroleum ether/ethyl acetate = 3/1, volume ratio) to obtain the intermediate **WX004-5.** MS-ESI m/z: 245.1 [M+H]⁺. ¹H NMR (400 MHz, CDCl₃) δ: 7.71 (d, *J* = 2.0 Hz, 1H), 7.70 (s, 1H), 7.42 (q, *J =* 9.0 Hz, 2H), 7.03 (d, *J =* 2.4 Hz, 1H), 4.19 (q, *J=* 7.0 Hz, 2H), 3.85 (s, 2H), 1.24 (t, *J =* 7.0 Hz, 3H).

### Step 5: Synthesis of compound WX004

At room temperature, the intermediate **WX004-5** (0.08 g, 327.54 µmol) was dissolved in tetrahydrofuran (5 mL), and then acrylamide (23.28 mg, 327.54 µmol) and potassium *tert-*butoxide (36.75 mg, 327.54 µmol) were added successively, and the reaction mixture was stirred and reacted at room temperature for 1 hour. After completion of the reaction, the reaction was quenched by adding water (20 mL), and extracted with ethyl acetate (80 mL × 2). The organic phase was combined, dried over anhydrous sodium sulfate and filtered, and the solvent was removed from the filtrate under reduced pressure. The resulting residue was purified by preparative HPLC (mobile phase: acetonitrile/water; acid system: 0.05% HCl) to obtain the compound **WX004.** MS-ESI *m*/*z:* 270.0 [M+H]⁺. ¹H NMR (400 MHz, DMSO_*d*₆) δ: 10.99 (s, 1H), 8.10 (d, *J =* 2.4 Hz, 1H), 7.99 (s, 1H), 7.56 (q, *J =* 9.0 Hz, 2H), 7.07 (d, *J* = 1.6 Hz, 1H), 4.35 (dd, *J=* 5.0, 12.6 Hz, 1H), 2.90-2.78 (m, 1H), 2.72-2.58 (m, 1H), 2.38-2.24 (m, 1H), 2.22-2.12 (m, 1H).

### Example 5: WX005

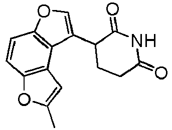

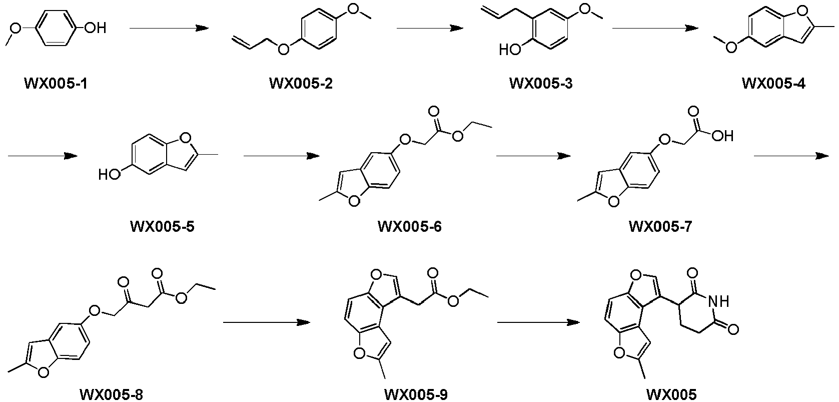

### Step 1: Synthesis of intermediate WX005-2

At room temperature and under nitrogen atmosphere, the compound **WX005-1** (30.00 g, 241.67 mmol) and 3-bromoprop-1-ene (35.08 g, 290.00 mmol) were dissolved in acetone (300 mL), potassium carbonate (66.80 g, 483.34 mmol) was added, and the reaction mixture was heated to 65 °C and stirred and reacted for 12 hours. After completion of the reaction, the reaction mixture was cooled to room temperature and filtered, and the filter cake was discarded, the solvent was removed from the filtrate under reduced pressure. The resulting residue was purified by column chromatography (eluent: petroleum ether/ethyl acetate = 1/0-100/1, volume ratio) to obtain the intermediate **WX005-2.** MS-ESI *m*/*z:* 165.0 [M+H]⁺. ¹H NMR (400 MHz, CDCl₃) δ: 6.92-6.78 (m, 4H), 6.13-6.00 (m, 1H), 5.41 (dq, *J =* 1.6, 17.2 Hz, 1H), 5.28 (dq, *J =* 1.4, 10.2 Hz, 1H), 4.51 (t, *J =* 1.6 Hz, 1H), 4.50 (t, *J =* 1.4 Hz, 1H), 3.78 (s, 3H).

### Step 2: Synthesis of intermediate WX005-3

At room temperature and under nitrogen atmosphere, the intermediate **WX005-2** (33.00 g, 196.63 mmol, purity: 97.84%) was added to a single-necked flask, and the reaction mixture was heated to 180 °C and stirred and reacted for 6 hours. After completion of the reaction, the reaction mixture was cooled to room temperature. The resulting residue was purified by column chromatography (eluent: petroleum ether/ethyl acetate = 100/1-10/1, volume ratio) to obtain the intermediate **WX005-3.** MS-ESI *m*/*z:* 165.0 [M+H]⁺. ¹H NMR (400 MHz, CDCl₃) δ: 6.79-6.73 (m, 1H), 6.72-6.64 (m, 2H), 6.11-5.92 (m, 1H), 5.23-5.17 (m, 1H), 5.16-5.13 (m, 1H), 4.63 (s, 1H), 3.77 (s, 3H), 3.39 (d, *J=* 6.0 Hz, 2H).

### Step 3: Synthesis of intermediate WX005-4

At room temperature, the intermediate **WX005-3** (5.00 g, 28.86 mmol, purity: 94.77%) was dissolved in dimethylacetamide (3 mL) and water (0.5 mL). Palladium chloride (102.35 mg, 577.16 µmol) and sodium acetate (4.73 g, 57.72 mmol) were added. The reaction mixture was evacuated and ventilated with oxygen several times. The reaction mixture was stirred and reacted for 1 hour at 25 °C and under the protection of oxygen (15 psi). Three batches were combined for treatment. After completion of the reaction, the reaction mixture was added with water (200 mL) and extracted with ethyl acetate (100 mL × 3). The organic phase was combined, washed with saturated brine (100 mL × 2), dried over anhydrous sodium sulfate and filtered, and the solvent was removed from the filtrate under reduced pressure. The resulting residue was purified by column chromatography (eluent: petroleum ether/ethyl acetate = 20/1-10/1, volume ratio) to obtain the intermediate **WX005-4.** MS-ESI *m*/*z:* 163.0 [M+H]⁺. ¹H NMR (400 MHz, CDCl₃) δ: 7.29 (d, *J=* 9.2 Hz, 1H), 6.96 (d, *J=* 2.8 Hz, 1H), 6.81 (dd, *J=* 2.6, 9.0 Hz, 1H), 6.32 (s, 1H), 3.84 (s, 3H), 2.44 (d, *J=* 0.8 Hz, 3H).

### Step 4: Synthesis of intermediate WX005-5

At -78 °C and under nitrogen atmosphere, the intermediate **WX005-4** (4.22 g, 25.42 mmol, purity: 97.69%) was dissolved in dichloromethane (40 mL), and the solution of boron tribromide (19.10 g, 76.26 mmol, 7.35 mL) in dichloromethane (10 mL) was slowly added, and the reaction mixture was warmed to 25 °C and stirred and reacted for 5 hours. After completion of the reaction, the reaction mixture was poured into water (100 mL) and extracted with dichloromethane (50 mL × 3). The organic phase was combined, washed with saturated brine (50 mL × 2), dried over anhydrous sodium sulfate and filtered, and the solvent was removed from the filtrate under reduced pressure. The resulting residue was purified by column chromatography (eluent: petroleum ether/ethyl acetate = 20/1-8/1, volume ratio) to obtain the intermediate **WX005-5.** MS-ESI *m*/*z:* 148.9 [M+H]⁺. ¹H NMR (400 MHz, CDCl₃) δ: 7.24 (d, *J =* 8.4 Hz, 1H), 6.89 (d, *J =* 2.4 Hz, 1H), 6.71 (dd, *J =* 2.4, 8.8 Hz, 1H), 6.28 (s, 1H), 4.80 (s, 1H), 2.43 (d, *J=* 0.8 Hz, 3H).

### Step 5: Synthesis of intermediate WX005-6

At room temperature and under nitrogen atmosphere, the intermediate **WX005-5** (3.08 g, 20.48 mmol, purity: 98.53%) was dissolved in N,N-dimethylformamide (30 mL), potassium carbonate (5.66 g, 40.97 mmol) was added, the reaction mixture was stirred at 0 °C for 0.5 hours, and ethyl 2-bromoacetate (3.42 g, 20.48 mmol, 2.27 mL) was added, the reaction mixture was stirred and reacted for 12 hours at 25 °C under nitrogen atmosphere. After completion of the reaction, the reaction mixture was added with water (100 mL) and extracted with ethyl acetate (50 mL × 3). The organic phase was combined, washed with saturated brine (50 mL × 2), dried over anhydrous sodium sulfate and filtered, and the solvent was removed from the filtrate under reduced pressure. The resulting residue was purified by column chromatography (eluent: petroleum ether/ethyl acetate = 20/1-10/1, volume ratio) to obtain the intermediate **WX005-6.** MS-ESI *m*/*z*: 235.1 [M+H]⁺. ¹H NMR (400 MHz, CDCl₃) δ: 7.30 (d, *J =* 9.2 Hz, 1H), 6.95 (d, *J =* 2.4 Hz, 1H), 6.86 (dd, *J =* 2.4, 8.8 Hz, 1H), 6.31 (s, 1H), 4.64 (s, 2H), 4.28 (q, *J=* 7.0 Hz, 2H), 2.43 (d, *J=* 0.8 Hz, 3H), 1.31 (t, *J =* 7.2 Hz, 3H).

### Step 6: Synthesis of intermediate WX005-7

At room temperature and under nitrogen atmosphere, the intermediate **WX005-6** (2.10 g, 8.67 mmol, purity: 96.74%) was dissolved in tetrahydrofuran (20 mL), ethanol (10 mL) and water (5 mL), sodium hydroxide (346.91 mg, 8.67 mmol) was added, and the reaction mixture was stirred and reacted at 25 °C for 12 hours. Tetrahydrofuran and ethanol were removed from the reaction mixture under reduced pressure, and water (100 mL) was added to the reaction mixture. Then 2 M dilute hydrochloric acid (10 mL) was added to adjust the pH to 2-3, and extraction with ethyl acetate (50 mL × 3) was performed. The organic phase was combined, washed with saturated brine (50 mL × 2), dried over anhydrous sodium sulfate and filtered, and the solvent was removed from the filtrate under reduced pressure to obtain the intermediate **WX005-7.** MS-ESI *m*/*z:* 207.1 [M+H]⁺. ¹H NMR (400 MHz, CDCl₃) δ: 7.32 (d, *J =* 8.8 Hz, 1H), 6.98 (d, *J =* 2.8 Hz, 1H), 6.86 (dd, *J =* 2.6, 9.0 Hz, 1H), 6.33 (s, 1H), 4.70 (s, 2H), 2.44 (d, *J =* 0.8 Hz, 3H).

### Step 7: Synthesis of intermediate WX005-8

At 10 °C and under nitrogen atmosphere, monoethyl malonate potassium salt (3.29 g, 19.31 mmol) was dissolved in acetonitrile (20 mL), and the mixture of triethylamine (3.14 g, 31.06 mmol, 4.32 mL) and magnesium chloride (2.16 g, 22.66 mmol) was added to the above reaction solution, and the reaction mixture was warmed to 25 °C and stirred and reacted for 2 hours. At 0 °C and under nitrogen atmosphere, the intermediate **WX005-7** (1.77 g, 8.39 mmol, purity: 97.79%) was dissolved in acetonitrile (10 mL), N,N'-carbonyldiimidazole (1.36 g, 8.39 mmol) and triethylamine (849.43 mg, 8.39 mmol, 1.17 mL) were added, and the reaction mixture was warmed to 25 °C and stirred and reacted for 2 hours. At 0 °C and under nitrogen atmosphere, the reaction mixture was added dropwise to the above solution, and the reaction mixture was warmed to 25 °C and stirred and reacted for 10 hours. After completion of the reaction, the reaction mixture was added to ice water (100 mL), and extracted with ethyl acetate (60 mL × 3). The organic phase was combined, washed with saturated brine (50 mL × 2), dried over anhydrous sodium sulfate and filtered, and the solvent was removed from the filtrate under reduced pressure. The resulting residue was purified by column chromatography (eluent: petroleum ether/ethyl acetate = 10/1-5/1, volume ratio) to obtain the intermediate WX005-8. MS-ESI *m*/*z*: 277.0 [M+H]⁺. ¹H NMR (400 MHz, CDCl₃) δ: 7.31 (d, *J* = 8.8 Hz, 1H), 6.91 (d, *J=* 2.8 Hz, 1H), 6.81 (dd, *J=* 2.8, 8.8 Hz, 1H), 6.32 (t, *J=* 0.8 Hz, 1H), 4.66 (s, 2H), 4.20 (q, *J=* 7.0 Hz, 2H), 3.66 (s, 2H), 2.44 (d, *J =* 1.2 Hz, 3H), 1.26 (t, *J=* 7.2 Hz, 3H).

### Step 8: Synthesis of intermediate WX005-9

At room temperature and under nitrogen atmosphere, the intermediate **WX005-8** (0.821 g, 2.44 mmol, purity: 82.04%) was dissolved in toluene (10 mL), polyphosphoric acid (0.300 g) was added, and the reaction mixture was heated to 110 °C and stirred and reacted for 1 hour. After completion of the reaction, the reaction mixture was cooled to room temperature, added with water (30 mL), and extracted with ethyl acetate (20 mL × 3). The organic phase was combined, washed with saturated brine (10 mL × 2), dried over anhydrous sodium sulfate and filtered, and the solvent was removed from the filtrate under reduced pressure. The resulting residue was subjected to supercritical fluid chromatography (separation conditions: column: ChiralPakAD-3 150 × 4.6 mm I.D., 3 µm; mobile phase: A: carbon dioxide, B: ethanol (0.05% diethylamine); flow rate: 2.5 mL/min; column temperature: 40 °C; wavelength: 220 nm) separation, the sample with retention time of 2.087 min was collected to obtain the intermediate **WX005-9.** MS-ESI *m*/*z:* 259.1 [M+H]⁺. ¹H NMR (400 MHz, CDCl₃) δ: 7.68 (s, 1H), 7.34 (q, *J =* 9.0 Hz, 2H), 6.66 (s, 1H), 4.21 (q, *J =* 7.0 Hz, 2H), 3.83 (d, *J =* 0.8 Hz, 2H), 2.52 (d, *J=* 0.8 Hz, 3H), 1.27 (t, *J=* 7.2 Hz, 3H).

### Step 9: Synthesis of WX005

At 0 °C and under nitrogen atmosphere, potassium tert-butoxide (83.66 mg, 745.58 µmol) was added to the solution of intermediate **WX005-9** (0.200 g, 745.58 µmol, purity: 96.28%) in N,N-dimethylformamide (10 mL), followed by acrylamide (52.99 mg, 745.58 µmol), and the reaction mixture was stirred and reacted for 1 hour at 0 °C under nitrogen atmosphere. After completion of the reaction, the reaction mixture was diluted by adding water (50 mL) and extracted with ethyl acetate (30 mL × 3). The organic phase was combined, washed with saturated brine (30 mL × 2), dried over anhydrous sodium sulfate and filtered, and the solvent was removed from the filtrate under reduced pressure. The resulting residue was subjected to preparative HPLC (mobile phase: acetonitrile/water; acidic system: 0.05% HCl) to obtain the target compound **WX005.** MS-ESI *m*/*z:* 284.1 [M+H]⁺. ¹H NMR (400 MHz, DMSO_*d*₆) δ: 10.95 (s, 1H), 7.94 (s, 1H), 7.47 (d, *J =* 9.2 Hz, 1H), 7.43 (d, *J=* 8.8 Hz, 1H), 6.69 (s, 1H), 4.29 (dd, *J =* 4.8, 12.4 Hz, 1H), 2.89-2.77 (m, 1H), 2.69-2.58 (m, 1H), 2.49 (s, 3H), 2.36-2.23 (m, 1H), 2.20-2.09 (m, 1H).

### Example 6: WX006

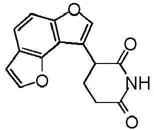

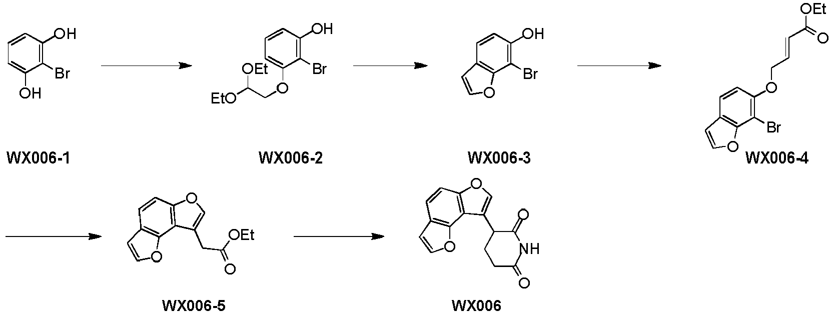

### Step 1: Synthesis of intermediate WX006-2

At room temperature and under nitrogen atmosphere, the compound **WX006-1** (4.48 g, 23.70 mmol) was dissolved in N,N-dimethylformamide (50 mL), sodium hydride (948.12 mg, 23.70 mmol, purity: 60%) was added, then bromoacetaldehyde diethyl acetal (4.67 g, 23.70 mmol, 3.57 mL) was added. The reaction mixture was heated to 100 °C and stirred and reacted for 16 hours. After completion of the reaction, the reaction solution was cooled to room temperature, poured into ice water (50 mL), and extracted with ethyl acetate (50 mL × 3). The organic phase was combined, washed with brine (100 mL × 2), dried over anhydrous sodium sulfate and filtered, and the solvent was removed from the filtrate under reduced pressure. The resulting residue was purified by column chromatography (eluent: petroleum ether/ethyl acetate = 20/1-10/1, volume ratio) to obtain the intermediate **WX006-2.** ¹H NMR (400 MHz, CDCl₃) δ: 7.15 (t, *J* = 8.4 Hz, 1H), 6.69 (dd, *J* = 1.2, 8.4 Hz, 1H), 6.48 (dd, *J* = 1.0, 8.2 Hz, 1H), 5.65 (s, 1H), 4.89 (t, *J=* 5.2 Hz, 1H), 4.05 (d, *J=* 5.2 Hz, 2H), 3.86-3.77 (m, 2H), 3.75-3.66 (m, 2H), 1.27 (t, *J=* 7.0 Hz, 6H).

### Step 2: Synthesis of intermediate WX006-3

At room temperature and under nitrogen atmosphere, the intermediate **WX006-2** (2.22 g, 7.27 mmol) was dissolved in toluene (10 mL), and then polyphosphoric acid (1.00 g) was added. The reaction mixture was heated to 120 °C and stirred and reacted for 0.4 hours. After completion of the reaction, the reaction solution was cooled to room temperature, added with water (40 mL), and extracted with ethyl acetate (30 mL × 3). The organic phase was combined, washed with brine (60 mL × 2), dried over anhydrous sodium sulfate and filtered, and the solvent was removed from the filtrate under reduced pressure. The resulting residue was purified by column chromatography (eluent: petroleum ether/ethyl acetate = 20/1-10/1, volume ratio) to obtain the intermediate **WX006-3.** ¹H NMR (400 MHz, CDCl₃) δ: 7.61 (d, *J =* 2.0 Hz, 1H), 7.41 (d, *J =* 8.4 Hz, 1H), 6.99 (d, *J =* 8.4 Hz, 1H), 6.78 (d, *J =* 2.0 Hz, 1H), 5.49 (s, 1H).

### Step 3: Synthesis of intermediate WX006-4

At room temperature and under nitrogen atmosphere, the intermediate **WX006-3** (739.00 mg, 3.47 mmol) was dissolved in acetonitrile (10 mL), then potassium carbonate (958.91 mg, 6.94 mmol) and ethyl 4-bromocrotonate (1.34 g, 6.94 mmol, 956.65 µL) were added, and the reaction mixture was stirred at room temperature for 16 hours. After completion of the reaction, the reaction mixture was added with water (30 mL) and extracted with ethyl acetate (30 mL × 3). The organic phase was combined, washed with saturated brine (60 mL × 2), dried over anhydrous sodium sulfate and filtered, and the solvent was removed from the filtrate under reduced pressure. The resulting residue was purified by column chromatography (eluent: petroleum ether/ethyl acetate = 100/1-50/1, volume ratio) to obtain the intermediate **WX006-4.** ¹H NMR (400 MHz, CDCl₃) δ: 7.65 (d, *J=* 2.0 Hz, 1H), 7.46 (d, *J =* 8.8 Hz, 1H), 7.12 (dt, *J =* 3.9, 15.6 Hz, 1H), 6.88 (d, *J =* 8.8 Hz, 1H), 6.79 (d, *J =* 2.4 Hz, 1H), 6.35 (dt, *J =* 2.0, 15.6 Hz, 1H), 4.83 (dd, *J =* 2.2, 3.8 Hz, 2H), 4.24 (q, *J =* 7.2 Hz, 2H), 1.32 (t, *J* = 7.0 Hz, 3H).

### Step 4: Synthesis of intermediate WX006-5

At room temperature and under nitrogen atmosphere, the intermediate **WX006-4** (940.00 mg, 2.89 mmol) was dissolved in N,N-dimethylformamide (10 mL), then sodium carbonate (766.03 mg, 7.23 mmol), sodium formate (196.61 mg, 2.89 mmol), palladium acetate (32.45 mg, 144.55 µmol) and tetrabutylammonium chloride (883.78 mg, 3.18 mmol) were added successively, and the reaction mixture was heated to 80 °C and stirred and reacted for 14 hours. After completion of the reaction, the reaction solution was cooled to room temperature, added with water (30 mL), and extracted with ethyl acetate (30 mL × 3). The organic phase was combined, washed with saturated brine (60 mL × 2), dried over anhydrous sodium sulfate and filtered, and the solvent was removed from the filtrate under reduced pressure. The resulting residue was purified by column chromatography (eluent: petroleum ether/ethyl acetate = 100/1-50/1, volume ratio) to obtain the intermediate **WX006-5.** MS-ESI m/z: 245.0 [M+H]⁺.

### Step 5: Synthesis of compound WX006

At 0 °C and under the nitrogen atmosphere, the intermediate **WX006-5** (43.00 mg, 166.97 µmol, purity: 94.84%) and the intermediate **WX006-5** (20.75 mg, 69.87 µmol, purity: 82.23%) were added to N,N-dimethylformamide (5 mL), then potassium tert-butoxide (26.58 mg, 236.84 µmol) was added. After stirring at 0 °C for 0.5 hours, acrylamide (16.83 mg, 236.84 µmol) was added to the above reaction solution, and the reaction mixture was stirred and reacted for additional 1 hour at 0 °C under nitrogen atmosphere. After completion of the reaction, the reaction mixture was added with water (30 mL) and extracted with ethyl acetate (30 mL × 3). The organic phase was combined, washed with saturated brine (60 mL × 2), dried over anhydrous sodium sulfate and filtered, and the solvent was removed from the filtrate under reduced pressure. The resulting residue was purified by preparative HPLC (mobile phase: acetonitrile/water; acidic system: 0.05% HCl) to obtain the target compound **WX006.** MS-ESI m/z: 270.1 [M+H]⁺. ¹H NMR (400 MHz, DMSO_*d*₆) δ: 10.98 (s, 1H), 8.02 (d, *J =* 2.0 Hz, 1H), 7.99 (s, 1H), 7.61-7.57 (m, 1H), 7.55-7.51 (m, 1H), 7.05 (d, *J* = 2.0 Hz, 1H), 4.25 (dd, *J* = 4.8, 12.8 Hz, 1H), 2.89-2.80 (m, 1H), 2.68-2.60 (m, 1H), 2.45-2.31 (m, 1H), 2.18-2.09 (m, 1H).

### Example 7: WX007

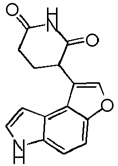

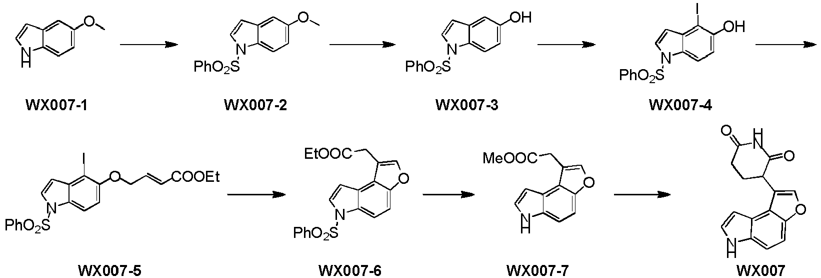

### Step 1: Synthesis of intermediate WX007-2

At room temperature and under nitrogen atmosphere, **WX007-1** (20 g, 135.89 mmol) was dissolved in tetrahydrofuran (300 mL), and the temperature was reduced to 0 °C. Sodium hydride (6.79 g, 169.87 mmol, purity: 60%) was added in batches, and the reaction mixture was warmed to 15 °C and stirred and reacted for 1 hour, then reduced to 5 °C. Benzenesulfonyl chloride (30.00 g, 169.87 mmol, 21.74 mL) was slowly added dropwise, and the reaction mixture was returned to 15 °C and stirred and reacted for 1 hour. After completion of the reaction, the reaction solution was poured into saturated ammonium chloride solution (200 mL), and extracted with ethyl acetate (100 mL × 3). The organic phase was combined, dried over anhydrous sodium sulfate, filtered, and concentrated under reduced pressure to remove the solvent. The resulting residue was stirred with 50 mL of methanol at room temperature for 30 minutes and then filtered. The solid was collected and concentrated under reduced pressure to obtain the intermediate **WX007-2.** ¹H NMR (400 MHz, CDCl₃) δ: 7.90 (d, *J =* 8.8 Hz, 1H), 7.87-7.83 (m, 2H), 7.58-7.49 (m, 2H), 7.47-7.38 (m, 2H), 6.98 (d, *J* = 2.4 Hz, 1H), 6.94 (dd, *J* = 2.6, 9.0 Hz, 1H), 6.60 (d, *J =* 3.6 Hz, 1H), 3.82 (s, 3H).

### Step 2: Synthesis of intermediate WX007-3

At room temperature, the intermediate **WX007-2** (5 g, 17.40 mmol) was dissolved in dichloromethane (100 mL), and the temperature was reduced to -30 °C. The solution of boron tribromide (5.67 g, 22.62 mmol, 2.18 mL) in dichloromethane (20 mL) was added dropwise, and the reaction mixture was returned to 15 °C and stirred and reacted for 3 hours. After completion of the reaction, the reaction solution was poured into 300 mL ice water, and extracted with dichloromethane (200 mL × 3). The organic phase was combined, dried over anhydrous sodium sulfate, filtered, and concentrated under reduced pressure to remove the solvent. The resulting residue was purified by column chromatography (eluent: petroleum ether/ethyl acetate = 1/0-7/3, volume ratio) to obtain the intermediate **WX007-3.** ¹H NMR (400 MHz, CDCl₃) δ: 7.90-7.79 (m, 3H), 7.58-7.49 (m, 2H), 7.43 (t, *J =* 7.6 Hz, 2H), 6.93 (d, *J =* 2.4 Hz, 1H), 6.85 (dd, *J =* 2.4, 8.8 Hz, 1H), 6.56 (d, *J =* 3.6 Hz, 1H), 4.81 (s, 1H).

### Step 3: Synthesis of intermediate WX007-4

At 0 °C and under nitrogen atmosphere, the intermediate **WX007-3** (3.2 g, 11.71 mmol) was dissolved in toluene (150 mL), and tert-butylamine (85.64 mg, 1.17 mmol, 123.04 µL) and N-iodosuccinimide (2.63 g, 11.71 mmol) were added successively under the protection from light and the reaction mixture was stirred for 1 hour at 0 °C. After completion of the reaction, the reaction solution was added with water (200 mL), and extracted with ethyl acetate (200 mL × 3). The organic phase was combined, dried over anhydrous sodium sulfate, filtered, and concentrated under reduced pressure to remove the solvent. The resulting residue was purified by column chromatography (eluent: petroleum ether/ethyl acetate = 1/0-5/1, volume ratio) to obtain the intermediate **WX007-4.**

### Step 4: Synthesis of intermediate WX007-5

At room temperature, the intermediate **WX007-4** (2.5 g, 6.26 mmol) was dissolved in acetonitrile (40 mL), then potassium carbonate (2.60 g, 18.79 mmol) and ethyl 4-bromocrotonate (3.45 g, 12.52 mmol, 2.47 mL) were added, and the reaction mixture was heated to 35 °C and stirred and reacted for 18 hours. After completion of the reaction, the reaction solution was directly filtered, the filter cake was washed with ethyl acetate (20 mL × 2), the mother liquor was collected, concentrated under reduced pressure to remove the solvent. The resulting residue was purified by column chromatography (eluent: petroleum ether/ethyl acetate = 1/0-5/1, volume ratio) to obtain the intermediate **WX007-5.** ¹H NMR (400 MHz, CDCl₃) δ: 7.88 (d, *J=* 8.8 Hz, 1H), 7.85-7.80 (m, 2H), 7.60 (d, *J=* 3.6 Hz, 1H), 7.57-7.50 (m, 1H), 7.47-7.38 (m, 2H), 7.06 (td, *J =* 4.0, 15.6 Hz, 1H), 6.81 (d, *J =* 9.2 Hz, 1H), 6.61 (d, *J =* 4.0 Hz, 1H), 6.34 (td, *J =* 2.4, 15.8 Hz, 1H), 4.73 (dd, *J =* 2.0, 4.0 Hz, 2H), 4.20 (q, *J =* 6.8 Hz, 2H), 1.29 (t, *J*= 7.2 Hz, 3H).

### Step 5: Synthesis of intermediate WX007-6

At room temperature and under nitrogen atmosphere, the intermediate **WX007-5** (2.1 g, 4.11 mmol) was dissolved in N,N-dimethylformamide (35 mL), and then sodium carbonate (1.09 g, 10.27 mmol), sodium formate (279.31 mg, 4.11 mmol), palladium acetate (46.10 mg, 205.35 µmol) and tetrabutylammonium chloride (1.26 g, 4.52 mmol) were added successively, and the reaction mixture was warmed to 80 °C and stirred and reacted for 8 hours. After completion of the reaction, the reaction mixture was cooled to room temperature, added with water (100 mL), and extracted with ethyl acetate (50 mL × 3). The organic phase was combined, washed with half-saturated brine (50 mL × 2), dried over anhydrous sodium sulfate, filtered, and concentrated under reduced pressure to remove the solvent. The resulting residue was purified by column chromatography (eluent: petroleum ether/ethyl acetate = 1/0-5/1, volume ratio) to obtain the intermediate **WX007-6.** ¹H NMR (400 MHz, CDCl₃) δ: 7.98 (d, *J=* 9.2 Hz, 1H), 7.90-7.86 (m, 2H), 7.71-7.67 (m, 2H), 7.56-7.50 (m, 1H), 7.48-7.39 (m, 3H), 6.96 (d, *J=* 4.0 Hz, 1H), 4.18 (q, *J=* 7.2 Hz, 2H), 3.83 (d, *J=* 0.8 Hz, 2H), 1.22 (t, *J=* 7.2 Hz, 3H).

### Step 6: Synthesis of intermediate WX007-7

At room temperature, the intermediate **WX007-6** (1.2 g, 3.13 mmol) was dissolved in a mixed solution of tetrahydrofuran (20 mL) and methanol (100 mL), and then ammonium chloride (58.60 mg, 1.10 mmol) and magnesium chips (2.66 g, 109.54 mmol) were added, and the reaction mixture was heated to 80 °C and stirred and reacted for 3 hours. After completion of the reaction, the reaction mixture was cooled to room temperature, and concentrated under reduced pressure to remove the solvent, and the resulting residue was diluted with saturated aqueous ammonium chloride (100 mL) and ethyl acetate (100 mL). The organic phase was collected after separation, and the aqueous phase was extracted with ethyl acetate (50 mL × 2). The organic phase was combined, dried over anhydrous sodium sulfate and filtered, and concentrated under reduced pressure to obtain a residue. The resulting residue was purified by column chromatography (eluent: petroleum ether/ethyl acetate = 1/0-5/1, volume ratio) to obtain the intermediate **WX007-7.** ¹H NMR (400 MHz, CDCl₃) δ: 8.37 (s, 1H), 7.68 (s, 1H), 7.35 (d, *J=* 8.8 Hz, 1H), 7.30 (d, *J=* 9.2 Hz, 1H), 7.26 (d, *J =* 3.2 Hz, 1H), 6.80-6.77 (m, 1H), 3.95 (s, 2H), 3.74 (s, 3H).

### Step 7: Synthesis of compound WX007

At room temperature, the intermediate **WX007-7** (210 mg, 916.11 µmol) was dissolved in N,N-dimethylformamide (10 mL), and then acrylamide (65.11 mg, 916.11 µmol) and potassium tert-butoxide (102.80 mg, 916.11 µmol) were added successively. The reaction mixture was stirred and reacted for 2 hours at 15 °C, additional potassium tert-butoxide (50 mg) was added, and the resulting reaction mixture was stirred and reacted for additional 1 hour. After completion of the reaction, the reaction solution was added with water (20 mL), and extracted with ethyl acetate (20 mL × 3). The organic phase was combined, dried over anhydrous sodium sulfate, filtered, and concentrated under reduced pressure to remove the solvent. The resulting residue was purified by column chromatography (eluent: petroleum ether/ethyl acetate = 5/1-1/1, volume ratio), and the resulting crude product was purified by preparative HPLC (mobile phase: acetonitrile/water; acid system: 0.05% HCl) to obtain the target compound **WX007.** MS-ESI *m*/*z:* 269.0 [M+H]⁺. ¹H NMR (400 MHz, DMSO_*d*₆) δ: 11.30 (s, 1H), 10.97 (s, 1H), 7.81 (s, 1H), 7.39 (t, *J =* 2.8 Hz, 1H), 7.33 (q, *J =* 8.8 Hz, 2H), 6.48 (s, 1H), 4.29 (dd, *J=* 5.2, 12.0 Hz, 1H), 2.89-2.79 (m, 1H), 2.69-2.56 (m, 1H), 2.36-2.24 (m, 1H), 2.20-2.11 (m, 1H).

### Example 8: Hydrochloride of WX008

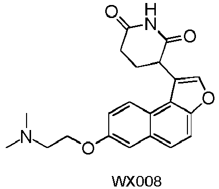

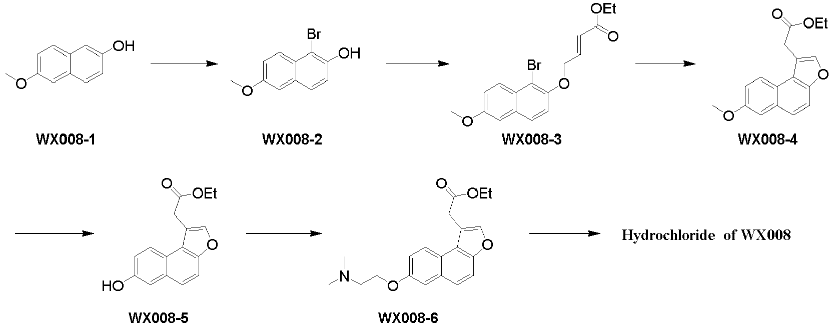

### Step 1: Synthesis of intermediate WX008-2

At room temperature and under nitrogen atmosphere, **WX008-1** (12.5 g, 71.76 mmol) was dissolved in N,N-dimethylformamide (125 mL), and then N-bromosuccinimide (13.28 g, 74.63 mmol) was added in batches. The reaction mixture was stirred and reacted at 15 °C for 0.5 hours. After completion of the reaction, the two batches were combined for treatment. The reaction solution was slowly poured into ice water (500 mL) and filtered, and the filter cake was concentrated under reduced pressure to remove the solvent to obtain the intermediate **WX008-2.** ¹H NMR (400 MHz, CDCl₃) δ: 8.04 (s, 1H), 7.95 (d, *J =* 8.8 Hz, 1H), 7.62 (d, *J* = 8.8 Hz, 1H), 7.27-7.23 (m, 1H), 7.22-7.20 (m, 1H), 7.10 (d, *J* = 2.8 Hz, 1H), 3.91 (s, 3H).

### Step 2: Synthesis of intermediate WX008-3

At room temperature and under nitrogen atmosphere, the intermediate **WX008-2** (15 g, 59.27 mmol) was dissolved in acetonitrile (350 mL), and then ethyl 4-bromocrotonate (16.78 g, 65.19 mmol, 11.99 mL) and potassium carbonate (16.38 g, 118.53 mmol) were added successively, and the reaction mixture was stirred and reacted at 15 °C for 12 hours. After completion of the reaction, the two batches were combined for treatment. The reaction solution was filtered, and the filtrate was concentrated under reduced pressure to remove the solvent. The resulting residue was purified by column chromatography (eluent: petroleum ether/ethyl acetate = 1/0-30/1, volume ratio) to obtain the intermediate **WX008-3.**

### Step 3: Synthesis of intermediate WX008-4

At room temperature and under nitrogen atmosphere, the intermediate **WX008-3** (3.6 g, 9.86 mmol) was dissolved in N,N-dimethylformamide (45 mL), and then palladium acetate (110.65 mg, 492.86 µmol), tetrabutylammonium chloride (3.01 g, 10.84 mmol), sodium formate (670.38 mg, 9.86 µmol) and sodium carbonate (2.61 g, 24.64 mmol) were added successively, and the reaction mixture was heated to 80 °C and stirred and reacted for 2 hours. After completion of the reaction, the reaction solution was cooled to room temperature, slowly poured into water (50 mL), and extracted with ethyl acetate (50 mL × 3). The organic phase was combined, dried over anhydrous sodium sulfate and filtered, and the filtrate was concentrated under reduced pressure to remove the solvent. The resulting residue was purified by column chromatography (eluent: petroleum ether/ethyl acetate = 1/0-30/1, volume ratio) to obtain the intermediate **WX008-4.**

### Step 4: Synthesis of intermediate WX008-5

At room temperature and under nitrogen atmosphere, the intermediate **WX008-4** (0.5 g, 1.76 mmol) was dissolved in dichloromethane (6 mL), and then boron tribromide (881.17 mg, 3.52 mmol, 338.91 µL) was added dropwise at -40 °C and the reaction mixture was returned to 20 °C and stirred and reacted for 1 hour. After completion of the reaction, the reaction solution was poured into ice water (50 mL), and extracted with ethyl acetate (50 mL×5). The organic phase was combined, dried over anhydrous sodium sulfate and filtered, and the filtrate was concentrated under reduced pressure to remove the solvent. The resulting residue was purified by column chromatography (eluent: petroleum ether/ethyl acetate = 20/1-15/1, volume ratio) to obtain the intermediate **WX008-5.** ¹H NMR (400 MHz, CDCl₃) δ: 8.14 (d, *J =* 9.2 Hz, 1H), 7.74 (s, 1H), 7.59 (q, *J =* 8.8 Hz, 2H), 7.31 (d, *J =* 2.8 Hz, 1H), 7.19 (dd, *J* = 2.6, 9.0 Hz, 1H), 5.22 (s, 1H), 4.22 (q, *J =* 7.2 Hz, 2H), 4.04 (s, 2H), 1.28 (t, *J =* 7.2 Hz, 3H).

### Step 5: Synthesis of intermediate WX008-6

At room temperature and under nitrogen atmosphere, the intermediate **WX008-5** (0.2 g, 739.98 µmol), 2-(dimethylamino) ethanol (131.92 mg, 1.48 mmol) and triphenylphosphine (388.17 mg, 1.48 mmol) were dissolved in tetrahydrofuran (6 mL), cooled down to 0 °C, and the solution of diisopropyl azodicarboxylate (299.26 mg, 1.48 mmol) in tetrahydrofuran (0.2 mL) was added dropwise, and the reaction mixture was heated to 50 °C and stirred and reacted for 6 hours. After completion of the reaction, the reaction mixture was cooled to room temperature, and concentrated under reduced pressure to remove the solvent. The resulting residue was purified by column chromatography (eluent: petroleum ether/ethyl acetate = 4/1-1/1, volume ratio) to obtain the intermediate **WX008-6.**

### Step 6: Synthesis of compound WX008

At room temperature, the intermediate **WX008-6** (130 mg, 380.79 µmol) was dissolved in tetrahydrofuran (2.5 mL), and then acrylamide (27.07 mg, 380.79 µmol) and the solution of potassium *tert*-butoxide (380.79 µL, 1 M) in tetrahydrofuran were added successively and the reaction mixture was stirred and reacted for 1 hour at 15 °C. After completion of the reaction, 2 M hydrochloric acid was added to adjust the pH = 5-6, the filtrate was concentrated under reduced pressure to remove the solvent, and the resulting residue was purified by preparative HPLC twice (mobile phase: acetonitrile/water; neutral system: 10 mM NH₄HCO₃) and (mobile phase: acetonitrile/water; acidic system: 0.05% HCl) to obtain the hydrochloride of the target compound **WX008.** MS-ESI *m*/*z:* 367.1 [M+H]⁺. ¹H NMR (400 MHz, MeOD_*d*₄) δ: 8.10 (d, *J =* 8.8 Hz, 1H), 7.81 (s, 1H), 7.74 (d, *J =* 9.2 Hz, 1H), 7.66 (d, *J* = 9.2 Hz, 1H), 7.53 (d, *J =* 2.4 Hz, 1H), 7.35 (dd, *J =* 2.4, 9.2 Hz, 1H), 4.58 (dd, *J =* 5.4, 10.6 Hz, 1H), 4.49 (t, *J =* 4.8 Hz, 2H), 3.67 (t, *J =* 4.8 Hz, 2H), 3.02 (s, 6H), 2.93-2.83 (m, 1H), 2.78-2.70 (m, 1H), 2.52-2.37 (m, 2H).

### Example 9: WX009

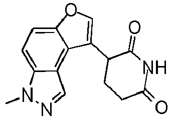

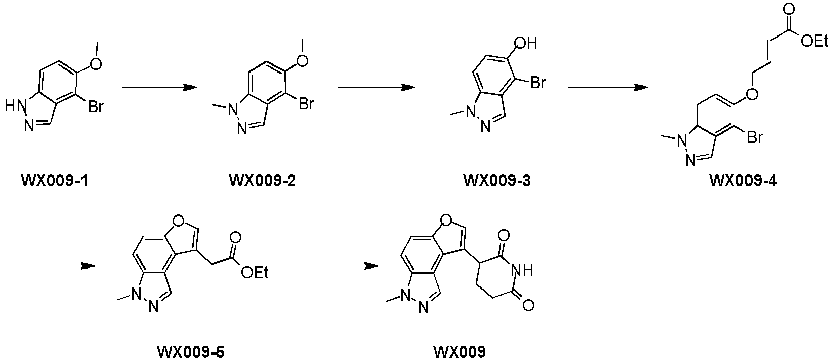

### Step 1: Synthesis of intermediate WX009-2

At room temperature and under nitrogen atmosphere, the compound **WX009-1** (2.5 g, 11.01 mmol) was dissolved in tetrahydrofuran (50 mL), and potassium tert-butoxide (1.85 g, 16.52 mmol) was added to the above solution in batches, and then methyl iodide (17.19 g, 121.11 mmol, 7.54 mL) was added dropwise to the above reaction solution, and the reaction mixture was stirred and reacted at 20 °C for 2 hours. After completion of the reaction, the reaction solution was added with ethyl acetate (100 mL) and deionized water (100 mL). The organic phase was separated, washed with saturated brine, dried over anhydrous sodium sulfate and filtered, and the solvent was removed from the filtrate under reduced pressure. The resulting residue was purified by column chromatography (eluent: petroleum ether/ethyl acetate = 5 : 1, volume ratio) to obtain the intermediate **WX009-2.** MS-ESI *m*/*z:* 240.8 [M+H]⁺, 242.8 [M+H+2]⁺. ¹H NMR (400 MHz, CDCl₃) δ: 7.94 (s, 1H), 7.31 (d*, J=* 8.8 Hz, 1H), 7.16 (d, *J=* 8.8 Hz, 1H), 4.06 (s, 3H), 3.96 (s, 3H).

### Step 2: Synthesis of intermediate WX009-3

At room temperature and under nitrogen atmosphere, the intermediate **WX009-2** (1.2 g, 4.98 mmol) was dissolved in dichloromethane (100 mL), and boron tribromide (3.74 g, 14.93 mmol, 1.44 mL) was added and the reaction mixture was stirred and reacted at 20 °C for 12 hours. After completion of the reaction, the reaction solution was added with water (20 mL), and extracted with dichloromethane (100 mL). The organic phase was combined, washed with saturated brine (50 mL), dried over anhydrous sodium sulfate and filtered, and the solvent was removed from the filtrate under reduced pressure to obtain intermediate **WX009-3.** MS-ESI *m*/*z:* 226.9 [M+H]⁺, 228.9 [M+H+2]⁺.

### Step 3: Synthesis of intermediate WX009-4

At room temperature and under nitrogen atmosphere, the intermediate **WX009-3** (0.15 g, 660.63 µmol), ethyl 4-bromocrotonate (191.29 mg, 990.94 µmol) and potassium carbonate (136.96 mg, 990.94 µmol) were added into N,N-dimethylformamide (20 mL), and the reaction mixture was stirred at room temperature for 12 hours. After completion of the reaction, the reaction solution was added with water (50 mL), and extracted with ethyl acetate (100 mL). The organic phase was separated, washed with saturated brine (20 mL × 2), dried over anhydrous sodium sulfate and filtered, and the solvent was removed from the filtrate under reduced pressure. The resulting residue was purified by column chromatography (eluent: petroleum ether/ethyl acetate = 1 : 1, volume ratio) to obtain intermediate **WX009-4.** ¹H NMR (400 MHz, CDCl₃) δ: 7.96 (s, 1H), 7.29 (d, *J =* 9.2 Hz, 1H), 7.14-7.09 (m, 2H), 6.38-6.28 (m, 1H), 4.83-4.76 (m, 2H), 4.24 (q, *J =* 14.4 Hz, 2H), 4.07 (s, 3H), 1.32 (t, *J =* 6.8 Hz, 3H).

### Step 4: Synthesis of intermediate WX009-5

At room temperature and under nitrogen atmosphere, the intermediate **WX009-4** (0.1 g, 294.83 µmol), palladium acetate (13.24 mg, 58.97 µmol), tetrabutylammonium chloride (98.32 mg, 353.79 µmol), sodium formate (40.10 mg, 589.65 µmol) and sodium carbonate (62.50 mg, 589.65 µmol) were dissolved in N,N-dimethylformamide (20 mL), and the reaction mixture was stirred and reacted at 80 °C for 2 hours. After completion of the reaction, the reaction solution was added with water (50 mL), and extracted with ethyl acetate (100 mL). The organic phase was separated, washed with saturated brine (20 mL), dried over anhydrous sodium sulfate and filtered, and the solvent was removed from the filtrate under reduced pressure. The resulting residue was purified by column chromatography (eluent: petroleum ether/ethyl acetate = 3 : 1, volume ratio) to obtain the intermediate **WX009-5.** ¹H NMR (400 MHz, CDCl₃) δ: 8.07 (s, 1H), 7.67 (s, 1H),7.46 (d, *J=* 9.6 Hz, 1H), 7.18 (d, *J=* 4.0 Hz, 1H), 4.12 (q, *J=* 14.0 Hz, 2H), 4.04 (s, 3H), 3.81 (d, *J=* 0.8 Hz, 2H), 1.18 (t, *J=* 7.2 Hz, 3H).

### Step 5: Synthesis of WX009

Under nitrogen atmosphere, the intermediate **WX009-5** (0.06 g, 232.31 µmol) was dissolved in N,N-dimethylformamide (10 mL), and potassium tert-butoxide (26.07 mg, 232.31 µmol) was added, and acrylamide (16.51 mg, 232.31 µmol) was added, and the reaction mixture was stirred and reacted at 0-5 °C for 1 hour. After completion of the reaction, the reaction solution was added with water (30 mL), and extracted with ethyl acetate (50 mL). The organic phase was separated, washed with saturated brine (20 mL × 2), dried over anhydrous sodium sulfate and filtered, and the solvent was removed from the filtrate under reduced pressure. The resulting residue was purified by preparative HPLC (mobile phase: acetonitrile/water; acidic system: 0.05% HCl) to obtain the target compound **WX009.** MS-ESI *m*/*z:* 284.0 [M+H]⁺. ¹HNMR (400 MHz, DMSO_*d*₆) δ: 10.98 (s,1H), 8.07 (s, 1H), 7.99 (s, 1H), 7.68 (d, *J =* 8.8 Hz, 1H), 7.57 (d, *J =* 9.2 Hz, 1H), 4.37 (dd, *J =* 4.6, 12.2 Hz, 1H), 4.11 (s, 3H), 2.85-2.75 (m, 1H), 2.65-2.50 (m, 1H), 2.35-2.31 (m, 1H), 2.24-2.15 (m, 1H).

### Example 10: WX010

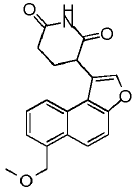

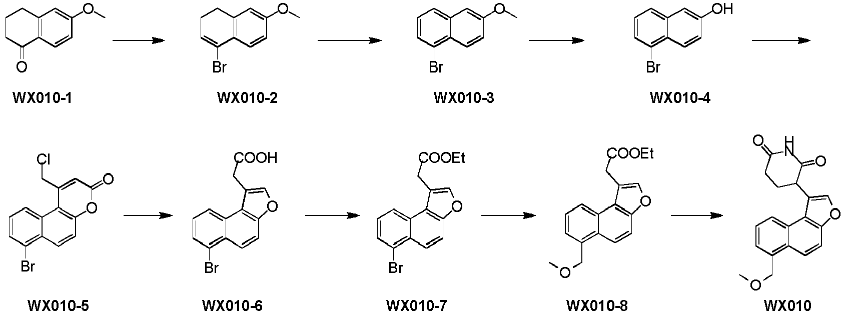

### Step 1: Synthesis of intermediate WX010-2

At room temperature and under nitrogen atmosphere, triphenyl phosphite (96.85 g, 312.13 mmol, 82.07 mL) was dissolved in dichloromethane (1000 mL), cooled to -78 °C, and then liquid bromine (54.41 g, 340.50 mmol, 17.55 mL) was added dropwise, and then triethylamine (368.88 mmol, 51.34 mL) was added dropwise. The reaction mixture was stirred and reacted for 30 minutes, then the compound **WX010-1** (50 g, 283.75 mmol) was added, and the reaction mixture was returned to room temperature and stirred and reacted for 12 hours. After completion of the reaction, the reaction solution was poured into saturated aqueous sodium sulfite solution (1500 mL), and extracted with dichloromethane (1000 mL × 3). The organic phase was combined, washed with saturated brine (1000 mL × 3) successively, dried over anhydrous sodium sulfate, filtered, and the filtrate was concentrated under reduced pressure to remove the solvent. The resulting residue was purified by column chromatography (eluent: petroleum ether/ethyl acetate = 1/0, volume ratio) to obtain intermediate **WX010-2.** ¹H NMR (400 MHz, CDCl₃) δ: 7.48 (d, *J=* 8.8 Hz, 1H), 6.75 (dd, *J* = 2.2, 8.6 Hz, 1H), 6.67 (d, *J =* 2.4 Hz, 1H), 6.30 (t, *J =* 4.8 Hz, 1H), 3.82 (s, 3H), 2.82 (t, *J =* 7.8 Hz, 2H), 2.41-2.28 (m, 2H).

### Step 2: Synthesis of intermediate WX010-3

At room temperature and under nitrogen atmosphere, the intermediate **WX010-2** (119.5 g, 499.77 mmol) was dissolved in toluene (5 mL), cooled to 0 °C, and then 2,3-dichloro-5,6-dicyano-*p*-benzoquinone (124.67 g, 549.19 mmol) was added, and the reaction mixture was stirred at room temperature for 12 hours. After completion of the reaction, the reaction solution was poured into a saturated aqueous sulfurous acid solution (1000 mL), and extracted with ethyl acetate (500 mL × 3). The organic phase was combined, washed with saturated aqueous sodium bicarbonate solution (500 mL × 3) and saturated brine (500 mL × 3), dried over anhydrous sodium sulfate and filtered, and the filtrate was concentrated under reduced pressure to remove the solvent. The resulting residue was purified by column chromatography (eluent: petroleum ether/ethyl acetate = 1/0, volume ratio) to obtain intermediate **WX010-3.** ¹H NMR (399 MHz, CDCl₃) δ: 8.13 (d, *J =* 9.2 Hz, 1H), 7.68 (d, *J* = 8.4 Hz, 1H), 7.60 (dd, *J =* 1.0, 7.4 Hz, 1H), 7.25 (d, *J =* 2.4 Hz, 1H), 7.23 (dd, *J =* 2.8, 6.4 Hz, 1H), 7.11 (d, *J* = 2.8 Hz, 1H), 3.92 (s, 3H).

### Step 3: Synthesis of intermediate WX010-4

At room temperature and under nitrogen atmosphere, the intermediate **WX010-3** (24 g, 101.23 mmol) was dissolved in dichloromethane (350 mL), cooled to 0 °C, and boron tribromide (30.43 g, 121.47 mmol, 11.70 mL) was added dropwise, and the reaction mixture was returned to room temperature and stirred and reacted for 3 hours. After completion of the reaction, the reaction solution was poured into ice water (1000 mL) for quenching, and extracted with dichloromethane (500 mL × 3). The organic phase was combined, washed with saturated brine (500 mL × 3) successively, dried over anhydrous sodium sulfate, filtered, and the filtrate was concentrated under reduced pressure to remove the solvent to obtain intermediate **WX010-4.** ¹H NMR (400 MHz, CDCl₃) δ: 8.17 (d, *J* = 9.2 Hz, 1H), 7.64 (t, *J* = 8.2 Hz, 2H), 7.27 (t, *J* = 7.8 Hz, 1H), 7.21 (dd, *J* = 2.6, 9.0 Hz, 1H), 7.16 (d, *J* = 2.4 Hz, 1H).

### Step 4: Synthesis of intermediate WX010-5

At room temperature and under nitrogen atmosphere, the intermediate **WX010-4** (6.5 g, 29.14 mmol) was dissolved in methanesulfonic acid (87.75 g, 913.06 mmol, 65.00 mL), and then ethyl 4-chloroacetoacetate (7.19 g, 43.71 mmol) was added, and the reaction mixture was stirred and reacted at room temperature for 12 hours. After completion of the reaction, the reaction solution was poured into ice water (500 mL) and extracted with ethyl acetate (400 mL × 3). The organic phase was combined, washed with saturated brine (400 mL × 3), dried over anhydrous sodium sulfate and filtered, and the filtrate was concentrated under reduced pressure to remove the solvent to obtain intermediate **WX010-5.**

### Step 5: Synthesis of intermediate WX010-6

At room temperature and under nitrogen atmosphere, the intermediate **WX010-5** (9.4 g, 29.05 mmol) was dissolved in an aqueous sodium hydroxide solution (2 M, 94.00 mL) and the reaction mixture was warmed to 80 °C and stirred and reacted for 2 hours. After completion of the reaction, the reaction solution was poured into water (500 mL) for dilution, and then extracted with methyl tert-butyl ether (300 mL). The organic phase was discarded, the aqueous phase was adjusted to pH 5 with 12 M concentrated hydrochloric acid, and extracted with ethyl acetate (500 mL × 3). The organic phase was combined, washed with saturated brine (300 mL × 3), dried over anhydrous sodium sulfate and filtered, and the filtrate was concentrated under reduced pressure to remove the solvent to obtain intermediate **WX010-6.**

### Step 6: Synthesis of intermediate WX010-7

At room temperature and under nitrogen atmosphere, the intermediate **WX010-6** (8.8 g, 28.84 mmol,) was dissolved in ethanol (63 mL), then concentrated sulfuric acid (2.57 g, 25.68 mmol, 1.40 mL, purity: 98%) was added, and the reaction mixture was warmed to 80 °C and stirred and reacted for 2 hours. After completion of the reaction, the reaction solution was concentrated under reduced pressure to remove the solvent, added with water (300 mL), and extracted with ethyl acetate (200 mL × 3). The organic phase was combined, washed with saturated brine (200 mL × 3), dried over anhydrous sodium sulfate and filtered, and the filtrate was concentrated under reduced pressure to remove the solvent. The resulting residue was purified by column chromatography (eluent: petroleum ether/ethyl acetate = 1/0-10/1, volume ratio) to obtain the intermediate **WX010-7.** ¹H NMR (400 MHz, CDCl₃) δ: 8.22 (t, *J* = 8.0 Hz, 2H), 7.81 (t, *J* = 3.6 Hz, 2H), 7.74 (d, *J* = 9.2 Hz, 1H), 7.41 (t, *J* = 8.0 Hz, 1H), 4.22 (q, *J* = 7.2 Hz, 2H), 4.06 (s, 2H), 1.26 (t, *J* = 7.2 Hz, 3H).

### Step 7: Synthesis of intermediate WX010-8

At room temperature and under nitrogen atmosphere, the intermediate **WX010-7** (0.5 g, 1.50 mmol) was dissolved in water (0.5 mL) and 1,4-dioxane (5 mL), and then potassium methoxy-methyltrifluoroborate salt (456.11 mg, 3.00 mmol), cesium carbonate (1.47 g, 4.50 mmol), 2-dicyclohexylphosphine-2,6-diisopropoxy-1,1-biphenyl (140.06 mg, 300.14 µ mol), palladium acetate (33.69 mg, 150.07 µmol) were added successively, the reaction mixture was heated to 100 °C and stirred and reacted for 12 hours. After completion of the reaction, the reaction solution was cooled to room temperature, added with water (100 mL), and extracted with ethyl acetate (50 mL × 3). The organic phase was combined, washed with saturated brine (50 mL × 3) successively, dried over anhydrous sodium sulfate, filtered, and the filtrate was concentrated under reduced pressure to remove the solvent. The resulting residue was purified by column chromatography (eluent: petroleum ether/ethyl acetate = 50/0-40/1, volume ratio) to obtain the intermediate **WX010-8**. ¹H NMR (399 MHz, CDCl₃) δ: 8.23 (d, *J* = 8.0 Hz, 1H), 8.05 (d, *J* = 9.2 Hz, 1H), 7.77 (s, 1H), 7.71 (d, *J* = 9.6 Hz, 1H), 7.55 (t, *J* = 7.4 Hz, 1H), 7.50 (d, *J* = 6.0 Hz, 1H), 4.96 (s, 2H), 4.22 (q, *J* = 6.8 Hz, 2H), 4.08 (s, 2H), 3.47 (s, 3H), 1.26 (t, *J* = 7.0 Hz, 3H).

### Step 8: Synthesis of WX010

At room temperature and under nitrogen atmosphere, the intermediate **WX010-8** (100 mg, 335.20 µmol) was dissolved in N,N-dimethylformamide (2 mL), and then acrylamide (23.83 mg, 335.20 µmol), potassium tert-butoxide (37.61 mg, 335.20 µmol) were added successively, and the reaction mixture was stirred and reacted at room temperature for 1 hour. After completion of the reaction, the reaction solution was added with water (50 mL), and extracted with ethyl acetate (30 mL × 3). The organic phase was combined, washed with half-saturated brine (30 mL) successively, dried over anhydrous sodium sulfate and filtered, and the filtrate was concentrated under reduced pressure to remove the solvent. The resulting residue was purified by preparative HPLC (mobile phase: acetonitrile/water; neutral system: 10 mM NH₄HCO₃) to obtain the target compound **WX010.** MS-ESI *m*/*z:* 341.1 [M+H₂O+H]⁺. ¹H NMR (400 MHz, CDCl₃) δ: 8.21 (s, 1H), 8.10 (d, *J* = 9.2 Hz, 1H), 7.99 (d, *J* = 8.0 Hz, 1H), 7.73 (d, *J* = 9.6 Hz, 1H), 7.67 (s, 1H), 7.62-7.47 (m, 2H), 5.04-4.87 (m, 2H), 4.53 (dd, *J* = 6.0, 8.0 Hz, 1H), 3.47 (s, 3H), 2.87-2.68 (m, 2H), 2.59-2.39 (m, 2H).

### Example 11: WX011

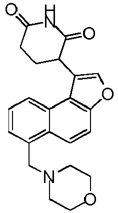

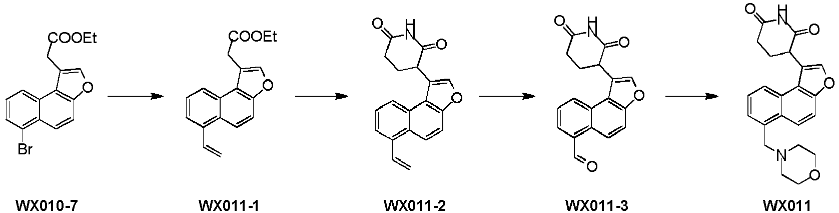

### Step 1: Synthesis of intermediate WX011-1

At room temperature and under nitrogen atmosphere, the intermediate **WX010-7** (2.8 g, 8.40 mmol) was dissolved in N,N-dimethylformamide (30 mL), and then potassium phosphate (1.96 g, 9.24 mmol), potassium vinyltrifluoborate (1.35 g, 10.08 mmol) and [1,1-bis(diphenylphosphine)ferrocene]palladium dichloride dichloromethane (686.30 mg, 840.40 µmol) were added successively, and the reaction mixture was warmed to 80 °C and stirred and reacted at 80 °C for 12 hours. After completion of the reaction, the reaction solution was poured into water (200 mL) and extracted with ethyl acetate (100 mL × 3). The organic phase was combined, washed with saturated brine (100 mL × 3), dried over anhydrous sodium sulfate and filtered, and the filtrate was concentrated under reduced pressure to remove the solvent. The resulting residue was purified by column chromatography (eluent: petroleum ether/ethyl acetate = 1/0-50/1, volume ratio) to obtain the intermediate **WX011-1.** ¹H NMR (400 MHz, CDCl₃) δ: 8.20 (d, *J* = 8.0 Hz, 1H), 8.04 (d, *J* = 9.2 Hz, 1H), 7.77 (s, 1H), 7.69 (d, *J* = 9.2 Hz, 1H), 7.62 (d, *J* = 6.8 Hz, 1H), 7.57 (d, *J* = 8.0 Hz, 1H), 7.55-7.47 (m, 1H), 5.79 (dd, *J* = 1.6, 17.2 Hz, 1H), 5.52 (dd, *J* = 1.2, 10.8 Hz, 1H), 4.23 (q, *J* = 7.0 Hz, 2H), 4.08 (s, 2H), 1.27 (t, *J* = 7.2 Hz, 3H).

### Step 2: Synthesis of intermediate WX011-2

At room temperature and under nitrogen atmosphere, the intermediate **WX011-1** (1 g, 3.57 mmol) was dissolved in N,N-dimethylformamide (10 mL), and then acrylamide (253.56 mg, 3.57 mmol) and potassium tert-butoxide (400.30 mg, 3.57 mmol) were added successively, and the reaction mixture was stirred and reacted at room temperature for 1 hour. After completion of the reaction, the reaction solution was poured into water (200 mL) and extracted with ethyl acetate (100 mL × 3). The organic phase was combined, washed with saturated brine (100 mL × 3) successively, dried over anhydrous sodium sulfate and filtered, and the filtrate was concentrated under reduced pressure to remove the solvent. The resulting residue was added to methanol (20 mL), and a solid was precipitated. The solid was collected by filtration and concentrated under reduced pressure to remove the solvent to obtain the intermediate **WX011-2**. ¹H NMR (400 MHz, CDCl₃) δ: 8.09 (d, *J* = 9.2 Hz, 2H), 7.96 (d, *J* = 8.0 Hz, 1H), 7.71 (d, *J* = 9.2 Hz, 1H), 7.67 (s, 1H), 7.64 (d, *J* = 7.2 Hz, 1H), 7.58 (t, *J* = 6.6 Hz, 1H), 7.52 (dd, *J* = 9.8, 16.2 Hz, 1H), 5.80 (dd, *J* = 1.2, 17.2 Hz, 1H), 5.54 (dd, *J* = 1.2, 10.8 Hz, 1H), 4.55 (dd, *J* = 5.6, 8.4 Hz, 1H), 2.85-2.73 (m, 2H), 2.58-2.44 (m, 2H).

### Step 3: Synthesis of intermediate WX011-3

At room temperature and under nitrogen atmosphere, the intermediate **WX011-2** (0.4 g, 1.31 mmol) was dissolved in tetrahydrofuran (3 mL) and water (1 mL), the temperature was reduced to 0 °C, and then sodium periodate (560.43 mg, 2.62 mmol) and potassium osmate dihydrate (96.54 mg, 262.01 µmol) were added, and the reaction mixture was stirred and reacted at 0 °C for 1 hour. After completion of the reaction, the reaction solution was added with water (100 mL), and extracted with ethyl acetate (50 mL × 3). The organic phase was combined, washed with saturated aqueous sodium sulfite solution (50 mL × 3) and saturated brine (50 mL × 3) successively, dried over anhydrous sodium sulfate and filtered, and the filtrate was concentrated under reduced pressure to remove the solvent. The resulting residue was purified by column chromatography (eluent: petroleum ether/ethyl acetate = 2/1-1/1, volume ratio) to obtain the intermediate **WX011-3**. MS-ESI *m*/*z:* 308.0 [M+H]⁺.

### Step 4: Synthesis of WX011

At room temperature and under nitrogen atmosphere, the intermediate **WX011-3** (120 mg, 390.50 µmol) was dissolved in 1,2-dichloroethane (2 mL), then morpholine (34.02 mg, 390.50 µmol) was added, and the reaction mixture was stirred and reacted at room temperature for 10 minutes. Sodium triacetoxyborohydride (165.53 mg, 781.00 µmol) was added, and the reaction mixture was stirred and reacted at room temperature for 1 hour. After completion of the reaction, the reaction solution was added with water (50 mL), and extracted with ethyl acetate (30 mL × 3). The organic phase was combined, washed with saturated brine (30 mL × 2) successively, dried over anhydrous sodium sulfate and filtered, and the filtrate was concentrated under reduced pressure to remove the solvent. The resulting residue was purified by preparative HPLC (mobile phase: acetonitrile/water; neutral system: 10 mM NH₄HCO₃) to obtain the target compound **WX011.** MS-ESI *m*/*z:* 379.1 [M+H]⁺. ¹H NMR (400 MHz, CDCl₃) δ: 8.35 (d, *J* = 9.6 Hz, 1H), 8.04 (s, 1H), 7.96 (d, *J* = 8.4 Hz, 1H), 7.72 (d, *J* = 9.2 Hz, 1H), 7.67 (s, 1H), 7.52 (t, *J* = 7.8 Hz, 1H), 7.46 (d, *J* = 7.2 Hz, 1H), 4.54 (t, *J* = 6.8 Hz, 1H), 3.95 (s, 2H), 3.70 (t, *J* = 4.6 Hz, 4H), 2.87-2.75 (m, 2H), 2.57-2.41 (m, 6H).

### Example 12: WX012

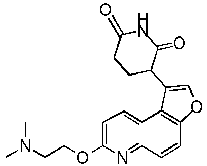

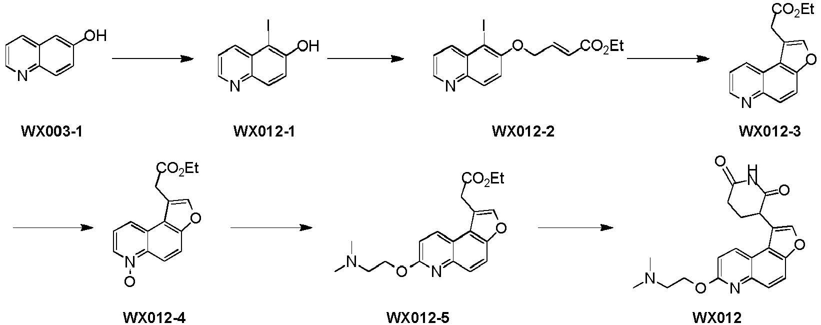

### Step 1: Synthesis of intermediate WX012-1

At room temperature and under nitrogen atmosphere, the intermediate **WX003-1** (20 g, 137.78 mmol) was dissolved in N,N- dimethylformamide (200 mL), and then N-iodosuccinimide (31.00 g, 137.78 mmol) was added, and the reaction mixture was stirred at room temperature for 4 hours. After completion of the reaction, a saturated aqueous sodium sulfite solution (200 mL) was added to the reaction solution, ethyl acetate (100 mL) was added for dilution, the organic phase was collected by separation, and the aqueous phase was extracted with ethyl acetate (100 mL × 3). The organic phase was combined, washed with half- saturated brine (150 mL × 3), and then with saturated brine (150 mL × 2), dried over anhydrous sodium sulfate and filtered, and the filtrate was concentrated under reduced pressure to obtain a residue. Dichloromethane (50 mL) was added to the resulting residue, stirred at room temperature for 0.5 hours and filtered, and the solid was concentrated under reduced pressure to remove the solvent to obtain the intermediate **WX012-1.**

### Step 2: Synthesis of intermediate WX012-2

At 20 °C and under nitrogen atmosphere, the intermediate **WX012-1** (17 g, 62.72 mmol) was dissolved in acetonitrile (170 mL), and then potassium carbonate (43.34 g, 313.59 mmol) and ethyl 4-bromocrotonate (15.34 g, 59.58 mmol) were added, and the reaction mixture was stirred and reacted at 20 °C for 12 hours. After completion of the reaction, water (300 mL) was added to the reaction solution, ethyl acetate (200 mL) was added for dilution, the organic phase was collected by separation, and the aqueous phase was extracted with ethyl acetate (150 mL × 3). The organic phase was combined, and washed with saturated brine (100 mL × 2) successively, dried over anhydrous sodium sulfate and filtered, and the filtrate was concentrated under reduced pressure to remove the solvent. The resulting residue was purified by column chromatography (eluent: petroleum ether/ethyl acetate = 1/0-1/1, volume ratio) to obtain the intermediate **WX012-2.** ¹H NMR (400 MHz, DMSO _*d*₆) δ: 8.78 (dd, *J* = 1.6, 4.2 Hz, 1H), 8.39 (d, *J* = 8.0 Hz, 1H), 8.08 (d, *J* = 9.2 Hz, 1H), 7.66 (d, *J* = 9.2 Hz, 1H), 7.61 (dd, *J* = 4.2, 8.6 Hz, 1H), 7.09 (dt, *J* = 3.6, 16.0 Hz, 1H), 6.33 (dt, *J* = 2.2, 16.0 Hz, 1H), 5.08 (dd, *J* = 2.2, 3.4 Hz, 2H), 4.16 (q, *J* = 7.2 Hz, 2H), 1.23 (t, *J* = 7.0 Hz, 3H).

### Step 3: Synthesis of intermediate WX012-3

At room temperature and under nitrogen atmosphere, the intermediate **WX012-2** (7.3 g, 19.05 mmol) was dissolved in N,N-dimethylformamide (80 mL), and then sodium carbonate (5.05 g, 47.63 mmol), tetrabutyl ammonium chloride (5.82 g, 20.96 mmol), sodium formate (1.30 g, 19.05 mmol) and palladium acetate (213.86 mg, 952.55 µmol) were added, and the reaction mixture was warmed to 70 °C and stirred and reacted for 2 hours. After completion of the reaction, the reaction mixture was cooled to room temperature and subjected to combination treatment. The reaction solution was added with half-saturated brine (200 mL) and ethyl acetate (100 mL), the organic phase was collected by separation, and the aqueous phase was extracted with ethyl acetate (100 mL × 3). The organic phase was combined, washed with saturated brine (50 mL × 2), dried over anhydrous sodium sulfate and filtered, and the filtrate was concentrated under reduced pressure to remove the solvent. The resulting residue was purified by column chromatography (eluent: petroleum ether/ethyl acetate = 1/0-7/3, volume ratio) to obtain the intermediate **WX012-3.** ¹H NMR (400 MHz, CDCl₃) δ: 8.93 (dd, *J* = 1.8, 4.2 Hz, 1H), 8.60 (d, *J* = 8.0 Hz, 1H), 8.03 (d, *J* = 9.2 Hz, 1H), 7.89 (d, *J* = 9.2 Hz, 1H), 7.83 (s, 1H), 7.50 (dd, *J* = 4.4, 8.4 Hz, 1H), 4.22 (q, *J* = 7.6 Hz, 2H), 4.05 (s, 2H), 1.25 (t, *J* = 7.0 Hz, 3H).

### Step 4: Synthesis of intermediate WX012-4

At room temperature, the intermediate **WX012-3** (5.6 g, 21.94 mmol) was dissolved in dichloromethane (100 mL), then m-chloroperoxybenzoic acid (5.21 g, 24.13 mmol, purity: 80%) was added, and the reaction mixture was stirred and reacted at room temperature for 12 hours. After completion of the reaction, the reaction solution was directly purified by column chromatography (eluent: dichloromethane/methanol = 1/0-15/1, volume ratio) to obtain the intermediate **WX012-4.** ¹H NMR (400 MHz, DMSO_*d*₆) δ: 8.61 (d, *J* = 6.0 Hz, 1H), 8.55 (d, *J* = 9.6 Hz, 1H), 8.22 (s, 1H), 8.09 (d, *J* = 9.6 Hz, 2H), 7.58 (dd, *J* = 6.0, 8.4 Hz, 1H), 4.18 (s, 2H), 4.13 (q, *J* = 7.0 Hz, 2H), 1.17 (t, *J* = 7.0 Hz, 3H).

### Step 5: Synthesis of intermediate WX012-5

At 20 °C and under nitrogen atmosphere, the intermediate **WX012-4** (500 mg, 1.84 mmol) was dissolved in carbon tetrachloride (5 mL), N,N-dimethylethanolamine (164.30 mg, 1.84 mmol) and N,N-diisopropylethylamine (476.43 mg, 3.69 mmol) were added, then the solution of diethyl phosphite (509.10 mg, 3.69 mmol) in acetonitrile (5 mL) was added dropwise to the reaction solution, and the reaction mixture was warmed to 40 °C and stirred and reacted for 12 hours. Additional N,N-dimethylethanolamine (82.15 mg, 921.60 µmol) and diethyl phosphite (509.10 mg, 3.69 mmol) were added, and the reaction mixture was warmed to 80 °C and stirred and reacted for 12 hours. Additional diethyl phosphite (509.10 mg, 3.69 mmol) was added, and the reaction mixture was warmed to 80 °C and stirred and reacted for 12 hours. After completion of the reaction, the reaction mixture was concentrated under reduced pressure to remove the solvent. The resulting residue was purified by preparative HPLC (mobile phase: acetonitrile/water; neutral system: 10 mM NH₄HCO₃) to obtain the intermediate **WX012-5.** ¹H NMR (400 MHz, CHCl₃) δ: 8.44 (d, *J* = 8.8 Hz, 1H), 7.81-7.72 (m, 3H), 7.04 (d, *J* = 9.2 Hz, 1H), 4.74 (t, *J* = 5.4 Hz, 2H), 4.21 (q, *J* = 7.2 Hz, 2H), 3.99 (s, 2H), 3.07 (t, *J* = 5.2 Hz, 2H), 2.58 (s, 6H), 1.25 (t, *J* = 7.0 Hz, 3H).

### Step 6: Synthesis of WX012

At 20 °C and under nitrogen atmosphere, the intermediate **WX012-5** (65 mg, 189.84 µmol) was dissolved in N,N-dimethylformamide (1 mL), and then acrylamide (13.49 mg, 189.84 µmol) and the solution of potassium tert-butyl (1 M, 189.84 µL) in tetrahydrofuran were added successively, and the reaction mixture was stirred and reacted at 20 °C for 3 hours. After completion of the reaction, the reaction solution was added dropwise with 2 N of dilute aqueous hydrochloric acid solution to adjust to pH = 6-7 and filtered, and the filtrate was collected. The resulting filtrate was purified by preparative HPLC (mobile phase: acetonitrile/water; acid system: 0.05% HCl), further purified by preparative HPLC (mobile phase: acetonitrile/water; neutral system: 10 mM NH₄HCO₃) to obtain the target compound **WX012.** MS-ESI *m*/*z:* 368.2 [M+H]⁺. ¹H NMR (400 MHz, DMSO_*d*₆) δ: 10.93 (s, 1H), 8.49 (d, *J* = 8.8 Hz, 1H), 8.04 (s, 1H), 7.93 (d, *J* = 9.2 Hz, 1H), 7.70 (d, *J* = 8.8 Hz, 1H), 7.05 (d, *J* = 8.8 Hz, 1H), 4.64 (dd, *J* = 4.4, 12.0 Hz, 1H), 4.57-4.46 (m, 2H), 2.93-2.80 (m, 1H), 2.70 (t, *J* = 6.0 Hz, 2H), 2.66-2.58 (m, 1H), 2.45-2.38 (m, 1H), 2.34-2.27 (m, 1H), 2.24 (s, 6H).

### Example 13: WX013

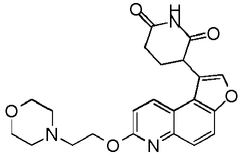

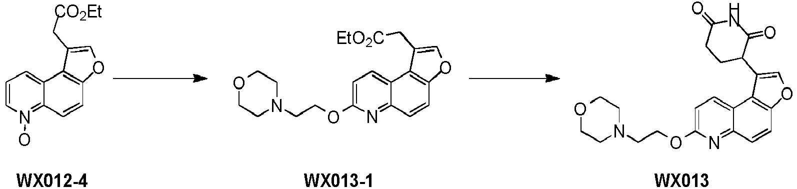

### Step 1: Synthesis of intermediate WX013-1

At room temperature and under nitrogen atmosphere, the intermediate **WX012-4** (500 mg, 1.84 mmol) was dissolved in carbon tetrachloride (5 mL), and N-(2-hydroxyethyl)morpholine (483.55 mg, 3.69 mmol) and N,N-diisopropylethylamine (952.88 mg, 7.37 mmol) were added, then the solution of diethyl phosphite (1.02 g, 7.37 mmol) in acetonitrile (5 mL) was added dropwise to the reaction, and the reaction mixture was warmed to 80 °C and stirred and reacted for 12 hours. After completion of the reaction, the reaction solution was directly concentrated under reduced pressure to remove the solvent. The resulting residue was purified by preparative HPLC (mobile phase: acetonitrile/water; neutral system: 10 mM NH₄HCO₃) to obtain the intermediate **WX013-1.** ¹H NMR (400 MHz, CHCl₃) δ: 8.44 (d, *J* = 9.2 Hz, 1H), 7.87-7.72 (m, 3H), 7.02 (d, *J* = 9.2 Hz, 1H), 4.65 (t, *J* = 5.8 Hz, 2H), 4.21 (q, *J* = 7.2 Hz, 2H), 4.03-3.97 (m, 2H), 3.76 (t, *J* = 4.6 Hz, 4H), 2.88 (t, *J* = 6.0 Hz, 2H), 2.63 (t, *J* = 4.4 Hz, 4H), 1.25 (t, *J* = 7.2 Hz, 3H).

### Step 2: Synthesis of WX013

At 20 °C and under nitrogen atmosphere, the intermediate **WX013-1** (97 mg, 252.32 µmol) was dissolved in N,N-dimethylformamide (2 mL), and then acrylamide (17.93 mg, 252.32 µmol) and the solution of potassium *tert*-butoxide (28.31 mg, 1 M, 252.32 µL) in tetrahydrofuran were added successively, and the reaction mixture was stirred at 20 °C for 3 hours. After completion of the reaction, the reaction solution was added dropwise with 2 N of dilute aqueous hydrochloric acid solution to adjust the pH to 6-7 and filtered, and the filtrate was collected. The resulting filtrate was purified by preparative HPLC (mobile phase: acetonitrile/water; neutral system: 10 mM NH₄HCO₃) to obtain the target compound **WX013.** MS-ESI *m*/*z:* 410.1 [M+H]⁺. ¹H NMR (400 MHz, DMSO_*d*₆) δ: 10.93 (s, 1H), 8.50 (d, *J* = 9.2 Hz, 1H), 8.05 (s, 1H), 7.93 (d, *J* = 8.8 Hz, 1H), 7.70 (d, *J* = 9.2 Hz, 1H), 7.06 (d, *J* = 9.2 Hz, 1H), 4.64 (dd, *J* = 4.6, 12.6 Hz, 1H), 4.58-4.52 (m, 2H), 3.57 (t, *J* = 4.4 Hz, 4H), 2.91-2.80 (m, 1H), 2.76 (t, *J* = 5.6 Hz, 2H), 2.69-2.64 (m, 1H), 2.63-2.58 (m, 1H), 2.57-2.52 (m, 2H), 2.45-2.38 (m, 1H), 2.37-2.31 (m, 1H), 2.30-2.22 (m, 1H).

### Example 14: WX014

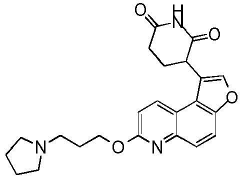

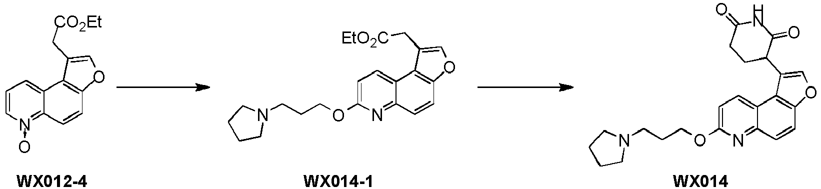

### Step 1: Synthesis of intermediate WX014-1

At room temperature and under nitrogen atmosphere, the intermediate **WX012-4** (500 mg, 1.84 mmol) was dissolved in carbon tetrachloride (5 mL), and 1-hydroxypropylpyrrolidine (476.28 mg, 3.69 mmol) and N,N-diisopropylethylamine (952.86 mg, 7.37 mmol) were added, then the solution of diethyl phosphite (1.02 g, 7.37 mmol) in acetonitrile (5 mL) was added dropwise to the reaction solution, and the reaction mixture was warmed to 80 °C and stirred and reacted for 12 hours. After completion of the reaction, the reaction solution was concentrated under reduced pressure to remove the solvent. The resulting residue was purified by preparative HPLC (mobile phase: acetonitrile/water; acid system: 0.225% FA) to obtain the intermediate **WX014-1.** ¹H NMR (400 MHz, DMSO_*d*₆) δ: 8.45 (d, *J* = 8.8 Hz, 1H), 8.16-8.07 (m, 1H), 7.94 (d, *J* = 9.2 Hz, 1H), 7.69 (d, *J* = 9.2 Hz, 1H), 7.10 (d, *J* = 8.8 Hz, 1H), 4.48 (t, *J* = 6.2 Hz, 2H), 4.17-4.07 (m, 4H), 3.03-2.87 (m, 6H), 2.15-2.02 (m, 2H), 1.90-1.75 (m, 4H), 1.16 (t, *J* = 7.0 Hz, 3H).

### Step 2: Synthesis of WX014

At 20 °C and under nitrogen atmosphere, the intermediate **WX014-1** (42 mg, 109.82 µmol) was dissolved in N,N-dimethylformamide (2 mL), and then acrylamide (7.81 mg, 109.82 µmol) and the solution of potassium *tert*-butoxide (1 M, 109.82 µL) in tetrahydrofuran were added successively, and the reaction mixture was stirred and reacted at 20 °C for 3 hours. After completion of the reaction, the reaction solution was added dropwise with 2 N of dilute aqueous hydrochloric acid solution to adjust the pH to 6-7 and filtered, and the filtrate was collected. The resulting filtrate was purified by preparative HPLC (mobile phase: acetonitrile/water; neutral system: 10 mM NH₄HCO₃) to obtain the target compound **WX014.** MS-ESI *m*/*z:* 408.1 [M+H]⁺. ¹H NMR (400 MHz, DMSO_*d*₆) δ: 10.93 (s, 1H), 8.49 (d, *J=* 8.8 Hz, 1H), 8.04 (s, 1H), 7.93 (d, *J* = 9.2 Hz, 1H), 7.70 (d, *J* = 8.8 Hz, 1H), 7.05 (d, *J* = 8.8 Hz, 1H), 4.64 (dd, *J* = 5.0, 12.2 Hz, 1H), 4.45 (t, *J=* 6.0 Hz, 2H), 2.91-2.80 (m, 1H), 2.69-2.64 (m, 1H), 2.63-2.53 (m, 4H), 2.47-2.44 (m, 2H), 2.43-2.36 (m, 1H), 2.31-2.22 (m, 1H), 2.05-1.92 (m, 2H), 1.72-1.66 (m, 4H).

### Example 15: WX015

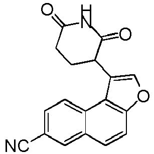

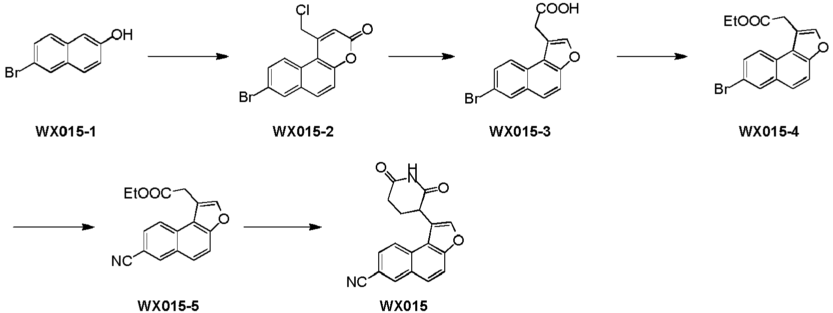

### Step 1: Synthesis of intermediate WX015-2

At 20 °C and under nitrogen atmosphere, concentrated sulfuric acid (220.80 g, 2.21 mol, 120 mL, purity: 98%) was added to ice water (40 mL) dropwise, then the compound **WX015-1** (10 g, 44.83 mmol) was added, and finally ethyl 4-chloroacetoacetate (7.38 g, 44.83 mmol) was added dropwise at 5-10 °C, and the reaction mixture was stirred and reacted at 20 °C for 16 hours. The reaction mixture was warmed to 50 °C and stirred and reacted for additional 16 hours. After completion of the reaction, the reaction solution was cooled to room temperature, poured into ice water (1000 mL) and a solid was precipitated. The solid was collected by filtration and the filtrate was discarded. Toluene (400 mL × 2) was added to the solid, and the solvent was removed by concentration under reduced pressure to obtain the intermediate **WX015-2.**

### Step 2: Synthesis of intermediate WX015-3

At room temperature and under nitrogen atmosphere, the intermediate **WX015-2** (14.5 g, 44.81 mmol) was dissolved in the solution of sodium hydroxide (8.70 g, 217.52 mmol) in water (150 mL), and the reaction mixture was warmed to 80 °C and stirred and reacted for 5 hours. After completion of the reaction, dichloromethane (150 mL) was added for dilution, the organic phase was collected after separation, and the aqueous phase was extracted with dichloromethane (150 mL × 3). The organic phase was combined, washed with saturated brine (50 mL × 2), dried over anhydrous sodium sulfate and filtered, and the filtrate was concentrated under reduced pressure to remove the solvent. The aqueous phase was adjusted to pH 4 with 2 M hydrochloric acid aqueous, and extracted with ethyl acetate (200 mL × 3). The organic phase was combined, washed with saturated brine (50 mL × 2), dried over anhydrous sodium sulfate and filtered, and the filtrate was concentrated under reduced pressure to remove the solvent to obtain the intermediate **WX015-3.**

### Step 3: Synthesis of intermediate WX015-4

At room temperature and under nitrogen atmosphere, the intermediate **WX015-3** (11.3 g, 37.03 mmol) was dissolved in ethanol (300 mL), then concentrated sulfuric acid (2.08 g, 20.78 mmol, 1.13 mL, purity: 98%) was added, and the reaction mixture was warmed to 80 °C and the reaction mixture was stirred and reacted for 12 hours. After completion of the reaction, the reaction solution was concentrated under reduced pressure to remove the solvent, water (150 mL) was added, and ethyl acetate (150 mL) was added for dilution. After separation, the organic phase was collected and the aqueous phase was extracted with ethyl acetate (100 mL × 3). The organic phase was combined, washed with saturated brine (50 mL × 2), dried over anhydrous sodium sulfate and filtered, and the filtrate was concentrated under reduced pressure to remove the solvent. The resulting residue was purified by column chromatography (eluent: petroleum ether/ethyl acetate = 1/0-32/1, volume ratio) to obtain the intermediate **WX015-4.** ¹H NMR (400 MHz, DMSO_*d*₆) δ: 8.35 (d, *J* = 2.0 Hz, 1H), 8.09 (t, *J* = 4.4 Hz, 2H), 7.86 (s, 2H), 7.73 (dd, *J* = 2.0, 8.8 Hz, 1H), 4.09-4.18 (m, 4H), 1.18 (t, *J* = 7.2 Hz, 3H).

### Step 4: Synthesis of intermediate WX015-5

At room temperature and under nitrogen atmosphere, the intermediate **WX015-4** (5 g, 15.01 mmol) was dissolved in N,N-dimethylformamide (80 mL), and then potassium hexacyanoferrate (II) (1.16 g, 3.15 mmol), sodium carbonate (1.59 g, 15.01 mmol) and palladium acetate (336.92 mg, 1.50 mmol) were added successively, and the reaction mixture was heated to 140 °C and stirred and reacted for 8 hours. After completion of the reaction, the reaction solution was cooled to room temperature, added with water (300 mL), and extracted with ethyl acetate (100 mL × 5). The organic phase was combined, dried over anhydrous sodium sulfate and filtered, and the filtrate was concentrated under reduced pressure to remove the solvent. The resulting residue was purified by column chromatography (eluent: petroleum ether/ethyl acetate = 1/0-9/1, volume ratio) to obtain the intermediate **WX015-5.** ¹H NMR (400 MHz, CDCl₃) δ: 8.32 (d, *J* = 1.2 Hz, 1H), 8.30 (d, *J* = 8.8 Hz, 1H), 7.84 (s, 1H), 7.77 (s, 2H), 7.72 (dd, *J* = 1.8, 8.6 Hz, 1H), 4.23 (q, *J* = 7.0 Hz, 2H), 4.05 (s, 2H), 1.27 (t, *J* = 7.2 Hz, 3H).

### Step 5: Synthesis of WX015

At 20 °C, the intermediate **WX015-5** (1.1 g, 3.94 mmol) was dissolved in N,N-dimethylformamide (20 mL), and then acrylamide (279.94 mg, 3.94 mmol) and potassium *tert-*butoxide (441.95 mg, 3.94 mmol) were added successively, and the reaction mixture was stirred and reacted at 20 °C for 2 hours. After completion of the reaction, the reaction solution was added with water (100 mL), and extracted with ethyl acetate (30 mL × 3). The organic phase was combined, washed with half-saturated brine (20 mL × 2) successively, dried over anhydrous sodium sulfate and filtered, and the filtrate was concentrated under reduced pressure to remove the solvent. The resulting residue was stirred with 5 mL of methanol at room temperature for 5 minutes and a solid was precipitated. The solid was collected by filtration and concentrated under reduced pressure to remove the solvent. The resulting residue was purified by preparative HPLC (mobile phase: acetonitrile/water; acid system: 0.05% HCl) to obtain the target compound **WX015.** MS-ESI *m*/*z:* 305.0 [M+H]⁺. ¹H NMR (400 MHz, DMSO_*d*₆) δ: 10.96 (s, 1H), 8.70 (d, *J* = 1.2 Hz, 1H), 8.36 (d, *J* = 8.4 Hz, 1H), 8.13 (s, 1H), 8.02 (d, *J* = 9.2 Hz, 1H), 7.98 (d, *J* = 8.8 Hz, 1H), 7.87 (dd, *J* = 1.6, 8.8 Hz, 1H), 4.72 (dd, *J* = 4.2, 12.6 Hz, 1H), 2.94-2.80 (m, 1H), 2.71-2.61 (m, 1H), 2.47-2.38 (m, 1H), 2.35-2.24 (m, 1H).

### Example 16: WX016

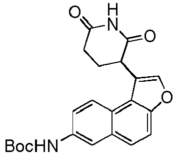

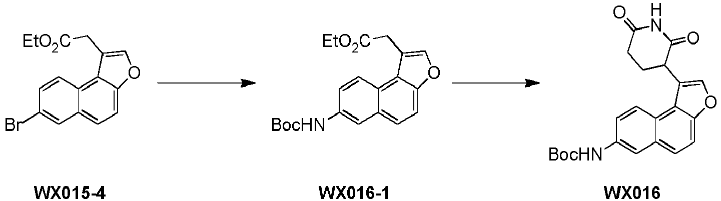

### Step 1: Synthesis of intermediate WX016-1

At room temperature and under nitrogen atmosphere, the intermediate **WX015-4** (2 g, 6.00 mmol) was dissolved in dioxane (35 mL), and then tert-butyl carbamate (1.05 g, 9.00 mmol), 4,5-bis-diphenylphosphine-9,9- dimethylxanthene (521.00 mg, 900.42 µmol), cesium carbonate (4.89 g, 15.01 mmol) and palladium acetate (202.15 mg, 900.42 µmol) were added successively, and the reaction mixture was slowly warmed to 80 °C and stirred and reacted for 12 hours. After completion of the reaction, water (80 mL) and ethyl acetate (80 mL) were added to the reaction solution for dilution, the organic phase was collected by separation, and the aqueous phase was extracted with ethyl acetate (50 mL × 3). The organic phase was combined, washed with saturated brine (50 mL × 2), dried over anhydrous sodium sulfate and filtered, and the filtrate was concentrated under reduced pressure to remove the solvent. The resulting residue was purified by column chromatography (eluent: petroleum ether/ethyl acetate = 1/0-9/1, volume ratio) to obtain the intermediate **WX016-1.**

### Step 2: Synthesis of WX016

At 20 °C and under nitrogen atmosphere, the intermediate **WX016-1** (200 mg, 541.40 µmol) was dissolved in tetrahydrofuran (4 mL), and then acrylamide (38.48 mg, 541.40 µmol) and potassium tert-butoxide (60.75 mg, 541.40 µmol) were added successively, and the reaction mixture was stirred and reacted at 20 °C for 4 hours. After completion of the reaction, water (15 mL) and 2-methyltetrahydrofuran (10 mL) were added to the reaction solution for dilution, the organic phase was collected by separation, and the aqueous phase was extracted with 2-methyltetrahydrofuran (15 mL × 3). The organic phase was combined, washed with saturated brine (10 mL × 2), dried over anhydrous sodium sulfate and filtered, and the filtrate was concentrated under reduced pressure to remove the solvent. The resulting residue was purified by preparative HPLC (mobile phase: acetonitrile/water; neutral system: 10 mM NH₄HCO₃) to obtain the target compound **WX016.** MS-ESI *m*/*z:* 417.1 [M+Na]⁺. ¹H NMR (400 MHz, DMSO_*d*₆) δ: 10.94 (s, 1H), 9.54 (s, 1H), 8.24 (s, 1H), 8.06 (d, *J* = 9.6 Hz, 1H), 7.96 (s, 1H), 7.71 (s, 2H), 7.55 (dd, *J* = 2.0, 8.8 Hz, 1H), 4.63 (dd, *J* = 4.6, 12.2 Hz, 1H), 2.93-2.80 (m, 1H), 2.70-2.55 (m, 1H), 2.45-2.36 (m, 1H), 2.30-2.20 (m, 1H), 1.52 (s, 9H).

### Example 17: Hydrochloride of WX017

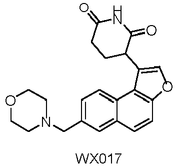

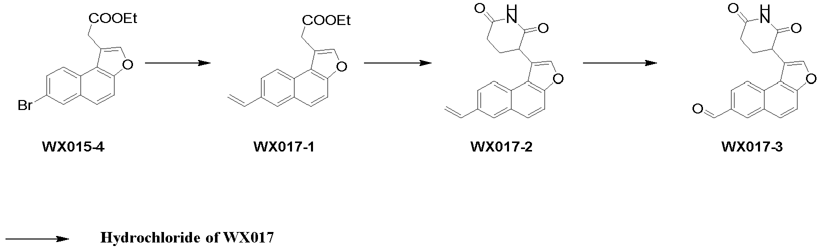

### Step 1: Synthesis of intermediate WX017-1

At room temperature and under nitrogen atmosphere, the intermediate **WX015-4** (5 g, 15.01 mmol) was dissolved in N,N-dimethylformamide (50 mL), and then [1,1-bis(diphenylphosphine)ferrocene]palladium dichloride dichloromethane (1.23 g, 1.50 mmol), potassium phosphate (3.50 g, 16.51 mmol) and potassium vinyltrifluoroborate (2.41 g, 18.01 mmol) were added successively, and the reaction mixture was heated to 80 °C and stirred and reacted for 12 hours. After completion of the reaction, the reaction solution was cooled to room temperature, added with water (200 mL), and extracted with ethyl acetate (100 mL × 3). The organic phase was combined, washed with half-saturated brine (100 mL × 3) successively, dried over anhydrous sodium sulfate and filtered, and the filtrate was concentrated under reduced pressure to remove the solvent. The resulting residue was purified by column chromatography (eluent: petroleum ether/ethyl acetate = 1/0-9/1, volume ratio) to obtain the intermediate **WX017-1.** ¹H NMR (400 MHz, CDCl₃) δ: 8.19 (d, *J* = 8.8 Hz, 1H), 7.89 (d, *J* = 0.8 Hz, 1H), 7.76 (s, 1H), 7.73 (dd, *J* = 1.6, 6.8 Hz, 1H), 7.01 (d, *J* = 5.2 Hz, 1H), 7.64 (d, *J* = 8.8 Hz, 1H), 6.91 (dd, *J =* 10.8, 17.6 Hz, 1H), 5.89 (d, *J =* 17.6 Hz, 1H), 5.35 (d, *J =* 10.8 Hz, 1H), 4.24 (q, *J =* 7.2 Hz, 2H), 4.07 (s, 2H), 1.27 (t, *J =* 7.2 Hz, 3H).

### Step 2: Synthesis of intermediate WX017-2

At room temperature, the intermediate **WX017-1** (2 g, 7.13 mmol) was dissolved in N,N-dimethylformamide (30 mL), and then acrylamide (506.79 mg, 7.13 mmol) and potassium *tert*-butoxide (800.07 mg, 7.13 mmol) were added successively, and the reaction mixture was stirred and reacted at room temperature for 2 hours. After completion of the reaction, the reaction solution was diluted by adding water (100 mL), and extracted with ethyl acetate (50 mL × 3). The organic phase was combined, washed with half-saturated brine (30 mL × 3) successively, dried over anhydrous sodium sulfate and filtered, and the filtrate was concentrated under reduced pressure to remove the solvent. The resulting residue was stirred with 10 mL of methanol at room temperature for 10 minutes, and a light yellow solid was precipitated. The solid was collected by filtration, and concentrated under reduced pressure to obtain the intermediate **WX017-2**. ¹H NMR (400 MHz, DMSO_*d*₆) δ: 10.98 (s, 1H), 8.13 (d, *J=* 8.4 Hz, 1H), 8.05 (s, 1H), 8.02 (s, 1H), 7.84 (d, *J* = 9.2 Hz, 1H), 7.81-7.75 (m, 2H), 6.92 (dd, *J* = 11.0, 17.8 Hz, 1H), 5.98 (d, *J* = 17.6 Hz, 1H), 5.35 (d, *J* = 11.2 Hz, 1H), 4.67 (dd, *J* = 4.4, 12.0 Hz, 1H), 2.96-2.82 (m, 1H), 2.69-2.59 (m, 1H), 2.44-2.35 (m, 1H), 2.33-2.20 (m, 1H).

### Step 3: Synthesis of intermediate WX017-3

At room temperature and under nitrogen atmosphere, the intermediate **WX017-2** (400 mg, 1.31 mmol) was dissolved in tetrahydrofuran (6 mL) and water (2 mL), the temperature was reduced to 0 °C, and sodium periodate (560.43 mg, 2.62 mmol) and potassium osmate dihydrate (96.54 mg, 262.01 µmol) were added, and the reaction mixture was slowly returned to room temperature and stirred and reacted for 1 hour. After completion of the reaction, the reaction solution was diluted by adding water (20 mL) and N,N-dimethylformamide (1 mL), and extracted with ethyl acetate (20 mL × 3). The organic phase was combined, washed with saturated sodium sulfite solution (20 mL × 3), dried with anhydrous sodium sulfate and filtered, and the filtrate was concentrated under reduced pressure. The resulting residue was purified by column chromatography (eluent: petroleum ether/ethyl acetate = 4/1-1/1, volume ratio) and the resulting crude product was stirred with 1 mL methanol at room temperature for 5 minutes and filtered, and the solid was collected and concentrated under reduced pressure to obtain the intermediate **WX017-3**. ¹H NMR (400 MHz, DMSO_*d*₆) δ: 10.93 (s, 1H), 10.17 (s, 1H), 8.71 (s, 1H), 8.37 (d, *J* = 8.8 Hz, 1H), 8.14-8.08 (m, 2H), 8.00-7.97 (m, 2H), 4.73 (dd, *J* = 4.4, 12.0 Hz, 1H), 2.69-2.62 (m, 1H), 2.47-2.38 (m, 1H), 2.37-2.25 (m, 2H).

### Step 4: Synthesis of WX017

At room temperature, the intermediate **WX017-3** (60 mg, 195.25 µmol) was dissolved in 1,2-dichloroethane (1 mL), then morpholine (17.01 mg, 195.25 µmol) was added, and the reaction mixture was stirred at room temperature for 10 minutes and then sodium borohydride acetate (82.76 mg, 390.50 µmol) was added, and the reaction mixture was stirred and reacted at room temperature for 12 hours. After completion of the reaction, the reaction solution was directly concentrated under reduced pressure to remove the solvent. The resulting residue was purified by preparative HPLC (mobile phase: acetonitrile/water; acid system: 0.05% HCl) to obtain the hydrochloride of the target compound **WX017.** MS-ESI *m*/*z:* 379.1 [M+H]⁺. ¹H NMR (400 MHz, DMSO_*d*₆) δ: 11.38 (s, 1H), 10.95 (s, 1H), 8.31-8.21 (m, 2H), 8.06 (s, 1H), 7.91-7.85 (m, 3H), 4.71 (dd, *J* = 4.4, 12.4 Hz, 1H), 4.51 (s, 2H), 3.93 (d, *J* = 12.0 Hz, 2H), 3.81 (t, *J* = 12.0 Hz, 2H), 3.31-3.22 (m, 2H), 3.22-3.07 (m, 2H), 2.96-2.83 (m, 1H), 2.69-2.58 (m, 1H), 2.46-2.37 (m, 1H), 2.33-2.21 (m, 1H).

### Example 18: WX018

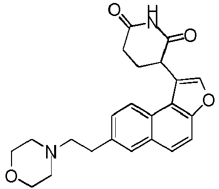

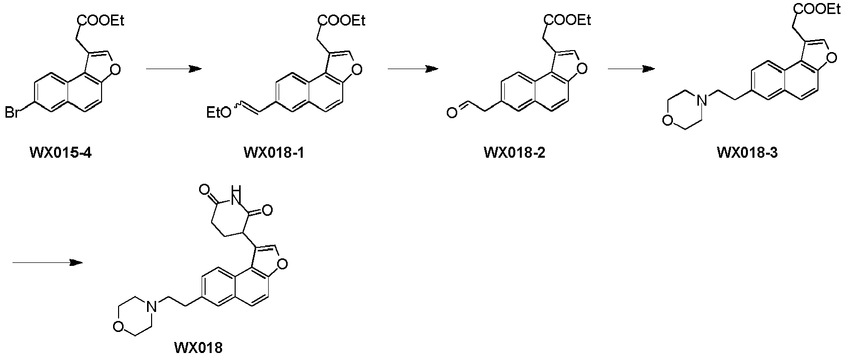

### Step 1: Synthesis of intermediate WX018-1

At room temperature, the intermediate **WX015-4** (5 g, 15.01 mmol) was dissolved in N,N-dimethylformamide (50 mL), and then potassium phosphate (3.19 g, 15.01 mmol), [1,1-bis(diphenylphosphine)ferrocene]palladium dichloride dichloromethane (1.23 g, 1.50 mmol) and (*E*)-1-ethoxyvinyl-2-boronic acid pinacol ester (3.86 g, 19.51 mmol) were added successively, and the reaction mixture was heated to 80 °C and stirred and reacted for 12 hours. After completion of the reaction, the reaction solution was cooled to room temperature, added with water (100 mL), and extracted with ethyl acetate (100 mL × 3). The organic phase was combined, washed with half-saturated brine (50 mL × 3) successively, dried over anhydrous sodium sulfate and filtered, and the filtrate was concentrated under reduced pressure to obtain a residue. The resulting residue was purified by column chromatography (eluent: petroleum ether/ethyl acetate = 1/0-19/1, volume ratio) to obtain the intermediate **WX018-1.** MS-ESI *m*/*z:* 325.2 [M+H]⁺.

### Step 2: Synthesis of intermediate WX018-2

At 0 °C, the intermediate **WX018-1** (200 mg, 616.58 µmol) was dissolved in chloroform (2 mL), and then ethanol (28.40 mg, 616.58 µmol, 36.05 µL), water (616.58 µmol, 11.11 µL) and oxalyl chloride (78.26 mg, 616.58 µmol, 53.97 µ L) were added successively, and the reaction mixture was returned to room temperature and stirred and reacted for 1 hour. After completion of the reaction, the reaction solution was added with water (10 mL), adjusted with saturated sodium bicarbonate to achieve pH 6-7, and extracted with dichloromethane (5 mL × 3). The organic phase was combined, dried over anhydrous sodium sulfate, and filtered to obtain a solution of the intermediate **WX018-2** in dichloromethane.

### Step 3: Synthesis of intermediate WX018-3

At room temperature and under nitrogen atmosphere, morpholine (78.41 mg, 900.00 µmol) was added to the solution of the intermediate **WX018-2** in dichloromethane (0.04 M, 15 mL), the reaction mixture was stirred and reacted at room temperature for 10 minutes, and then sodium borohydride acetate (254.33 mg, 1.20 mmol) was added, and the reaction mixture was stirred and reacted for additional 4 hours at room temperature. After completion of the reaction, the reaction solution was directly concentrated under reduced pressure to remove the solvent. The resulting residue was purified by column chromatography (eluent: petroleum ether/ethyl acetate = 7/3-3/7, volume ratio) to obtain the intermediate **WX018-3.** ¹H NMR (400 MHz, CDCl₃) δ: 8.17 (d, *J* = 8.4 Hz, 1H), 7.76 (d, *J* = 6.0 Hz, 2H), 7.68 (d, *J* = 9.2 Hz, 1H), 7.63 (d, *J* = 8.8 Hz, 1H), 7.45 (dd, *J* = 1.8, 8.6 Hz, 1H), 4.23 (q, *J* = 7.2 Hz, 2H), 4.06 (s, 2H), 3.79 (t, *J* = 4.6 Hz, 4H), 3.08-2.95 (m, 2H), 2.81-2.70 (m, 2H), 2.67-2.55 (m, 4H), 1.27 (t, *J* = 7.0 Hz, 3H).

### Step 4: Synthesis of WX018

At room temperature and under nitrogen atmosphere, the intermediate **WX018-3** (100 mg, 272.16 µmol) was dissolved in N,N-dimethylformamide (2 mL), and then acrylamide (19.34 mg, 272.16 µmol) and potassium *tert*-butoxide (30.54 mg, 272.16 µmol) were added successively, and the reaction mixture was stirred and reacted at room temperature for 1 hour. After completion of the reaction, 2 M hydrochloric acid aqueous was added dropwise to the reaction solution to adjust the pH to 6-7. After filtration, the filtrate was collected. The resulting filtrate was purified by preparative HPLC (mobile phase: acetonitrile/water; neutral system: 10 mM NH₄HCO₃) to obtain the target compound **WX018.** MS-ESI *m*/*z:* 393.1 [M+H]⁺. ¹H NMR (400 MHz, DMSO_*d*₆) δ: 10.96 (s, 1H), 8.08 (d, *J* = 8.4 Hz, 1H), 7.99 (s, 1H), 7.87 (s, 1H), 7.78 (d, *J* = 9.2 Hz, 1H), 7.74 (d, *J* = 8.8 Hz, 1H), 7.48 (dd, *J* = 1.4, 8.6 Hz, 1H), 4.65 (dd, *J* = 4.2, 11.8 Hz, 1H), 3.58 (t, *J* = 4.4 Hz, 4H), 2.92 (t, *J =* 5.8 Hz, 2H), 2.89-2.82 (m, 1H), 2.69-2.64 (m, 1H), 2.63-2.55 (m, 2H), 2.48-2.42 (m, 4H), 2.41-2.22 (m, 2H).

### Example 19: WX019

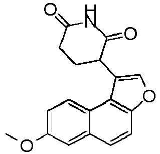

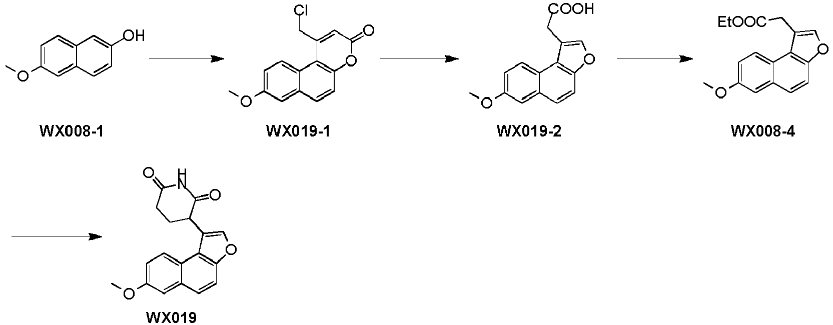

### Step 1: Synthesis of intermediate WX019-1

At 19 °C, concentrated sulfuric acid (110.40 g, 1.10 mol, 60 mL, purity: 98%) was added dropwise to ice water (20 mL), and then the compound **WX008-1** (5 g, 28.70 mmol) was added, and finally ethyl 4-chloroacetoacetate (5.20 g, 31.57 mmol) was added dropwise at 5-10 °C, and the reaction mixture was returned to 19 °C and stirred and reacted for 12 hours. After completion of the reaction, the reaction solution was poured into ice water (200 mL), a light yellow solid was precipitated. The solid was collected by filtration, and the obtained solid was diluted with 2-methyltetrahydrofuran (500 mL) and water (200 mL). The organic phase was collected after separation, and the aqueous phase was extracted with 2-methyltetrahydrofuran (300 mL × 4). The organic phase was combined and concentrated under reduced pressure to remove the solvent. The resulting residue was stirred with 50 mL of methyl tert-butyl ether at room temperature for 15 minutes and filtered, and the solid was collected and concentrated under reduced pressure to remove the solvent to obtain the intermediate **WX019-1.**

### Step 2: Synthesis of intermediate WX019-2

At room temperature, sodium hydroxide (1.60 g, 40.04 mmol) was dissolved in water (20 mL), and then the intermediate **WX019-1** (1 g, 3.64 mmol) was added, and the reaction mixture was heated to 80 °C and stirred and reacted for 12 hours. After completion of the reaction, the reaction mixture was cooled to room temperature, added with water (100 mL), and extracted with ethyl acetate (50 mL). The organic phase was removed, the pH of the aqueous phase was adjusted to 5-6 with concentrated hydrochloric acid (12 M), a solid was precipitated, and extraction with ethyl acetate (50 mL × 3) was performed. The organic phase was combined, dried over anhydrous sodium sulfate, filtered, and the filtrate was concentrated under reduced pressure to remove the solvent to obtain the intermediate **WX019-2.**

### Step 3: Synthesis of intermediate WX008-4

At room temperature, the intermediate **WX019-2** (0.9 g, 3.51 mmol) was dissolved in ethanol (10 mL), then concentrated sulfuric acid (368.00 mg, 3.68 mmol, 0.2 mL, purity: 98%) was added, and the reaction mixture was heated to 80 °C and stirred and reacted for 3 hours. After completion of the reaction, the reaction solution was directly concentrated under reduced pressure to remove most of the ethanol. The resulting residue was diluted with ethyl acetate (30 mL) and water (50 mL). The organic phase was collected after separation, and the aqueous phase was extracted with ethyl acetate (30 mL × 2). The organic phase was combined, dried over anhydrous sodium sulfate and filtered, and the filtrate was concentrated under reduced pressure to remove the solvent. The resulting residue was purified by column chromatography (eluent: petroleum ether/ethyl acetate = 1/0-9/1, volume ratio) to obtain the intermediate **WX008-4.** ¹H NMR (400 MHz, CDCl₃) δ: 8.04 (d, *J* = 9.2 Hz, 1H), 7.64 (s, 1H), 7.56-7.49 (m, 2H), 7.20 (d, *J* = 2.8 Hz, 1H), 7.15 (dd, *J* = 2.4, 9.2 Hz, 1H), 4.12 (q, *J* = 7.0 Hz, 2H), 3.94 (s, 2H), 3.84 (s, 3H), 1.16 (t, *J =* 7.0 Hz, 3H).

### Step 4: Synthesis of WX019

At 15 °C, the intermediate **WX008-4** (200 mg, 703.47 µmol) was dissolved in tetrahydrofuran (10 mL), and then acrylamide (50.00 mg, 703.47 µmol) and potassium *tert-*butoxide (78.94 mg, 703.47 µmol) were added successively, and the mixture was stirred and reacted at 15 °C for 3 hours. After completion of the reaction, the reaction solution was added with water (50 mL) and extracted with ethyl acetate (20 mL × 3). The organic phase was combined, dried over anhydrous sodium sulfate, filtered, and concentrated under reduced pressure to remove the solvent. The resulting residue was purified by preparative HPLC (mobile phase: acetonitrile/water; neutral system: 10 mM NH₄HCO₃), further purified by preparative HPLC (mobile phase: acetonitrile/water; acid system: 0.05% HCl) to obtain the target compound **WX019**. MS-ESI *m*/*z:* 310.0 [M+H]⁺. ¹H NMR (400 MHz, DMSO_*d*₆) δ: 10.94 (s, 1H), 8.08 (d, *J* = 8.8 Hz, 1H), 7.96 (s, 1H), 7.85-7.70 (m, 2H), 7.50 (d, *J* = 1.6 Hz, 1H), 7.23 (dd, *J* = 2.4, 9.2 Hz, 1H), 4.63 (dd, *J* = 4.0, 12.0 Hz, 1H), 3.89 (s, 3H), 3.01-2.82 (m, 1H), 2.70-2.56 (m, 2H), 2.33-2.19 (m, 1H).

### Example 20: Hydrochloride of WX020

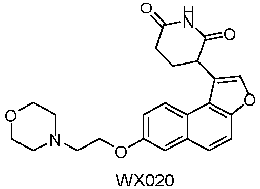

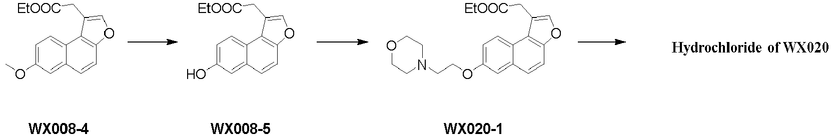

### Step 1: Synthesis of intermediate WX008-5

At 15 °C and under nitrogen atmosphere, the intermediate **WX008-4** (5 g, 17.59 mmol) was dissolved in dichloromethane (50 mL), the temperature was reduced to -60 °C, and boron tribromide (11.88 g, 47.43 mmol, 4.57 mL) was added, and the reaction mixture was returned to 15 °C and stirred and reacted for 2 hours. After completion of the reaction, the reaction solution was poured into ice water (200 mL) and extracted with dichloromethane (50 mL × 3). The organic phase was combined, dried over anhydrous sodium sulfate and filtered, and the filtrate was concentrated under reduced pressure to remove the solvent. The resulting residue was purified by column chromatography (eluent: petroleum ether/ethyl acetate = 1/0-4/1, volume ratio) to obtain the intermediate **WX008-5**. ¹H NMR (400 MHz, CDCl₃) δ: 8.14 (d, *J* = 9.2 Hz, 1H), 7.75 (s, 1H), 7.59 (q, *J* = 8.8 Hz, 2H), 7.36-7.25 (m, 1H), 7.19 (dd, *J* = 2.0, 8.8 Hz, 1H), 4.25 (q, *J* = 7.2 Hz, 2H), 4.06 (s, 2H), 1.28 (t, *J* = 7.0 Hz, 3H).

### Step 2: Synthesis of intermediate WX020-1

At 0 °C and under nitrogen atmosphere, the intermediate **WX008-5** (0.5 g, 1.85 mmol) was dissolved in tetrahydrofuran (15 mL), and then triphenylphosphine (630.79 mg, 2.40 mmol) and 2-morpholinoethanol (266.93 mg, 2.03 mmol) were added successively, the temperature was reduced to 0 °C, diisopropyl azodicarboxylate (486.30 mg, 2.40 mmol, 467.59 µL) was added dropwise, and the reaction mixture was returned to room temperature and stirred and reacted for 12 hours. After completion of the reaction, the reaction solution was directly concentrated under reduced pressure to remove the solvent and the resulting residue was purified by column chromatography (eluent: petroleum ether/ethyl acetate = 4/1-0/1, volume ratio) to obtain the intermediate **WX020-1.** MS-ESI *m*/*z*: 384.2 [M+H]⁺.

### Step 3: Synthesis of WX020

At 20 °C, the intermediate **WX020-1** (500 mg, 678.08 µmol, purity: 52%) was dissolved in tetrahydrofuran (20 mL), and then acrylamide (92.6 mg, 1.30 mmol) and the solution of potassium tert-butoxide (1 M, 1.30 mL) in tetrahydrofuran were added successively, and the reaction mixture was stirred and reacted at 20 °C for 2 hours. After completion of the reaction, the reaction solution was added dropwise with 4 M hydrogen chloride in ethyl acetate to adjust the pH to 5-6, and directly concentrated under reduced pressure to remove the solvent. The resulting residue was purified by preparative HPLC (mobile phase: acetonitrile/water; neutral system: 10 mM NH₄HCO₃), further purified by preparative HPLC (mobile phase: acetonitrile/water; acid system: 0.05% HCl) to obtain the hydrochloride of the target compound WX020. MS-ESI *m*/*z:* 409.2 [M+H]⁺. ¹H NMR (400 MHz, DMSO_*d*₆) δ: 11.26 (s, 1H), 10.95 (s, 1H), 8.12 (d, *J* = 8.4 Hz, 1H), 7.99 (s, 1H), 7.78 (s, 2H), 7.60 (d, *J* = 2.4 Hz, 1H), 7.30 (dd, *J* = 2.4, 9.2 Hz, 1H), 4.64 (dd, *J* = 4.2, 12.2 Hz, 1H), 4.57 (t, *J* = 4.6 Hz, 2H), 3.97 (d, *J* = 11.6 Hz, 2H), 3.84 (t, *J* = 11.8 Hz, 2H), 3.62 (s, 2H), 3.53 (d, *J* = 12.0 Hz, 2H), 3.31-3.16 (m, 2H), 2.94-2.81 (m, 1H), 2.70-2.58 (m, 1H), 2.45-2.35 (m, 1H), 2.34-2.20 (m, 1H).

### Example 21: Hydrochloride of WX021

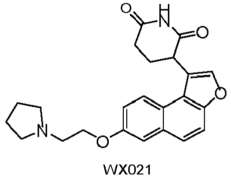

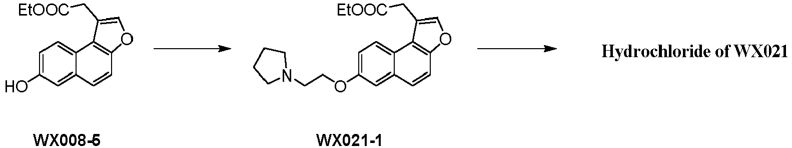

### Step 1: Synthesis of intermediate WX021-1

At 0 °C and under nitrogen atmosphere, the intermediate **WX008-5** (0.5 g, 1.85 mmol) was dissolved in tetrahydrofuran (15 mL), and then triphenylphosphine (630.79 mg, 2.40 mmol), N-(2-hydroxyethyl)-pyrrolidine (234.38 mg, 2.04 mmol) were added successively, the temperature was reduced to 0 °C, diisopropyl azodicarboxylate (486.30 mg, 2.40 mmol, 467.59 µL) was added dropwise, and the reaction mixture was returned to room temperature and stirred and reacted for 12 hours. After completion of the reaction, the reaction solution was directly concentrated under reduced pressure to remove the solvent. The resulting residue was purified by column chromatography (eluent: petroleum ether/ethyl acetate = 4/1-0/1, volume ratio) to obtain the intermediate **WX021-1.** ¹H NMR (400 MHz, CDCl₃) δ: 8.15 (d, *J* = 8.8 Hz, 1H), 7.75 (s, 1H), 7.63 (s, 2H), 7.34 (d, *J* = 2.8 Hz, 1H), 7.30 (dd, *J* = 2.4, 8.8 Hz, 1H), 4.27 (t, *J* = 6.0 Hz, 2H), 4.23 (q, *J* = 7.2 Hz, 2H), 4.06 (s, 2H), 3.01 (t, *J* = 6.0 Hz, 2H), 2.81-2.62 (m, 4H), 1.89-1.84 (m, 4H), 1.28 (t, *J* = 7.0 Hz, 3H).

### Step 2: Synthesis of WX021

At 20 °C, the intermediate **WX021-1** (250 mg, 680.39 µmol) was dissolved in tetrahydrofuran (10 mL), and then acrylamide (48.36 mg, 680.39 µmol) and the solution of potassium *tert*-butoxide (1 M, 680.39 µL) in tetrahydrofuran were added successively, and the reaction mixture was stirred and reacted at 20 °C for 2 hours. After completion of the reaction, the reaction solution was added with 4 M hydrogen chloride in ethyl acetate to adjust the pH to 5-6, and concentrated under reduced pressure to removed the solvent. The resulting residue was purified by preparative HPLC (mobile phase: acetonitrile/water; neutral system: 10 mM NH₄HCO₃), the resulting residue was further purified by preparative HPLC (mobile phase: acetonitrile/water; acid system: 0.05% HCl) to obtain the hydrochloride of the target compound **WX021.** MS-ESI *m*/*z:* 393.2 [M+H]⁺. ¹H NMR (400 MHz, DMSO_*d*₆) δ: 10.95 (s, 1H), 10.76 (s, 1H), 8.12 (d, *J* = 8.8 Hz, 1H), 7.99 (s, 1H), 7.78 (s, 2H), 7.59 (d, *J* = 2.4 Hz, 1H), 7.30 (dd, *J* = 2.8, 9.2 Hz, 1H), 4.64 (dd, *J* = 4.4, 12.0 Hz, 1H), 4.49 (t, *J* = 4.8 Hz, 2H), 3.70-3.56 (m, 4H), 3.21-3.08 (m, 2H), 2.95-2.81 (m, 1H), 2.70-2.58 (m, 1H), 2.45-2.31 (m, 1H), 2.30-2.21 (m, 1H), 2.10-1.83 (m, 4H).

### Example 22: Hydrochloride of WX022

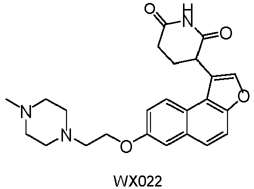

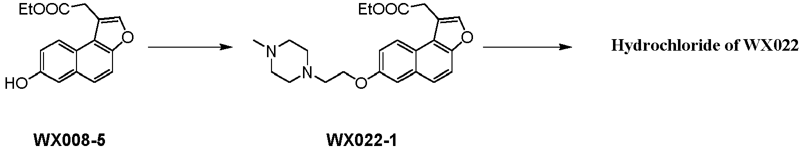

### Step 1: Synthesis of intermediate WX022-1

At 0 °C and under nitrogen atmosphere, the intermediate **WX008-5** (0.5 g, 1.85 mmol) was dissolved in tetrahydrofuran (15 mL), and then triphenylphosphine (630.79 mg, 2.40 mmol) and 1-hydroxyethyl-4-methylpiperazine (293.48 mg, 2.04 mmol) were added, the temperature was reduced to 0 °C, diisopropyl azodicarboxylate (486.30 mg, 2.40 mmol, 467.59 µL) was added dropwise, and the reaction mixture was slowly returned to room temperature and stirred and reacted for 12 hours. After completion of the reaction, the reaction solution was directly concentrated under reduced pressure to remove the solvent. The resulting residue was purified by column chromatography (eluent: petroleum ether/ethyl acetate = 1/1-0/1 to methanol/dichloromethane = 1/9, volume ratio) to obtain the intermediate **WX022-1.** ¹H NMR (400 MHz, CDCl₃) δ: 8.12 (d, *J* = 9.2 Hz, 1H), 7.72 (s, 1H), 7.60 (s, 2H), 7.30 (d, *J* = 2.4 Hz, 1H), 7.25 (dd, *J* = 2.6, 9.0 Hz, 1H), 4.24 (t, *J* = 6.0 Hz, 2H), 4.20 (q, *J* = 7.2 Hz, 2H), 4.03 (s, 2H), 2.89 (t, *J* = 5.8 Hz, 2H), 2.78-2.40 (m, 8H), 2.31 (s, 3H), 1.25 (t, *J* = 7.2 Hz, 3H).

### Step 2: Synthesis of WX022

At room temperature, the intermediate **WX022-1** (400 mg, 1.01 mmol) was dissolved in tetrahydrofuran (20 mL), and then acrylamide (71.71 mg, 1.01 mmol) and potassium *tert-*butoxide (113.21 mg, 1.01 mmol) were added, and the reaction mixture was stirred and reacted at room temperature for 2 hours. After completion of the reaction, the reaction solution was added dropwise with 4 M hydrogen chloride in ethyl acetate to adjust the pH to 5-6, and concentrated under reduced pressure to obtain a residue. The resulting residue was purified by preparative HPLC (mobile phase: acetonitrile/water; neutral system: 10 mM NH₄HCO₃), further purified by preparative HPLC (mobile phase: acetonitrile/water; acid system: 0.05% HCl) to obtain the hydrochloride of the target compound **WX022.** MS-ESI *m*/*z:* 422.2 [M+H]⁺. ¹H NMR (400 MHz, DMSO_*d*₆) δ: 11.91 (s, 1H), 10.95 (s, 1H), 8.11 (d, *J* = 8.8 Hz, 1H), 7.98 (s, 1H), 7.78 (s, 2H), 7.59 (d, *J* = 2.4 Hz, 1H), 7.32 (dd, *J* = 2.8, 9.2 Hz, 1H), 4.64 (dd, *J* = 4.4, 12.0 Hz, 1H), 4.57 (t, *J* = 4.0 Hz, 2H), 3.85-3.58 (m, 10H), 2.95-2.86 (m, 1H), 2.84 (s, 3H), 2.68-2.58 (m, 1H), 2.46-2.32 (m, 1H), 2.31-2.20 (m, 1H).

### Example 23: Hydrochloride of WX023

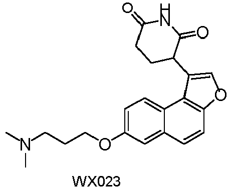

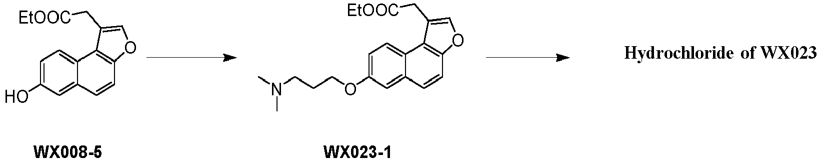

### Step 1: Synthesis of intermediate WX023-1

At room temperature and under nitrogen atmosphere, the intermediate **WX008-5** (0.5 g, 1.85 mmol) was dissolved in tetrahydrofuran (20 mL), and then 3-dimethylamino-1-propanol (209.94 mg, 2.04 mmol) and triphenylphosphine (630.81 mg, 2.40 mmol) were added successively, the temperature was reduced to 0 °C, diisopropyl azodicarboxylate (486.32 mg, 2.40 mmol, 467.61 µL) was added dropwise, and the reaction mixture was slowly returned to room temperature and stirred and reacted for 12 hours. After completion of the reaction, the reaction solution was directly concentrated under reduced pressure and the resulting residue was purified by column chromatography (eluent: petroleum ether/ethyl acetate = 1/1-0/1, then methanol/dichloromethane = 1/9, volume ratio) to obtain the intermediate **WX023-1.** ¹H NMR (400 MHz, CDCl₃) δ: 8.13 (d, *J* = 8.8 Hz, 1H), 7.73 (s, 1H), 7.61 (s, 2H), 7.32 (d, *J* = 2.8 Hz, 1H), 7.25 (dd, *J* = 2.4, 9.2 Hz, 1H), 4.22 (d, *J* = 7.2 Hz, 2H), 4.16 (t, *J* = 6.6 Hz, 2H), 4.04 (s, 2H), 2.54 (t, *J* = 7.2 Hz, 2H), 2.30 (s, 6H), 2.10-2.00 (m, 2H), 1.26 (t, *J* = 7.2 Hz, 3H).

### Step 2: Synthesis of WX023

At room temperature and under nitrogen atmosphere, the intermediate **WX023-1** (200 mg, 562.70 µmol) was dissolved in dry tetrahydrofuran (10 mL), and then acrylamide (40.00 mg, 562.70 µmol) and potassium tert-butoxide (63.14 mg, 562.70 µmol) were added, and the reaction mixture was stirred and reacted at room temperature for 2 hours. After completion of the reaction, the reaction solution was added dropwise with 4 M hydrogen chloride in ethyl acetate to adjust the pH to 5-6, and concentrated under reduced pressure to remove the solvent. The resulting residue was purified by preparative HPLC (mobile phase: acetonitrile/water; neutral system: 10 mM NH₄HCO₃), further purified by preparative HPLC (mobile phase: acetonitrile/water; acid system: 0.05% HCl) to obtain the hydrochloride of the target compound **WX023.** MS-ESI *m*/*z*: 381.2 [M+H]⁺. ¹H NMR (400 MHz, DMSO_*d*₆) δ: 10.94 (s, 1H), 10.33 (s, 1H), 8.10 (d, *J* = 8.8 Hz, 1H), 7.97 (s, 1H), 7.76 (s, 2H), 7.52 (d, *J* = 2.4 Hz, 1H), 7.23 (dd, *J* = 2.4, 9.2 Hz, 1H), 4.64 (dd, *J* = 4.0, 11.6 Hz, 1H), 4.20 (t, *J* = 5.8 Hz, 2H), 3.31-3.22 (m, 2H), 2.95-2.83 (m, 1H), 2.81 (s, 3H), 2.79 (s, 3H), 2.70-2.58 (m, 1H), 2.45-2.32 (m, 1H), 2.31-2.16 (m, 3H).

### Example 24: Hydrochloride of WX024

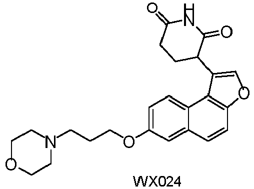

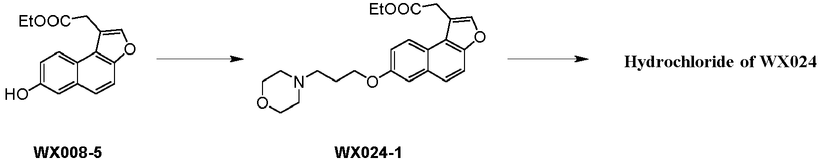

### Step 1: Synthesis of intermediate WX024-1

At room temperature and under nitrogen atmosphere, the intermediate **WX008-5** (0.5 g, 1.85 mmol) was dissolved in tetrahydrofuran (15 mL), and then triphenylphosphine (630.79 mg, 2.40 mmol) and 3-(4-morpholine)-1-propanol (349.21 mg, 2.40 mmol) were added successively, the temperature was reduced to 0 °C, diisopropyl azodicarboxylate (486.30 mg, 2.40 mmol, 467.59 µL) was added dropwise, and the reaction mixture was returned to room temperature and stirred and reacted for 12 hours. After completion of the reaction, the reaction solution was directly concentrated under reduced pressure to remove the solvent. The resulting residue was purified by column chromatography (eluent: petroleum ether/ethyl acetate = 3/2-1/4, volume ratio) to obtain the intermediate **WX024-1.** ¹H NMR (400 MHz, CDCl₃) δ: 8.13 (d, *J* = 9.2 Hz, 1H), 7.74 (s, 1H), 7.62 (s, 2H), 7.31 (d, *J* = 2.8 Hz, 1H), 7.24 (dd, *J* = 2.8, 9.2 Hz, 1H), 4.22 (q, *J* = 7.4 Hz, 2H), 4.17 (d, *J* = 6.4 Hz, 2H), 4.04 (s, 2H), 3.75 (t, *J* = 4.6 Hz, 4H), 2.59 (t, *J* = 7.2 Hz, 2H), 2.54-2.45 (m, 4H), 2.11-2.00 (m, 2H), 1.26 (t, *J* = 7.0 Hz, 3H).

### Step 2: Synthesis of WX024

At 20 °C, the intermediate **WX024-1 (350** mg, 880.58 µmol) was dissolved in tetrahydrofuran (10 mL), and then acrylamide (62.59 mg, 880.58 µmol) and potassium *tert-*butoxide (98.81 mg, 880.58 µmol) were added successively, and the mixture was stirred and reacted at 20 °C for 2 hours. After completion of the reaction, the reaction solution was added dropwise with 4 M hydrogen chloride in ethyl acetate to adjust the pH to 5-6, and concentrated under reduced pressure to remove the solvent. The resulting residue was purified by preparative HPLC (mobile phase: acetonitrile/water; neutral system: 10 mM NH₄HCO₃), further purified by preparative HPLC (mobile phase: acetonitrile/water; acid system: 0.05% HCl) to obtain the hydrochloride of the target compound **WX024.** MS-ESI *m*/*z:* 423.1 [M+H]⁺. ¹H NMR (400 MHz, DMSO_*d₆*) δ: 11.09 (s, 1H), 10.94 (s, 1H), 8.10 (d, *J* = 9.2 Hz, 1H), 7.97 (s, 1H), 7.76 (s, 2H), 7.53 (d, *J* = 2.4 Hz, 1H), 7.23 (dd, *J* = 2.4, 9.2 Hz, 1H), 4.64 (dd, *J* = 4.2, 11.8 Hz, 1H), 4.21 (t, *J* = 6.0 Hz, 2H), 3.97 (d, *J* = 10.4 Hz, 2H), 3.83 (t, *J* = 11.6 Hz, 2H), 3.52-3.46 (m, 2H), 3.35-3.25 (m, 2H), 3.16-3.03 (m, 2H), 2.93-2.81 (m, 1H), 2.69-2.57 (m, 1H), 2.43-2.22 (m, 4H).

### Example 25: WX025

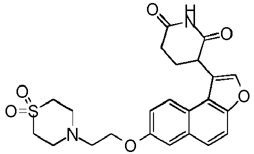

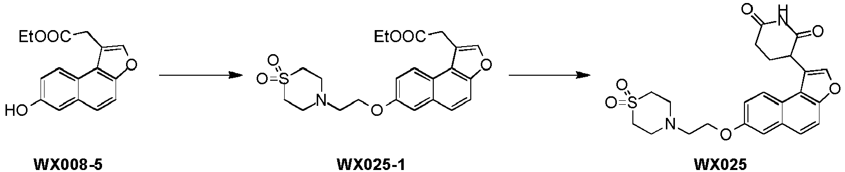

### Step 1: Synthesis of intermediate WX025-1

At room temperature and under nitrogen atmosphere, the intermediate **WX008-5** (0.5 g, 1.85 mmol) was dissolved in tetrahydrofuran (15 mL), and then triphenylphosphine (630.79 mg, 2.40 mmol) and 4-(2-hydroxyethyl)thiomorpholine-1,1-dioxide (464.22 mg, 2.59 mmol) were added successively, the temperature was reduced to 0 °C, diisopropyl azodicarboxylate (486.30 mg, 2.40 mmol, 467.59 µL) was added dropwise, and the reaction mixture was returned to room temperature and stirred and reacted for 12 hours. After completion of the reaction, the reaction solution was directly concentrated under reduced pressure to remove the solvent. The resulting residue was purified by column chromatography (eluent: petroleum ether/ethyl acetate = 3/2-3/7, volume ratio) to obtain the intermediate **WX025-1.**

### Step 2: Synthesis of WX025

At room temperature and under nitrogen atmosphere, the intermediate **WX025-1** (650 mg, 1.51 mmol) was dissolved in tetrahydrofuran (20 mL), and then acrylamide (107.07 mg, 1.51 mmol) and potassium *tert*-butoxide (169.03 mg, 1.51 mmol) were added successively, and the reaction mixture was stirred and reacted at room temperature for 1 hour. After completion of the reaction, the reaction solution was added dropwise with 4 M hydrogen chloride in ethyl acetate to adjust the pH to 5-6, and concentrated under reduced pressure to remove the solvent. The resulting residue was purified by preparative HPLC (mobile phase: acetonitrile/water; neutral system: 10 mM NH₄HCO₃), further purified by preparative HPLC (mobile phase: acetonitrile/water; acid system: 0.05% HCl) to obtain the target compound **WX025**. MS-ESI *mlz:* 457.1 [M+H]⁺. ¹H NMR (400 MHz, DMSO_*d*₆) δ: 10.95 (s, 1H), 8.11 (d, *J* = 8.4 Hz, 1H), 7.98 (s, 1H), 7.78 (s, 2H), 7.58 (d, *J* = 2.4 Hz, 1H), 7.31 (dd, *J* = 2.6, 9.0 Hz, 1H), 4.64 (dd, *J* = 4.4, 12.0 Hz, 1H), 4.57-4.49 (m, 2H), 3.88-3.75 (m, 4H), 3.74-3.64 (m, 6H), 2.94-2.81 (m, 1H), 2.70-2.58 (m, 1H), 2.45-2.35 (m, 1H), 2.31-2.20 (m, 1H).

### Example 26: Hydrochloride of WX026

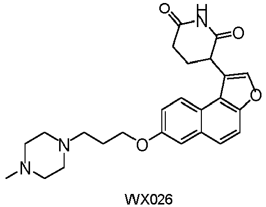

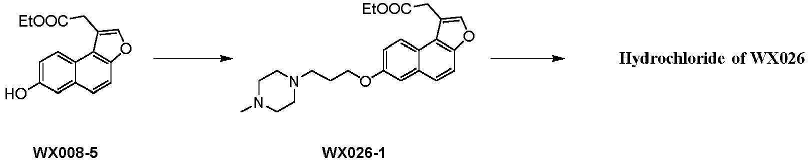

### Step 1: Synthesis of intermediate WX026-1

At room temperature and under nitrogen atmosphere, the intermediate **WX008-5** (0.5 g, 1.85 mmol) was dissolved in tetrahydrofuran (15 mL), and then triphenylphosphine (630.79 mg, 2.40 mmol) and 1-(3-hydroxypropyl)-4-methylpiperazine (409.84 mg, 2.59 mmol) were added successively, the temperature was reduced to 0 °C, diisopropyl azodicarboxylate (486.30 mg, 2.40 mmol, 467.59 µL) was added dropwise, and the reaction mixture was returned to room temperature and stirred and reacted for 12 hours. After completion of the reaction, the reaction solution was directly concentrated under reduced pressure to remove the solvent. The resulting residue was purified by column chromatography (eluent: petroleum ether/ethyl acetate = 1/1-0/1 to dichloromethane/methanol = 9/1, volume ratio) to obtain the intermediate **WX026-1.** ¹H NMR (400 MHz, CDCl₃) δ: 8.13 (d, *J* = 9.2 Hz, 1H), 7.73 (s, 1H), 7.61 (s, 2H), 7.30 (d, *J* = 2.4 Hz, 1H), 7.24 (dd, *J* = 2.6, 9.0 Hz, 1H), 4.22 (q, *J* = 7.0 Hz, 2H), 4.15 (t, *J* = 6.4 Hz, 2H), 4.04 (s, 2H), 2.60 (t, *J* = 7.4 Hz, 3H), 2.59-2.40 (m, 7H), 2.32 (s, 3H), 2.11-2.01 (m, 2H), 1.26 (t, *J* = 7.0 Hz, 3H).

### Step 2: Synthesis of WX026

At room temperature, the intermediate **WX026-1** (400 mg, 974.41 µmol) was dissolved in tetrahydrofuran (10 mL), and then acrylamide (69.26 mg, 974.41 µmol) and potassium tert-butoxide (109.34 mg, 974.41 µmol) were added successively, and the reaction mixture was stirred and reacted at room temperature for 2 hours. After completion of the reaction, the reaction solution was added dropwise with 4 M hydrogen chloride in ethyl acetate to adjust the pH to 5-6, and concentrated under reduced pressure to remove the solvent. The resulting residue was purified by preparative HPLC (mobile phase: acetonitrile/water; neutral system: 10 mM NH₄HCO₃), further purified by preparative HPLC (mobile phase: acetonitrile/water; acid system: 0.05% HCl) to obtain the hydrochloride of the target compound WX026. MS-ESI *mlz:* 436.1 [M+H]⁺. ¹H NMR (400 MHz, DMSO_*d*₆) δ: 11.94 (s, 1H), 10.94 (s, 1H), 8.10 (d, *J* = 8.8 Hz, 1H), 7.97 (s, 1H), 7.75 (s, 2H), 7.53 (d, *J* = 2.4 Hz, 1H), 7.24 (dd, *J* = 2.4, 8.8 Hz, 1H), 4.64 (dd, *J =* 4.4, 12.0 Hz, 1H), 4.22 (t, *J* = 5.8 Hz, 2H), 3.90-3.61 (m, 6H), 3.47-3.35 (m, 4H), 2.96-2.87 (m, 1H), 2.85 (s, 3H), 2.70-2.57 (m, 1H), 2.46-2.19 (m, 4H).

### Example 27: WX027

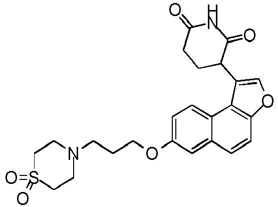

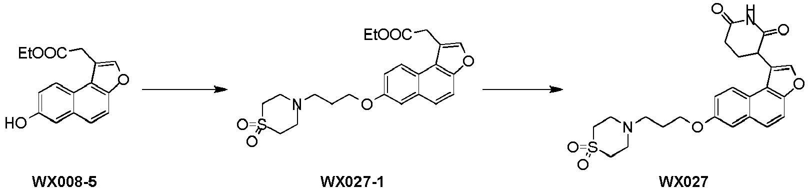

### Step 1: Synthesis of intermediate WX027-1

At room temperature and under nitrogen atmosphere, the intermediate **WX008-5** (1 g, 3.70 mmol) was dissolved in tetrahydrofuran (30 mL), and then triphenylphosphine (1.26 g, 4.81 mmol) and 4-(3-hydroxypropyl)thiomorpholine-1,1-dioxide (1.00 g, 5.18 mmol) were added successively, the temperature was reduced to 0 °C, diisopropyl azodicarboxylate (972.60 mg, 4.81 mmol, 935.19 µL) was added dropwise, the reaction mixture was returned to room temperature and stirred and reacted for 12 hours. After completion of the reaction, the reaction solution was directly concentrated under reduced pressure to remove the solvent. The resulting residue was purified by column chromatography (eluent: petroleum ether/ethyl acetate = 4/1-2/3, volume ratio), the resulting compound was further purified by preparative HPLC (mobile phase: acetonitrile/water; acid system: 0.05% HCl), the fraction was concentrated under reduced pressure to remove acetonitrile, adjusted with saturated sodium carbonate solution to achieve pH 6-7, and extracted with ethyl acetate (50 mL × 2). The organic phase was combined, dried over anhydrous sodium sulfate, filtered, and concentrated under reduced pressure to obtain the intermediate **WX027-1.** ¹H NMR (400 MHz, CDCl₃) δ: 8.14 (d, *J* = 9.2 Hz, 1H), 7.74 (s, 1H), 7.62 (s, 2H), 7.29 (d, *J* = 2.8 Hz, 1H), 7.23 (dd, *J* = 2.6, 9.0 Hz, 1H), 4.22 (q, *J* = 7.0 Hz, 2H), 4.16 (t, *J* = 6.0 Hz, 2H), 4.04 (s, 2H), 3.13-3.02 (m, 8H), 2.77 (t, *J* = 7.0 Hz, 2H), 2.05-1.99 (m, 2H), 1.26 (t, *J* = 7.2 Hz, 3H).

### Step 2: Synthesis of WX027

At room temperature and under nitrogen atmosphere, the intermediate **WX027-1** (200 mg, 448.91 µmol) was dissolved in tetrahydrofuran (5 mL), and then acrylamide (31.91 mg, 448.91 µmol) and potassium *tert*-butoxide (50.37 mg, 448.91 µmol) were added successively, and the reaction mixture was stirred and reacted at room temperature for 15 hours. After completion of the reaction, the reaction solution was added with 4 M hydrogen chloride in ethyl acetate to adjust the pH to 5-6, and concentrated under reduced pressure to remove the solvent. The resulting residue was purified by preparative HPLC (mobile phase: acetonitrile/water; neutral system: 10 mM NH₄HCO₃) to obtain the target compound **WX027.** MS-ESI *m*/*z:* 471.2 [M+H]⁺. ¹H NMR (400 MHz, DMSO_*d*₆) δ: 10.93 (s, 1H), 8.07 (d, *J* = 8.4 Hz, 1H), 7.96 (s, 1H), 7.79-7.70 (m, 2H), 7.50 (d, *J* = 2.4 Hz, 1H), 7.22 (dd, *J* = 2.6, 9.0 Hz, 1H), 4.62 (dd, *J* = 4.6, 11.8 Hz, 1H), 4.15 (t, *J* = 6.2 Hz, 2H), 3.13-3.04 (m, 4H), 2.98-2.89 (m, 4H), 2.88-2.80 (m, 1H), 2.72-2.63 (m, 3H), 2.43-2.34 (m, 1H), 2.29-2.21 (m, 1H), 2.01-1.89 (m, 2H).

### Example 28: Hydrochloride of WX028

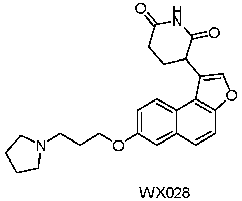

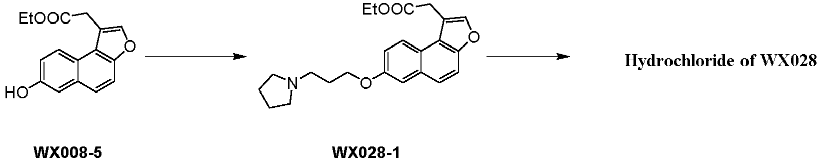

### Step 1: Synthesis of intermediate WX028-1

At room temperature and under nitrogen atmosphere, the intermediate **WX008-5** (1 g, 3.70 mmol) was dissolved in tetrahydrofuran (10 mL), and then triphenylphosphine (1.26 g, 4.81 mmol) and 3-(1-pyrrolidinyl)-1-propanol (525.82 mg, 4.07 mmol) were added successively, the temperature was reduced to 0 °C, diisopropyl azodicarboxylate (972.60 mg, 4.81 mmol, 935.19 µL) was added dropwise, and the reaction mixture was returned to room temperature and stirred and reacted for 12 hours. After completion of the reaction, the reaction solution was concentrated under reduced pressure to remove the solvent. The resulting residue was purified by column chromatography (eluent: petroleum ether/ethyl acetate = 1/1-1/2, dichloromethane/methanol = 20/1, volume ratio) to obtain the intermediate **WX028-1.** ¹H NMR (400 MHz, CDCl₃) δ: 8.13 (d, *J* = 8.8 Hz, 1H), 7.73 (s, 1H), 7.61 (s, 2H), 7.31 (d, *J* = 2.4 Hz, 1H), 7.24 (dd, *J* = 2.4, 9.2 Hz, 1H), 4.22 (q, *J* = 6.8 Hz, 2H), 4.18 (t, *J* = 6.6 Hz, 2H), 4.04 (s, 2H), 2.74 (t, *J* = 7.4 Hz, 2H), 2.69-2.57 (m, 4H), 2.21-2.09 (m, 2H), 1.90-1.78 (m, 4H), 1.26 (t, *J* = 7.2 Hz, 3H).

### Step 2: Synthesis of WX028

At room temperature and under nitrogen atmosphere, the intermediate **WX028-1** (460 mg, 1.21 mmol) was dissolved in N,N-dimethylformamide (5 mL), and then acrylamide (85.71 mg, 1.21 mmol) and potassium *tert*-butoxide (135.31 mg, 1.21 mmol) were added successively, and the reaction mixture was stirred and reacted at room temperature for 1 hour. After completion of the reaction, 2 M hydrochloric acid aqueous was added dropwise to the reaction solution to adjust the pH to 6-7. The resulting residue was purified by preparative HPLC (mobile phase: acetonitrile/water; neutral system: 10 mM NH₄HCO₃), further purified by preparative HPLC (mobile phase: acetonitrile/water; acid system: 0.05% HCl) to obtain the hydrochloride of the target compound **WX028**. MS-ESI *m*/*z:* 406.9 [M+H]⁺. ¹H NMR (400 MHz, DMSO_*d*₆) δ: 10.93 (s, 1H), 10.78 (s, 1H), 8.10 (d, *J* = 9.2 Hz, 1H), 7.97 (s, 1H), 7.76 (s, 2H), 7.53 (d, *J* = 2.8 Hz, 1H), 7.23 (dd, *J* = 2.6, 9.0 Hz, 1H), 4.64 (dd, *J* = 4.4, 12.0 Hz, 1H), 4.21 (t, *J* = 6.0 Hz, 2H), 3.64-3.51 (m, 2H), 3.33-3.27 (m, 2H), 3.11-2.97 (m, 2H), 2.93-2.81 (m, 1H), 2.70-2.57 (m, 1H), 2.47-2.31 (m, 1H), 2.30-2.17 (m, 3H), 2.08-1.95 (m, 2H), 1.94-1.83 (m, 2H).

### Example 29: WX029

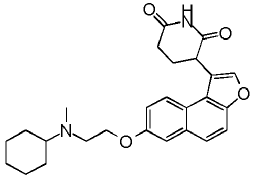

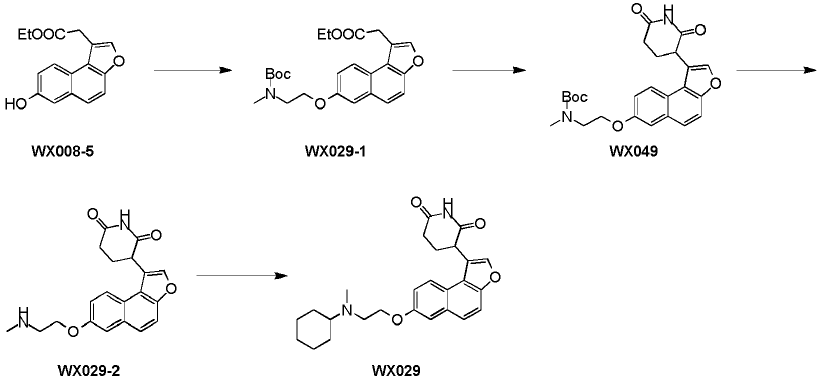

### Step 1: Synthesis of intermediate WX029-1

At room temperature and under nitrogen atmosphere, the intermediate **WX008-5** (6 g, 22.20 mmol) and N-Boc-N-methylaminoethanol (5.06 g, 28.86 mmol) were dissolved in tetrahydrofuran (100 mL), and triphenylphosphine (8.73 g, 33.30 mmol) was added, the temperature was reduced to 0 °C, diisopropyl azodicarboxylate (6.73 g, 33.30 mmol, 6.47 mL) was added dropwise, and the reaction mixture was warmed to room temperature and stirred and reacted for 12 hours. After completion of the reaction, the reaction mixture was concentrated under reduced pressure to remove the solvent. The resulting residue was purified by column chromatography (eluent: petroleum ether/ ethyl acetate = 1/0-4/1, volume ratio) to obtain the intermediate **WX029-1**. ¹H NMR (400 MHz, DMSO_*d*₆) δ: 8.05 (d, *J* = 8.8 Hz, 1H), 8.00 (s, 1H), 7.73 (s, 2H), 7.53 (d, *J* = 2.4 Hz, 1H), 7.25 (dd, *J* = 2.4, 9.2 Hz, 1H), 4.26-4.18 (m, 2H), 4.12 (q, *J* = 7.2 Hz, 2H), 4.10 (s, 2H), 3.60 (t, *J* = 4.8 Hz, 2H), 2.91 (d, *J* = 12.4 Hz, 3H), 1.35 (s, 9H), 1.17 (t, *J* = 7.2 Hz, 3H).

### Step 2: Synthesis of intermediate WX049

At room temperature and under nitrogen atmosphere, the intermediate **WX029-1** (4.1 g, 9.57 mmol) was dissolved in tetrahydrofuran (80 mL), the temperature was reduced to 0 °C, acrylamide (646.12 mg, 9.09 mmol) and potassium *tert*-butoxide (8.30 mL, 1 M) in tetrahydrofuran were added, and the reaction mixture was returned to room temperature and stirred and reacted for 1 hour. After completion of the reaction, the reaction solution was added with water (100 mL), and then extracted with ethyl acetate (200 mL × 3). The organic phase was combined, and the organic phase was washed with saturated brine (100 mL) successively, dried over anhydrous sodium sulfate, filtered, concentrated under reduced pressure to remove the solvent to obtain a residue. The resulting residue was purified by column chromatography (eluent: petroleum ether/ ethyl acetate = 1/0-2/3, volume ratio) to obtain the intermediate **WX049**. ¹H NMR (400 MHz, DMSO_*d*₆) δ: 10.94 (s, 1H), 8.09 (d, *J* = 8.8 Hz, 1H), 7.96 (s, 1H), 7.75 (s, 2H), 7.53 (d, *J* = 2.4 Hz, 1H), 7.21 (dd, *J* = 2.4, 9.2 Hz, 1H), 4.63 (dd, *J* = 4.4, 12.0 Hz, 1H), 4.27-4.17 (m, 2H), 3.67-3.55 (m, 2H), 2.94-2.86 (m, 3H), 2.71-2.53 (m, 2H), 2.42-2.20 (m, 2H), 1.36 (s, 9H).

### Step 3: Synthesis of intermediate WX029-2

At room temperature, the intermediate **WX049** (4.4 g, 9.72 mmol) was dissolved in ethyl acetate (10 mL), hydrogen chloride in ethyl acetate (100 mL, 4 M) was added, and the reaction mixture was stirred and reacted at room temperature for 2 hours. After completion of the reaction, the reaction solution was filtered. The filter cake was collected and concentrated under reduced pressure to remove the solvent to obtain the hydrochloride of the intermediate **WX029-2.** ¹H NMR (400 MHz, DMSO_*d*₆) δ: 10.93 (s, 1H), 8.95 (s, 2H), 8.14 (d, *J* = 9.2 Hz, 1H), 7.98 (s, 1H), 7.78 (s, 2H), 7.58 (d, *J* = 2.4 Hz, 1H), 7.28 (dd, *J* = 2.4, 8.8 Hz, 1H), 4.64 (dd, *J* = 4.4, 11.6 Hz, 1H), 4.39 (t, *J* = 5.0 Hz, 2H), 3.40 (t, *J* = 5.0 Hz, 2H), 2.92-2.83 (m, 1H), 2.66 (s, 3H), 2.62-2.58 (m, 1H), 2.46-2.32 (m, 1H), 2.31-2.22 (m, 1H).

### Step 4: Synthesis of WX029

At room temperature, the intermediate **WX029-2** (100 mg, 257.17 µmol, hydrochloride) was dissolved in 1,2-dichloroethane (4 mL), cyclohexanone (25.24 mg, 257.17 umol, 26.65 µL) and sodium acetate (105.48 mg, 1.29 mmol) were added, and the reaction mixture was warmed to 50 °C and stirred and reacted for 30 minutes. Sodium borohydride acetate (109.01 mg, 514.35 µmol) was added, and the reaction mixture was warmed to 50 °C and stirred and reacted for 12 hours. Additional Sodium borohydride acetate (109.01 mg, 514.35 µmol) was added, and the reaction mixture was warmed to 75 °C and stirred and reacted for 2 hours. After completion of the reaction, the reaction mixture was cooled to room temperature, and concentrated under reduced pressure to remove the solvent. The resulting residue was purified by preparative HPLC (mobile phase: acetonitrile/water; acid system: 0.05% HCl). The target compound **WX029** was obtained. MS-ESI *m*/*z*: 435.1 [M+H]⁺. ¹H NMR (400 MHz, DMSO_*d*₆) δ: 10.94 (s, 1H), 8.13 (d, *J* = 8.8 Hz, 1H), 7.99 (s, 1H), 7.78 (s, 2H), 7.59 (d, *J* = 2.8 Hz, 1H), 7.28 (dd, *J* = 2.6, 9.0 Hz, 1H), 4.64 (dd, *J* = 4.4, 11.6 Hz, 1H), 4.59-4.49 (m, 2H), 3.78-3.63 (m, 1H), 3.33-3.25 (m, 1H), 2.93-2.85 (m, 1H), 2.84-2.79 (m, 3H), 2.69-2.58 (m, 2H), 2.44-2.22 (m, 2H), 2.16-2.02 (m, 2H), 1.89-1.78 (m, 2H), 1.68-1.57 (m, 1H), 1.53-1.40 (m, 2H), 1.36-1.24 (m, 2H), 1.21-1.05 (m, 1H).

### Example 30: Hydrochloride of WX030

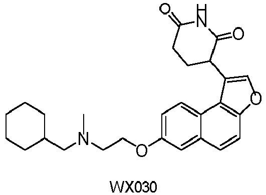

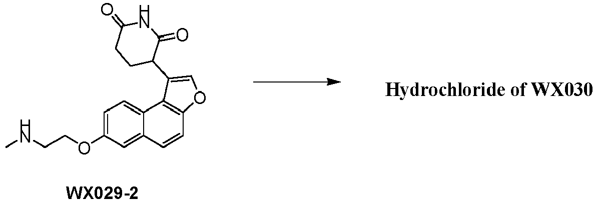

At room temperature, the intermediate **WX029-2** (100 mg, 257.17 µmol, hydrochloride) was dissolved in 1,2-dichloroethane (4 mL), and cyclohexylformaldehyde (57.69 mg, 514.35 µmol) and sodium acetate (21.10 mg, 257.17 µmol) were added, and the reaction mixture was stirred and reacted at room temperature for 30 minutes. Sodium borohydride acetate (109.01 mg, 514.35 µmol) was added, and the reaction mixture was stirred and reacted at room temperature for additional 1 hour. After completion of the reaction, the reaction mixture was concentrated under reduced pressure to remove the solvent. The resulting residue was purified by preparative HPLC (mobile phase: acetonitrile/water; acidic system: 0.05% HCl) to obtain the hydrochloride of the target compound **WX030.** MS-ESI *m*/*z:* 449.1 [M+H]⁺. ¹H NMR (400 MHz, DMSO_*d*₆) δ: 10.94 (s, 1H), 10.19 (s, 1H), 8.13 (d, *J* = 9.2 Hz, 1H), 7.98 (s, 1H), 7.78 (s, 2H), 7.59 (d, *J* = 2.4 Hz, 1H), 7.26 (dd, *J* = 2.8, 9.2 Hz, 1H), 4.65 (dd, *J* = 4.2, 11.8 Hz, 1H), 4.60-4.48 (m, 2H), 3.67-3.51 (m, 2H), 3.19-3.08 (m, 1H), 3.01-2.94 (m, 1H), 2.93-2.81 (m, 4H), 2.69-2.59 (m, 1H), 2.46-2.34 (m, 1H), 2.31-2.22 (m, 1H), 1.97-1.78 (m, 3H), 1.75-1.55 (m, 3H), 1.34-1.06 (m, 3H), 1.04-0.85 (m, 2H).

### Example 31: WX031

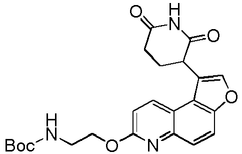

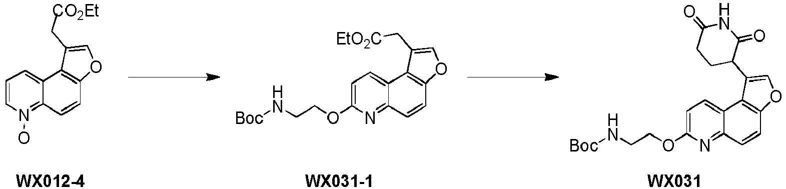

### Step 1: Synthesis of intermediate WX031-1

At room temperature and under nitrogen atmosphere, the intermediate **WX012-4** (4 g, 14.75 mmol) was dissolved in carbon tetrachloride (40 mL), and N-Boc-ethanolamine (4.75 g, 29.49 mmol, 4.57 mL) and N,N-diisopropyl ethylamine (7.62 g, 58.98 mmol) were added, and then the solution of diethyl phosphite (8.15 g, 58.98 mmol) in acetonitrile (40 mL) was added dropwise to the reaction solution, and the reaction mixture was warmed to 80 °C and stirred and reacted for 12 hours. After completion of the reaction, the reaction solution was cooled to room temperature and concentrated under reduced pressure to remove the solvent. The resulting residue was purified by column chromatography (eluent: petroleum ether/ethyl acetate = 1/0-5/1, volume ratio) to obtain the intermediate **WX031-1.** MS-ESI *m*/*z:* 415.2 [M+H]⁺.

### Step 2: Synthesis of WX031

At room temperature and under nitrogen atmosphere, the intermediate **WX031-1** (100 mg, 241.28 µmol) was dissolved in tetrahydrofuran (2 mL), the temperature was reduced to 0 °C, and acrylamide (15.43 mg, 217.15 µmol) and the solution of potassium *tert*-butoxide (1 M, 217.15 µL) in tetrahydrofuran were added successively, and the reaction mixture was returned to 20 °C and stirred and reacted for 2 hours. After completion of the reaction, the reaction solution was poured into water (15 mL), diluted by adding 2-methyltetrahydrofuran (10 mL). The organic phase was collected by separation, and the aqueous phase was extracted with 2-methyltetrahydrofuran (15 mL × 3). The organic phase was combined, washed with saturated brine (10 mL × 2), dried over anhydrous sodium sulfate and filtered, and the filtrate was concentrated under reduced pressure to remove the solvent. The resulting residue was purified by preparative HPLC (mobile phase: acetonitrile/water; neutral system: 10 mM NH₄HCO₃) to obtain the target compound **WX031.** MS-ESI *m*/*z:* 440.1 [M+H]⁺. ¹H NMR (400 MHz, DMSO *d₆*) δ: 10.93 (s, 1H), 8.51 (d, *J* = 9.2 Hz, 1H), 8.05 (s, 1H), 7.94 (d, *J* = 9.2 Hz, 1H), 7.70 (d, *J* = 8.8 Hz, 1H), 7.04 (d, *J* = 9.2 Hz, 2H), 4.64 (dd, *J* = 4.4, 12.6 Hz, 1H), 4.41 (t, *J* = 5.6 Hz, 2H), 3.42-3.38 (m, 2H), 2.91-2.79 (m, 1H), 2.69-2.59 (m, 1H), 2.45-2.35 (m, 1H), 2.34-2.21 (m, 1H), 1.38 (s, 9H).

### Example 32: WX032

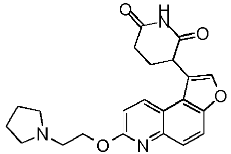

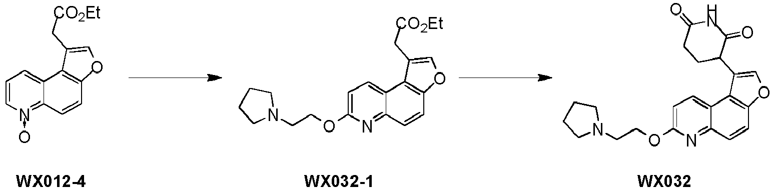

### Step 1: Synthesis of intermediate WX032-1

At 20 °C and under nitrogen atmosphere, the intermediate **WX012-4** (500 mg, 1.84 mmol) was dissolved in carbon tetrachloride (5 mL), and N-hydroxyethylpyrrolidine (424.57 mg, 3.69 mmol) and N,N-diisopropylethylamine (952.86 mg, 7.37 mmol) were added, then the solution of diethyl phosphite (1.02 g, 7.37 mmol) in acetonitrile (5 mL) was added dropwise to the reaction, and the reaction mixture was warmed to 80 °C and stirred and reacted for 12 hours. After completion of the reaction, the reaction solution was cooled to room temperature and concentrated under reduced pressure to remove the solvent. The resulting residue was purified by preparative HPLC (mobile phase: acetonitrile/water; neutral system: 0.2% FA) to obtain the intermediate **WX032-1**. ¹H NMR (400 MHz, DMSO_*d*₆) δ: 8.50 (d, *J* = 9.2 Hz, 1H), 8.15-8.08 (m, 1H), 7.97 (d, *J* = 8.8 Hz, 1H), 7.72 (d, *J* = 9.2 Hz, 1H), 7.17 (d, *J* = 9.2 Hz, 1H), 4.70 (t, *J* = 4.8 Hz, 2H), 4.16-4.08 (m, 4H), 4.02-3.97 (m, 2H), 3.80-3.71 (m, 2H), 1.96-1.87 (m, 4H), 1.20-1.16 (m, 5H).

### Step 2: Synthesis of WX032

At 20 °C and under nitrogen atmosphere, the intermediate **WX032-1** (70 mg, 190.00 µmol) was dissolved in N,N-dimethylformamide (1 mL), and then acrylamide (13.50 mg, 190.00 µmol) and the solution of potassium *tert*-butoxide (1 M, 190.00 µL) in tetrahydrofuran were added successively, and the reaction mixture was stirred and reacted at 20 °C for 3 hours. After completion of the reaction, the reaction solution was added dropwise with 2 N of dilute aqueous hydrochloric acid solution to adjust the pH to 6-7 and filtered, and the filtrate was collected. The resulting filtrate was purified by preparative HPLC (mobile phase: acetonitrile/water; neutral system: 10 mM NH₄HCO₃) to obtain the target compound **WX032**. ¹H NMR (400 MHz, DMSO_*d*₆) δ: 10.93 (s, 1H), 8.51 (d, *J =* 9.2 Hz, 1H), 8.05 (s, 1H), 7.94 (d, *J* = 9.2 Hz, 1H), 7.71 (d, *J =* 9.2 Hz, 1H), 7.07 (d, *J =* 8.8 Hz, 1H), 4.64 (dd, *J =* 4.0, 12.0 Hz, 1H), 4.59-4.52 (m, 2H), 3.00-2.79 (m, 3H), 2.72-2.58 (m, 5H), 2.46-2.21 (m, 2H), 1.76-1.66 (m, 4H).

### Example 33: WX033

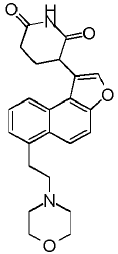

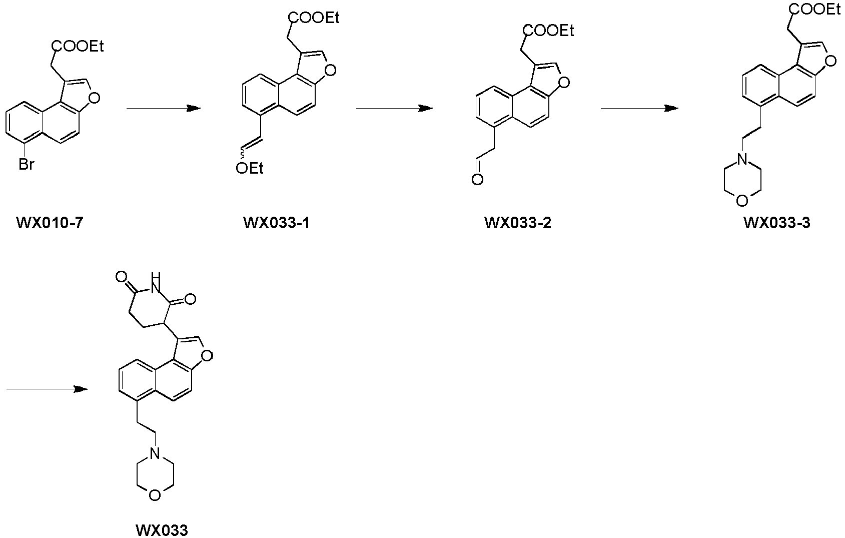

### Step 1: Synthesis of intermediate WX033-1

At room temperature and under nitrogen atmosphere, the intermediate **WX010-7** (1 g, 3.00 mmol) was dissolved in N,N-dimethylformamide (10 mL), and then potassium phosphate (637.10 mg, 3.00 mmol), (*E*)-1-ethoxyvinyl-2-boronic acid pinacol ester (772.82 mg, 3.90 mmol) and [1,1-bis(diphenylphosphine)ferrocene]palladium dichloride dichloromethane (245.11 mg, 300.14 µmol) were added successively, and the reaction mixture was warmed to 80 °C and stirred and reacted for 12 hours. After completion of the reaction, the reaction solution was poured into water (200 mL) and extracted with ethyl acetate (150 mL × 3). The organic phase was combined, washed with half-saturated brine (150 mL × 3), dried over anhydrous sodium sulfate and filtered, and the filtrate was concentrated under reduced pressure to remove the solvent. The resulting residue was purified by column chromatography (eluent: petroleum ether/ethyl acetate = 1/0-50/1, volume ratio) to obtain the intermediate **WX033-1.** MS-ESI *m*/*z*: 325.1 [M+H]⁺.

### Step 2: Synthesis of intermediate WX033-2

At room temperature and under nitrogen atmosphere, the intermediate **WX033-1** (0.7 g, 2.16 mmol) was dissolved in chloroform (7 mL), the temperature was reduced to 0 °C, and then ethanol (99.42 mg, 2.16 mmol, 126.16 µL), water (2.16 mmol, 38.88 µL) and oxalyl chloride (273.91 mg, 2.16 mmol, 188.90 µL) were added successively, and the reaction mixture was returned to room temperature and stirred and reacted for 1 hour. After completion of the reaction, the reaction solution was added with water (20 mL), adjusted with saturated sodium bicarbonate solution to pH 7, and extracted with dichloromethane (10 mL × 3). The organic phase was combined, washed with saturated brine (20 mL × 3) successively, dried over anhydrous sodium sulfate and filtered, and the filtrate (30 mL) was directly used in the next reaction to obtain a solution of the intermediate **WX033-2** in dichloromethane.

### Step 3: Synthesis of intermediate WX033-3

At room temperature and under nitrogen atmosphere, morpholine (365.90 mg, 4.20 mmol) was added to the solution of the intermediate **WX033-2** in dichloromethane (0.07 M, 30 mL), and the reaction mixture was stirred and reacted at room temperature for 10 minutes. Then sodium triacetoxyborohydride (890.15 mg, 4.20 mmol) was added, and the reaction mixture was stirred and reacted at room temperature for 12 hours. After completion of the reaction, the reaction solution was added with water (100 mL), and extracted with dichloromethane (50 mL × 3). The organic phase was combined, washed with saturated brine (50 mL × 3) successively, dried over anhydrous sodium sulfate and filtered, and the filtrate was concentrated under reduced pressure to remove the solvent. The resulting residue was purified by column chromatography (eluent: petroleum ether/ethyl acetate = 20/1-0/1, volume ratio), and the resulting residue was further purified by preparative HPLC (mobile phase: acetonitrile/water; neutral system: 10 mM NH₄HCO₃) to obtain the intermediate **WX033-3.** ¹H NMR (400 MHz, DMSO_*d*₆) δ: 8.04 (s, 1H), 8.01 (t, *J* = 7.4 Hz, 2H), 7.82 (d, *J* = 9.2 Hz, 1H), 7.51 (t, *J* = 7.8 Hz, 1H), 7.42 (d, *J* = 6.8 Hz, 1H), 4.23-4.06 (m, 4H), 3.61 (t, *J* = 4.4 Hz, 4H), 3.27 (t, *J* = 7.8 Hz, 2H), 2.62 (t, *J* = 7.8 Hz, 2H), 2.53-2.49 (m, 4H), 1.18 (t, *J* = 7.2 Hz, 3H).

### Step 4: Synthesis of WX033

At room temperature and under nitrogen atmosphere, the intermediate **WX033-3** (120 mg, 326.59 µmol) was dissolved in N,N-dimethylformamide (2 mL), and then acrylamide (23.21 mg, 326.59 µmol) and potassium *tert*-butoxide (36.65 mg, 326.59 µmol) were added successively, and the reaction mixture was stirred and reacted at room temperature for 1 hour. After completion of the reaction, the reaction solution was added with water (50 mL), and extracted with ethyl acetate (30 mL × 3). The organic phase was combined, washed with saturated brine (30 mL × 2) successively, dried over anhydrous sodium sulfate and filtered, and the filtrate was concentrated under reduced pressure to remove the solvent. The resulting residue was purified by preparative HPLC (mobile phase: acetonitrile/water; neutral system: 10 mM NH₄HCO₃) to obtain the target compound **WX033.** MS-ESI m/z: 393.2 [M+H]⁺. ¹H NMR (400 MHz, DMSO_*d*₆) δ: 10.95 (s, 1H), 8.06 (d, *J* = 7.6 Hz, 1H), 8.03-7.97 (m, 2H), 7.83 (d, *J* = 9.2 Hz, 1H), 7.50 (t, *J* = 7.8 Hz, 1H), 7.42 (d, *J* = 6.8 Hz, 1H), 4.68 (dd, *J* = 4.4, 12.0 Hz, 1H), 3.61 (t, *J* = 4.4 Hz, 4H), 3.31-3.23 (m, 5H), 2.93-2.84 (m, 1H), 2.69-2.60 (m, 3H), 2.47-2.35 (m, 2H), 2.31-2.22 (m, 1H).

### Example 34: WX034

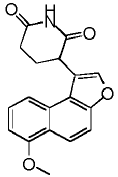

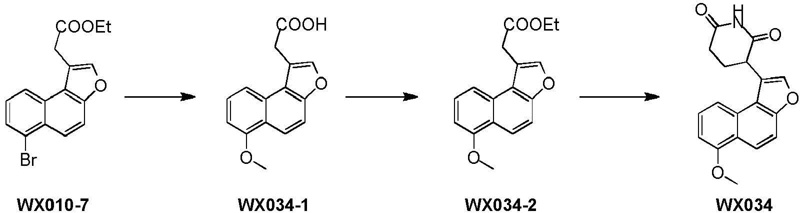

### Step 1: Synthesis of intermediate WX034-1

At room temperature and under nitrogen atmosphere, the intermediate **WX010-7** (5 g, 15.01 mmol) was dissolved in methanol (25 mL) and toluene (25 mL), and then cesium carbonate (7.33 g, 22.51 mmol), 2-di-*tert*-butylphosphine-2,4,6-triisopropylbiphenyl (382.36 mg, 900.42 µmol) and palladium acetate (101.08 mg, 450.21 µmol) were added successively, and the reaction mixture was warmed to 80 °C and stirred and reacted for 12 hours. After completion of the reaction, the reaction solution was added with water (200 mL), extracted with ethyl acetate (150 mL), and the organic phase was discarded. The aqueous phase was adjusted to pH 5 with concentrated hydrochloric acid (12 M), then the aqueous phase was extracted with ethyl acetate (150 mL × 3). The organic phase was combined, washed with saturated brine (150 mL × 3) successively, dried over anhydrous sodium sulfate and filtered, and the filtrate was concentrated under reduced pressure to remove the solvent. The intermediate **WX034-1** was obtained. MS-ESI *m*/*z:* 257.1 [M+H]⁺.

### Step 2: Synthesis of intermediate WX034-2

At room temperature and under nitrogen atmosphere, the intermediate **WX034-1** (2.8 g, 10.93 mmol) was dissolved in ethanol (28 mL), then concentrated sulfuric acid (1.07 g, 10.93 mmol, 582.43 µL, purity: 98%) was added, and the reaction mixture was warmed to 80 °C and stirred and reacted for 3 hours. After completion of the reaction, the reaction solution was directly concentrated under reduced pressure to remove the solvent. The resulting residue was purified by column chromatography (eluent: petroleum ether/ethyl acetate = 49/1-19/1, volume ratio) to obtain the intermediate **WX034-2.** ¹H NMR (400 MHz, CDCl₃) δ: 8.25 (d, *J=* 9.2 Hz, 1H), 7.81 (d, *J* = 8.4 Hz, 1H), 7.76 (s, 1H), 7.64 (d, *J* = 9.2 Hz, 1H), 7.50 (t*, J* = 8.2 Hz, 1H), 6.87 (d, *J* = 7.6 Hz, 1H), 4.24 (q, *J* = 7.2 Hz, 2H), 4.07 (s, 2H), 4.03 (s, 3H), 1.27 (t, *J* = 7.0 Hz, 3H).

### Step 3: Synthesis of WX034

At room temperature and under nitrogen atmosphere, the intermediate **WX034-2** (60 mg, 211.04 µmol) was dissolved in N,N-dimethylformamide (2 mL), and then acrylamide (15.00 mg, 211.04 µmol) and potassium *tert*-butoxide (23.68 mg, 211.04 µmol) were added successively, and the reaction mixture was stirred and reacted at room temperature for 1 hour. After completion of the reaction, the reaction solution was added with water (50 mL), and extracted with ethyl acetate (30 mL × 3). The organic phase was combined, washed with half-saturated brine (30 mL × 3) successively, dried over anhydrous sodium sulfate and filtered, and the filtrate was concentrated under reduced pressure to remove the solvent. The resulting residue was purified by preparative HPLC (mobile phase: acetonitrile/water; acid system: 0.04% HCl) to obtain the target compound **WX034.** MS-ESI *m*/*z:* 310.1 [M+H]⁺. ¹H NMR (400 MHz, DMSO_*d*₆) δ: 10.95 (s, 1H), 8.15 (d, *J* = 9.6 Hz, 1H), 8.00 (s, 1H), 7.78-7.70 (m, 2H), 7.51 (t, *J* = 8.0 Hz, 1H), 7.02 (d, *J* = 8.0 Hz, 1H), 4.65 (dd, *J* = 4.4, 12.0 Hz, 1H), 4.00 (s, 3H), 2.98-2.82 (m, 1H), 2.68-2.59 (m, 1H), 2.46-2.33 (m, 1H), 2.31-2.20 (m, 1H).

### Example 35: WX035

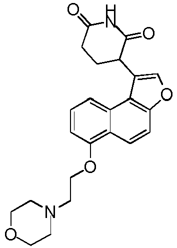

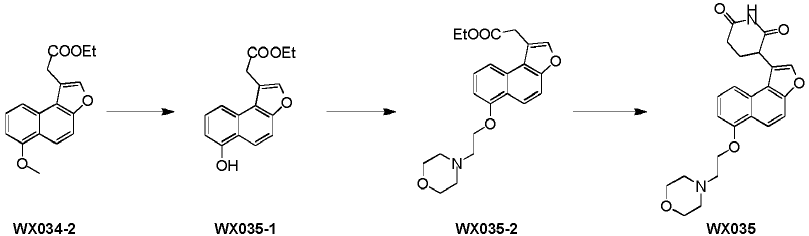

### Step 1: Synthesis of intermediate WX035-1

At room temperature and under nitrogen atmosphere, the intermediate **WX034-2** (2 g, 7.03 mmol) was dissolved in dichloromethane (40 mL), the temperature was reduced to -78 °C, and then boron tribromide (2.11 g, 8.44 mmol, 813.39 µL) was added dropwise, and the reaction mixture was returned to room temperature and stirred for 2 hours. Then the reaction mixture was cooled to -78 °C, boron tribromide (1.76 g, 7.03 mmol, 677.83 µL) was added dropwise, and the reaction mixture was returned to room temperature and stirred and reacted for 2 hours. After completion of the reaction, the reaction solution was poured into ice water (200 mL) and extracted with dichloromethane (150 mL × 3). The organic phase was combined, washed with saturated brine (150 mL × 3), dried over anhydrous sodium sulfate and filtered, and the filtrate was concentrated under reduced pressure to remove the solvent. The resulting residue was purified by column chromatography (eluent: petroleum ether/ethyl acetate = 9/1-4/1, volume ratio) to obtain the intermediate **WX035-1.** ¹H NMR (400 MHz, CDCl₃) δ: 8.14 (d, *J* = 9.2 Hz, 1H), 7.80 (d, *J* = 8.4 Hz, 1H), 7.76 (s, 1H), 7.63 (d, *J* = 9.2 Hz, 1H), 7.39 (t, *J* = 7.8 Hz, 1H), 6.81 (d, *J* = 7.6 Hz, 1H), 5.49 (s, 1H), 4.24 (q, *J* = 7.0 Hz, 2H), 4.07 (s, 2H), 1.27 (t, *J* = 7.2 Hz, 3H).

### Step 2: Synthesis of intermediate WX035-2

At room temperature and under nitrogen atmosphere, the intermediate **WX035-1** (300 mg, 1.11 mmol) was dissolved in tetrahydrofuran (3 mL), and then triphenylphosphine (378.47 mg, 1.44 mmol) and N-(2-hydroxyethyl)morpholine (189.28 mg, 1.44 mmol) were added successively, the temperature was reduced to 0 °C, diisopropyl azodicarboxylate (291.78 mg, 1.44 mmol, 280.56 µL) was added dropwise, and the reaction mixture was returned to room temperature and stirred and reacted for 12 hours. After completion of the reaction, the reaction solution was added with water (100 mL), and extracted with ethyl acetate (50 mL × 3). The organic phase was combined, washed with saturated brine (50 mL × 3) successively, dried over anhydrous sodium sulfate and filtered, and the filtrate was concentrated under reduced pressure to remove the solvent. The resulting residue was purified by column chromatography (eluent: petroleum ether/ethyl acetate = 10/1-0/1, volume ratio) to obtain the intermediate **WX035-2.** MS-ESI m/z: 384.3 [M+H]⁺.

### Step 3: Synthesis of WX035

At room temperature and under nitrogen atmosphere, the intermediate **WX035-2** (400 mg, 1.04 mmol) was dissolved in N,N-dimethylformamide (2 mL), then acrylamide (74.15 mg, 1.04 mmol) and potassium tert-butoxide (117.06 mg, 1.04 mmol) were added successively, and the reaction mixture was stirred and reacted at room temperature for 3 hours. After completion of the reaction, the reaction solution was added with water (50 mL), and extracted with ethyl acetate (30 mL × 3). The organic phase was combined, washed with half-saturated brine (30 mL × 2) successively, dried over anhydrous sodium sulfate and filtered, and the filtrate was concentrated under reduced pressure to remove the solvent. The resulting residue was purified by preparative HPLC (mobile phase: acetonitrile/water; neutral system: 10 mM NH₄HCO₃) to obtain the target compound **WX035.** MS-ESI *m*/*z:* 409.2 [M+H]⁺. ¹H NMR (400 MHz, DMSO_*d*₆) δ: 10.95 (s, 1H), 8.16 (d, *J* = 9.2 Hz, 1H), 8.00 (s, 1H), 7.76 (d, *J* = 9.2 Hz, 1H), 7.73 (d, *J* = 8.0 Hz, 1H), 7.49 (t, *J* = 8.2 Hz, 1H), 7.03 (d, *J* = 8.0 Hz, 1H), 4.64 (dd, *J* = 4.2, 11.8 Hz, 1H), 4.31 (t, *J* = 5.8 Hz, 2H), 3.60 (t, *J* = 4.6 Hz, 4H), 2.93-2.83 (m, 3H), 2.68-2.61 (m, 1H), 2.58-2.55 (m, 3H), 2.46-2.34 (m, 2H), 2.30-2.22 (m, 1H).

### Example 36: WX036

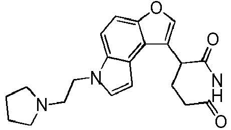

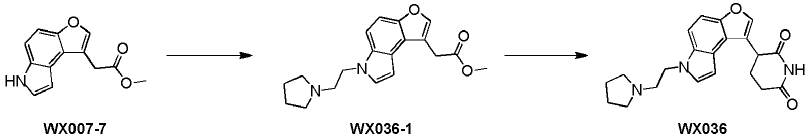

### Step 1: Synthesis of intermediate WX036-1

At 20 °C, the intermediate **WX007-7** (1 g, 4.36 mmol) was dissolved in N,N-dimethylformamide (15 mL), then 1-(2-chloroethyl)pyrrolidine (1.48 g, 8.72 mmol) and potassium carbonate (2.23 g, 16.14 mmol) were added, and the reaction mixture was stirred and reacted at 20 °C for 12 hours. Additional 1-(2-chloroethyl)pyrrolidine (741.96 mg, 4.36 mmol) and potassium carbonate (1.12 g, 8.07 mmol) were added, and the reaction mixture was stirred and reacted at 20 °C for 16 hours. Additional 1-(2-chloroethyl)pyrrolidine (741.96 mg, 4.36 mmol) and potassium carbonate (1.12 g, 8.07 mmol) were added, and the reaction mixture was stirred and reacted at 20 °C for 36 hours. After completion of the reaction, the reaction solution was poured into half-saturated brine (50 mL), diluted by addeing ethyl acetate (50 mL). The organic phase was collected by separation, and the aqueous phase was extracted with ethyl acetate (50 mL × 3). The organic phase was combined, washed with half-saturated brine (80 mL × 3), saturated brine (80 mL × 2) successively, dried over anhydrous sodium sulfate and filtered, and the filtrate was concentrated under reduced pressure to remove the solvent. The resulting residue was purified by preparative HPLC (mobile phase: acetonitrile/water; acid system: 0.05% HCl), the resulting crude product was further purified by preparative HPLC (mobile phase: acetonitrile/water; neutral system: 10 mM NH₄HCO₃) to obtain the intermediate **WX036-1.** ¹H NMR (400 MHz, DMSO_*d*₆) δ: 7.85 (s, 1H) 7.44 (d, *J* = 4.9 Hz, 1H) 7.42 (d, *J* = 8.8 Hz, 1H) 7.34 (d, *J* = 9.2 Hz, 1H) 6.55 (d, *J* = 3.2 Hz, 1H) 4.33 (t, *J* = 6.8 Hz, 2H) 3.93 (s, 2H) 3.64 (s, 3H) 2.79 (t, *J* = 6.6 Hz, 2H), 2.48-2.42 (m, 4H) 1.68-1.61 (m, 4H).

### Step 2: Synthesis of WX036

At 0 °C and under nitrogen atmosphere, the intermediate **WX036-1** (49 mg, 150.13 µmol) was dissolved in tetrahydrofuran (1 mL), and acrylamide (10.67 mg, 150.13 µmol) and the solution of potassium *tert*-butoxide (1 M, 150.13 µL) in tetrahydrofuran were added successively, and the reaction mixture was stirred at 20 °C for 3 hours. After completion of the reaction, water (2 mL) was added to the reaction solution, ethyl acetate (2 mL) was added for dilution, the organic phase was collected by separation, and the aqueous phase was extracted with ethyl acetate (3 mL × 3). The organic phase was combined, washed with saturated brine (10 mL × 2), dried over anhydrous sodium sulfate and filtered, and the filtrate was concentrated under reduced pressure to remove the solvent to obtain a residue. The resulting residue was purified by preparative HPLC (mobile phase: acetonitrile/water; neutral system: 10 mM NH₄HCO₃) to obtain the target compound **WX036.** MS-ESI *m*/*z:* 366.1 [M+H]⁺. ¹H NMR (400 MHz, DMSO_*d₆*) δ: 10.97 (s, 1H), 7.83 (s, 1H), 7.45 (s, 1H), 7.42 (d, *J* = 5.2 Hz, 1H), 7.35 (d, *J* = 9.2 Hz, 1H), 6.45 (d, *J* = 3.2 Hz, 1H), 4.33 (t, *J* = 6.8 Hz, 2H), 4.28 (dd, *J* = 5.2, 12.0 Hz, 1H), 2.89-2.82 (m, 1H), 2.79 (t, *J* = 6.6 Hz, 2H), 2.64-2.55 (m, 1H), 2.48-2.43 (m, 4H), 2.36-2.24 (m, 1H), 2.19-2.09 (m, 1H), 1.67-1.62 (m, 4H).

### Example 37: WX037

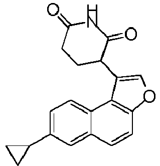

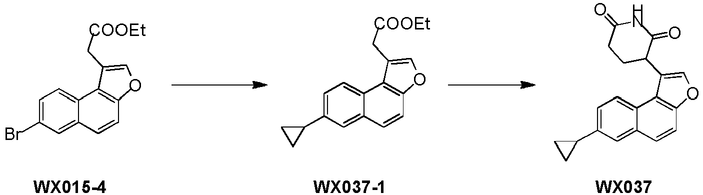

### Step 1: Synthesis of intermediate WX037-1

At room temperature and under nitrogen atmosphere, the intermediate **WX015-4** (500 mg, 1.50 mmol) was dissolved in 1,4-dioxane (15 mL), and then potassium cyclopropyltrifluoroborate (444.14 mg, 3.00 mmol), tetratriphenylphosphine palladium (86.71 mg, 75.04 µmol) and sodium carbonate (556.71 mg, 5.25 mmol) were added successively, and the reaction mixture was heated to 110 °C and stirred and reacted for 12 hours. After completion of the reaction, the reaction mixture was cooled to room temperature and filtered. The filtrate was collected and concentrated under reduced pressure to remove the solvent. The resulting residue was purified by column chromatography (eluent: petroleum ether/ethyl acetate = 1/0-19/1, volume ratio), and the resulting compound was further purified by preparative HPLC (mobile phase: acetonitrile/water; acid system: 0.05% HCl), concentrated under reduced pressure to remove most of acetonitrile, and extracted with ethyl acetate (20 mL × 3). The organic phase was combined, dried over anhydrous sodium sulfate and filtered, and the filtrate was concentrated under reduced pressure to remove the solvent to obtain the intermediate **WX037-1**. ¹H NMR (400 MHz, CDCl₃) δ: 8.12 (d, *J* = 8.8 Hz, 1H), 7.74 (s, 1H), 7.68-7.59 (m, 3H), 7.30 (dd, *J* = 1.8, 8.6 Hz, 1H), 4.22 (q, *J* = 7.0 Hz, 2H), 4.05 (s, 2H), 2.14-2.05 (m, 1H), 1.27 (t, *J* = 7.2 Hz, 3H), 1.10-0.99 (m, 2H), 0.87-0.75 (m, 2H).

### Step 2: Synthesis of WX037

At room temperature, the intermediate **WX037-1** (150 mg, 509.61 µmol) was dissolved in N,N-dimethylformamide (3 mL), and then acrylamide (36.22 mg, 509.61 µmol) and potassium tert-butoxide (57.18 mg, 509.61 µmol) were added successively, and the reaction mixture was stirred and reacted at room temperature for 1 hour. After completion of the reaction, the reaction solution was added with water (20 mL), and extracted with ethyl acetate (20 mL × 3). The organic phase was combined, dried over anhydrous sodium sulfate, filtered, and concentrated under reduced pressure to obtain a residue, and the residue was purified by preparative HPLC (mobile phase: acetonitrile/water; neutral system: 10 mM NH₄HCO₃) to obtain the target compound **WX037.** MS-ESI *m*/*z:* 320.2 [M+H]⁺. ¹H NMR (400 MHz, DMSO_*d*₆) δ: 10.95 (s, 1H), 8.04 (d, *J* = 8.8 Hz, 1H), 7.97 (s, 1H), 7.82-7.66 (m, 3H), 7.29 (dd, *J* = 1.6, 8.8 Hz, 1H), 4.63 (dd, *J* = 4.4, 12.0 Hz, 1H), 2.94-2.82 (m, 1H), 2.70-2.59 (m, 1H), 2.43-2.34 (m, 1H), 2.30-2.20 (m, 1H), 2.14-2.05 (m, 1H), 1.06-0.97 (m, 2H), 0.82-0.75 (m, 2H).

### Example 38: WX038

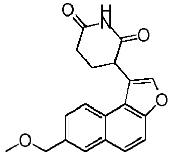

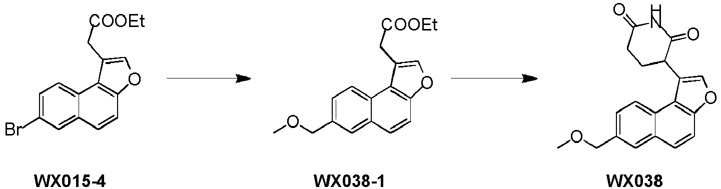

### Step 1: Synthesis of intermediate WX038-1

At room temperature and under nitrogen atmosphere, the intermediate **WX015-4** (500 mg, 1.50 mmol) was dissolved in water (0.5 mL) and 1,4-dioxane (5 mL), and then potassium methoxy-methyltrifluoroborate salt (456.11 mg, 3.00 mmol), palladium acetate (33.69 mg, 150.07 µmol), cesium carbonate (1.47 g, 4.50 mmol) and 2-dicyclohexylphosphine-2,6-diisopropoxy-1,1-biphenyl (140.06 mg, 300.14 µmol) were added successively, and the reaction mixture was heated to 100 °C and stirred and reacted for 12 hours. After completion of the reaction, the reaction solution was added with water (20 mL) and extracted with ethyl acetate (30 mL × 3). The organic phase was combined, dried over anhydrous sodium sulfate and filtered, and the filtrate was concentrated under reduced pressure to remove the solvent. The resulting residue was purified by column chromatography (eluent: petroleum ether/ethyl acetate = 1/0-19/1, volume ratio) to obtain the intermediate **WX038-1.** ¹H NMR (400 MHz, CDCl₃) δ: 8.22 (d, *J* = 8.4 Hz, 1H), 7.91 (s, 1H), 7.77 (s, 1H), 7.73 (d, *J* = 9.2 Hz, 1H), 7.65 (d, *J* = 9.2 Hz, 1H), 7.57 (dd, *J* = 1.4, 8.6 Hz, 1H), 4.66 (s, 2H), 4.23 (q, *J* = 7.0 Hz, 2H), 4.08 (s, 2H), 3.45 (s, 3H), 1.27 (t, *J* = 7.0 Hz, 3H).

### Step 2: Synthesis of WX038

At room temperature and under nitrogen atmosphere, the intermediate **WX038-1** (130 mg, 435.76 µmol) was dissolved in N,N-dimethylformamide (2 mL), and then acrylamide (30.97 mg, 435.76 µmol) and potassium tert-butoxide (48.90 mg, 435.76 µmol) were added successively, and the reaction mixture was stirred and reacted at room temperature for 2 hours. After completion of the reaction, the reaction solution was added with water (20 mL) and extracted with ethyl acetate (20 mL × 3). The organic phase was combined, dried over anhydrous sodium sulfate and filtered, and the filtrate was concentrated under reduced pressure to remove the solvent. The resulting residue was purified by preparative HPLC (mobile phase: acetonitrile/water; neutral system: 10 mM NH₄HCO₃) to obtain the target compound **WX038.** MS-ESI *m*/*z*: 324.0 [M+H]⁺. ¹H NMR (400 MHz, CDCl₃) δ: 8.11 (s, 1H), 7.98 (d, *J* = 8.4 Hz, 1H), 7.94 (s, 1H), 7.84-7.53 (m, 4H), 4.66 (s, 2H), 4.57-4.46 (m, 1H), 3.46 (s, 3H), 2.92-2.72 (m, 2H), 2.61-2.38 (m, 2H).

### Example 39: WX039

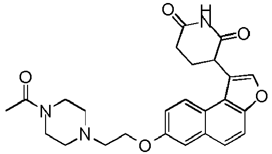

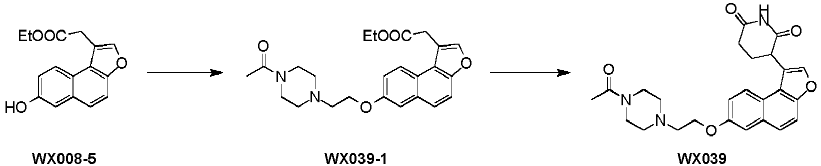

### Step 1: Synthesis of intermediate WX039-1

At room temperature and under nitrogen atmosphere, the intermediate **WX008-5** (0.5 g, 1.85 mmol) was dissolved in tetrahydrofuran (15 mL), and then triphenylphosphine (630.79 mg, 2.40 mmol), 1-acetyl-4-(2-hydroxyethyl)piperazine (414.19 mg, 2.40 mmol) and 4 Å molecular sieve (0.2 g) were added successively, the temperature was reduced to 0 °C, diisopropyl azodicarboxylate (486.30 mg, 2.40 mmol, 467.59 µL) was added dropwise, and the reaction mixture was slowly returned to room temperature and stirred and reacted for 12 hours. After completion of the reaction, the reaction solution was directly concentrated under reduced pressure to remove the solvent, and the resulting residue was purified by column chromatography (eluent: petroleum ether/ethyl acetate = 1/1-0/1 to methanol/dichloromethane = 1/9, volume ratio) to obtain the intermediate **WX039-1.** MS-ESI *m*/*z:* 425.2 [M+H]⁺._{.}

### Step 2: Synthesis of WX039

At room temperature, the intermediate **WX039-1** (200 mg, 365.62 µmol) was dissolved in tetrahydrofuran (4 mL), and then acrylamide (33.49 mg, 471.17 µmol) and the solution of potassium tert-butoxide (1 M, 471.17 µL) in tetrahydrofuran were added, and the reaction mixture was stirred at room temperature for 1 hour. After completion of the reaction, the reaction solution was added dropwise with 4 M hydrogen chloride in ethyl acetate to adjust the pH to 6-7, and concentrated under reduced pressure to remove the solvent. The resulting residue was purified by preparative HPLC (mobile phase: acetonitrile/water; neutral system: 10 mM NH₄HCO₃) to obtain the target compound **WX039.** MS-ESI m/z: 450.2 [M+H]⁺. ¹H NMR (400 MHz, DMSO_*d*₆) δ: 10.93 (s, 1H), 8.08 (d, *J* = 9.2 Hz, 1H), 7.96 (s, 1H), 7.75 (s, 2H), 7.53 (d, *J* = 2.4 Hz, 1H), 7.22 (dd, *J* = 2.4, 9.2 Hz, 1H), 4.63 (dd, *J* = 4.4, 11.6 Hz, 1H), 4.23 (t, *J* = 5.6 Hz, 2H), 3.50-3.39 (m, 5H), 2.93-2.83 (m, 1H), 2.80 (t, *J* = 5.4 Hz, 2H), 2.69-2.54 (m, 2H), 2.46-2.22 (m, 4H), 1.99 (s, 3H).

### Example 40: WX040

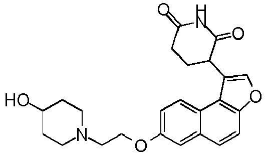

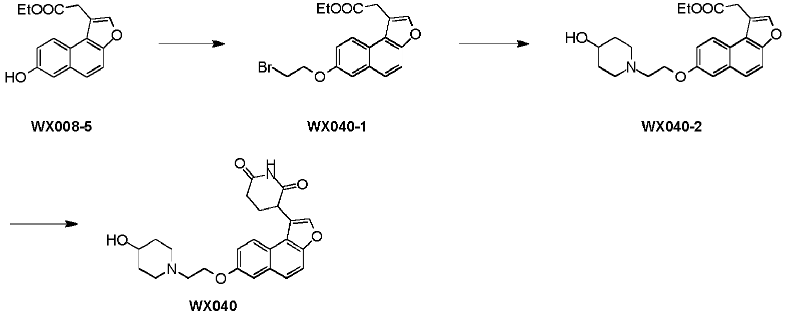

### Step 1: Synthesis of intermediate WX040-1

At room temperature and under nitrogen atmosphere, the intermediate **WX008-5** (3 g, 11.10 mmol) was dissolved in tetrahydrofuran (50 mL), and then triphenylphosphine (3.78 g, 14.43 mmol) and 2-bromoethanol (1.80 g, 14.43 mmol, 1.02 mL) were added successively, the temperature was reduced to 0 °C, diisopropyl azodicarboxylate (2.92 g, 14.43 mmol, 2.81 mL) was added dropwise, and the reaction mixture was slowly returned to room temperature and stirred and reacted for 12 hours. The reaction mixture was warmed to 40 °C and stirred and reacted for 3 hours. After completion of the reaction, the reaction solution was directly concentrated under reduced pressure to remove the solvent, and the resulting residue was purified by column chromatography (eluent: petroleum ether/ethyl acetate = 9/1-4/1, volume ratio) to obtain the intermediate **WX040-1.** ¹H NMR (400 MHz, CDCl₃) δ: 8.18 (d, *J* = 9.2 Hz, 1H), 7.77 (s, 1H), 7.68-7.61 (m, 2H), 7.34-7.28 (m, 2H), 4.46 (t, *J* = 6.2 Hz, 2H), 4.24 (q, *J* = 7.0 Hz, 2H), 4.06 (s, 2H), 3.74 (t, *J* = 6.4 Hz, 2H), 1.28 (t, *J* = 7.0 Hz, 3H).

### Step 2: Synthesis of intermediate WX040-2

At room temperature, the intermediate **WX040-1** (300 mg, 795.27 µmol) was dissolved in N,N-dimethylformamide (3 mL), and then potassium carbonate (384.69 mg, 2.78 mmol), potassium iodide (66.01 mg, 397.64 µmol) and 4-hydroxypiperidine (160.88 mg, 1.59 mmol) were added successively, and the reaction mixture was stirred and reacted at room temperature for 2 hours. After completion of the reaction, the reaction solution was directly filtered, the filtrate was collected, and the obtained filtrate was directly purified by preparative HPLC (mobile phase: acetonitrile/water; acid system: 0.05% HCl) to obtain the hydrochloride of the intermediate **WX040-2.** ¹H NMR (400 MHz, D₂O) δ: 7.86-7.71 (m, 1H), 7.65 (s, 1H), 7.53 (s, 2H), 7.27 (s, 1H), 7.12 (d, *J* = 8.8 Hz, 1H), 4.38-4.27 (m, 2H), 4.22-4.05 (m, 3H), 4.00-3.82 (m, 3H), 3.66 (d, *J* = 12.8 Hz, 1H), 3.57-3.42 (m, 3H), 3.38-3.27 (m, 1H), 3.10 (t, *J* = 12.2 Hz, 1H), 2.16 (d, *J* = 13.6 Hz, 1H), 2.05-1.85 (m, 2H), 1.82-1.67 (m, 1H), 1.16 (t, *J* = 7.0 Hz, 3H).

### Step 3: Synthesis of WX040

At room temperature and under nitrogen atmosphere, the intermediate **WX040-2** (260 mg, 599.18 µmol, hydrochloride) was dissolved in N,N-dimethylformamide (5 mL), and then acrylamide (42.59 mg, 599.18 µmol) and potassium tert-butoxide (134.47 mg, 1.20 mmol) were added successively, and the reaction mixture was stirred and reacted at room temperature for 1 hour. After completion of the reaction, the reaction solution was directly added with 1 M hydrochloric acid aqueous to adjust the pH to 6-7, and the resulting solution was directly purified by preparative HPLC (mobile phase: acetonitrile/water; neutral system: 10 mM NH₄HCO₃) to obtain the target compound **WX040.** MS-ESI *m*/*z:* 423.2 [M+H]⁺. ¹H NMR (400 MHz, DMSO_*d₆*) δ: 10.94 (s, 1H), 8.08 (d, *J* = 8.8 Hz, 1H), 8.01-7.90 (m, 1H), 7.75 (s, 2H), 7.54 (d, *J* = 2.4 Hz, 1H), 7.23 (dd, *J* = 2.2, 9.0 Hz, 1H), 4.63 (dd, *J* = 4.6, 11.4 Hz, 2H), 4.24 (s, 2H), 3.62-3.43 (m, 1H), 2.96-2.79 (m, 4H), 2.71-2.58 (m, 1H), 2.44-2.17 (m, 5H), 1.83-1.68 (m, 2H), 1.52-1.37 (m, 2H).

### Example 41: WX041

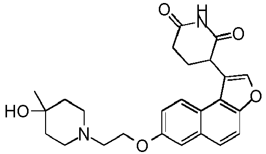

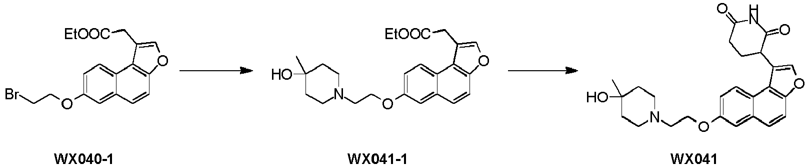

### Step 1: Synthesis of intermediate WX041-1

At room temperature, the intermediate **WX040-1** (230 mg, 609.71 µmol) was dissolved in N,N-dimethylformamide (3 mL), and then potassium carbonate (379.21 mg, 2.74 mmol), potassium iodide (50.61 mg, 304.86 µmol) and 4-methyl-4-hydroxypiperidine (184.91 mg, 1.22 mmol, hydrochloride) were added successively, and the reaction mixture was stirred and reacted at room temperature for 3 hours. After completion of the reaction, the reaction solution was directly filtered, the filtrate was collected, and the obtained filtrate was directly purified by preparative HPLC (mobile phase: acetonitrile/water; acid system: 0.05% HCl) to obtain the hydrochloride of the intermediate **WX041-1.**

### Step 2: Synthesis of WX041

At room temperature and under nitrogen atmosphere, the intermediate **WX041-1** (180 mg, 401.83 µmol, hydrochloride) was dissolved in N,N-dimethylformamide (3 mL), and then acrylamide (28.56 mg, 401.83 µmol) and potassium *tert*-butoxide (90.18 mg, 803.66 µmol) were added successively, and the reaction mixture was stirred and reacted at room temperature for 1 hour. After completion of the reaction, the reaction solution was added dropwise with 2 M hydrochloric acid aqueous to adjust the pH to 6-7, and the resulting solution was directly purified by preparative HPLC (mobile phase: acetonitrile/water; neutral system: 10 mM NH₄HCO₃) to obtain the target compound **WX041.** MS-ESI *m*/*z:* 437.1 [M+H]⁺. ¹H NMR (400 MHz, CDCl₃) δ: 8.12 (s, 1H), 7.89 (d, *J* = 9.2 Hz, 1H), 7.70-7.59 (m, 3H), 7.37-7.28 (m, 2H), 4.56-4.43 (m, 1H), 4.26 (t, *J* = 5.8 Hz, 2H), 2.93 (t, *J* = 5.6 Hz, 2H), 2.84-2.70 (m, 4H), 2.64-2.40 (m, 4H), 1.80-1.66 (m, 4H), 1.28 (s, 3H).

### Example 42: WX042

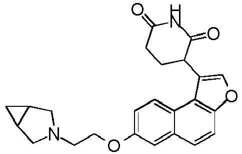

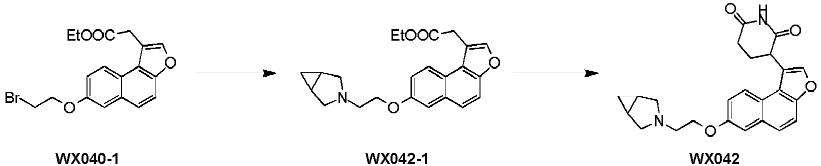

### Step 1: Synthesis of intermediate WX042-1

At room temperature, the intermediate **WX040-1** (500 mg, 1.33 mmol) was dissolved in N,N-dimethylformamide (5 mL), and 3-azabicyclo[3.1.0]hexane (165.28 mg, 1.38 mmol, hydrochloride) and potassium carbonate (824.34 mg, 5.96 mmol) were addedsuccessively, and the reaction mixture was stirred and reacted at room temperature for 12 hours. After completion of the reaction, the reaction solution was added with water (100 mL) and extracted with ethyl acetate (150 mL × 3). The organic phase was combined, dried over anhydrous sodium sulfate and filtered, and the filtrate was concentrated under reduced pressure to remove the solvent. The resulting residue was purified by column chromatography (eluent: petroleum ether/ethyl acetate = 5/1-1/1, volume ratio) to obtain the intermediate **WX042-1.** ¹H NMR (400 MHz, CDCl₃) δ: 8.11 (d, *J* = 9.2 Hz, 1H), 7.72 (s, 1H), 7.60 (s, 2H), 7.29 (s, 1H), 7.23 (d, *J* = 8.8 Hz, 1H), 4.23-4.17 (m, 2H), 4.11 (q, *J* = 7.2 Hz, 4H), 4.02 (s, 2H), 3.29-3.17 (m, 2H), 2.67-2.58 (m, 2H), 1.47-1.37 (m, 3H), 0.95-0.78 (m, 2H), 0.49-0.36 (m, 1H), 0.06 (s, 1H).

### Step 2: Synthesis of WX042

At room temperature and under nitrogen atmosphere, the intermediate **WX042-1** (0.1330 g, 350.51 µmol) was dissolved in N,N-dimethylformamide (1 mL), and then acrylamide (24.91 mg, 350.51 µmol) and potassium tert-butoxide (39.33 mg, 350.51 µmol) were added successively, and the reaction mixture was stirred and reacted at room temperature for 1 hour. After completion of the reaction, hydrochloric acid aqueous (2 M) was added to the reaction solution to adjust the pH to 5-6. The resulting residue was purified by preparative HPLC (mobile phase: acetonitrile/water; neutral system: 10 mM NH₄HCO₃) to obtain the target compound **WX042.** MS-ESI *m*/*z:* 404.9 [M+H]⁺. ¹H NMR (400 MHz, CDCl₃) δ: 8.12 (s, 1H), 7.88 (d, *J* = 8.8 Hz, 1H), 7.67-7.63 (m, 2H), 7.32 (d, *J* = 2.4 Hz, 1H), 7.26-7.24 (m, 1H), 4.49 (dd, *J* = 5.2, 8.8 Hz, 1H), 4.19 (t, *J* = 6.0 Hz, 2H), 3.14 (d, *J* = 8.8 Hz, 2H), 2.95 (t, *J* = 6.0 Hz, 2H), 2.84-2.71 (m, 2H), 2.57-2.38 (m, 4H), 1.44-1.34 (m, 2H), 0.78-0.69 (m, 1H), 0.43-0.34 (m, 1H).

### Example 43: WX043

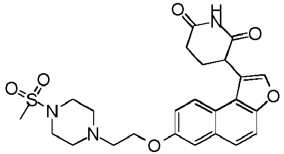

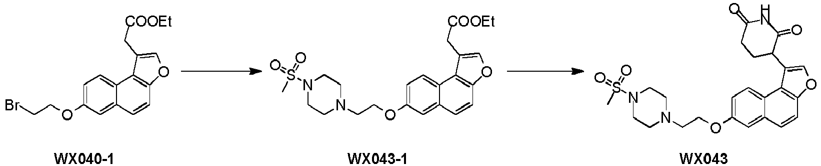

### Step 1: Synthesis of intermediate WX043-1

At room temperature and under nitrogen atmosphere, the intermediate **WX040-1** (0.5 g, 1.33 mmol) was dissolved in N,N-dimethylformamide (5 mL), and then potassium iodide (110.01 mg, 662.73 µmol) and 1-methanesulfonylpiperazine (435.35 mg, 2.65 mmol) were added successively, and the reaction mixture was stirred and reacted at room temperature for 12 hours. After completion of the reaction, the reaction solution was added with water (100 mL), and extracted with ethyl acetate (50 mL × 3). The organic phase was combined, washed with saturated brine (50 mL × 3) successively, dried over anhydrous sodium sulfate, filtered, and the filtrate was concentrated under reduced pressure to remove the solvent. The resulting residue was purified by column chromatography (eluent: petroleum ether/ethyl acetate = 20/1-1/1, volume ratio) to obtain the intermediate **WX043-1.** ¹H NMR (400 MHz, CDCl₃) δ: 8.15 (d, *J* = 9.2 Hz, 1H), 7.74 (s, 1H), 7.62 (s, 2H), 7.30 (d, *J* = 2.4 Hz, 1H), 7.26-7.23 (m, 1H), 4.25 (t, *J* = 6.0 Hz, 2H), 4.22 (q, *J* = 7.6 Hz, 2H), 4.04 (s, 2H), 3.30 (t, *J* = 5.0 Hz, 4H), 2.95 (t, *J* = 5.4 Hz, 2H), 2.79 (s, 3H), 2.76 (t, *J* = 8.8 Hz, 4H), 1.26 (t, *J* = 7.2 Hz, 3H).

### Step 2: Synthesis of WX043

At room temperature and under nitrogen atmosphere, the intermediate **WX043-1** (0.3 g, 651.41 µmol) was dissolved in N,N-dimethylformamide (3 mL), and then acrylamide (46.30 mg, 651.41 µmol) and potassium *tert*-butoxide (73.10 mg, 651.41 µmol) were added successively, and the reaction mixture was stirred at room temperature for 1 hour. After completion of the reaction, the pH of the reaction solution was adjusted to 6-7 with concentrated hydrochloric acid (12 M). The resulting residue was purified by preparative HPLC (mobile phase: acetonitrile/water; neutral system: 10 mM NH₄HCO₃) to obtain the target compound **WX043.** MS-ESI *m*/*z:* 486.1 [M+H]⁺. ¹H NMR (400 MHz, DMSO_*d₆*) δ: 10.89 (s, 1H), 8.03 (d, *J* = 9.2 Hz, 1H), 7.91 (s, 1H), 7.70 (s, 2H), 7.48 (d, *J* = 2.4 Hz, 1H), 7.18 (dd, *J* = 2.8, 9.2 Hz, 1H), 4.58 (dd, *J* = 4.2, 12.2 Hz, 1H), 4.18 (t, *J* = 5.8 Hz, 2H), 3.08 (t, *J* = 5.0 Hz, 4H), 2.82 (s, 3H), 2.79 (t, *J* = 5.6 Hz, 2H), 2.58 (t, *J* = 4.8 Hz, 4H), 2.38-2.19 (m, 2H), 1.22-1.01 (m, 2H).

### Example 44: WX044

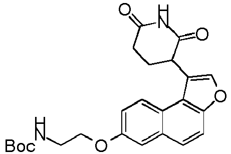

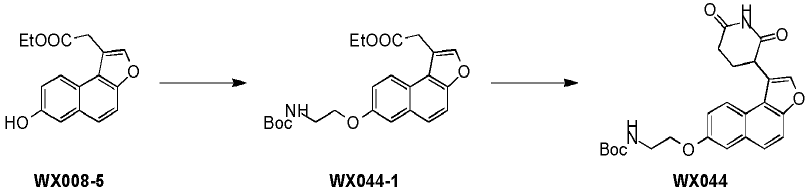

### Step 1: Synthesis of intermediate WX044-1

At room temperature and under nitrogen atmosphere, the intermediate **WX008-5** (1.5 g, 5.55 mmol) was dissolved in tetrahydrofuran (20 mL), and then triphenylphosphine (1.89 g, 7.21 mmol) and N-Boc-ethanolamine (1.16 g, 7.21 mmol, 1.12 mL) were added successively, the temperature was reduced to 0 °C, diisopropyl azodicarboxylate (1.46 g, 7.21 mmol, 1.40 mL) was added dropwise, and the reaction mixture was slowly returned to room temperature and stirred and reacted for 4 hours. After completion of the reaction, the reaction solution was directly concentrated under reduced pressure to remove the solvent, and the resulting residue was purified by column chromatography (eluent: petroleum ether/ethyl acetate = 9/1-4/1, volume ratio) to obtain the intermediate **WX044-1.** ¹H NMR (400 MHz, CDCl₃) δ: 8.15 (d, *J* = 9.2 Hz, 1H), 7.74 (s, 1H), 7.62 (s, 2H), 7.29 (d, *J* = 2.4 Hz, 1H), 7.24 (dd, *J* = 2.6, 9.0 Hz, 1H), 4.22 (q, *J* = 7.0 Hz, 2H), 4.19-4.13 (m, 2H), 4.04 (s, 2H), 3.69-3.57 (m, 2H), 1.47 (s, 9H), 1.26 (t, *J* = 7.2 Hz, 3H).

### Step 2: Synthesis of WX044

At room temperature and under nitrogen atmosphere, the intermediate **WX044-1** (2 g, 4.84 mmol) was dissolved in tetrahydrofuran (30 mL), and then acrylamide (343.82 mg, 4.84 mmol) and the solution of potassium *tert*-butoxide (1 M, 4.84 mL) in tetrahydrofuran were added successively, and the reaction mixture was stirred and reacted at room temperature for 2 hours. After completion of the reaction, the reaction solution was added with water (20 mL) and extracted with ethyl acetate (20 mL × 3). The organic phase was combined, dried over anhydrous sodium sulfate and filtered, and the filtrate was concentrated under reduced pressure to remove the solvent. The resulting residue was purified by column chromatography (eluent: petroleum ether/ethyl acetate = 4/1-1/1, volume ratio), and the resulting crude product was stirred with 5 mL methanol at room temperature for 15 minutes and a white solid was precipitated. The solid was collected by filtration and concentrated under reduced pressure to remove the solvent to obtain the target compound **WX044.** MS-ESI *m*/*z:* 338.9 [M-Boc+H]⁺. ¹H NMR (400 MHz, CDCl₃) δ: 8.18 (s, 1H), 7.89 (d, *J* = 8.8 Hz, 1H), 7.68-7.62 (m, 3H), 7.32 (d, *J* = 2.4 Hz, 1H), 7.25 (dd, *J* = 2.6, 9.0 Hz, 1H), 5.07 (s, 1H), 4.48 (dd, *J* = 5.2, 8.8 Hz, 1H), 4.17 (t, *J* = 5.0 Hz, 2H), 3.67-3.55 (m, 2H), 2.88-2.70 (m, 2H), 2.57-2.39 (m, 2H), 1.47 (s, 9H).

### Example 45: Hydrochloride of WX045

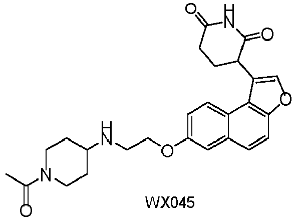

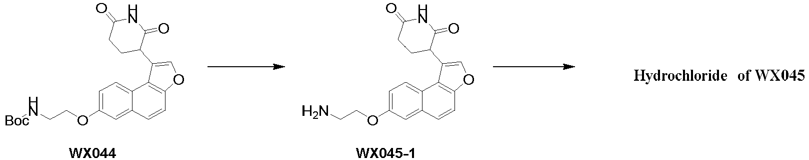

### Step 1: Synthesis of intermediate WX045-1

At room temperature, the intermediate **WX044** (800 mg, 1.81 mmol, purity: 99.07%) was dissolved in hydrochloric acid/ethyl acetate (4 M, 10 mL), and the reaction mixture was stirred and reacted at room temperature for 2 hours. A white solid was precipitated. After completion of the reaction, the reaction solution was directly filtered, the solid was collected, and concentrated under reduced pressure to remove the solvent to obtain the hydrochloride of intermediate **WX045-1.** ¹H NMR (400 MHz, DMSO_*d₆*) δ: 10.93 (s, 1H), 8.35 (s, 3H), 8.14 (d, *J* = 9.2 Hz, 1H), 7.98 (s, 1H), 7.85-7.73 (m, 2H), 7.57 (d, *J* = 2.4 Hz, 1H), 7.28 (dd, *J* = 2.8, 8.8 Hz, 1H), 4.65 (dd, *J* = 4.6, 12.2 Hz, 1H), 4.34 (t, *J* = 5.2 Hz, 2H), 3.34-3.23 (m, 2H), 2.94-2.82 (m, 1H), 2.69-2.57 (m, 1H), 2.46-2.33 (m, 1H), 2.32-2.21 (m, 1H).

### Step 2: Synthesis of WX045

At room temperature, the intermediate **WX045-1** (100 mg, 266.80 µmol, hydrochloride) was dissolved in 1,2-dichloroethane (3 mL), and then 1-acetylpiperidin-4-one (37.66 mg, 266.80 µmol) and sodium acetate (43.77 mg, 533.60 µmol) were added successively, and the reaction mixture was stirred and reacted at room temperature for 10 minutes. Sodium borohydride acetate (113.09 mg, 533.60 µmol) was added, and the reaction mixture was stirred and reacted at room temperature for 12 hours. After completion of the reaction, the reaction solution was directly concentrated under reduced pressure to remove the solvent, and the resulting residue was purified by preparative HPLC (mobile phase: acetonitrile/water; acid system: 0.05% HCl) to obtain the hydrochloride of the target compound **WX045.** MS-ESI *m*/*z:* 464.1 [M+H]⁺. ¹H NMR (400 MHz, DMSO_*d₆*) δ: 10.94 (s, 1H), 9.50 (s, 2H), 8.13 (d, *J* = 8.8 Hz, 1H), 7.98 (s, 1H), 7.83-7.74 (m, 2H), 7.58 (d, *J* = 2.4 Hz, 1H), 7.30 (dd, *J* = 2.4, 9.2 Hz, 1H), 4.65 (dd, *J* = 4.4, 11.6 Hz, 1H), 4.52-4.39 (m, 3H), 3.92 (d, *J* = 13.6 Hz, 1H), 3.49-3.33 (m, 3H), 3.06 (t, *J* = 12.6 Hz, 1H), 2.95-2.82 (m, 1H), 2.69-2.54 (m, 2H), 2.44-2.32 (m, 1H), 2.31-2.21 (m, 1H), 2.13 (t, *J* = 14.4 Hz, 2H), 2.01 (s, 3H), 1.68-1.54 (m, 1H), 1.53-1.39 (m, 1H).

### Example 46: Hydrochloride of WX046

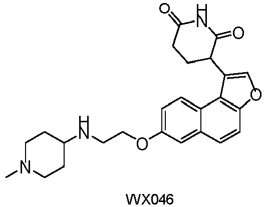

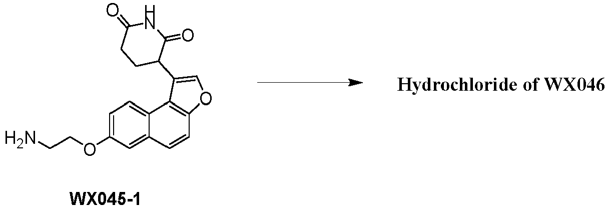

At room temperature and under nitrogen atmosphere, the intermediate **WX045-1** (0.1 g, 266.80 µmol, hydrochloride) was dissolved in 1,2-dichloroethane (3 mL), and then 1-methyl-4-piperidone (30.19 mg, 266.80 µmol) and sodium acetate (43.77 mg, 533.59 µmol) were added successively, and the reaction mixture was stirred and reacted at room temperature for 10 minutes, then sodium triacetoxyborohydride (113.09 mg, 533.59 µmol) was added, and the reaction mixture was stirred and reacted at room temperature for 12 hours. After completion of the reaction, the reaction solution was concentrated under reduced pressure to remove the solvent. The resulting residue was purified by preparative HPLC (mobile phase: acetonitrile/water; acid system: 0.05% HCl) to obtain the hydrochloride of the target compound **WX046.** MS-ESI *m*/*z:* 436.1 [M+H]⁺. ¹H NMR (400 MHz, DMSO_*d₆*) δ: 10.94 (s, 1H), 10.61 (s, 1H), 9.54 (s, 2H), 8.13 (d, *J* = 8.8 Hz, 1H), 7.99 (s, 1H), 7.85-7.73 (m, 2H), 7.58 (d, *J* = 2.8 Hz, 1H), 7.30 (dd, *J* = 2.8, 9.2 Hz, 1H), 4.64 (dd, *J* = 4.6, 11.8 Hz, 1H), 4.44 (t, *J* = 5.0 Hz, 2H), 3.58-3.44 (m, 4H), 3.08-2.95 (m, 2H), 2.91-2.82 (m, 1H), 2.79-2.62 (m, 4H), 2.45-2.18 (m, 5H), 2.12-1.95 (m, 2H).

### Example 47: WX047

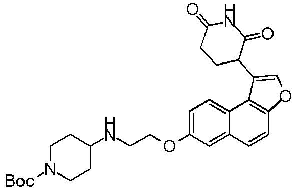

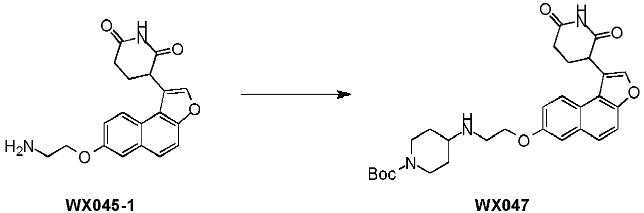

At room temperature and under nitrogen atmosphere, the intermediate **WX045-1** (0.18 g, 480.23 µmol, hydrochloride) was dissolved in 1,2-dichloroethane (3 mL), then N*-tert-*butoxycarbonyl-4-piperidone (95.68 mg, 480.23 µmol) and sodium acetate (78.79 mg, 960.47 µmol) were added successively, and the reaction mixture was stirred at room temperature for 10 minutes, then sodium triacetoxyborohydride (203.56 mg, 960.47 µmol) was added, and the reaction mixture was stirred and reacted at room temperature for 12 hours. After completion of the reaction, the reaction solution was concentrated under reduced pressure to remove the solvent. The resulting residue was purified by preparative HPLC (mobile phase: acetonitrile/water; neutral system: 10 mM NH₄HCO₃) to obtain the target compound **WX047.** MS-ESI *m*/*z:* 522.2 [M+H]⁺. ¹H NMR (400 MHz, DMSO_*d₆*) δ: 10.93 (s, 1H), 8.08 (d, *J* = 9.2 Hz, 1H), 7.96 (s, 1H), 7.80-7.68 (m, 2H), 7.51 (d, *J* = 2.8 Hz, 1H), 7.23 (dd, *J* = 2.4, 9.2 Hz, 1H), 4.63 (dd, *J* = 4.4, 12.0 Hz, 1H), 4.14 (t, *J* = 5.8 Hz, 2H), 3.83 (d, *J* = 13.2 Hz, 2H), 2.97 (t, *J* = 5.6 Hz, 2H), 2.93-2.74 (m, 3H), 2.70-2.52 (m, 3H), 2.46-2.31 (m, 1H), 2.30-2.18 (m, 1H), 1.84-1.75 (m, 2H), 1.39 (s, 9H), 1.19-1.05 (m, 2H).

### Example 48: Hydrochloride of WX048

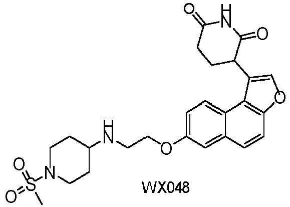

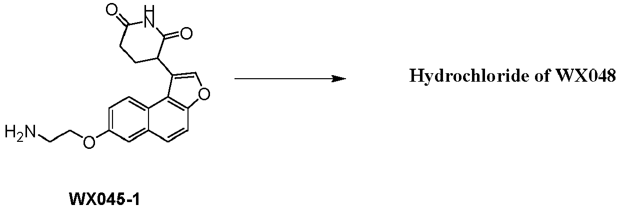

At room temperature and under nitrogen atmosphere, the intermediate **WX045-1** (0.1 g, 266.80 µmol, hydrochloride) was dissolved in 1,2-dichloroethane (3 mL), and then 1-methanesulfonyl-4-piperidone (47.28 mg, 266.80 µmol) and sodium acetate (43.77 mg, 533.60 µmol) were added successively, and the reaction mixture was stirred and reacted at room temperature for 10 minutes, then sodium triacetoxyborohydride (113.09 mg, 533.60 µmol) was added, and the reaction mixture was stirred and reacted at room temperature for 12 hours. After completion of the reaction, the reaction solution was concentrated under reduced pressure to remove the solvent. The resulting residue was purified by preparative HPLC (mobile phase: acetonitrile/water; acid system: 0.05% HCl) to obtain the hydrochloride of the target compound **WX048.** MS-ESI *m*/*z*: 500.0 [M+H]⁺. ¹H NMR (400 MHz, DMSO_*d₆*) δ: 10.94 (s, 1H), 9.20 (s, 2H), 8.14 (d, *J* = 9.2 Hz, 1H), 7.99 (s, 1H), 7.85-7.72 (m, 2H), 7.59 (d, *J* = 2.4 Hz, 1H), 7.30 (dd, *J* = 2.6, 9.0 Hz, 1H), 4.64 (dd, *J* = 4.2, 11.8 Hz, 1H), 4.43 (t, *J* = 4.6 Hz, 2H), 3.67 (d, *J* = 12.0 Hz, 2H), 3.47 (s, 2H), 3.30-3.23 (m, 1H), 2.91 (s, 3H), 2.90-2.84 (m, 1H), 2.83-2.76 (m, 2H), 2.66-2.59 (m, 1H), 2.46-2.38 (m, 1H), 2.31-2.24 (m, 1H), 2.19 (d, *J* = 11.2 Hz, 2H), 1.76-1.60 (m, 2H).

### Example 49: WX049

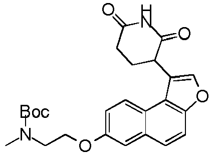

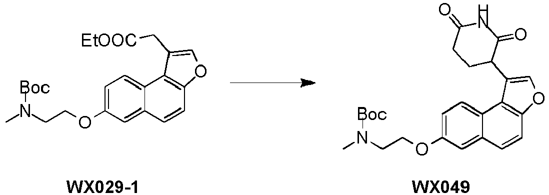

At room temperature and under nitrogen atmosphere, the intermediate **WX029-1** (0.3 g, 700.15 µmol) was dissolved in tetrahydrofuran (8 mL), the temperature was reduced to 0 °C, acrylamide (49.77 mg, 700.15 µmol) and the solution of potassium tert-butoxide (700.15 µL, 1 M) in tetrahydrofuran were added, and the reaction mixture was returned to room temperature and stirred and reacted for 1 hour. After completion of the reaction, the reaction solution was added with water (10 mL), and then extracted with ethyl acetate (10 mL × 3). The organic phase was combined, washed successively with saturated brine (10 mL), dried with anhydrous sodium sulfate, filtered, concentrated under reduced pressure to remove the solvent. The resulting residue was purified by preparative HPLC (mobile phase: acetonitrile/water; neutral system: 10 mM NH₄HCO₃) to obtain the target compound **WX049.** MS-ESI *m*/*z:* 475.0 [M+Na]⁺. ¹H NMR (400 MHz, DMSO_*d₆*) δ: 10.94 (s, 1H), 8.09 (d, *J* = 8.8 Hz, 1H), 7.97 (s, 1H), 7.75 (s, 2H), 7.54 (d, *J* = 2.8 Hz, 1H), 7.22 (dd, *J* = 2.4, 9.2 Hz, 1H), 4.63 (dd, *J* = 4.4, 12.4 Hz, 1H), 4.27-4.18 (m, 2H), 3.66-3.56 (m, 2H), 2.94-2.88 (m, 3H), 2.88-2.83 (m, 1H), 2.68-2.59 (m, 1H), 2.46-2.31 (m, 1H), 2.29-2.22 (m, 1H), 1.38 (s, 9H).

### Example 50: Hydrochloride of WX050

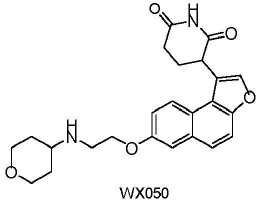

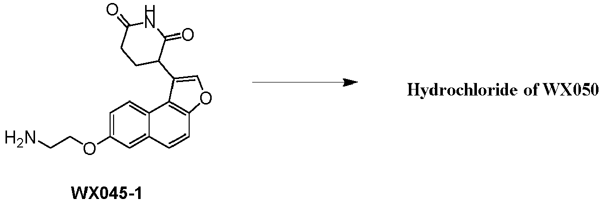

At room temperature, the intermediate **WX045-1** (100 mg, 266.80 µmol, hydrochloride) was dissolved in 1,2-dichloroethane (3 mL), and then tetrahydropyrone (26.71 mg, 266.80 µmol, 24.51 µL) and sodium acetate (43.77 mg, 533.60 µmol) were added successively, and the reaction mixture was stirred and reacted at room temperature for 10 minutes. Sodium borohydride acetate (113.09 mg, 533.60 µmol) was added, and the reaction mixture was stirred and reacted at room temperature for 12 hours. After completion of the reaction, the reaction solution was directly concentrated under reduced pressure to remove the solvent, and the resulting residue was purified by preparative HPLC (mobile phase: acetonitrile/water; acid system: 0.05% HCl) to obtain the hydrochloride of the target compound **WX050.** MS-ESI *m*/*z:* 423.1 [M+H]⁺. ¹H NMR (400 MHz, DMSO_*d₆*) δ: 10.94 (s, 1H), 9.18 (s, 2H), 8.14 (d, *J* = 9.2 Hz, 1H), 7.99 (s, 1H), 7.83-7.75 (m, 2H), 7.59 (d, *J* = 2.4 Hz, 1H), 7.30 (dd, *J* = 2.4, 9.2 Hz, 1H), 4.64 (dd, *J* = 4.4, 12.0 Hz, 1H), 4.43 (t, *J* = 4.4 Hz, 2H), 3.94 (dd, *J* = 4.0, 11.2 Hz, 2H), 3.51-3.36 (m, 4H), 3.31-3.26 (m, 1H), 2.97-2.81 (m, 1H), 2.71-2.58 (m, 1H), 2.45-2.35 (m, 1H), 2.31-2.22 (m, 1H), 2.07-1.97 (m, 2H), 1.77-1.58 (m, 2H).

### Example 51: Hydrochloride of WX051

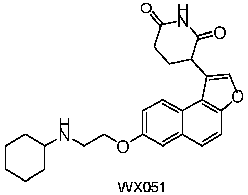

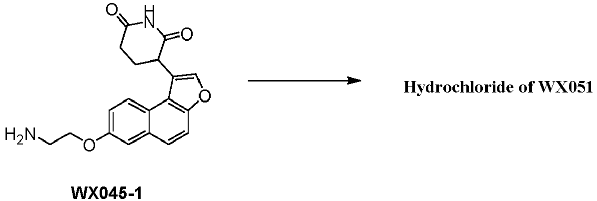

At room temperature, the intermediate **WX045-1** (100 mg, 266.80 µmol, hydrochloride) was dissolved in 1,2-dichloroethane (3 mL), and then cyclohexanone (26.18 mg, 266.80 µmol, 27.65 µL) and sodium acetate (43.77 mg, 533.60 µmol) were added successively, and the reaction mixture was stirred at room temperature for 10 minutes. Sodium borohydride acetate (113.09 mg, 533.60 µmol) was added, and the reaction was stirred and reacted at room temperature for 12 hours. After completion of the reaction, the reaction solution was directly concentrated under reduced pressure to remove the solvent, and the resulting residue was purified by preparative HPLC (mobile phase: acetonitrile/water; acid system: 0.05% HCl) to obtain the hydrochloride of the target compound **WX051.** MS-ESI *m*/*z:* 421.1 [M+H]⁺. ¹H NMR (400 MHz, DMSO_*d₆*) δ: 10.94 (s, 1H), 8.88 (s, 2H), 8.14 (d, *J* = 9.2 Hz, 1H), 7.99 (s, 1H), 7.84-7.75 (m, 2H), 7.58 (d, *J* = 2.4 Hz, 1H), 7.29 (dd, *J* = 2.4, 9.2 Hz, 1H), 4.64 (dd, *J* = 4.4, 11.6 Hz, 1H), 4.41 (t, *J* = 4.8 Hz, 2H), 3.51-3.39 (m, 2H), 3.20-3.04 (m, 1H), 2.96-2.80 (m, 1H), 2.73-2.58 (m, 1H), 2.45-2.35 (m, 1H), 2.30-2.22 (m, 1H), 2.15-2.04 (m, 2H), 1.86-1.74 (m, 2H), 1.68-1.57 (m, 1H), 1.44-1.19 (m, 4H), 1.18-1.04 (m, 1H).

### Example 52: WX052

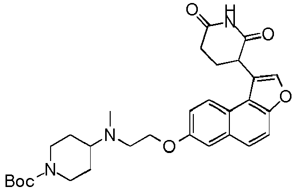

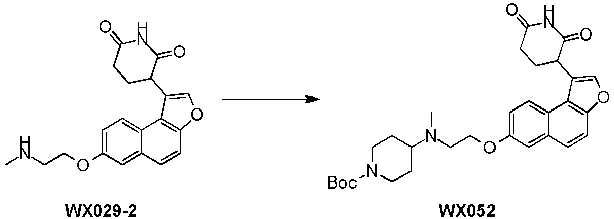

At room temperature and under nitrogen atmosphere, the intermediate **WX029-2** (150 mg, 385.76 µmol, hydrochloride) was dissolved in 1,2-dichloroethane (5 mL), and N*-tert-*butoxycarbonyl-4-piperidone (76.86 mg, 385.76 µmol) and sodium acetate (31.64 mg, 385.76 µmol) were added, and the reaction mixture was warmed to 50 °C and stirred and reacted for 0.5 hours. Then sodium borohydride acetate (163.52 mg, 771.52 µmol) was added, and the reaction mixture was stirred and reacted at 50 °C for additional 4 hours. Additional sodium acetate (31.64 mg, 385.76 µmol) was added, and the reaction mixture was warmed to 70 °C and stirred and reacted for 12 hours. After completion of the reaction, the reaction solution was cooled to room temperature and concentrated under reduced pressure to remove the solvent. The resulting residue was purified by preparative HPLC (mobile phase: acetonitrile/water; neutral system: 10 mM NH₄HCO₃) to obtain the target compound **WX052.** MS-ESI *m*/*z:* 536.2 [M+H]⁺. ¹H NMR (400 MHz, DMSO_*d₆*) δ: 10.93 (s, 1H), 8.07 (d, *J* = 8.8 Hz, 1H), 7.96 (s, 1H), 7.77-7.71 (m, 2H), 7.51 (d, *J* = 2.4 Hz, 1H), 7.20 (dd, *J* = 2.6, 9.0 Hz, 1H), 4.63 (dd, *J* = 4.4, 12.0 Hz, 1H), 4.15 (t, *J* = 6.0 Hz, 2H), 3.97 (d, *J* = 12.8 Hz, 2H), 2.93-2.80 (m, 3H), 2.78-2.63 (m, 2H), 2.62-2.56 (m, 1H), 2.43-2.37 (m, 1H), 2.36-2.30 (m, 1H), 2.29 (s, 3H), 2.28-2.21 (m, 1H), 1.76-1.65 (m, 2H), 1.38 (s, 9H), 1.34-1.21 (m, 2H).

### Example 53: Hydrochloride of WX053

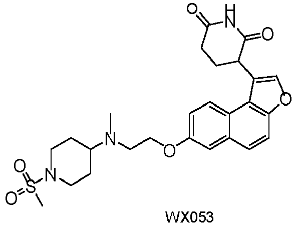

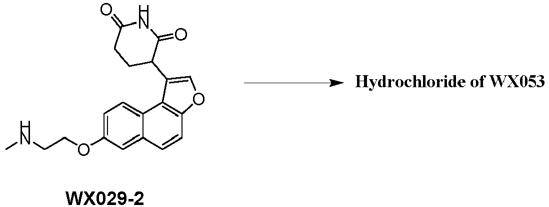

At room temperature and under nitrogen atmosphere, the intermediate **WX029-2** (100 mg, 257.17 µmol, hydrochloride) was dissolved in 1,2-dichloroethane (4 mL), and 1-methylsulfonyl-4-piperidone (45.58 mg, 257.17 µmol) and sodium acetate (21.10 mg, 257.17 µmol) were added, and the reaction mixture was warmed to 50 °C and stirred and reacted for 0.5 hours. Then sodium borohydride acetate (109.01 mg, 514.35 µmol) was added, and the reaction mixture was stirred and reacted at 50 °C for additional 4 hours. Additional sodium acetate (21.10 mg, 257.17 µmol) was added, and the reaction mixture was warmed to 70 °C and stirred and reacted for 12 hours. After completion of the reaction, the reaction solution was cooled to room temperature and concentrated under reduced pressure to remove the solvent. The resulting residue was purified by preparative HPLC (mobile phase: acetonitrile/water; acid system: 0.05% HCl) to obtain the hydrochloride of the target compound **WX053.** MS-ESI *m*/*z:* 514.2 [M+H]⁺. ¹H NMR (400 MHz, DMSO_*d₆*) δ: 11.12 (s, 1H), 10.94 (s, 1H), 8.12 (d, *J* = 8.8 Hz, 1H), 7.98 (s, 1H), 7.78 (s, 2H), 7.60 (d, *J* = 2.8 Hz, 1H), 7.30 (dd, *J* = 2.8, 9.2 Hz, 1H), 4.64 (dd, *J* = 4.2, 12.2 Hz, 1H), 4.60-4.51 (m, 2H), 3.78-3.65 (m, 3H), 2.92 (s, 3H), 2.90-2.76 (m, 6H), 2.68-2.58 (m, 1H), 2.49-2.35 (m, 2H), 2.34-2.13 (m, 4H), 1.88-1.72 (m, 2H).

### Example 54: Hydrochloride of WX054

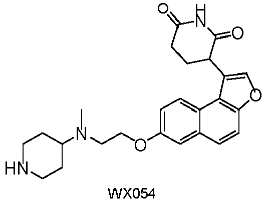

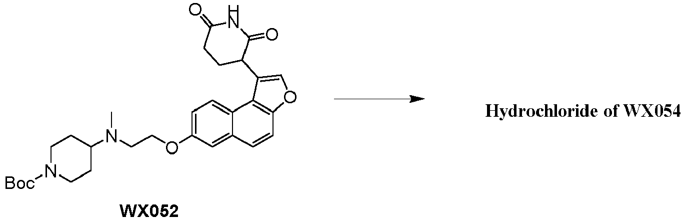

At 0 °C and under nitrogen atmosphere, the intermediate **WX052** (100 mg, 186.70 µmol) was mixed with hydrogen chloride in ethyl acetate (4 M, 21.05 mL), and the reaction mixture was stirred and reacted at 20 °C for 2 hours. After completion of the reaction, the reaction mixture was filtered, and the resulting filter cake was concentrated under reduced pressure to remove the solvent to obtain the hydrochloride of the target compound **WX054.** MS-ESI *m*/*z:* 436.2 [M+H]⁺. ¹H NMR (400 MHz, DMSO_*d₆*) δ: 11.25 (s, 1H), 10.94 (s, 1H), 9.21 (d, *J* = 10.0 Hz, 1H), 9.08 (d, *J=* 10.8 Hz, 1H), 8.12 (d, *J* = 8.4 Hz, 1H), 7.99 (s, 1H), 7.78 (s, 2H), 7.60 (d, *J* = 2.4 Hz, 1H), 7.30 (dd, *J* = 2.6, 9.0 Hz, 1H), 4.69-4.50 (m, 3H), 3.72-3.55 (m, 2H), 3.42-3.38 (m, 2H), 3.03-2.88 (m, 3H), 2.84 (d, *J* = 4.4 Hz, 3H), 2.70-2.58 (m, 1H), 2.54-2.52 (m, 1H), 2.47-2.21 (m, 4H), 2.12-1.95 (m, 2H).

### Example 55: WX055

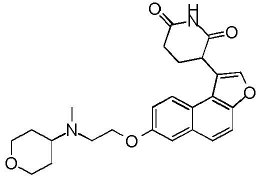

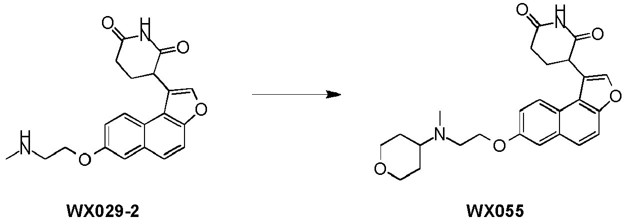

At room temperature, the intermediate **WX029-2** (100 mg, 257.17 µmol, hydrochloride) was dissolved in 1,2-dichloroethane (4 mL), tetrahydropyran-4-one (51.49 mg, 514.35 umol, 47.24 µL) and sodium acetate (21.10 mg, 257.17 µmol) were added, and the reaction mixture was warmed to 50 °C and stirred and reacted for 30 minutes. Sodium borohydride acetate (109.01 mg, 514.35 µmol) was added, and the reaction mixture was stirred and reacted at 50 °C for 2 hours. Additional sodium borohydride acetate (109.01 mg, 514.35 µmol) was added, and the reaction mixture was warmed to 75 °C and stirred and reacted for 2 hours. After completion of the reaction, the reaction solution was cooled to room temperature. The solvent was removed by concentration under reduced pressure. The resulting residue was purified by preparative HPLC (mobile phase: acetonitrile/water; neutral system: 10 mM NH₄HCO₃) to obtain the target compound **WX055.** MS-ESI *m*/*z:* 436.9 [M+H]⁺. ¹H NMR (400 MHz, DMSO_*d₆*) δ: 10.94 (s, 1H), 8.08 (d, *J* = 8.8 Hz, 1H), 7.96 (s, 1H), 7.75 (d, *J* = 1.6 Hz, 2H), 7.52 (d, *J* = 2.4 Hz, 1H), 7.21 (dd, *J* = 2.6, 9.0 Hz, 1H), 4.63 (dd, *J* = 4.6, 12.2 Hz, 1H), 4.17 (t, *J* = 6.2 Hz, 2H), 3.89 (dd, *J* = 4.0, 10.8 Hz, 2H), 3.32-3.25 (m, 2H), 2.92-2.82 (m, 3H), 2.70-2.58 (m, 2H), 2.45-2.36 (m, 1H), 2.32 (s, 3H), 2.29-2.22 (m, 1H), 1.72-1.63 (m, 2H), 1.52-1.38 (m, 2H).

### Example 56: Hydrochloride of WX056

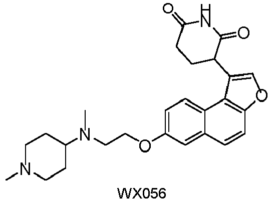

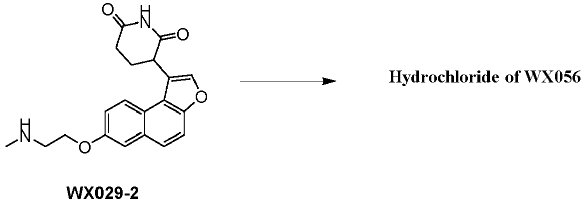

At room temperature, the intermediate **WX029-2** (100 mg, 257.17 µmol, hydrochloride) was dissolved in 1,2-dichloroethane (4 mL), and 1-methyl-4-piperidone (58.20 mg, 514.35 µmol) and sodium acetate (21.10 mg, 257.17 µmol) were added, and the reaction mixture was heated to 50 °C and stirred and reacted for 30 minutes. Sodium borohydride acetate (109.01 mg, 514.35 µmol) was added, and the reaction mixture was stirred and reacted at 50 °C for 2 hours. Additional sodium borohydride acetate (109.01 mg, 514.35 µmol) was added, and the reaction mixture was heated to 75 °C and stirred and reacted for 2 hours. After completion of the reaction, the reaction solution was cooled to room temperature and concentrated under reduced pressure to remove the solvent. The resulting residue was purified by preparative HPLC (mobile phase: acetonitrile/water; acid system: 0.05% HCl) to obtain the hydrochloride of the target compound **WX056.** MS-ESI *m*/*z:* 449.9 [M+H]⁺. ¹H NMR (400 MHz, DMSO_*d₆*) δ: 11.50 (s, 1H), 11.14 (s, 1H), 10.96 (s, 1H), 8.14 (d, *J* = 8.4 Hz, 1H), 8.01 (s, 1H), 7.80 (s, 2H), 7.62 (d, *J* = 2.4 Hz, 1H), 7.33 (dd, *J* = 2.4, 9.2 Hz, 1H), 4.67 (dd, *J* = 4.6, 12.2 Hz, 1H), 4.63-4.58 (m, 2H), 3.70-3.52 (m, 5H), 3.13-2.99 (m, 2H), 2.96-2.89 (m, 1H), 2.87 (d, *J* = 4.4 Hz, 3H), 2.74 (d, *J* = 4.4 Hz, 3H), 2.70-2.59 (m, 1H), 2.45-2.37 (m, 2H), 2.36-2.18 (m, 4H).

### Example 57: WX057

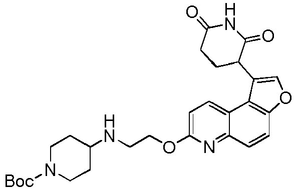

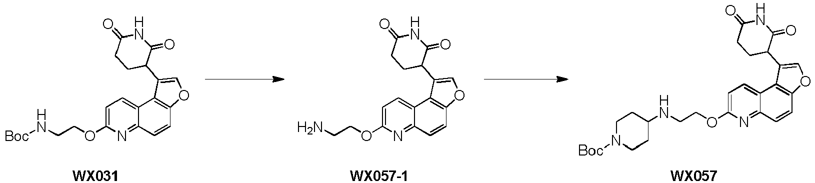

### Step 1: Synthesis of intermediate WX057-1

At 20 °C and under nitrogen atmosphere, the intermediate **WX031** (300 mg, 682.66 µmol) was mixed with hydrogen chloride in ethyl acetate (4 M, 20 mL), and the reaction mixture was stirred and reacted at 20 °C for 3 hours. After completion of the reaction, the reaction solution was filtered, and the resulting filter cake was concentrated under reduced pressure to remove the solvent to obtain the hydrochloride of the intermediate **WX057-1.** ¹H NMR (400 MHz, DMSO_*d₆*) δ: 10.95 (s, 1H), 8.58 (d, *J* = 8.8 Hz, 1H), 8.36 (s, 3H), 8.08 (s, 1H), 7.98 (d, *J* = 8.8 Hz, 1H), 7.75 (d, *J* = 9.2 Hz, 1H), 7.11 (d, *J* = 9.2 Hz, 1H), 4.70-4.63 (m, 3H), 3.36-3.23 (m, 2H), 2.95-2.82 (m, 1H), 2.71-2.61 (m, 1H), 2.47-2.38 (m, 1H), 2.36-2.23 (m, 1H).

### Step 2: Synthesis of WX057

At room temperature and under nitrogen atmosphere, the intermediate **WX057-1** (165 mg, 439.06 µmol, hydrochloride) was dissolved in 1,2-dichloroethane (5 mL), and N*-tert-*butoxycarbonyl-4-piperidone (87.48 mg, 439.06 µmol) and sodium acetate (39.89 mg, 486.23 µmol) were added, and the reaction mixture was warmed to 50 °C and stirred and reacted for 0.5 hours. Then sodium borohydride acetate (186.11 mg, 878.11 µmol) was added, and the reaction mixture was warmed to 50 °C and stirred and reacted for 12 hours. After completion of the reaction, the reaction solution was cooled to room temperature and concentrated under reduced pressure to remove the solvent. The resulting residue was purified by preparative HPLC twice (mobile phase: acetonitrile/water; neutral system: 10 mM NH₄HCO₃) to obtain the target compound **WX057.** MS-ESI *m*/*z*: 523.3 [M+H]⁺. ¹H NMR (400 MHz, DMSO_*d₆*) δ: 10.93 (s, 1H), 8.50 (d, *J* = 9.2 Hz, 1H), 8.05 (s, 1H), 7.93 (d, *J* = 8.8 Hz, 1H), 7.69 (d, *J* = 8.8 Hz, 1H), 7.06 (d, *J* = 9.2 Hz, 1H), 4.64 (dd, *J* = 4.0, 12.4 Hz, 1H), 4.45 (t, *J* = 5.6 Hz, 2H), 3.82 (d, *J* = 12.8 Hz, 2H), 2.97 (t, *J* = 5.8 Hz, 2H), 2.91-2.71 (m, 3H), 2.69-2.58 (m, 2H), 2.48-2.35 (m, 2H), 2.34-2.20 (m, 1H), 1.88-1.75 (m, 2H), 1.39 (s, 9H), 1.18-1.05 (m, 2H).

### Experimental example 1: In vitro test of IKZF3 protein level in multiple myeloma cells

### Experiment object:

The WB method was used to study the regulation of IKZF3 protein level in multiple myeloma cells MM.1S with compounds treatment at different concentrations.

### Protocols:

1) The MM.1S cells were thawed and passaged twice;
2) The MM.1S cells were inoculated in a 6-well plate with 1 × 10⁶ cells per well, and then treated with a certain concentration of the test compound;
3) After 16 hours of treatment, the cultured cell sample was dissolved in RIPA buffer (Sigma-Aldrich) or NETN buffer (150 mM NaCl, 1 % NP-40, 50 mM Tris-HCl, pH = 8.0) with a complete protease inhibitor (Roche) on ice and standing for 20 minutes;
4) After 15 minutes of centrifugation (rotating speed: 17950 rpm), the supernatant was collected and a protein quantitative test (Pierce BCA Protein Assay Kit, Thermo) was performed;
5) The same amount of 20 µg protein by SDS-PAGE was separated and transfered to PVDF or nylon membrane (Invitrogen);
6) 5% skimmed milk powder was added, and then incubated overnight at 4 °C in 5% BSA containing primary antibody anti-IKZF3 (NBP2-24495, Novµs Biologicals) and anti-Actin (1844-1, Epitomics);
7) Finally, after reaction using HRP-linked secondary antibody (Goat-anti-rabbit IgG (sc-2004, Santa Cruz)) for 1 hour, a chemiluminescent substrate (Thermo Scientific) was used to detect bands on the membrane.

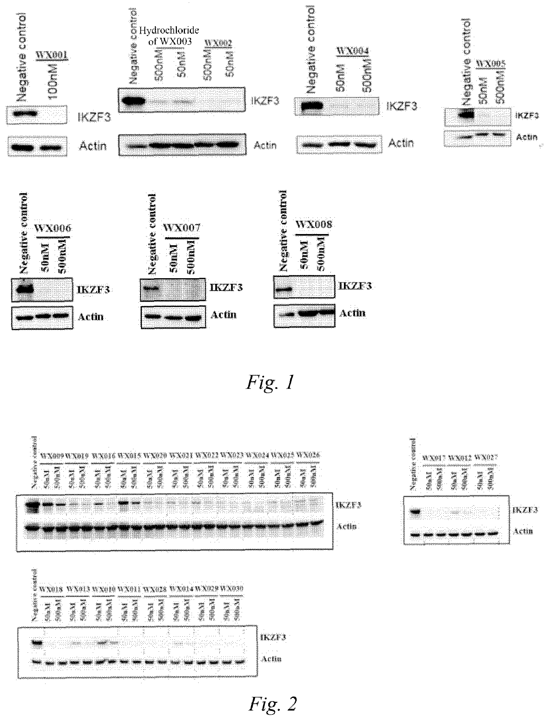

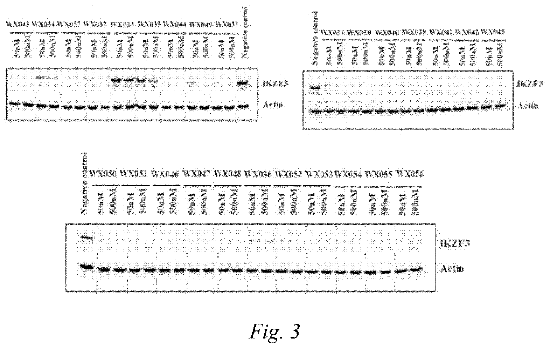

The experimental results are shown in Figures **1-3****.**

### Conclusion:

After the multiple myeloma cells MM.1S are treated with the compounds of the present disclosure at concentrations of 100 nM, or 500 nM and 50 nM, WB detection shows that the level of IKZF3 protein in the cells is significantly decreased.

### Experimental example 2: Evaluation of anti-proliferative effects in lymphoma cell lines OCI-LY10, DOHH2 and Mino

**Experiment object:** In this experiment, the inhibitory effects of the test compounds on cell proliferation in the diffuse large B-cell lymphoma cell lines **OCI-LY10** and **DOHH2,** and the mantle cell lymphoma cell line **Mino** were tested.

### Experimental materials:

### 1. Cell lines and culture methods

| Cell lines | Tumor types | Growth characteristics | Culture methods |
|---|---|---|---|
| OCI-LY10 | Lymphoma | Suspension | RPMI 1640 + 10% FBS |
| DOHH2 | Lymphoma | Suspension | RPMI 1640 + 10% FBS |
| Mino | Lymphoma | Suspension | RPMI 1640 + 15% FBS |

### 2. Media and reagents

| Media and reagents | Manufacturer | Catalog No. |
|---|---|---|
| RPMI 1640 | GIBCO | 22400-089 |
| Dulbecco's PBS | Hyclone | SH30256.01 |
| FBS | Hyclone | SY30087.03 |
| Antibiotic-antimycotic | GIBCO | 15240-062 |
| 0.25% Trypsin | GIBCO | 25200072 |
| DMSO | SIGMA | D2650 |
| 2-mercaptoethanol | SIGMA | 60-24-2 |

### 3. Multi-well plate

Greiner CELLSTAR^{®} 96-well plate, flat-bottomed black plate (with lid and transparent bottom), # 655090.

### 4. Reagents and instruments used in cell viability experiments

(1) Promega CellTiter-Glo Luminescence Cell Viability Detection Kit (Promega-G7573).
(2) 2104 EnVision^{®} plate reader, PerkinElmer.

### Protocols:

### 1. Cells culture

The tumor cell lines were cultured in an incubator at 37 °C and 5% CO₂ under the above-mentioned culture conditions. The cells were passaged regularly, and cells in the logarithmic growth phase were taken for planting.

### 2. Cell planking

(1). The cells were stained with trypan blue and live cells were counted.
(2). The cell concentration was adjusted to an appropriate concentration.

| **Cell line** | **Density (per 96-well)** |
|---|---|
| OCI-LY10 | 5000 |
| DOHH2 | 5000 |
| Mino | 6000 |

(3). 90 µL of cell suspension was added to each well of the culture plate as shown in the table above, and cell-free culture solution was added to the blank control well.
(4). The culture plates were incubated overnight at 37 °C, 5% CO₂, and 100% relative humidity in an incubator.

### 3. Preparation of compound storage solution

The mother liquor storage plate with a compound concentration of 400 times the working concentration of the compound was prepared: The compound was gradually diluted with DMSO from the highest concentration to the lowest concentration. The compound was freshly formulated when use every time.

### 4. Formulation of 10 times compound working solution and treatment of cells with the compound

(1). Formulation of a working solution with a compound concentration of 10 times the working concentration of the compound: 76 µL of cell culture solution was added to a 96-well plate with a V-shaped bottom, and 4 µL of the compound was pipetted from the mother liquor storage plate with a compound concentration of 200 times the working concentration of the compound to the cell culture solution of the 96-well plate. 4 µL of DMSO was added to the vehicle control and blank control. After adding the compound or DMSO, a multi-channel pipette was used for pipetting and mixing well. 78 µL of cell culture solution was added to a 96-well plate with a V-shaped bottom, and 2 µL of the compound was pipetted from the mother liquor storage plate with a compound concentration of 400 times the working concentration of the compound to the cell culture solution of the 96-well plate. 2 µL of DMSO was added to the vehicle control and blank control. After adding the compound or DMSO, a multi-channel pipette was used for mixing well.
(2). Dosing: 10 µL of the working solution of 10 times the working concentration of the compound was taken and added to the cell culture plate. 10 µL mixture of DMSO-cell culture solution was added to the vehicle control and blank control.
(3). The 96-well cell plate was placed back into the incubator to culture OCI-LY10 (5 times dilution, incubate with the compound for 5 days), DOHH2 (3 times dilution, incubate with the compound for 4 days), Mino (3 times dilution, incubate with the compound for 4 days).

### 5. CellTiter-Glo luminescence cell viability detection

The following steps followed the instructions of Promega CellTiter-Glo Luminescence Cell Viability Detection Kit (Promega-G7573).
(1). The CellTiter-Glo buffer was thawed and standing to reach room temperature.
(2). CellTiter-Glo substrate was standing to reach room temperature.
(3). 10 mL CellTiter-Glo buffer was added to CellTiter-Glo substrate in a bottle to dissolve the substrate to formulate CellTiter-Glo working solution.
(4). The working solution was vortexed slowly for fully dissolution.
(5). The cell culture plates were taken out and standing for 30 minutes to equilibrate to room temperature.
(6). 50 µL (equal to half the volume of cell culture solution in each well) of CellTiter-Glo working solution was added into each well. The cell plates were wrapped with aluminum foil to protect the cell plate from light.
(7). The culture plates were shaken on an orbital shaker for 2 minutes to induce cell lysis.
(8). The culture plates were left at room temperature for 10 minutes to stabilize the luminescence signal.
(9). The luminous signal was detected on the 2104 EnVision plate reader.

### 6. Data analysis

The following formula was used to calculate the inhibition rate (IR) of the test compound: IR (%) = (RLU of vehicle control-RLU of compound)/(RLU vehicle control-RLU blank control)* 100%. The inhibition rate of different concentrations of the compound was calculated in Excel, and then GraphPad Prism software was used to draw the inhibition curve and calculate the relevant parameters, including the minimum inhibition rate, the maximum inhibition rate and IC₅₀.

**Experimental results:** The test results are shown in Table **1.**

**Table 1 The inhibitory effect of the compound of the present disclosure on cell proliferation in OCI-LY10, DOHH2 and Mino cell lines**

| Compound | OCI-LY10 IC₅₀ (nM) | DOHH2 IC₅₀ (nM) | Mino IC₅₀ (nM) |
|---|---|---|---|
| **WX002** | 26 | 97 | 8 |
| **WX004** | 17 | 144 | 7 |
| **WX005** | / | 165 | 10 |

| | | | |
|---|---|---|---|
| "/" means not detected. | | | |

### Conclusion:

The compound of the present disclosure exhibits an excellent inhibitory effect on cell proliferation in the lymphoma cell lines OCI-LY10, DOHH2 and Mino.

### Experimental example 3: Evaluation the pharmacokinetic properties of test compound in mice

### Experiment object:

In this study, C57BL male mice were selected as the test animals. A LC-MS/MS method was used to quantitatively determine the drug concentration in the plasma after oral administration of the test compound and the reference compound, to evaluate the pharmacokinetic profile of the test compound in mice.

### Experimental materials:

C57Balb/c (C57) mice (male, 20-30 g, 7-10 weeks old, Beijing Vital River or Shanghai SLAC).

### Experiment operation:

A clear solution or suspension of the test compound was administrated to C57 mice (fasted overnight) by oral gavage. Blood was collected from jugular vein at pre-dose and 0.5, 1, 2, 4, 6, 8, 24 hours post-dose, the collected blood samples were placed in an anticoagulant tube (Jiangsu Kangjian Medical Co., Ltd.) supplemented with EDTA-K₂, and the mixture was vortexed and centrifuged at 13000 rpm for 10 minutes. The LC-MS/MS method was used to determine the plasma concentration of test compounds, and the relevant pharmacokinetic parameters were calculated using non-compartmental model linear logarithmic trapezoidal method by WinNonlin^{™} Version 6.3 (Pharsight, Mountain View, CA).

**Experimental results:** The test results are shown in Table **2.**

**Table 2 Pharmacokinetic parameters of the test compounds in mice**

| Mouse pharmacokinetic parameters | Oral (10 mg/kg) | | |
|---|---|---|---|
| | Peak concentration (µM) | Time to peak (h) | Area under the drug-time curve (0-inf, µM·h) |
| **WX002** | 2.83 | 0.50 | 2.49 |
| **WX004** | 6.54 | 0.25 | 5.50 |
| **WX007** | 12.95 | 0.08 | 7.79 |
| **Hydrochloride of WX008** | 4.88 | 1.00 | 10.27 |
| **Hydrochloride of WX020** | 8.56 | 0.25 | 18.38 |

### Conclusion:

The experimental results show higher oral plasma systemic exposures (AUC_{0-inf}) of **WX004, WX007, hydrochloride of WX008** and **hydrochloride of WX020.** In rodent mice, **WX004, WX007, hydrochloride of WX008** and **hydrochloride of WX020** have better pharmacokinetic properties.

### Experimental example 4: In vivo pharmacodynamic study of the compound in subcutaneous xenograft tumor CB-17 SCID model of human lymphoma OCI-LY10 cells

Cells culture: Human lymphoma OCI-LY10 cells (National Cancer Institute) were cultured in a monolayer *in vitro.* The culture conditions were RPMI-1640 medium with 10% fetal bovine serum, 100 U/mL penicillin and 100 µg/mL streptomycin, 37 °C, 5% CO₂ incubator culture. Conventional digestion treatment with pancreatin-EDTA for passage was carried out twice a week. When the cell saturation was 80% to 90%, and the number reached the requirement, the cells were collected, counted and inoculated.

Animal: CB-17 SCID mouse, female, 6-8 weeks old, weighing 18-22 grams.

### Protocols:

0.2 mL (10 × 10⁶ cells) of OCI-LY10 cells (with matrigel in a volume ratio of 1 : 1) were subcutaneously inoculated on the right back of each mouse. The grouping and administration were started when the average tumor volume reached about 139 mm³. One dosing cycle was seven days, and the compound was administered once a day with an interval of 24 hours. The test compound was administered orally for a total of four cycles. The dose of the test compound WX002 was 60 mg/kg, the tumor volume was measured twice a week with a two-dimensional caliper, and the volume was measured in cubic millimeters, calculated by the following formula: V = 0.5a × b², in which a and b were the long diameter and short diameter of the tumor, respectively. The anti-tumor efficacy was determined by dividing average increase of tumor volume of animals treated with the compound by that of untreated animals.

**Experimental results:** The test results are shown in Table **3.**

**Table 3 The test results of the compound of the present disclosure in subcutaneous xenograft tumor CB-17 SCID model of human lymphoma OCI-LY10 cells**

| Groups | Dosage | Tumor volume (mm³) (Day 1) | Tumor volume (mm³) (Day 27) | TGI (%) (Day 27) |
|---|---|---|---|---|
| Vehicle control | 0 mg/kg | 139 ± 10 | 1088 ± 101 | / |
| **WX002** | 60 mg/kg | 139 ± 10 | 35 ± 10 | 111 |

| | | | | |
|---|---|---|---|---|
| TGI: Tumor Growth Inhibition. TGI (%) = [1- (average tumor volume at the end of administration in a treatment group-average tumor volume at the beginning of administration in this treatment group)/(average tumor volume at the end of administration in the solvent control group-average tumor volume at the beginning of administration in the solvent control group)] × 100%. | | | | |

### Conclusion:

The compound **WX002** of the present disclosure has shown a significant tumor-shrinking effect on the human lymphoma OCI-LY10 in vivo pharmacodynamic model.

## Claims

1. A compound represented by formula (I) or a pharmaceutically acceptable salt thereof, 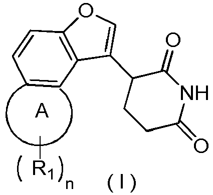 wherein,
n is selected from 0, 1, 2 and 3;
R₁ is selected from H, halogen, OH, NH₂, CN, C₃₋₆ cycloalkyl, C₁₋₆ alkyl, and C₁₋₆ alkoxy and the NH₂, C₃₋₆ cycloalkyl, C₁₋₆ alkyl and C₁₋₆ alkoxy are optionally substituted with 1, 2 or 3 Rₐ;
ring A is selected from 5- to 6-membered heteroaryl, phenyl, C₄₋₆ cycloalkyl, and 4- to 7-membered heterocycloalkyl;
Rₐ is independently selected from F, Cl, Br, I, OH, NH₂, C₁₋₁₀ alkylamino, C₁₋₁₀ alkoxy, -C(=O)O-C₁₋₁₀ alkyl, 4- to 10-membered heterocycloalkyl, 4- to 10-membered heterocycloalkylamino, C₄₋₇ cycloalkylamino, C₄₋₇ cycloalkylmethylamino, wherein the NH₂, C₁₋₁₀ alkylamino, C₁₋₁₀ alkoxy, -C(=O)O-C₁₋₁₀ alkyl, 4- to 10-membered heterocycloalkyl, 4-to 10-membered heterocycloalkylamino, C₄₋₇ cycloalkylamino and C₄₋₇ cycloalkylmethylamino are optionally substituted with 1, 2 or 3 R;
R is independently selected from F, Cl, Br, I, OH, NH₂, Me, 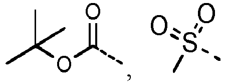 and 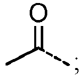
the 5- to 6-membered heteroaryl, 4- to 7-membered heterocycloalkyl, 4- to 10-membered heterocycloalkyl and 4- to 10-membered heterocycloalkylamino comprise 1, 2, 3 or 4 heteroatoms or heteroatom groups independently selected from -NH-, -O-, -S-, 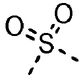 and N, respectively.

2. The compound as defined in claim 1 or a pharmaceutically acceptable salt thereof, wherein Rₐ is selected from F, Cl, Br, I, OH, NH₂, C₁₋₆ alkylamino, C₁₋₆ alkoxy, -C(=O)O-C₁₋₆ alkyl, 4- to 7-membered heterocycloalkyl, 4- to 7-membered heterocycloalkylamino, C₄₋₇ cycloalkylamino, C₄₋₇ cycloalkylmethylamino, wherein the NH₂, C₁₋₆ alkylamino, C₁₋₆ alkoxy, -C(=O)O-C₁₋₆ alkyl, 4- to 7-membered heterocycloalkyl, C₄₋₇ cycloalkylamino, 4- to 7-membered heterocycloalkylamino and C₄₋₇ cycloalkylmethylamino are optionally substituted with 1, 2 or 3 R.

3. The compound as defined in claim 2 or a pharmaceutically acceptable salt thereof, wherein Rₐ is selected from F, Cl, Br, I, OH, NH₂, C₁₋₃ alkylamino, C₁₋₃ alkoxy, -C(=O)O-C₁₋₄ alkyl, pyrrolidinyl, morpholinyl, piperazinyl, piperidinyl, 3-azabicyclo[3,1,0]hexyl, thiomorpholine-1,1-dioxide group, cyclohexylamino, piperidinylamino, tetrahydropyranyl, tetrahydropyranylamino and cyclohexylmethylamino, wherein the NH₂, C₁₋₃ alkylamino, C₁₋₃ alkoxy, -C(=O)O-C₁₋₄ alkyl, tetrahydropyrrolyl, morpholinyl, piperazinyl, piperidinyl, 3-azabicyclo[3,1,0]hexyl, thiomorpholine-1,1-dioxide group, cyclohexylamino, piperidinylamino, tetrahydropyranyl, tetrahydropyranylamino and cyclohexylmethylamino are optionally substituted with 1, 2 or 3 R.

4. The compound as defined in claim 3 or a pharmaceutically acceptable salt thereof, wherein Rₐ is selected from

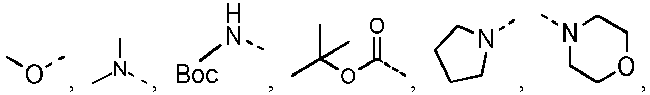

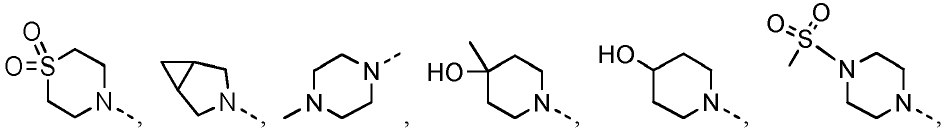

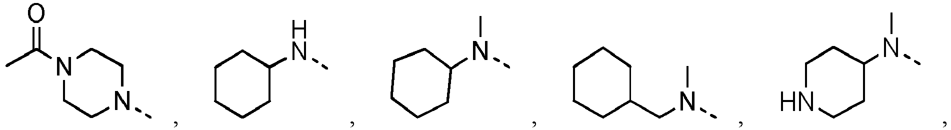

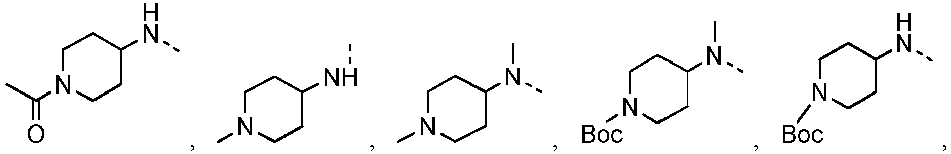

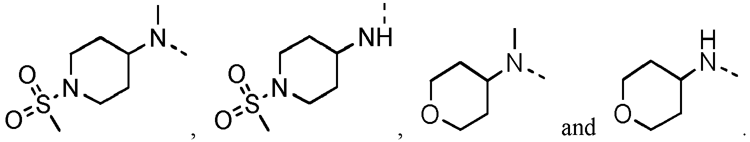

5. The compound as defined in any one of claims 1 to 4 or a pharmaceutically acceptable salt thereof, wherein R₁ is selected from H, NH₂, CN, C₃₋₆ cycloalkyl, C₁₋₃ alkyl and C₁₋₃ alkoxy, wherein the NH₂, C₃₋₆ cycloalkyl, C₁₋₃ alkyl and C₁₋₃ alkoxy are optionally substituted with 1, 2 or 3 Rₐ.

6. The compound as defined in claim 5 or a pharmaceutically acceptable salt thereof, wherein R₁ is selected from H, Me, CN,

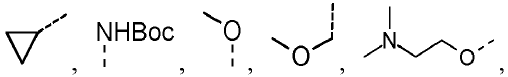

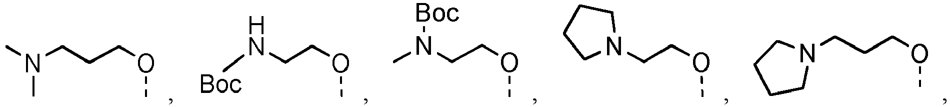

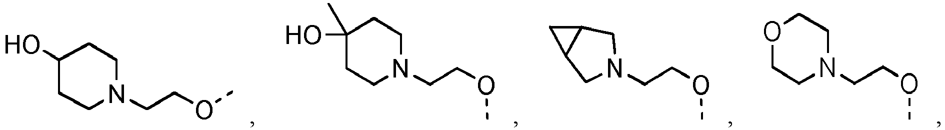

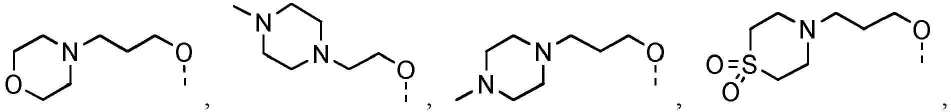

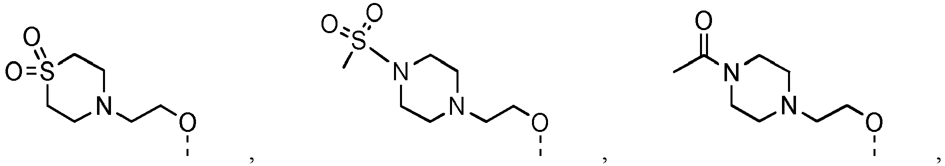

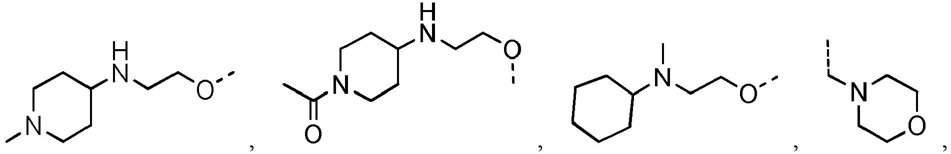

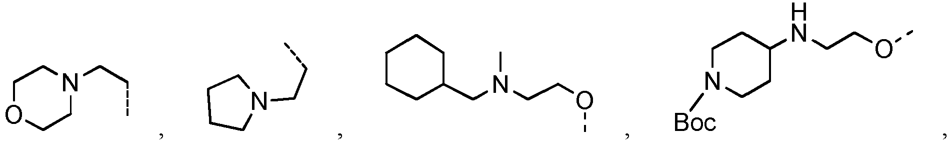

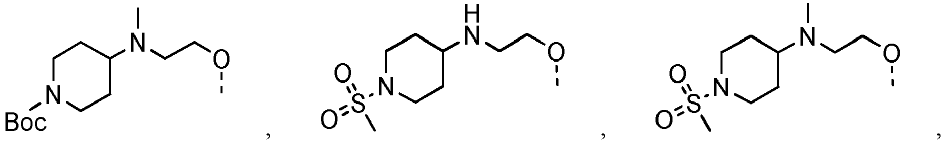

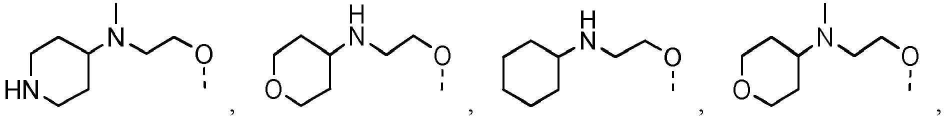

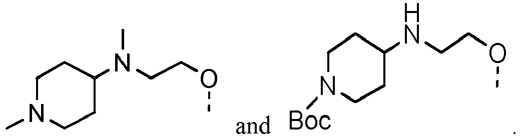

7. The compound as defined in any one of claims 1 to 3 or a pharmaceutically acceptable salt thereof, wherein ring A is selected from phenyl, pyridyl, pyrrolyl, pyrazolyl, 1,3-dioxolane, morpholinyl, oxazolylcyclobutyl, oxepanyl, furanyl, tetrahydrofuranyl and 1,4-oxazepinyl.

8. The compound as defined in claim 7 or a pharmaceutically acceptable salt thereof, wherein ring Ais selected from phenyl, pyridyl, pyrrolyl, pyrazolyl, 1,3-dioxolane, furanyl and tetrahydrofuranyl.

9. The compound as defined in claim 8 or a pharmaceutically acceptable salt thereof, wherein structural unit 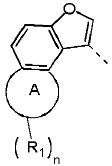 is selected from

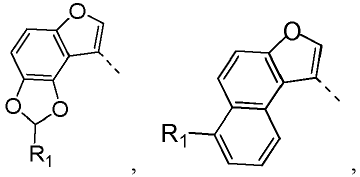

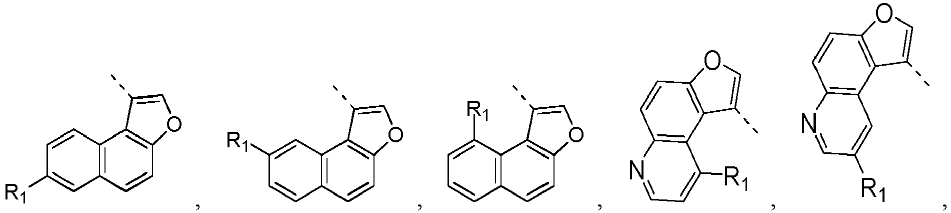

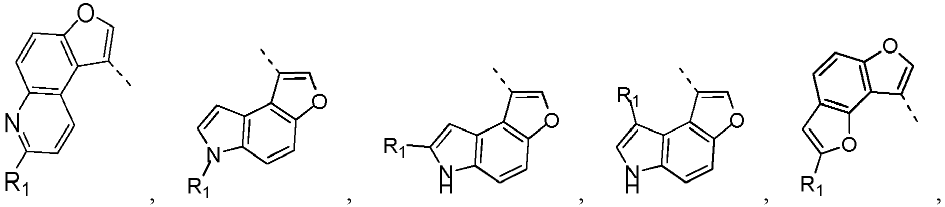

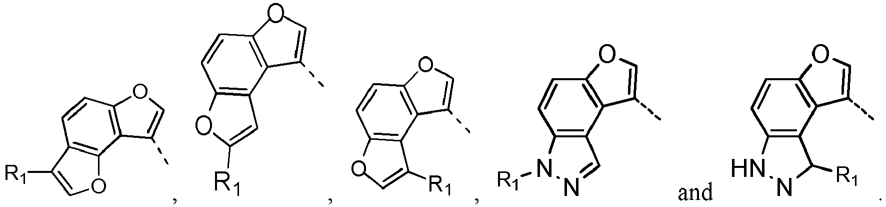

10. The compound as defined in any one of claims 1 to 9 or a pharmaceutically acceptable salt thereof, selected from 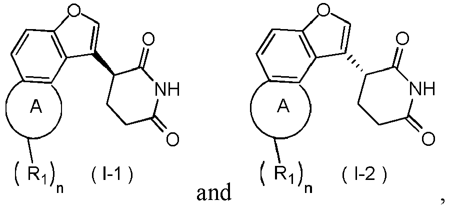 wherein,
n, R₁ and ring A are as defined in any one of claims 1 to 9.

11. The compound as defined in claim 1 or a pharmaceutically acceptable salt thereof, selected from

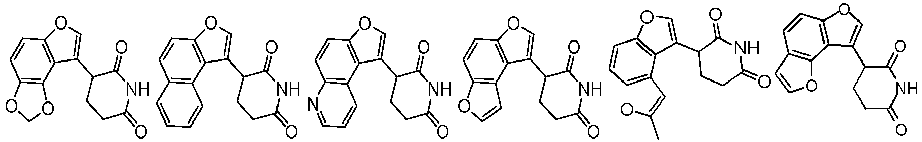

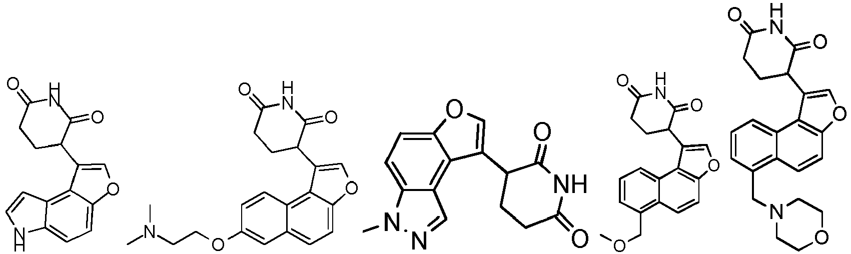

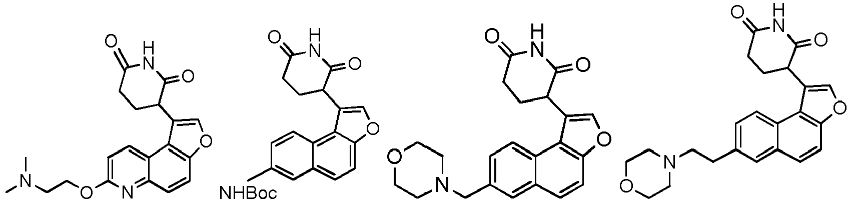

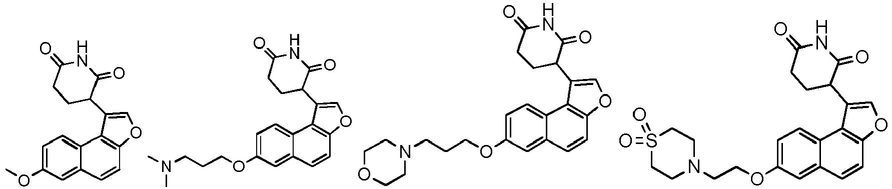

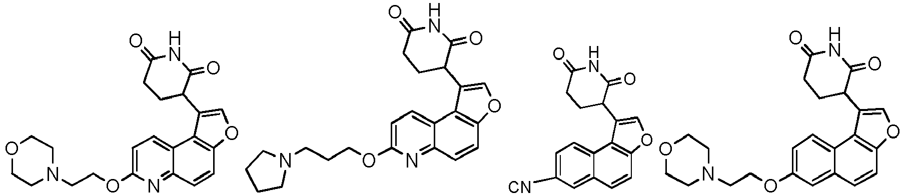

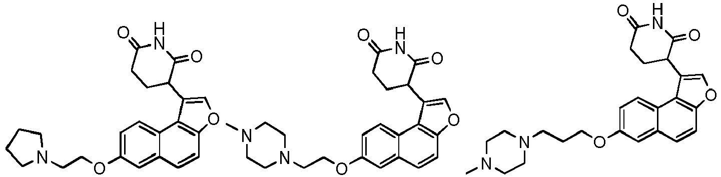

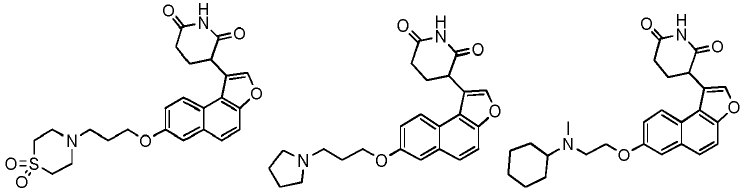

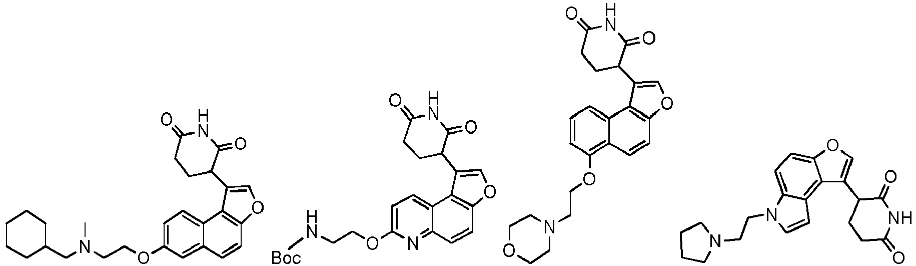

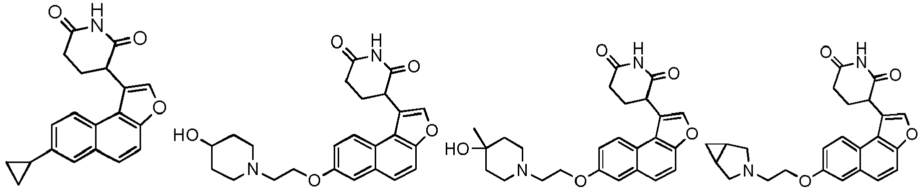

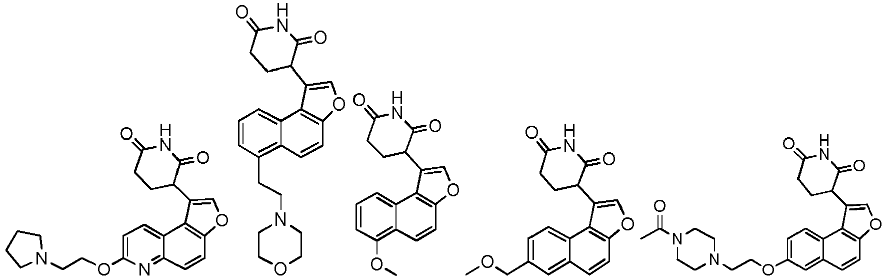

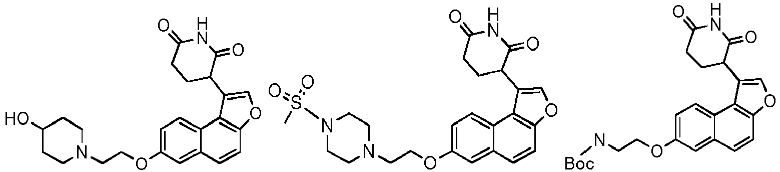

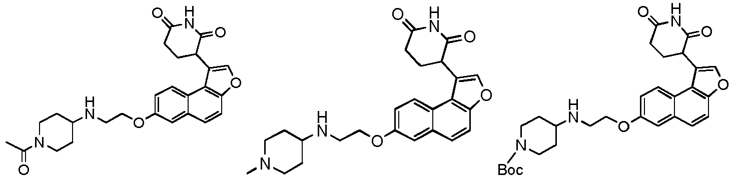

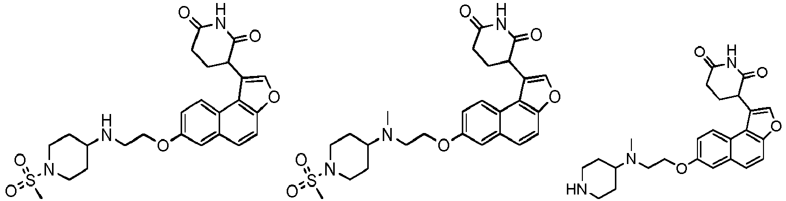

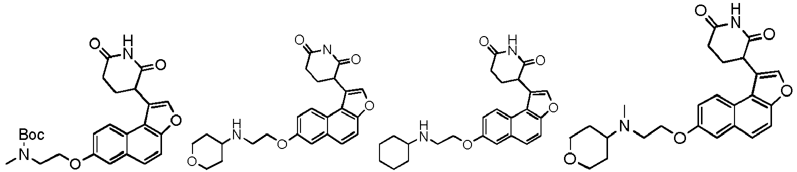

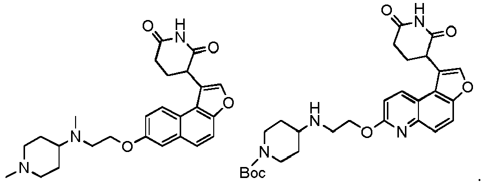

12. The compound as defined in claim 11 or a pharmaceutically acceptable salt thereof, selected from

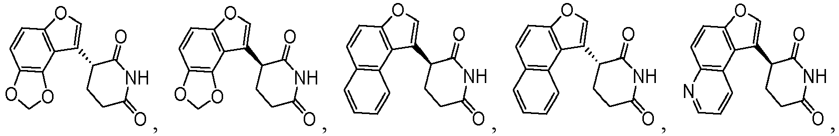

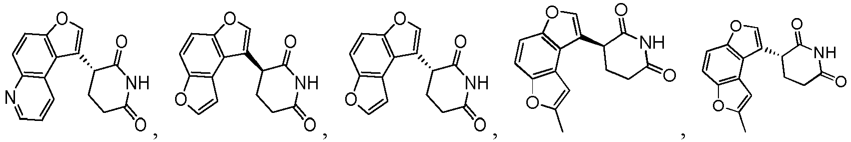

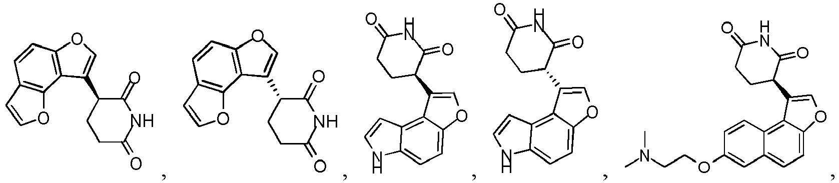

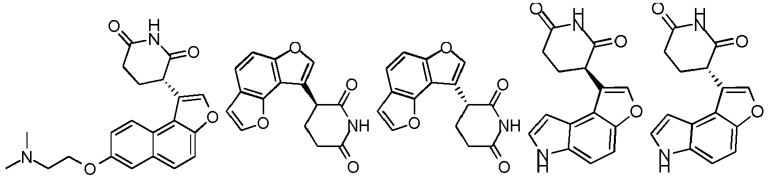

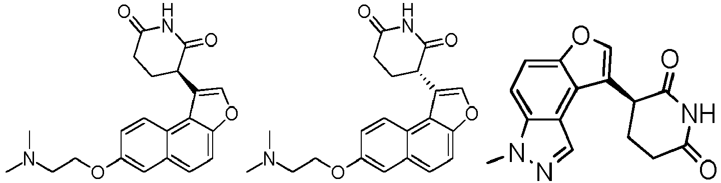

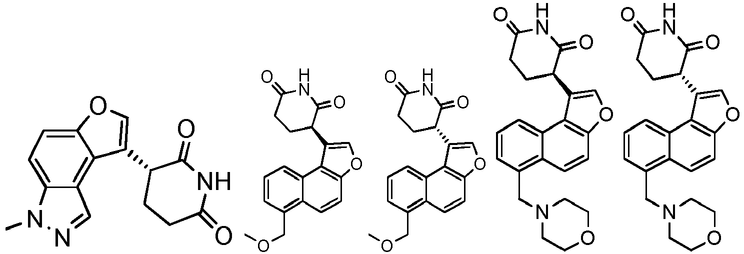

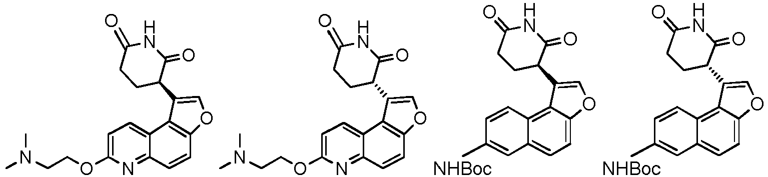

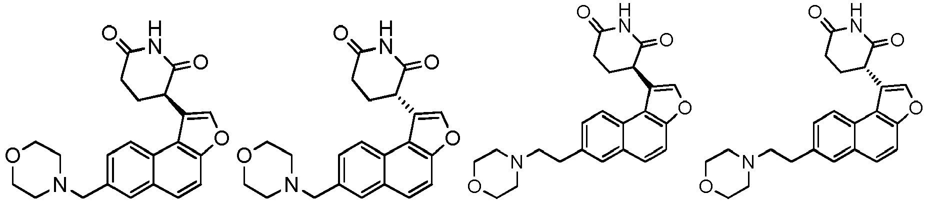

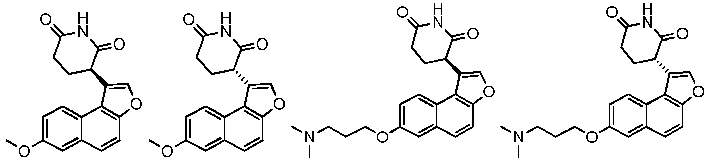

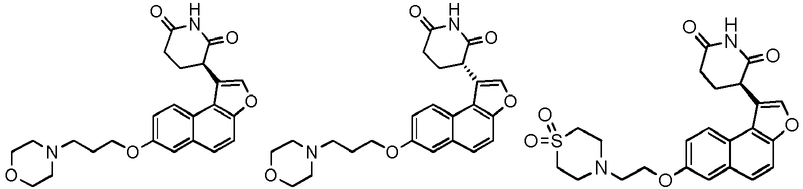

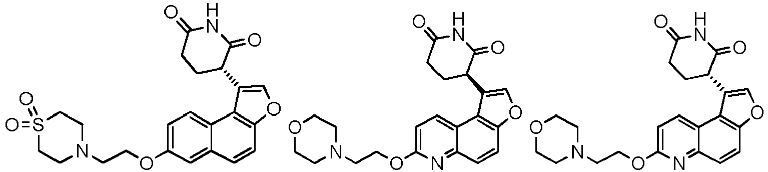

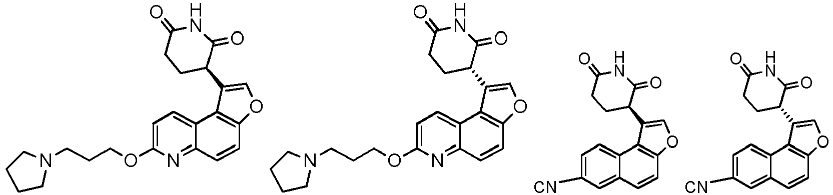

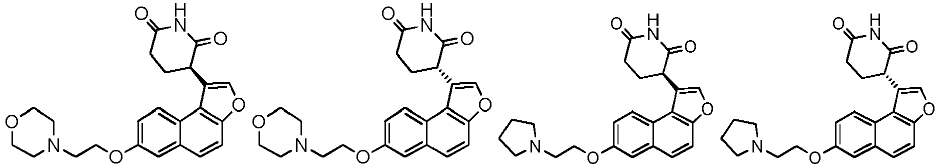

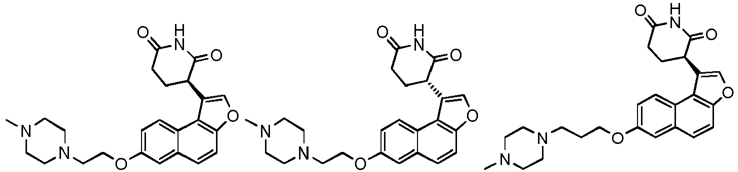

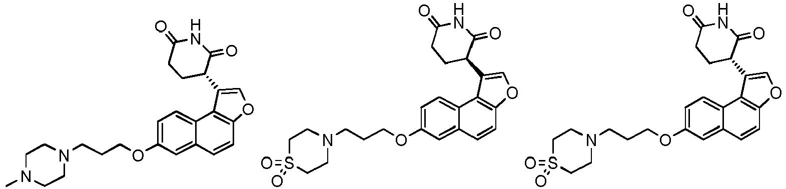

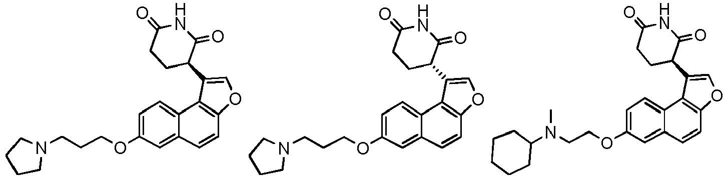

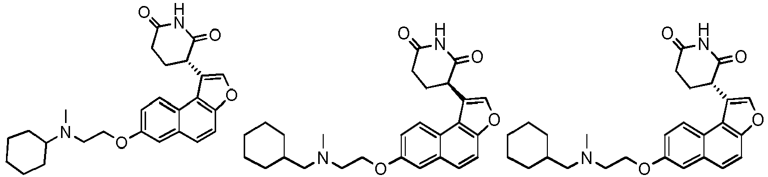

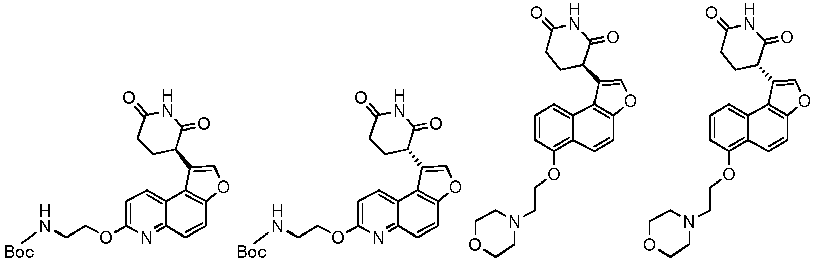

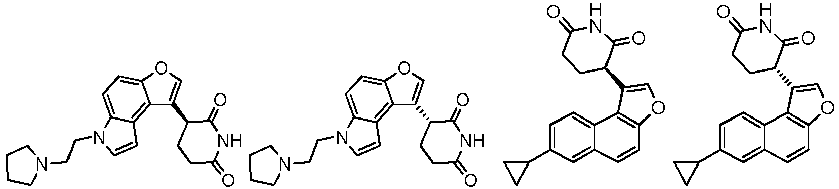

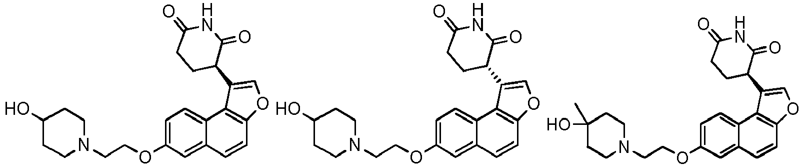

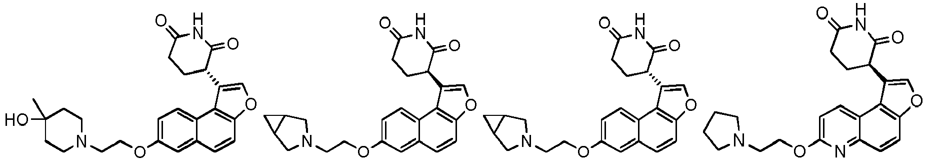

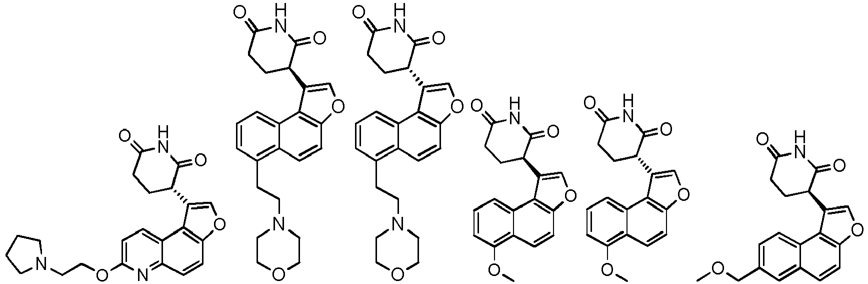

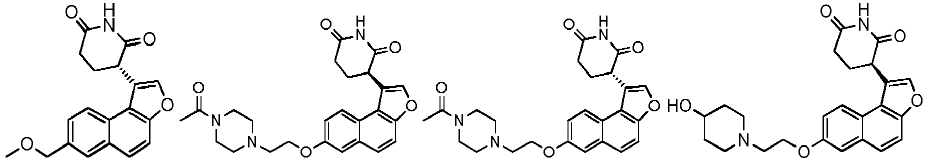

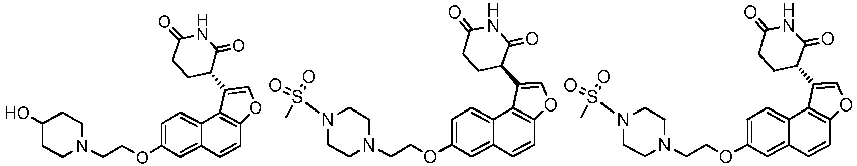

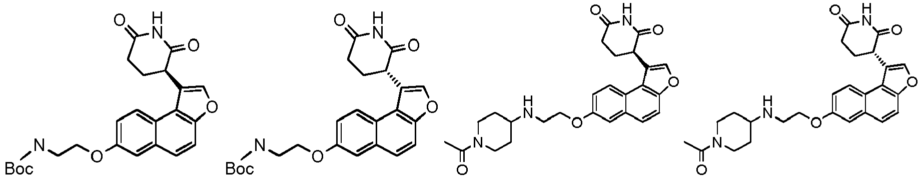

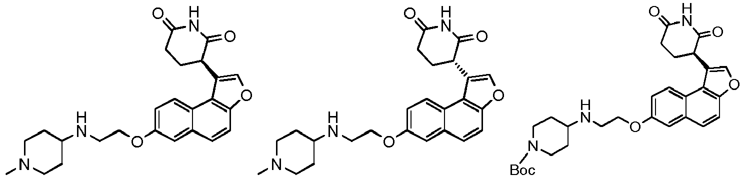

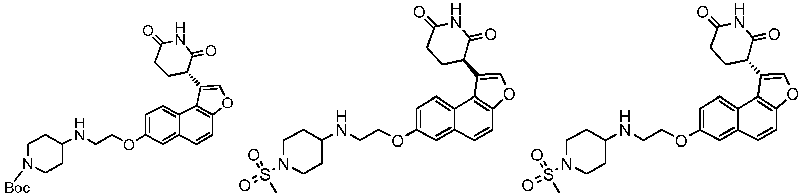

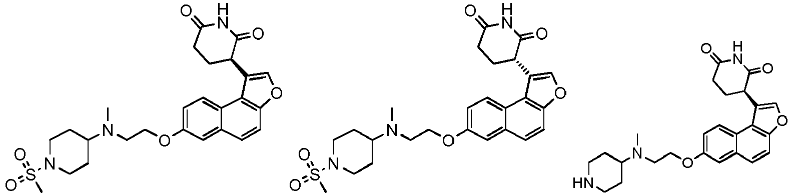

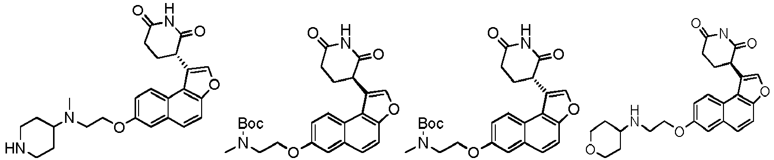

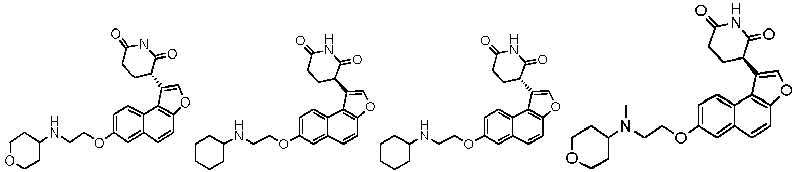

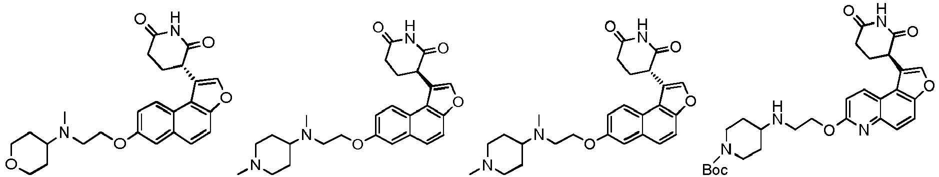

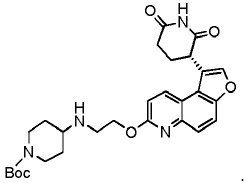

13. A pharmaceutical composition comprising a therapeutically effective amount of the compound as defined in any one of claims 1 to 12 or a pharmaceutically acceptable salt thereof as an active ingredient and a pharmaceutically acceptable carrier.

## Patentansprüche

1. Verbindung, dargestellt durch die Formel (!), oder ein pharmazeutisch verträgliches Salz davon, 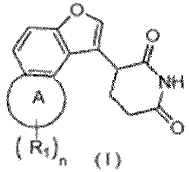 wobei,
n ausgewählt ist aus 0, 1, 2 und 3;
R₁ ausgewählt ist aus H, Halogen, OH, NH₂, CN, C₃₋₆-Cycloalkyl, C₁₋₆-Alkyl und C₁₋₆-Alkoxy und wobei NH₂, C₃₋₆-Cycloalkyl, C₁₋₆-Alkyl und C₁₋₆-Alkoxy gegebenenfalls mit 1, 2 oder 3 Rₐ substituiert sind;
Ring A ausgewählt ist aus 5- bis 6-gliedrigem Heteroaryl, Phenyl, C₄₋₆-Cycloalkyl und 4- bis 7-gliedrigem Heterocycloalkyl;
Rₐ unabhängig ausgewählt ist aus F, Cl, Br, I, OH, NH₂, C₁₋₁₀-Alkylamino, C₁₋₁₀-Alkoxy, -C(=O)O-C₁₋₁₀-Alkyl, 4- bis 10-gliedrigem Heterocycloalkyl, 4- bis 10-gliedrigem Heterocycloalkylamino, C₄₋₇-Cycloalkylamino, C₄₋₇-Cycloalkylmethylamino, wobei NH₂, C₁₋₁₀-Alkylamino, C₁₋₁₀-Alkoxy, -C(=O)O-C₁₋₁₀-Alkyl, 4- bis 10-gliedriges Heterocycloalkyl, 4- bis 10-gliedriges Heterocycloalkylamino, C₄₋₇-Cycloalkylamino und C₄₋₇-Cycloalkylmethylamino gegebenenfalls mit 1, 2 oder 3 R substituiert sind;
R unabhängig ausgewählt ist aus F, Cl, Br, I, OH, NH₂, Me, 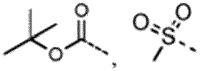 und 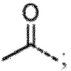
das 5- bis 6-gliedrige Heteroaryl, 4- bis 7-gliedrige Heterocycloalkyl, 4- bis 10-gliedrige Heterocycloalkyl und 4- bis 10-gliedrige Heterocycloalkylamino 1, 2, 3 oder 4 Heteroatome oder Heteroatomgruppen umfassen, die unabhängig ausgewählt sind aus - NH-, -O-, -S- 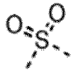 bzw. N.

2. Verbindung nach Anspruch 1 oder ein pharmazeutisch verträgliches Salz davon, wobei Rₐ ausgewählt ist aus F, Cl, Br, I, OH, NH₂, C₁₋₆-Alkylamino, C₁₋₆-Alkoxy, -C(=O)O-C₁₋₆-Alkyl, 4- bis 7-gliedrigem Heterocycloalkyl, 4- bis 7-gliedrigem Heterocycloalkylamino, C₄₋₇-Cycloalkylamino, C₄₋₇-Cycloalkylmethylamino, wobei NH₂, C₁₋₆-Alkylamino, C₁₋₆-Alkoxy, -C(=O)O-C₁₋₆-Alkyl, 4- bis 7-gliedriges Heterocycloalkyl, C₄₋₇-Cycloalkylamino, 4- bis 7-gliedriges Heterocycloalkylamino und C₄₋₇-Cycloalkylmethylamino gegebenenfalls mit 1, 2 oder 3 R substituiert sind.

3. Verbindung nach Anspruch 2 oder ein pharmazeutisch verträgliches Salz davon, wobei Rₐ ausgewählt ist aus F, Cl, Br, I, OH, NH₂, C₁₋₃-Alkylamino, C₁₋₃-Alkoxy, -C(=O)O-C₁₋₄-Alkyl, Pyrrolidinyl, Morpholinyl, Piperazinyl, Piperidinyl, 3-Azabicyclo[3,1,0]hexyl, Thiomorpholin-1,1-dioxid-Gruppe, Cyclohexylamino, Piperidinylamino, Tetrahydropyranyl, Tetrahydropyranylamino und Cyclohexylmethylamino, wobei die NH₂-, C₁₋₃-Alkylamino-, C₁₋₃-Alkoxy-, -C(=O)O-C₁₋₄-Alkyl-, Tetrahydropyrrolyl-, Morpholinyl-, Piperazinyl-, Piperidinyl-, 3-Azabicyclo[3,1,0]hexyl-, Thiomorpholin-1,1-Dioxydgruppe, Cyclohexylamino, Piperidinylamino, Tetrahydropyranyl, Tetrahydropyranylamino und Cyclohexylmethylamino gegebenenfalls mit 1, 2 oder 3 R substituiert sind.

4. Verbindung nach Anspruch 3 oder ein pharmazeutisch verträgliches Salz davon, wobei Rₐ ausgewählt ist aus

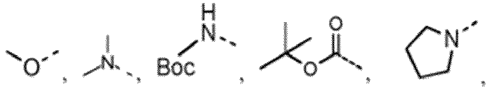

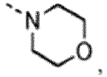

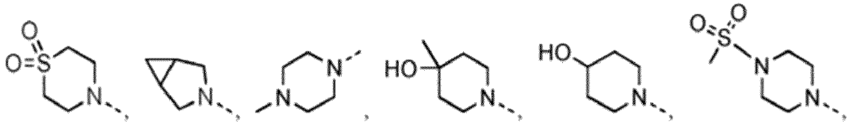

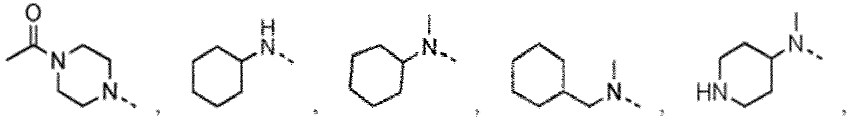

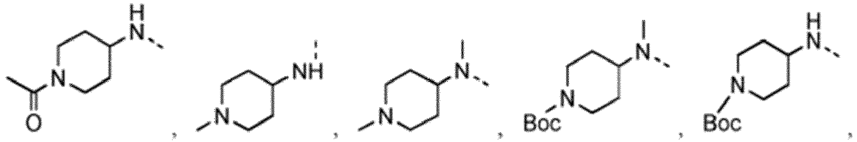

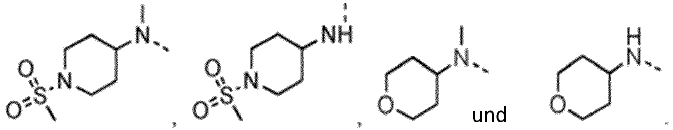

5. Verbindung nach einem der Ansprüche 1 bis 4 oder ein pharmazeutisch verträgliches Salz davon, wobei R₁ aus H, NH₂, CN, C₃₋₆-Cycloalkyl, C₁₋₃-Alkyl und C₁₋₃-Alkoxy ausgewählt ist, wobei NH₂, C₃₋₆-Cycloalkyl, C₁₋₃-Alkyl und C₁₋₃-Alkoxy gegebenenfalls mit 1, 2 oder 3 Rₐ substituiert sind.

6. Verbindung nach Anspruch 5 oder ein pharmazeutisch verträgliches Salz davon, wobei R₁ ausgewählt ist aus H, Me, CN,

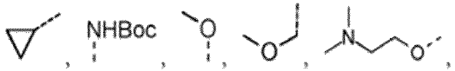

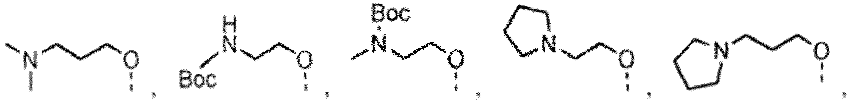

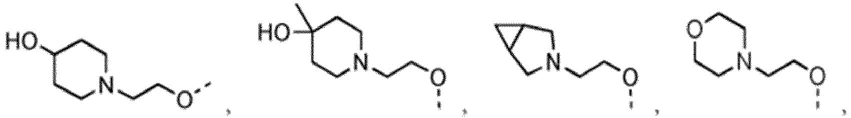

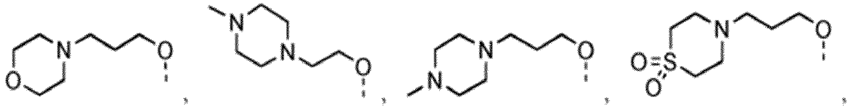

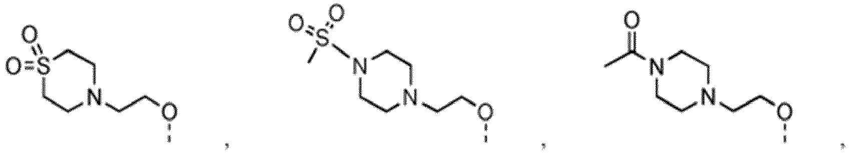

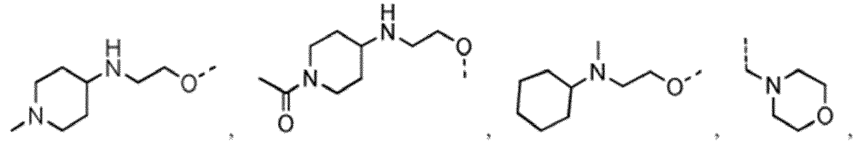

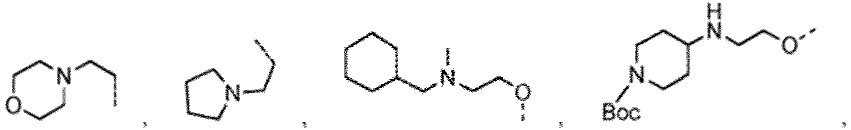

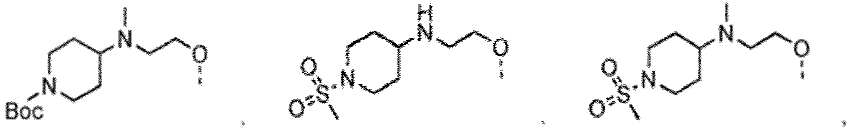

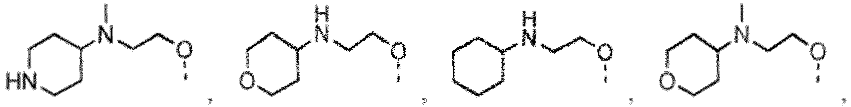

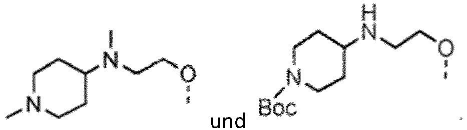

7. Verbindung nach einem der Ansprüche 1 bis 3 oder ein pharmazeutisch verträgliches Salz davon, wobei Ring A ausgewählt ist aus Phenyl, Pyridyl, Pyrrolyl, Pyrazolyl, 1,3-Dioxolan, Morpholinyl, Oxazolylcyclobutyl, Oxepanyl, Furanyl, Tetrahydrofuranyl und 1,4-Oxazepinyl.

8. Verbindung nach Anspruch 7 oder ein pharmazeutisch verträgliches Salz davon, wobei der Ring A ausgewählt ist aus Phenyl, Pyridyl, Pyrrolyl, Pyrazolyl, 1,3-Dioxolan, Furanyl und Tetrahydrofuranyl.

9. Verbindung nach Anspruch 8 oder ein pharmazeutisch verträgliches Salz davon, wobei die Struktureinheit 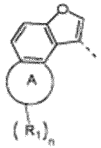 ausgewählt ist aus

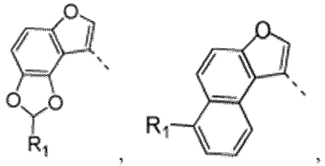

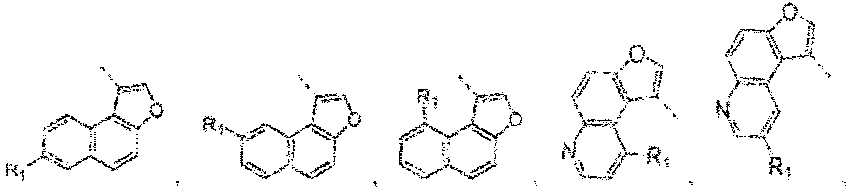

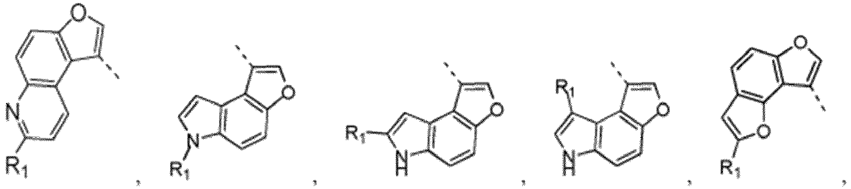

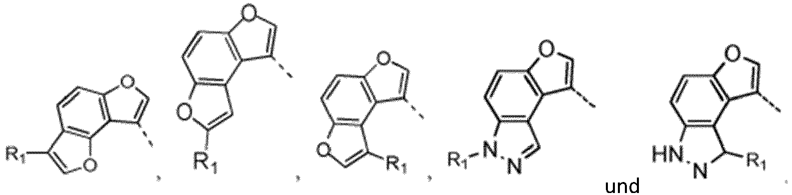

10. Verbindung nach einem der Ansprüche 1 bis 9 oder ein pharmazeutisch verträgliches Salz davon, ausgewählt aus 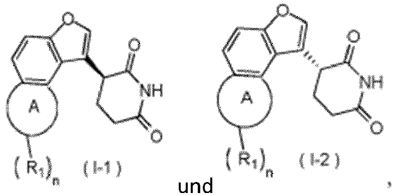 wobei,
n, R₁ und Ring A nach einem der Ansprüche 1 bis 9 definiert sind.

11. Verbindung nach Anspruch 1 oder ein pharmazeutisch verträgliches Salz davon, ausgewählt aus

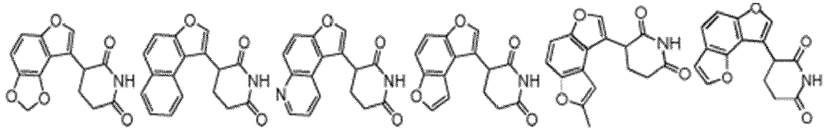

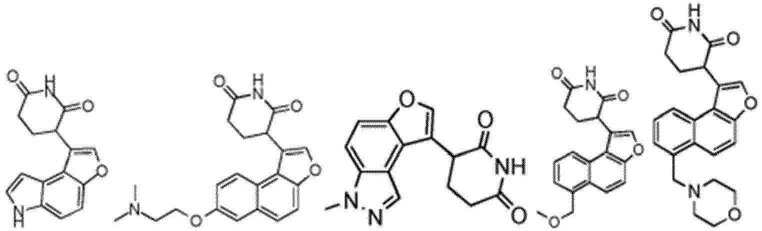

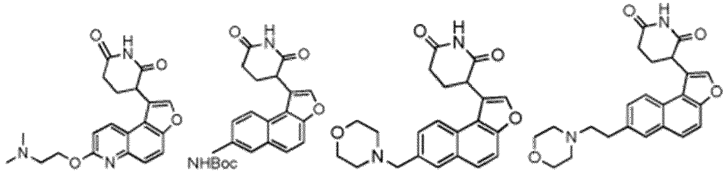

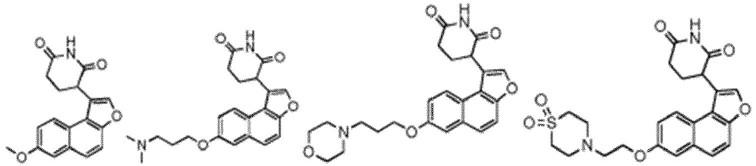

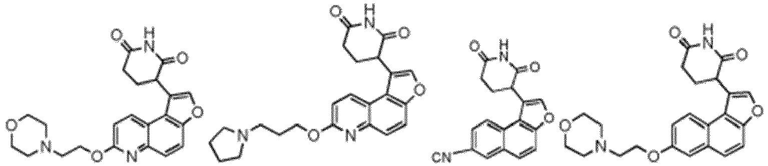

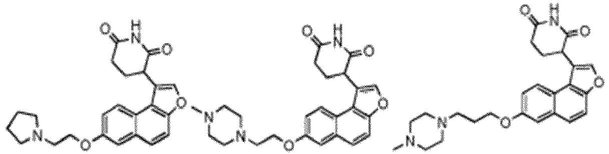

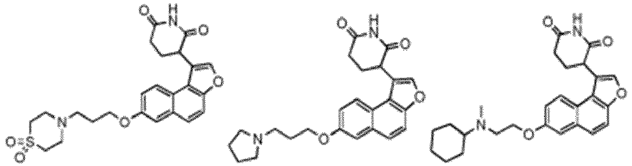

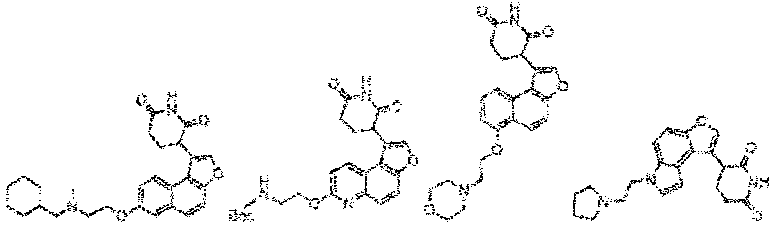

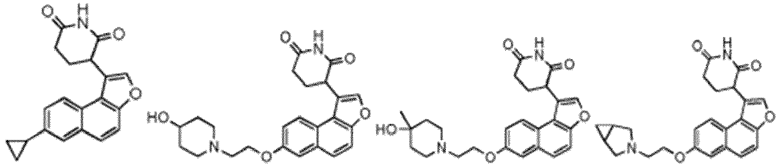

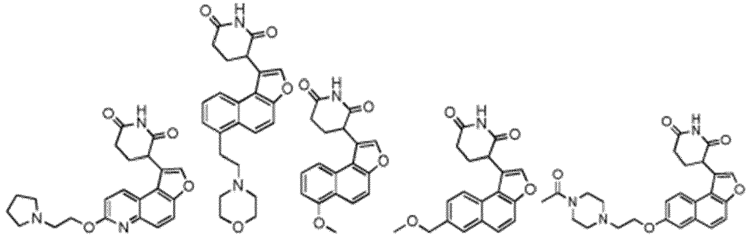

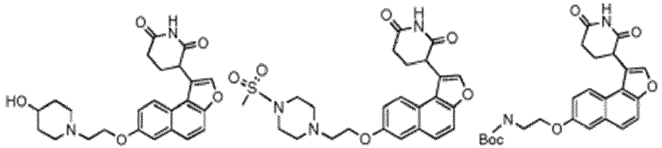

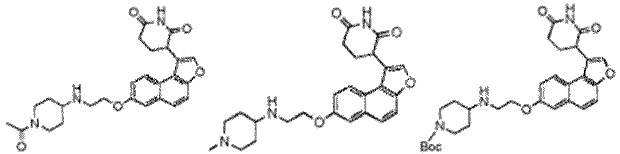

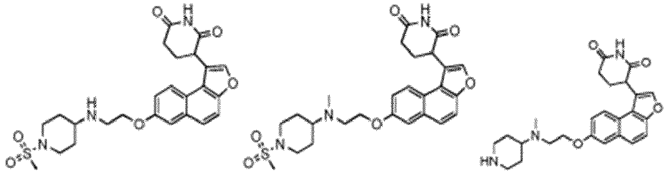

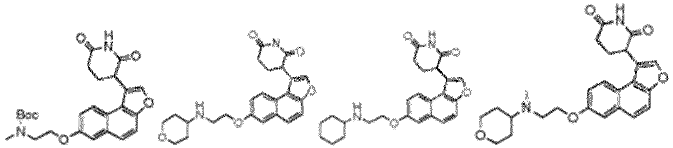

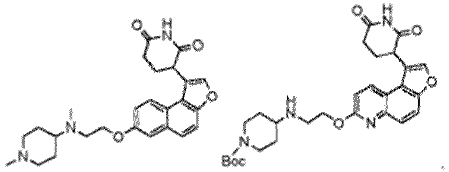

12. Verbindung nach Anspruch 11 oder ein pharmazeutisch verträgliches Salz davon, ausgewählt aus

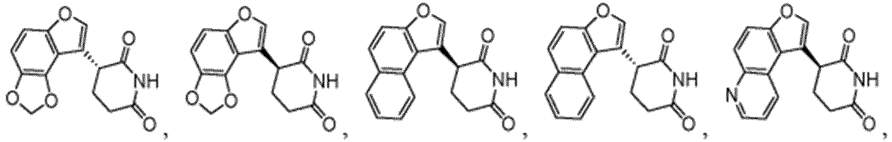

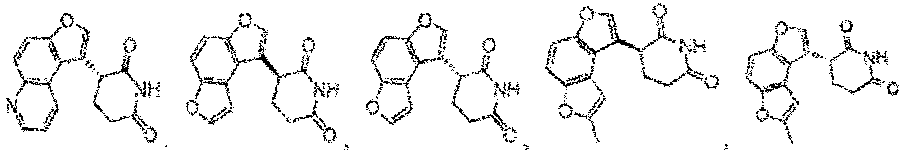

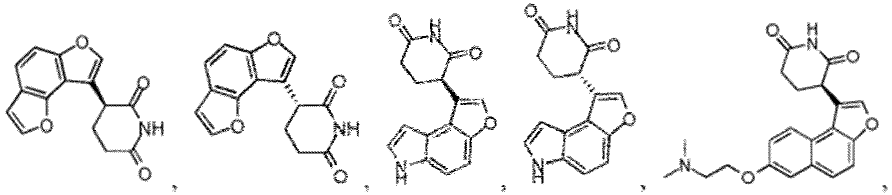

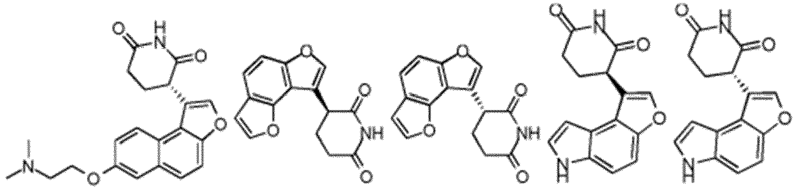

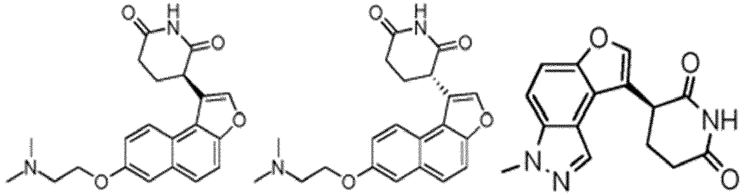

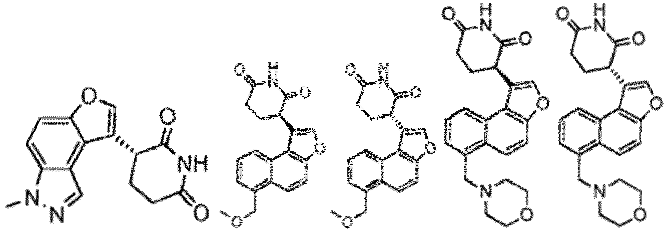

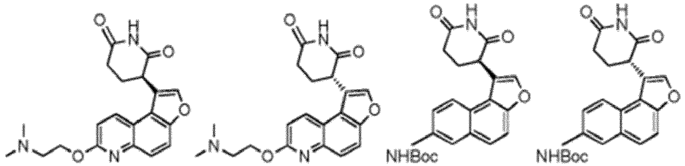

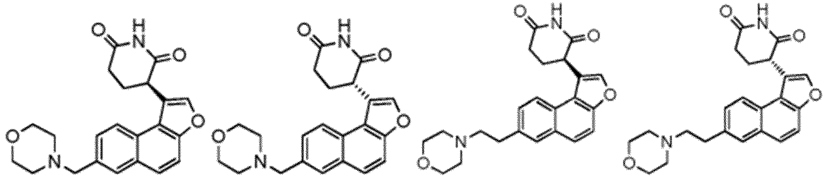

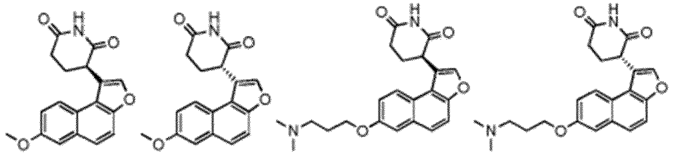

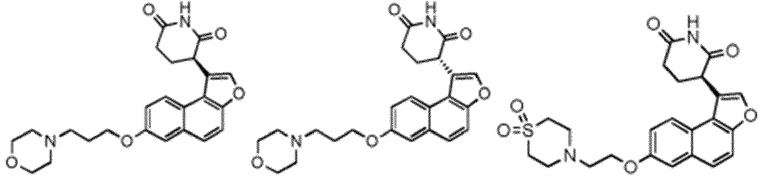

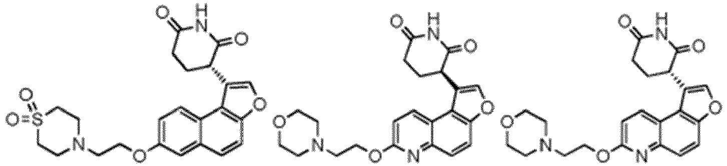

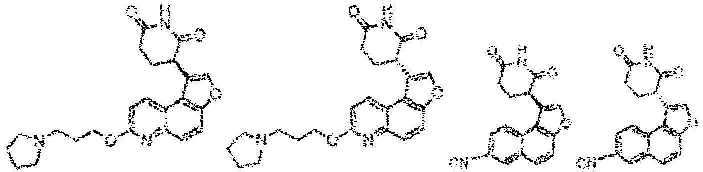

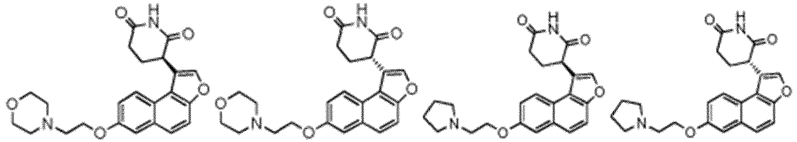

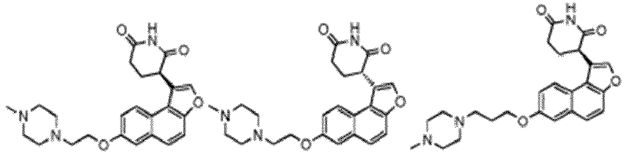

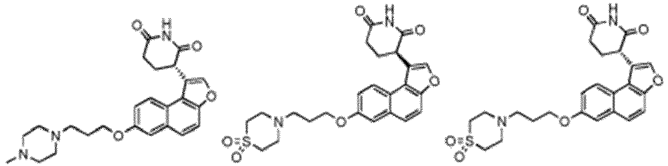

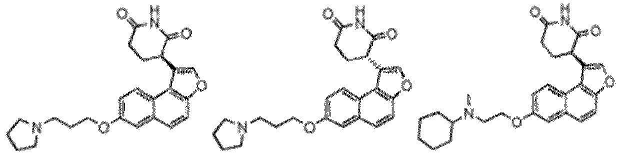

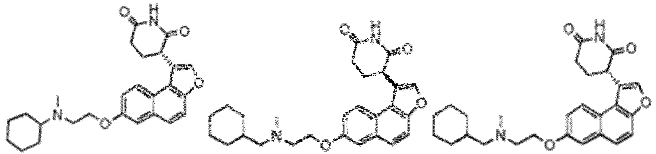

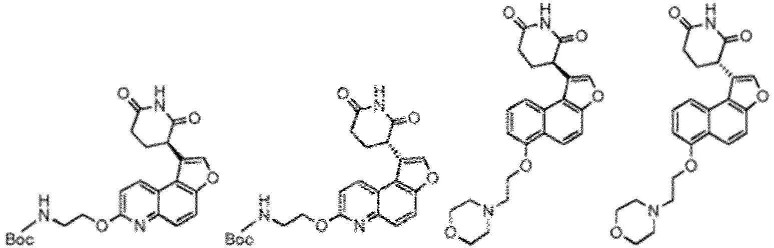

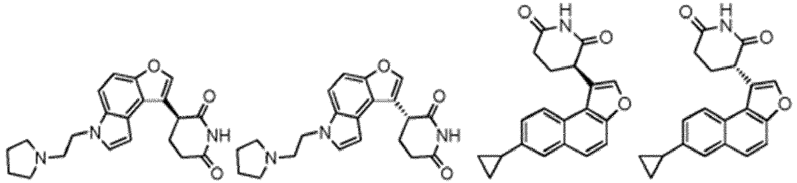

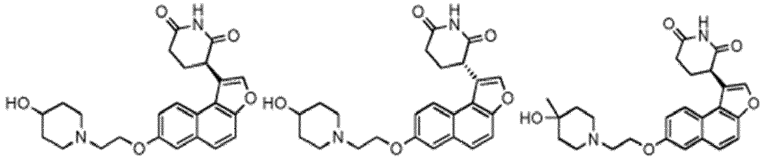

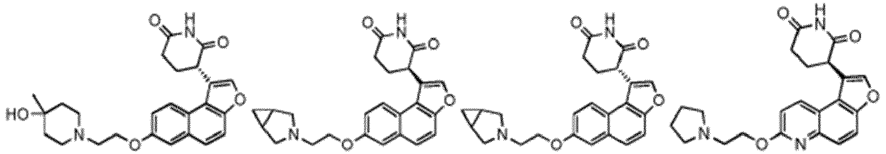

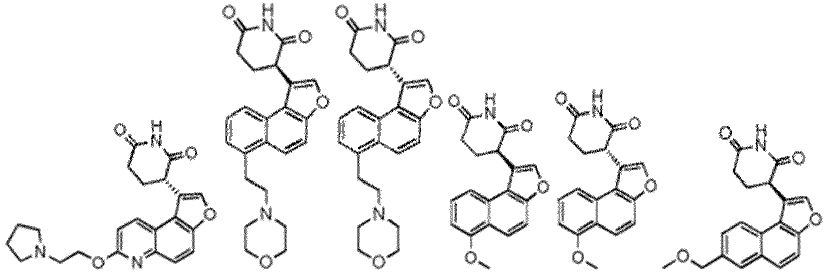

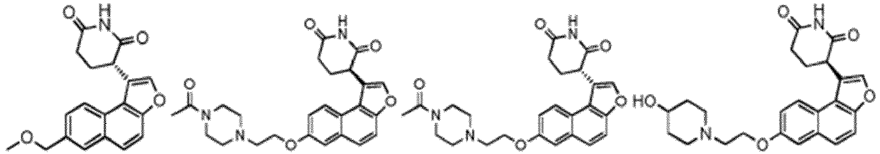

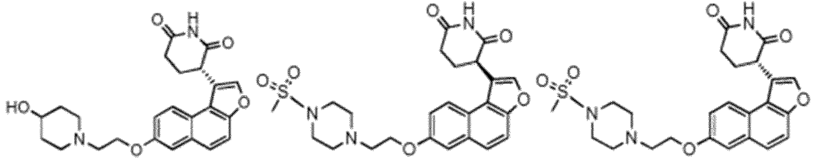

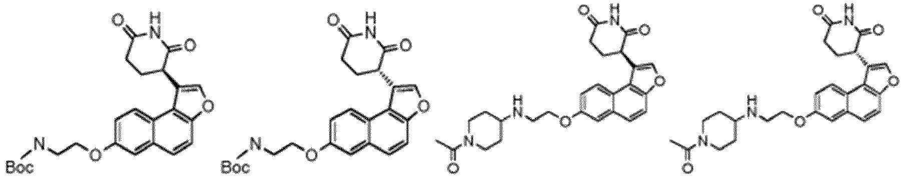

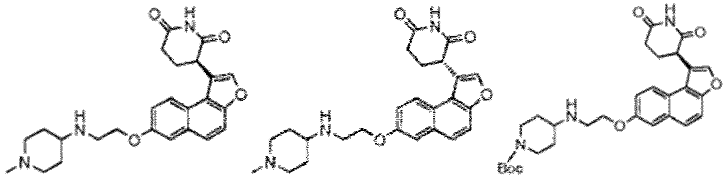

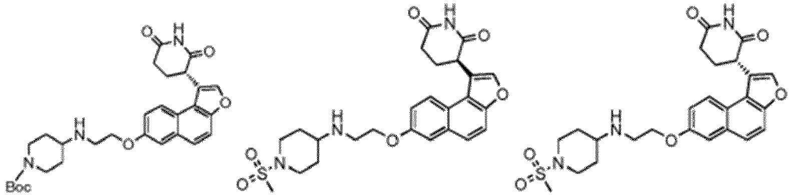

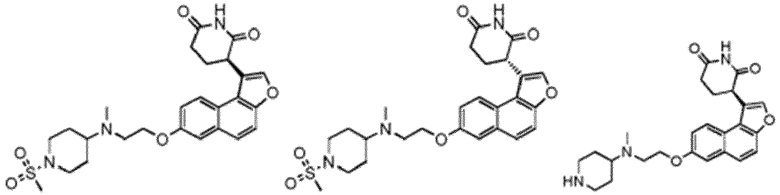

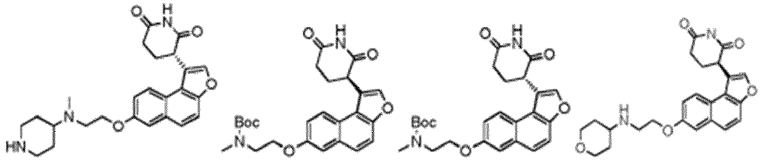

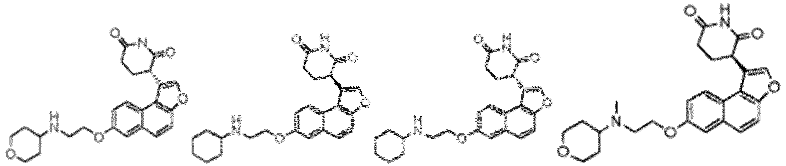

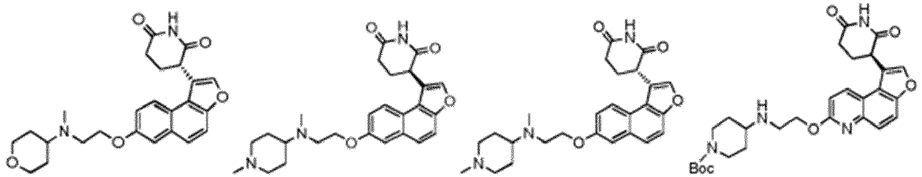

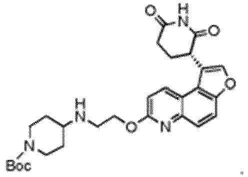

13. Pharmazeutische Zusammensetzung, umfassend eine therapeutisch wirksame Menge der in einem der Ansprüche 1 bis 12 definierten Verbindung oder ein pharmazeutisch verträgliches Salz davon als Wirkstoff und einen pharmazeutisch verträglichen Träger.

## Revendications

1. Composé représenté par la formule (I) ou sel pharmaceutiquement acceptable de celui-ci, 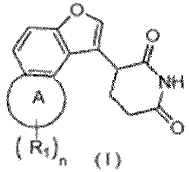 dans lequel,
n est sélectionné parmi 0, 1, 2 et 3 ;
R₁ est sélectionné parmi H, un halogène, OH, NH₂, CN, un cycloalkyle en C₃₋₆, un alkyle en C₁₋₆, et un alcoxy en C₁₋₆ et le NH₂, le cycloalkyle en C₃₋₆, l'alkyle en C₁₋₆ et l'alcoxy en C₁₋₆ sont facultativement substitués avec 1, 2 ou 3 Rₐ ;
le cycle A est sélectionné parmi un hétéroaryle de 5 à 6 chaînons, un phényle, un cycloalkyle en C₄₋₆, et un hétérocycloalkyle de 4 à 7 chaînons ;
Rₐ est sélectionné indépendamment parmi F, Cl, Br, I, OH, NH₂, un alkylamino en C₁₋₁₀, un alcoxy en C₁₋₁₀, un -C(=O)O-alkyle en C₁₋₁₀, un hétérocycloalkyle de 4 à 10 chaînons, un hétérocycloalkylamino de 4 à 10 chaînons, un cycloalkylamino en C₄₋₇, un cycloalkylméthylamino en C₄₋₇, dans lequel le NH₂, l'alkylamino en C₁₋₁₀, l'alcoxy en C₁₋₁₀, le -C(=O)O-alkyle en C₁₋₁₀, l'hétérocycloalkyle de 4 à 10 chaînons, l'hétérocycloalkylamino de 4 à 10 chaînons, le cycloalkylamino en C₄₋₇ et le cycloalkylméthylamino en C₄₋₇ sont facultativement substitués avec 1, 2 ou 3 R ;
R est sélectionné indépendamment parmi F, Cl, Br, I, OH, NH₂, Me, 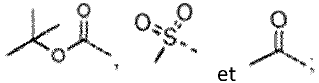
l'hétéroaryle de 5 à 6 chaînons, l'hétérocycloalkyle de 4 à 7 chaînons, l'hétérocycloalkyle de 4 à 10 chaînons et l'hétérocycloalkylamino de 4 à 10 chaînons comprennent 1, 2, 3 ou 4 hétéroatomes ou groupes d'hétéroatomes sélectionnés indépendamment parmi -NH-, -O-, -S-, 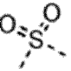 et N, respectivement.

2. Composé selon la revendication 1 ou sel pharmaceutiquement acceptable de celui-ci, dans lequel Rₐ est sélectionné parmi F, Cl, Br, I, OH, NH₂, un alkylamino en C₁₋₆, un alcoxy en C₁₋₆, un -C(=O)O-alkyle en C₁₋₆, un hétérocycloalkyle de 4 à 7 chaînons, un hétérocycloalkylamino de 4 à 7 chaînons, un cycloalkylamino en C₄₋₇, un cycloalkylméthylamino en C₄₋₇, dans lequel le NH₂, l'alkylamino en C₁₋₆, l'alcoxy en C₁₋₆, le -C(=O)O-alkyle en C₁₋₆, l'hétérocycloalkyle de 4 à 7 chaînons, le cycloalkylamino en C₄₋₇, l'hétérocycloalkylamino de 4 à 7 chaînons et le cycloalkylméthylamino en C₄₋₇ sont facultativement substitués avec 1, 2 ou 3 R.

3. Composé selon la revendication 2 ou sel pharmaceutiquement acceptable de celui-ci, dans lequel Rₐ est sélectionné parmi F, Cl, Br, I, OH, NH₂, un alkylamino en C₁₋₃, un alcoxy en C₁₋₃, un -C(=O)O-alkyle en C₁₋₄, un pyrrolidinyle, un morpholinyle, un pipérazinyle, un pipéridinyle, un 3-azabicyclo[3,1,0]hexyle, un groupe thiomorpholine-1,1-dioxyde, un cyclohexylamino, un pipéridinylamino, un tétrahydropyranyle, un tétrahydropyranylamino et un cyclohexylméthylamino, dans lequel le NH₂, l'alkylamino en C₁₋₃, l'alcoxy en C₁₋₃, le -C(=O)O-alkyle en C₁₋₄, le tétrahydropyrrolyle, le morpholinyle, le pipérazinyle, le pipéridinyle, le 3-azabicyclo[3,1,0]hexyle, le groupe thiomorpholine-1,1-dioxyde, le cyclohexylamino, le pipéridinylamino, le tétrahydropyranyle, le tétrahydropyranylamino et le cyclohexylméthylamino sont facultativement substitués avec 1, 2 ou 3 R.

4. Composé selon la revendication 3 ou sel pharmaceutiquement acceptable de celui-ci, dans lequel Rₐ est sélectionné parmi

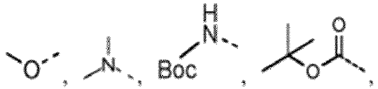

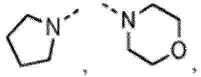

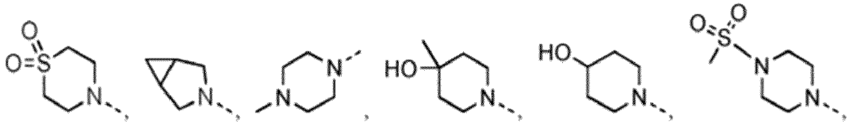

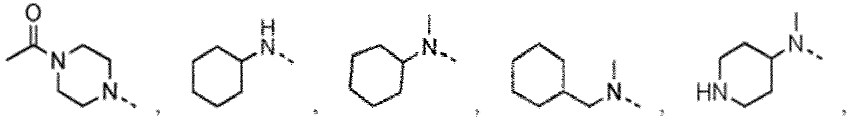

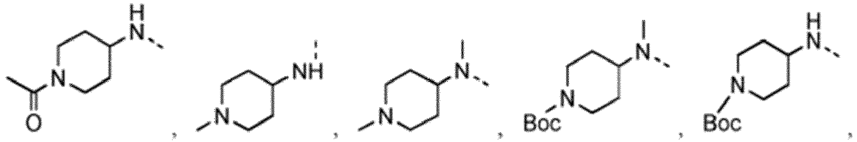

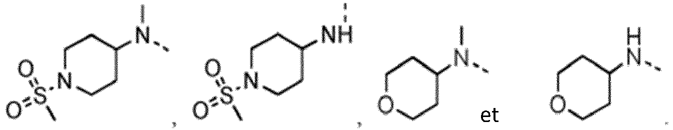

5. Composé selon l'une quelconque des revendications 1 à 4 ou sel pharmaceutiquement acceptable de celui-ci, dans lequel R₁ est sélectionné parmi H, NH₂, CN, un cycloalkyle en C₃₋₆, un alkyle en C₁₋₃ et un alcoxy en C₁₋₃, dans lequel le NH₂, le cycloalkyle en C₃₋₆, l'alkyle en C₁₋₃ et l'alcoxy en C₁₋₃ sont facultativement substitués avec 1, 2 ou 3 Rₐ.

6. Composé selon la revendication 5 ou sel pharmaceutiquement acceptable de celui-ci, dans lequel R₁ est sélectionné parmi H, Me, CN,

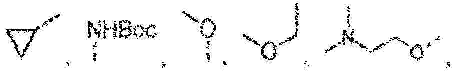

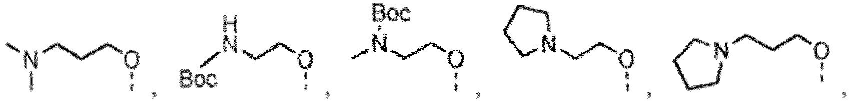

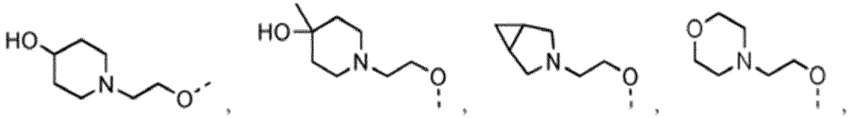

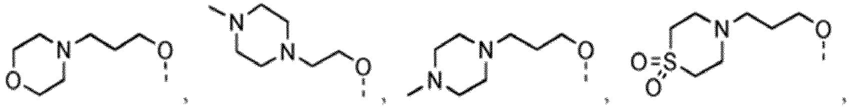

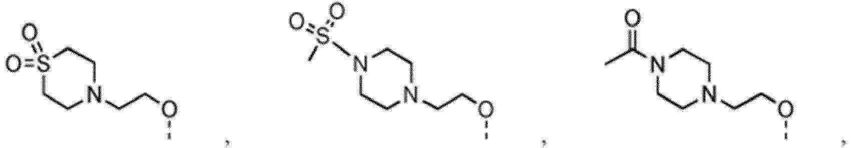

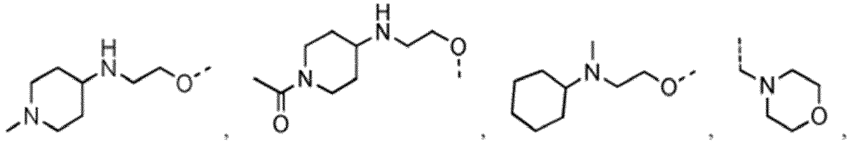

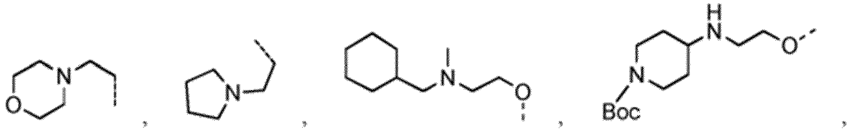

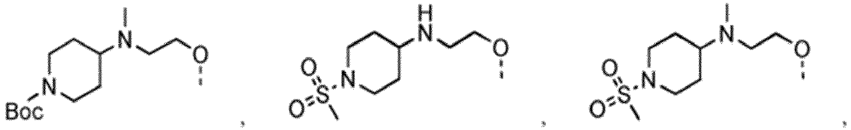

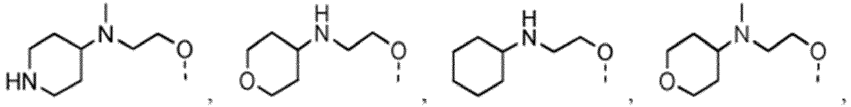

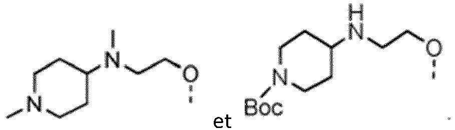

7. Composé selon l'une quelconque des revendications 1 à 3 ou sel pharmaceutiquement acceptable de celui-ci, dans lequel le cycle A est sélectionné parmi un phényle, un pyridyle, un pyrrolyle, un pyrazolyle, un 1,3-dioxolane, un morpholinyle, un oxazolylcyclobutyle, un oxépanyle, un furanyle, un tétrahydrofuranyle et un 1,4-oxazépinyle.

8. Composé selon la revendication 7 ou sel pharmaceutiquement acceptable de celui-ci, dans lequel le cycle A est sélectionné parmi un phényle, un pyridyle, un pyrrolyle, un pyrazolyle, un 1,3-dioxolane, un furanyle et un tétrahydrofuranyle.

9. Composé selon la revendication 8 ou sel pharmaceutiquement acceptable de celui-ci, dans lequel le motif de structure 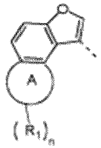 est sélectionné parmi

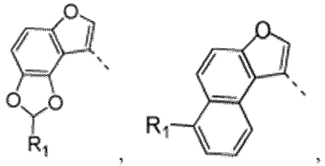

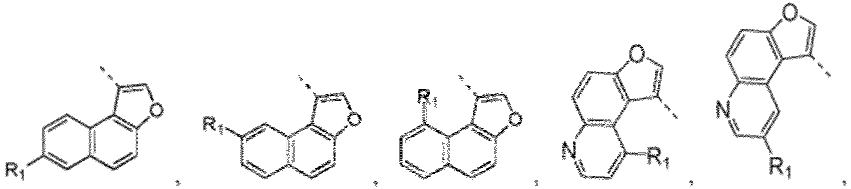

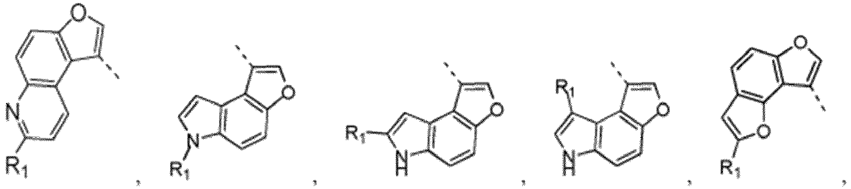

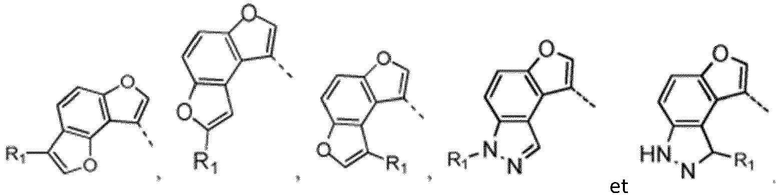

10. Composé selon l'une quelconque des revendications 1 à 9 ou sel pharmaceutiquement acceptable de celui-ci, sélectionné parmi 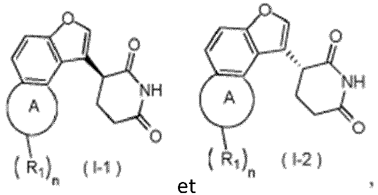 dans lequel,
n, R₁ et le cycle A sont tels que définis dans l'une quelconque des revendications 1 à 9.

11. Composé selon la revendication 1 ou sel pharmaceutiquement acceptable de celui-ci, sélectionné parmi

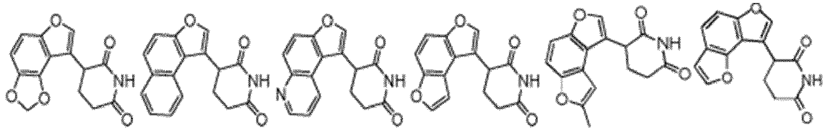

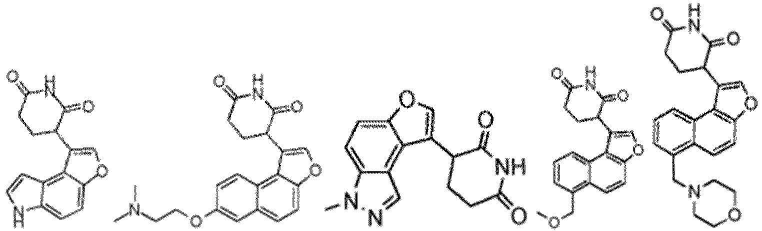

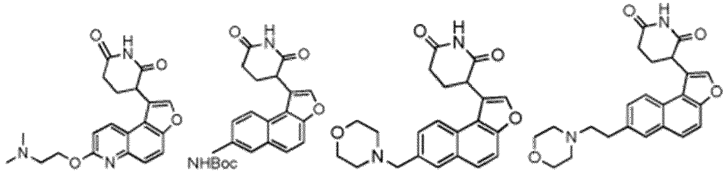

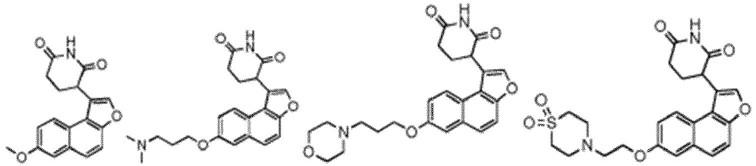

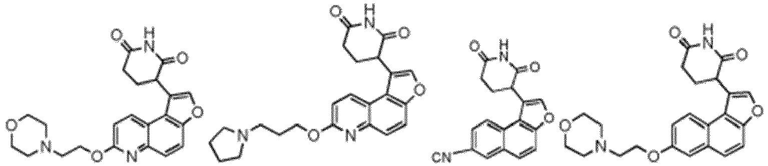

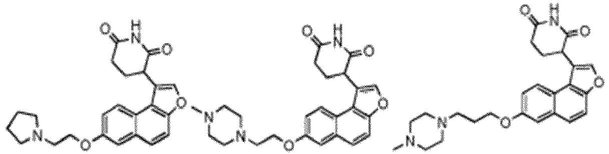

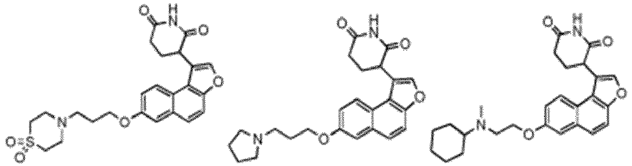

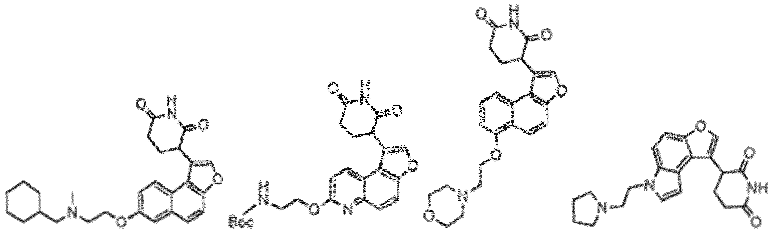

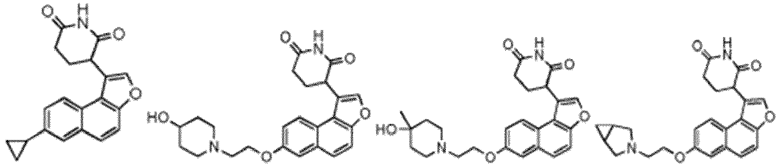

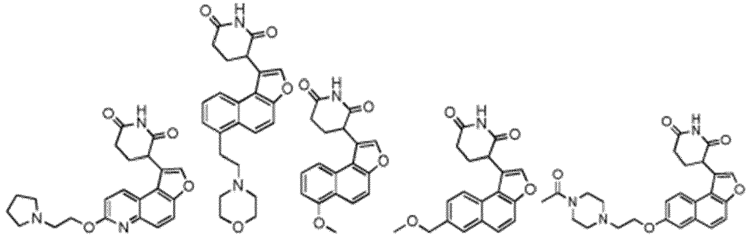

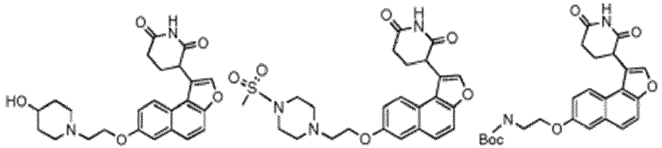

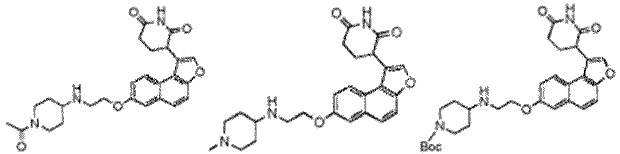

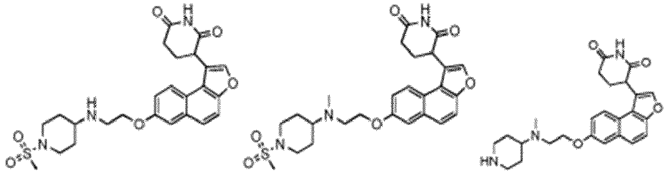

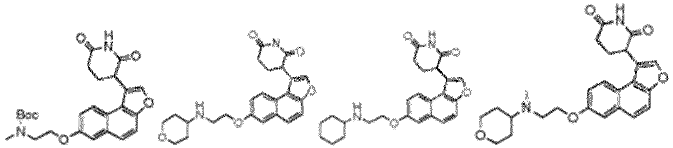

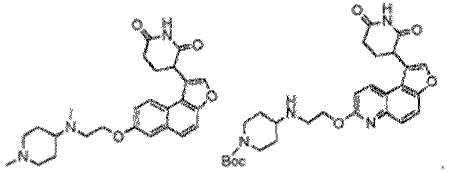

12. Composé selon la revendication 11 ou sel pharmaceutiquement acceptable de celui-ci, sélectionné parmi

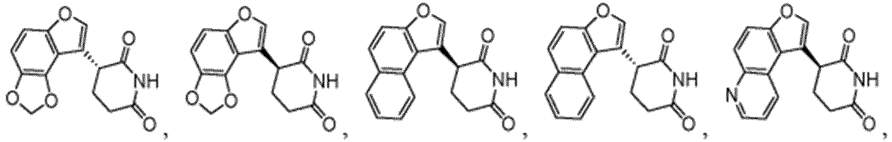

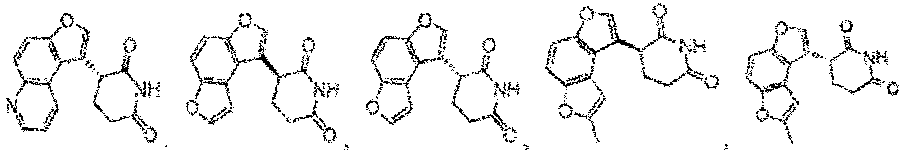

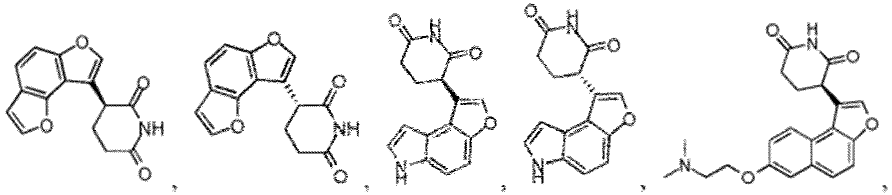

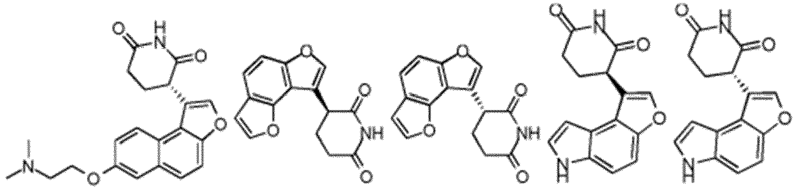

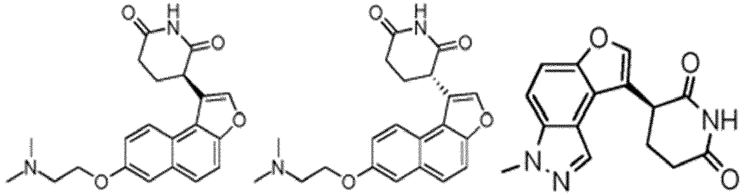

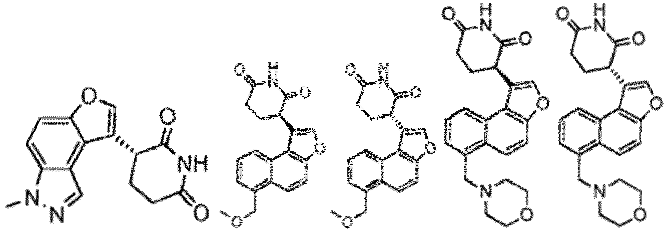

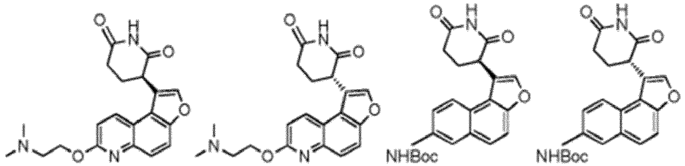

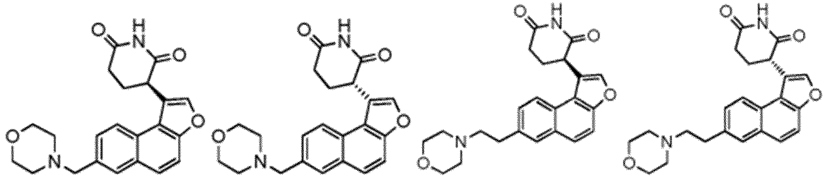

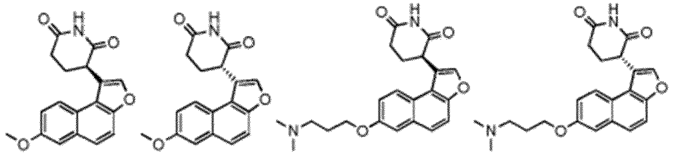

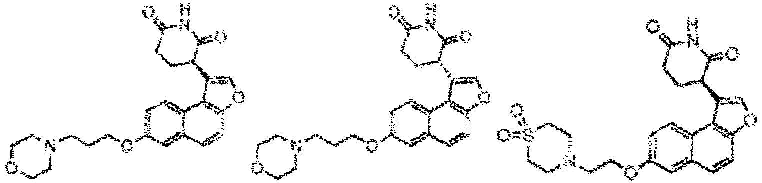

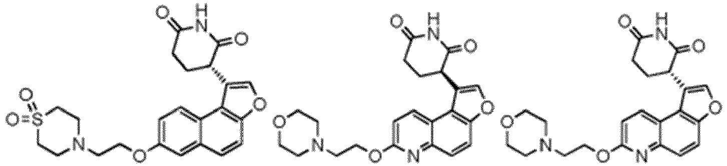

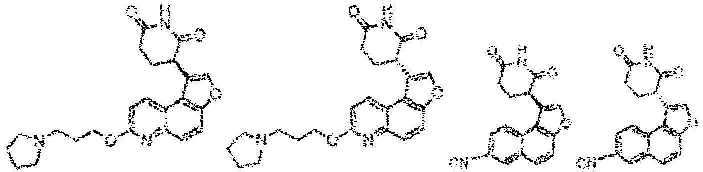

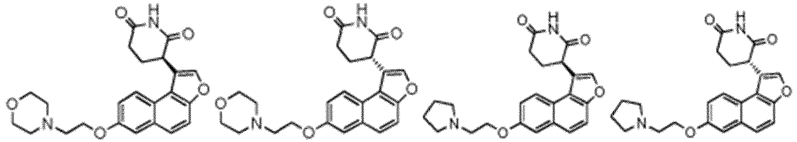

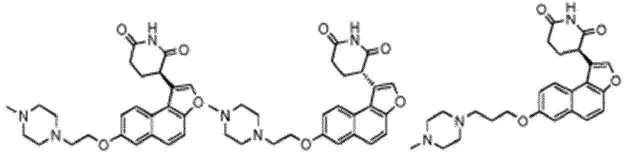

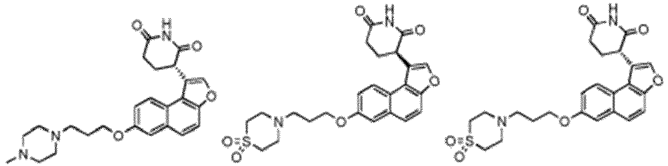

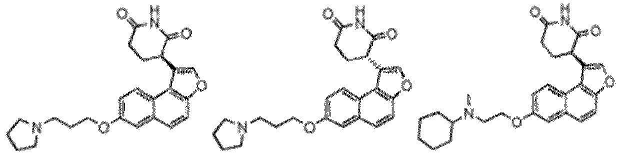

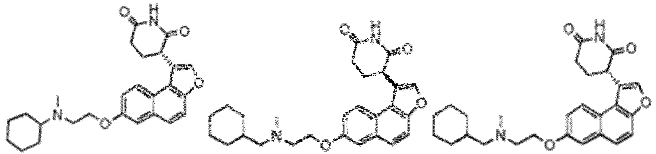

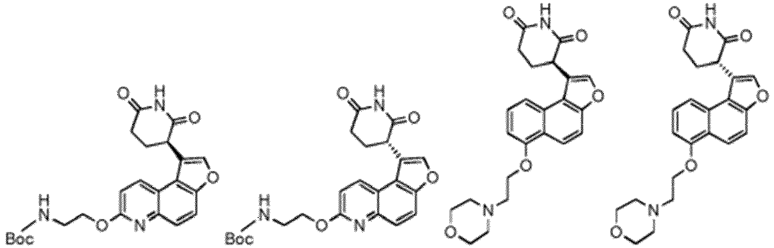

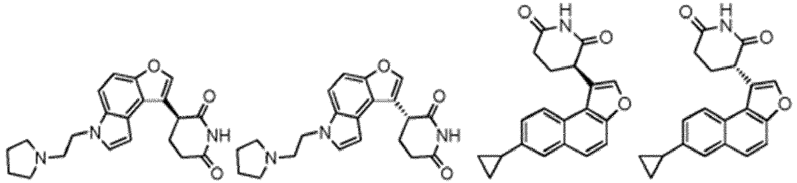

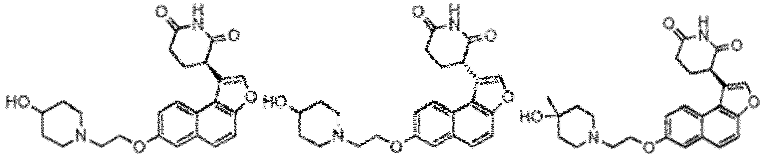

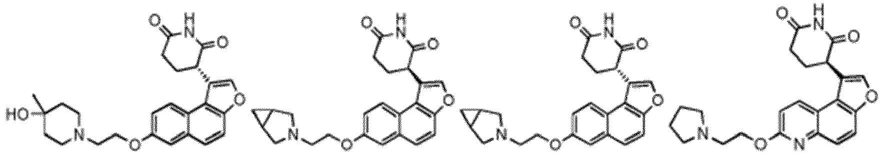

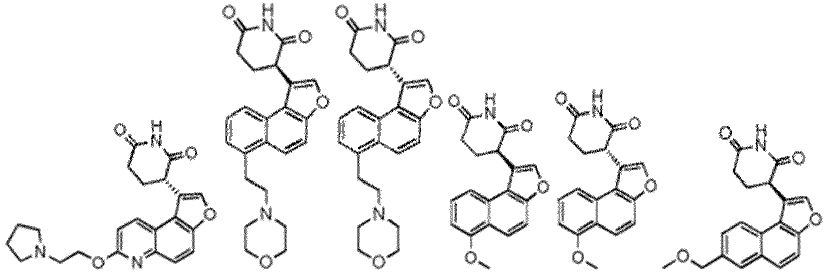

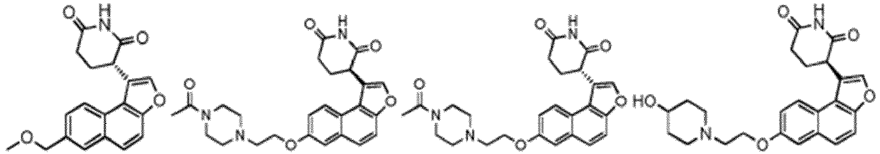

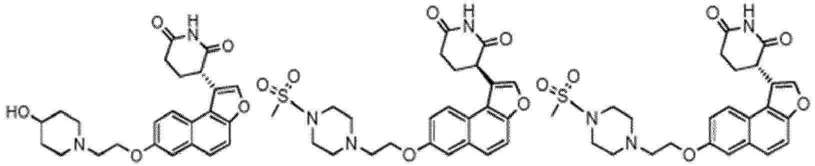

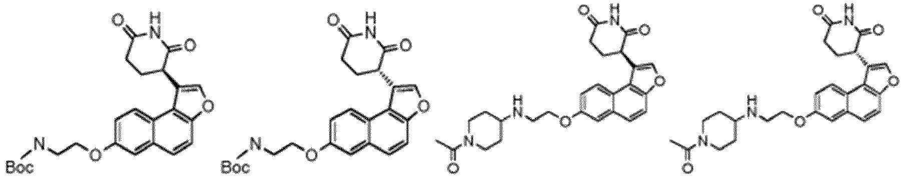

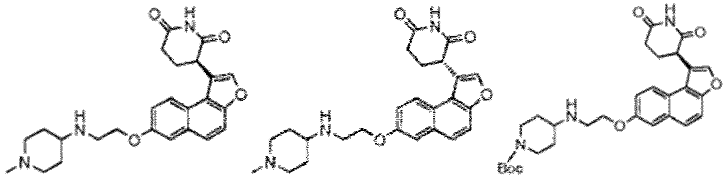

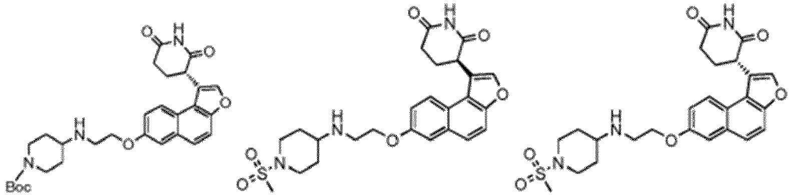

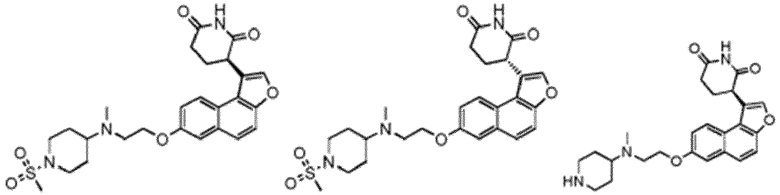

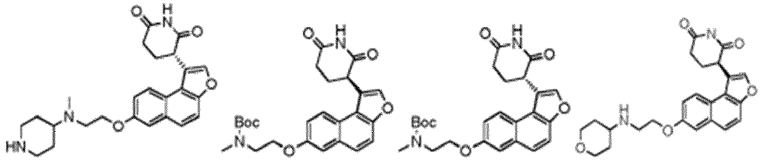

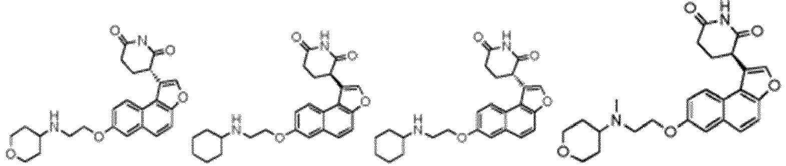

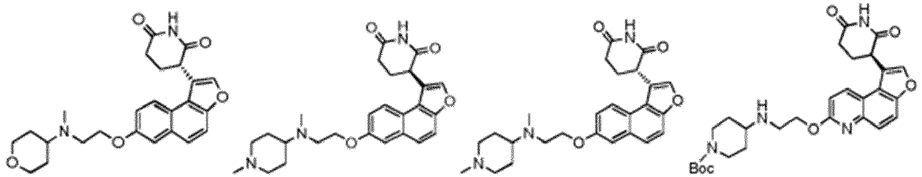

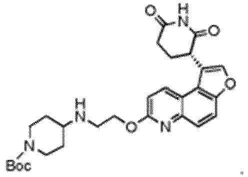

13. Composition pharmaceutique comprenant une quantité thérapeutiquement efficace du composé selon l'une quelconque des revendications 1 à 12 ou d'un sel pharmaceutiquement acceptable de celui-ci en tant qu'ingrédient actif et un support pharmaceutiquement acceptable.
